# EUROPEAN PATENT SPECIFICATION

(11) **EP 2 222 668 B1**
(45) Date of publication and mention of the grant of the patent: **02.11.2011**
(21) Application number: 08861137.1
(22) Date of filing: 15.12.2008
(51) Int. Cl.: C07D 413/04, C07D 413/14, A61K 31/4245, A61P 37/00

(54) **TETRAHYDROCYCLOPENTA[B]INDOL-3-YL CARBOXYLIC ACID DERIVATIVES USEFUL IN THE TREATMENT OF AUTOIMMUNE AND INFLAMMATORY DISORDERS**
FÜR DIE BEHANDLUNG VON AUTOIMMUNERKRANKUNGEN UND ENTZÜNDLICHEN ERKRANKUNGEN GEEIGNETE TETRAHYDROCYCLOPENTA[B]INDOL-3-YLCARBONSÄUREDERIVATE
DÉRIVÉS D'ACIDE TÉTRAHYDROCYCLOPENTA[B]INDOL-3-YLCARBOXYLIQUE UTILES DANS LE TRAITEMENT DE TROUBLES AUTO-IMMUNS ET INFLAMMATOIRES

(30) Priority: 18.12.2007 US 8079
(43) Date of publication of application: 01.09.2010
(73) Proprietor: Arena Pharmaceuticals, Inc., San Diego, CA 92121-3223 (US)
(72) Inventor: JONES, Robert, M., San Diego California 92130 (US); BUZARD, Daniel, J., San Diego California 92129 (US); KAWASAKI, Andrew, M., San Diego California 92130 (US); LOPEZ, Luis, A., San Diego California 92154 (US); MOODY, Jeanne, V., San Diego California 92122 (US); THORESEN, Lars, San Diego California 92107 (US); ULLMAN, Brett, San Diego California 92105 (US)
(74) Representative: Wytenburg, Wilhelmus Johannes
(86) International application number: PCT/US2008/013753
(87) International publication number: WO 2009/078983

(56) References cited:
- WO-A-2005/032465
- WO-A-2007/116866
- WO-A-2008/128951
- VACHAL PETR ET AL: "Highly selective and potent agonists of sphingosine-1-phosphate 1 (S1P1) receptor" BIOORGANIC & MEDICINAL CHEMISTRY LETTERS 15 JUL 2006,, vol. 16, no. 14, 15 July 2006 (2006-07-15), pages 3684-3687, XP002515397

## Description

### FIELD OF THE INVENTION

The present invention relates to certain (1,2,4-oxadiazol-3-yl)-1,2,3,4-tetrahydrocyclopenta[b]indol-3-yl carboxylic acid derivatives of Formula (**Ia**) and pharmaceutically acceptable salts thereof, which exhibit useful pharmacological properties, for example, as agonists of the S1P1 receptor. Also provided by the present invention are pharmaceutical compositions containing compounds of the invention, and methods of using the compounds and compositions of the invention in the treatment of S1P1 associated disorders, for example, psoriasis, rheumatoid arthritis, Crohn's disease, transplant rejection, multiple sclerosis, systemic lupus erythematosus, ulcerative colitis, type I diabetes, acne, microbial infections or diseases and viral infections or diseases.

### BACKGROUND OF THE INVENTION

The present invention relates to compounds that are S1P1 receptor agonists having at least immunosuppressive, anti-inflammatory and/or hemostatic activities, *e.g.* by virtue of modulating leukocyte trafficking, sequestering lymphocytes in secondary lymphoid tissues, and/or enhancing vascular integrity.

The present application is in part focused on addressing an unmet need for immunosuppressive agents such as may be orally available which have therapeutic efficacy for at least autoimmune diseases and disorders, inflammatory diseases and disorders (*e.g.*, acute and chronic inflammatory conditions), transplant rejection, cancer, and/or conditions that have an underlying defect in vascular integrity or that are associated with angiogenesis such as may be pathologic (*e.g.*, as may occur in inflammation, tumor development and atherosclerosis) with fewer side effects such as the impairment of immune responses to systemic infection.

The sphingosine-1-phosphate (S1P) receptors 1-5 constitute a family of G protein-coupled receptors with a seven-transmembrane domain. These receptors, referred to as S1P1 to S1P5 (formerly termed endothelial differentiation gene (EDG) receptor-1, -5, -3, -6 and -8, respectively; Chun et al., Pharmacological Reviews, 54:265-269, 2002), are activated via binding by sphingosine-1-phosphate, which is produced by the sphingosine kinase-catalyzed phosphorylation of sphingosine. S1P1, S1P4 and S1P5 receptors activate Gi but not Gq, whereas S1P2 and S1P3 receptors activate both Gi and Gq. The S1P3 receptor, but not the S1P1 receptor, responds to an agonist with an increase in intracellular calcium.

S1P receptor agonists having agonist activity on the S1P1 receptor have been shown to rapidly and reversibly induce lymphopenia (also referred to as peripheral lymphocyte lowering (PLL); Hale et al., Bioorg. Med. Chem. Lett., 14:3351-3355, 2004). This is attended by clinically useful immunosuppression by virtue of sequestering T- and B-cells in secondary lymphoid tissue (lymph nodes and Peyer's patches) and thus apart from sites of inflammation and organ grafts (Rosen et al., Immunol. Rev., 195:160-177, 2003; Schwab et al., Nature Immunol., 8:1295-1301, 2007). This lymphocyte sequestration, for example in lymph nodes, is thought to be a consequence of concurrent agonist-driven functional antagonism of the S1P1 receptor on T-cells (whereby the ability of S1P to mobilize T-cell egress from lymph nodes is reduced) and persistent agonism of the S1P1 receptor on lymph node endothelium (such that barrier function opposing transmigration of lymphocytes is increased) (Matloubian et al., Nature, 427:355-360, 2004; Baumruker et al., Expert Opin. Investig. Drugs, 16:283-289, 2007). It has been reported that agonism of the S1P1 receptor alone is sufficient to achieve lymphocyte sequestration (Sanna et al., J Biol Chem., 279:13839-13848, 2004) and that this occurs without impairment of immune responses to systemic infection (Brinkmann et al., Transplantation, 72:764-769,2001; Brinkmann et al., Transplant Proc., 33:530-531, 2001).

That agonism of the endothelial S1P1 receptor has a broader role in promoting vascular integrity is supported by work implicating the S1P1 receptor in capillary integrity in mouse skin and lung (Sanna et al., Nat Chem Biol., 2:434-441, 2006). Vascular integrity can be compromised by inflammatory processes, for example as may derive from sepsis, major trauma and surgery so as to lead to acute lung injury or respiratory distress syndrome (Johan Groeneveld, Vascul. Pharmacol., 39:247-256, 2003).

An exemplary S1P receptor agonist having agonist activity on the S1P1 receptor is FTY720 (fingolimod), an immunosuppressive agent currently in clinical trials (Martini et al., Expert Opin. Investig. Drugs, 16:505-518, 2007). FTY720 acts as a prodrug which is phosphorylated *in vivo;* the phosphorylated derivative is an agonist for S1P1, S1P3, S1P4 and S1P5 receptors (but not the S1P2 receptor) (Chiba, Pharmacology & Therapeutics, 108:308-319, 2005). FTY720 has been shown to rapidly and reversibly induce lymphopenia (also referred to as peripheral lymphocyte lowering (PLL); Hale et al., Bioorg. Med. Chem. Lett., 14:3351-3355, 2004). This is attended by clinically useful immunosuppression by virtue of sequestering T- and B-cells in secondary lymphoid tissue (lymph nodes and Peyer's patches) and thus apart from sites of inflammation and organ grafts (Rosen et al., Immunol. Rev., 195:160-177, 2003; Schwab et al., Nature Immunol., 8:1295-1301, 2007).

In clinical trials, FTY720 elicited an adverse event (i.e., transient asymptomatic bradycardia) due to its agonism of the S1P3 receptor (Budde et al., J. Am. Soc. Nephrol., 13:1073-1083, 2002; Sanna et al., J. Biol. Chem., 279:13839-13848, 2004; Ogawa et al., BBRC, 361:621-628,2007).

FTY720 has been reported to have therapeutic efficacy in at least: a rat model for autoimmune myocarditis and a mouse model for acute viral myocarditis (Kiyabayashi et al., J. Cardiovasc. Pharmacol., 35:410-416, 2000; Miyamoto et al., J. Am. Coll. Cardiol., 37:1713-1718, 2001); mouse models for inflammatory bowel disease including colitis (Mizushima et al., Inflamm. Bowel Dis., 10:182-192, 2004; Deguchi et al., Oncology Reports, 16:699-703, 2006; Fujii et al., Am. J. Physiol. Gastrointest. Liver Physiol., 291:G267-G274, 2006; Daniel et al., J. Immunol., 178:2458-2468, 2007); a rat model for progressive mesangioproliferative glomerulonephritis (Martini et al., Am. J. Physiol. Renal Physiol., 292:F1761-F1770, 2007); a mouse model for asthma, suggested to be primarily through the S1P1 receptor on the basis of work using the the S1P1 receptor agonist SEW2871 (Idzko et al, J. Clin. Invest., 116:2935-2944, 2006); a mouse model for airway inflammation and induction of bronchial hyperresponsiveness (Sawicka et al., J. Immunol., 171;6206-6214, 2003); a mouse model for atopic dermatitis (Kohno et al., Biol. Pharm. Bull., 27:1392-1396, 2004); a mouse model for ischemia-reperfusion injury (Kaudel et al., Transplant. Proc, 39:499-502, 2007); a mouse model for systemic lupus erythematosus (SLE) (Okazaki et al., J. Rheumatol., 29:707-716, 2002; Herzinger et al, Am. J. Clin. Dermatol., 8:329-336, 2007); rat models for rheumatoid arthritis (Matsuura et al., Int. J. Immunopharmacol., 22:323-331, 2000; Matsuura et al., Inflamm. Res., 49:404-410, 2000); a rat model for autoimmune uveitis (Kurose et al., Exp. Eye Res., 70:7-15, 2000); mouse models for type I diabetes (Fu et al, Transplantation, 73:1425-1430, 2002; Maki et al., Transplantation, 74:1684-1686, 2002; Yang et al., Clinical Immunology, 107:30-35, 2003; Maki et al., Transplantation, 79:1051-1055, 2005); mouse models for atherosclerosis (Nofer et al., Circulation, 115:501-508, 2007; Keul et al., Arterioscler. Thromb. Vasc. Biol., 27:607-613, 2007); a rat model for brain inflammatory reaction following traumatic brain injury (TBI) (Zhang et al., J. Cell. Mol. Med., 11:307-314, 2007); and mouse models for graft coronary artery disease and graft-versus-host disease (GVHD) (Hwang et al., Circulation, 100:1322-1329, 1999; Taylor et al., Blood, 110:3480-3488,2007). *In vitro* results suggest that FTY720 may have therapeutic efficacy for β-amyloid-related inflammatory diseases including Alzheimer's disease (Kaneider et al., FASEB J., 18:309-311, 2004). KRP-203, an S1P receptor agonist having agonist activity on the S1P1 receptor, has been reported to have therapeutic efficacy in a rat model for autoimmune myocarditis (Ogawa et al., BBRC, 361:621-628, 2007). Using the S1P1 receptor agonist SEW2871, it has been shown that agonism of the endothelial S1P1 receptor prevents proinflammatory monocyte/endothelial interactions in type I diabetic vascular endothelium (Whetzel et al., Circ. Res., 99:731-739, 2006) and protects the vasculature against TNFα-mediated monocyte/endothelial interactions (Bolick et al., Arterioscler. Thromb. Vasc. Biol., 25:976-981, 2005).

Additionally, FTY720 has been reported to have therapeutic efficacy in experimental autoimmune encephalomyelitis (EAE) in rats and mice, a model for human multiple sclerosis (Brinkmann et al., J. Biol. Chem., 277:21453-21457, 2002; Fujino et al., J. Pharmacol. Exp. Ther., 305:70-77, 2003; Webb et al., J. Neuroimmunol., 153:108-121, 2004; Rausch et al., J. Magn. Reson. Imaging, 20:16-24, 2004; Kataoka et al., Cellular & Molecular Immunology, 2:439-448, 2005; Brinkmann et al., Pharmacology & Therapeutics, 115:84-105, 2007; Baumruker et al., Expert Opin. Investig. Drugs, 16:283-289, 2007; Balatoni et al., Brain Research Bulletin, 74:307-316, 2007). Furthermore, FTY720 has been found to have therapeutic efficacy for multiple sclerosis in clinical trials. In Phase II clinical trials for relapsing-remitting multiple sclerosis, FTY720 was found to reduce the number of lesions detected by magnetic resonance imaging (MRI) and clinical disease activity in patients with multiple sclerosis (Kappos et al., N. Engl. J. Med, 355:1124-1140, 2006; Martini et al., Expert Opin. Investig. Drugs, 16:505-518, 2007; Zhang et al., Mini-Reviews in Medicinal Chemistry, 7:845-850, 2007; Brinkmann, Pharmacology & Therapeutics, 115:84-105, 2007). FTY720 is currently in Phase III studies of remitting-relapsing multiple sclerosis (Brinkmann, Pharmacology & Therapeutics, 115:84-105, 2007; Baumruker et al., Expert. Opin. Investig. Drugs, 16:283-289, 2007; Dev et al., Pharmacology and Therapeutics, 117:77-93, 2008).

Recently, FTY720 has been reported to have anti-viral activity. Specific data has been presented in the lymphocytic choriomeningitis virus (LCMV) mouse model, wherein the mice were infected with either the Armstrong or the clone 13 strain of LCMV (Premenko-Lanier et al., Nature, 454, 894, 2008).

FTY720 has been reported to impair migration of dendritic cells infected with *Francisella tularensis* to the mediastinal lymph node, thereby reducing the bacterial colonization of it. *Francisella tularensis* is associated with tularemia, ulceroglandular infection, respiratory infection and a typhoidal disease (E. Bar-Haim et al, PLoS Pathogens, 4(11): e1000211. doi:10.1371/journal.ppat.1000211, 2008).

It has also been recently reported that a short-term high dose of FTY720 rapidly reduced ocular infiltrates in experimental autoimmune uveoretinitis. When given in the early stages of ocular inflammation, FTY720 rapidly prevented retinal damage. It was reported to not only prevent infiltration of target organs, but also reduce existing infiltration (Raveney et al., Arch. Ophthalmol. 126(10), 1390, 2008).

Agonism of the S1P1 receptor has been implicated in enhancement of survival of oligodendrocyte progenitor cells. Survival of oligodendrocyte progenitor cells is a required component of the remyelination process. Remyelination of multiple sclerosis lesions is considered to promote recovery from clinical relapses. (Miron et al., Ann. Neurol., 63:61-71, 2008; Coelho et al., J. Pharmacol. Exp. Ther., 323:626-635, 2007; Dev et al., Pharmacology and Therapeutics, 117:77-93, 2008). It also has been shown that the S1P1 receptor plays a role in platelet-derived growth factor (PDGF)-induced oligodendrocyte progenitor cell mitogenesis (Jung et al., Glia, 55:1656-1667, 2007).

Agonism of the S1P1 receptor has also been reported to mediate migration of neural stem cells toward injured areas of the central nervous system (CNS), including in a rat model of spinal cord injury (Kimura et al., Stem Cells, 25:115-124, 2007).

Agonism of the S1P1 receptor has been implicated in the inhibition of keratinocyte proliferation (Sauer et al., J. Biol. Chem., 279:38471-38479, 2004), consistent with reports that S1P inhibits keratinocyte proliferation (Kim et al., Cell Signal, 16:89-95, 2004). The hyperproliferation of keratinocytes at the entrance to the hair follicle, which can then become blocked, and an associated inflammation are significant pathogenetic factors of acne (Koreck et al., Dermatology, 206:96-105, 2003; Webster, Cutis, 76:4-7, 2005).

FTY720 has been reported to have therapeutic efficacy in inhibiting pathologic angiogenesis, such as that as may occur in tumor development. Inhibition of angiogenesis by FTY720 is thought to involve agonism of the S1P1 receptor (Oo et al., J. Biol. Chem., 282;9082-9089, 2007; Schmid et al., J. Cell Biochem., 101:259-270,2007). FTY720 has been reported to have therapeutic efficacy for inhibiting primary and metastatic tumor growth in a mouse model of melanoma (LaMontagne et al., Cancer Res., 66:221-231, 2006). FTY720 has been reported to have therapeutic efficacy in a mouse model for metastatic hepatocellular carcinoma (Lee et al., Clin. Cancer Res., 11:84588466, 2005).

It has been reported that oral administration of FTY720 to mice potently blocked VEGF-induced vascular permeability, an important process associated with angiogenesis, inflammation, and pathological conditions such as sepsis, hypoxia, and solid tumor growth (T Sanchez et al, J. Biol. Chem., 278(47), 47281-47290, 2003).

Cyclosporin A and FK506 (calcineurin inhibitors) are drugs used to prevent rejection of transplanted organs. Although they are effective in delaying or suppressing transplant rejection, classical immunosuppressants such as cyclosporin A and FK506 are known to cause several undesirable side effects including nephrotoxicity, neurotoxicity, β-cell toxicity and gastrointestinal discomfort. There is an unmet need in organ transplantation for an immunosuppressant without these side effects which is effective as a monotherapy or in combination with a classical immunosuppressant for inhibiting migration of, *e.g.*, alloantigen-reactive T-cells to the grafted tissue, thereby prolonging graft survival.

FTY720 has been shown to have therapeutic efficacy in transplant rejection both as a monotherapy and in synergistic combination with a classical immunosuppressant, including cyclosporin A, FK506 and RAD (an mTOR inhibitor). It has been shown that, unlike the classical immunosuppressants cyclosporin A, FK506 and RAD, FTY720 has effcacy for prolonging graft survival without inducing general immunosuppression, and this difference in drug action is believed to be relevant to the synergism observed for the combination (Brinkmann et al., Transplant Proc., 33:530-531, 2001; Brinkmann et al., Transplantation, 72:764-769, 2001).

Agonism of the S1P1 receptor has been reported to have therapeutic efficacy for prolonging allograft survival in mouse and rat skin allograft models (Lima et al., Transplant Proc., 36:1015-1017, 2004; Yan et al., Bioorg. & Med. Chem. Lett., 16:3679-3683,2006). FTY720 has been reported to have therapeutic efficacy for prolonging allograft survival in a rat cardiac allograft model (Suzuki et al., Transpl. Immunol., 4:252-255, 1996). FTY720 has been reported to act synergistically with cyclosporin A to prolong rat skin allograft survival (Yanagawa et al., J. Immunol., 160:5493-5499, 1998), to act synergistically with cyclosporin A and with FK506 to prolong rat cardiac allograft survival, and to act synergistically with cyclosporin A to prolong canine renal allograft survival and monkey renal allograft survival (Chiba et al., Cell Mol. Biol., 3:11-19, 2006). KRP-203, an S1P receptor agonist has been reported to have therapeutic efficacy for prolonging allograft survival in a rat skin allograft model and both as monotherapy and in synergistic combination with cyclosporin A in a rat cardiac allograft model (Shimizu et al., Circulation, 111:222-229, 2005). KRP-203 also has been reported to have therapeutic efficacy in combination with mycophenolate mofetil (MMF; a prodrug for which the active metabolite is mycophenolic acid, an inhibitor of purine biosynthesis) for prolonging allograft survival both in a rat renal allograft model and in a rat cardiac allograft model (Suzuki et al., J. Heart Lung Transplant, 25:302-209, 2006; Fujishiro et al., J. Heart Lung Transplant, 25:825-833, 2006). It has been reported that an agonist of the S1P1 receptor, AUY954, in combination with a subtherapeutic dose of RAD001 (Certican/Everolimus, an mTOR inhibitor) can prolong rat cardiac allograft survival (Pan et al., Chemistry & Biology, 13:1227-1234, 2006). In a rat small bowel allograft model, FTY720 has been reported to act synergistically with cyclosporin A to prolong small bowel allograft survival (Sakagawa et al., Transpl. Immunol., 13:161-168, 2004). FTY720 has been reported to have therapeutic efficacy in a mouse islet graft model (Fu et al., Transplantation, 73:1425-1430, 2002; Liu et al., Microsurgery, 27:300-304; 2007) and in a study using human islet cells to evidence no detrimental effects on human islet function (Truong et al., American Journal of Transplantation, 7:2031-2038, 2007).

FTY720 has been reported to reduce the nociceptive behavior in the spared nerve injury model for neuropathic pain which does not depend on prostaglandin synthesis (O. Costu et al, Journal of Cellular and Molecular Medicine 12(3), 995-1004, 2008).

FTY720 has been reported to impair initiation of murine contact hypersensitivity (CHS). Adoptive transfer of immunized lymph node cells from mice treated with FTY720 during the sensitization phase was virtually incapable of inducing CHS response in recipients (D. Nakashima et al., J. Investigative Dermatology (128(12), 2833-2841, 2008).

It has been reported that prophylactic oral administration of FTY720 (1 mg/kg, three times a week), completely prevented the development of experimental autoimmune myasthenia gravis (EAMG) in C57BL/6 mice (T. Kohono et al, Biological & Pharmaceutical Bulletin, 28(4), 736-739, 2005).

S1P agonist are disclosed in WO2005032465, WO2007116866 and Bioorg. Med. chem. lett. 16 (2006), 3684. In one embodiment, the present invention encompasses compounds which are agonists of the S1P1 receptor having selectivity over the S1P3 receptor. The S1P3 receptor, and not the S1P1 receptor, has been directly implicated in bradycardia (Sanna et al., J. Biol. Chem., 279:13839-13848, 2004). An S1P1 receptor agonist selective over at least the S1P3 receptor has advantages over current therapies by virtue of an enhanced therapeutic window, allowing better tolerability with higher dosing and thus improving efficacy as therapy. The present invention encompasses compounds which are agonists of the S1P1 receptor and which exhibit no or substantially no activity for bradycardia.

S1P1 receptor agonists are useful to treat or prevent conditions where suppression of the immune system or agonism of the S1P1 receptor is in order, such as diseases and disorders mediated by lymphocytes, transplant rejection, autoimmune diseases and disorders, inflammatory diseases and disorders, and conditions that have an underlying defect in vascular integrity or that relate to angiogenesis such as may be pathologic.

In one embodiment, the present invention encompasses compounds which are agonists of the S1P1 receptor having good overall physical properties and biological activities and having an effectiveness that is substantially at least that of prior compounds with activity at the S1P1 receptor.

Citation of any reference throughout this application is not to be construed as an admission that such reference is prior art to the present application.

### SUMMARY OF THE INVENTION

The present invention encompasses compounds of Formula (Ia) and pharmaceutically acceptable salts, solvates and hydrates thereof 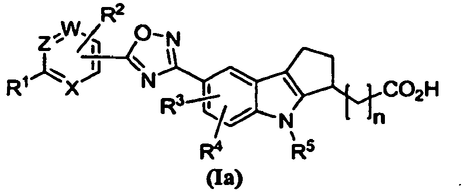 wherein:
n is 0 or 1;
W is N or CR⁶;
Z isN or CR⁷;
X is N or CR⁸; provided that W, Z and X are not all N;
R¹, R², R⁶, R⁷ and R⁸ are each independently selected from the group consisting of H, C₁-C₆ acyl, C₁-C₆ acyloxy, C₁-C₆ alkoxy, C₁-C₆ alkoxycarbonylamino, C₁-C₆ alkyl, C₂-C₆ alkynyl, C₁-C₆ alkylamino, C₂-C₈ dialkylamino, C₁-C₆ alkylcarboxamide, C₁-C₆ alkylsulfonamide, C₁-C₆ alkylsulfinyl, C₁-C₆ alkylsulfonyl, C₁-C₆ alkylthio, C₁-C₆ alkylureyl, amino, carbo-C₁-C₆-alkoxy, carboxamide, carboxy, cyano, C₃-C₇ cycloalkyl, C₃-C₇ cycloalkyloxy, C₃-C₇ cycloalkylthio, C₃-C₇ cycloalkylsulfinyl, C₃-C₇ cycloalkylsulfonyl, C₂-C₆ dialkylcarboxamide, C₁-C₆ haloalkoxy, C₁-C₆ haloalkyl, C₁-C₆ haloalkylsulfinyl, C₁-C₆ haloalkylsulfonyl, halogen, heteroaryl, heterocyclyl, hydroxyl, nitro and sulfonamide, wherein C₁-C₆ alkoxy, C₁-C₆ alkylthio, C₁-C₆ alkyl, C₂-C₆ alkynyl and heteroaryl are each optionally substituted with 1, 2 or 3 substituents selected independently from C₁-C₆ alkoxy, carbo-C₁-C₆-alkoxy, C₁-C₆ alkyl, cyano, C₃-C₇ cycloalkyl, halogen and phenyl;
R³ and R⁴ are each independently selected from the group consisting of H, C₁-C₂ alkyl, fluoro and chloro; and
R⁵ is H or C₁-C₆ alkyl.

In some embodiments, the present invention encompasses compounds of Formula (**Ia**) and pharmaceutically acceptable salts, solvates and hydrates thereof: 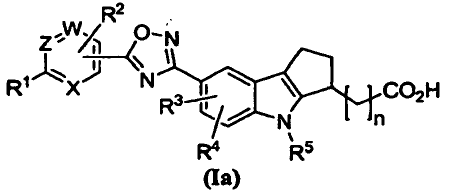 wherein:
n is 0 or 1;
W is N or CR⁶;
Z is N or CR⁷;
X is N or CR⁸; provided that W, Z and X are not all N;
R¹, R², R⁶, R⁷ and R⁸ are each independently selected from the group consisting of H, C₁-C₆ acyl, C₁-C₆ acyloxy, C₁-C₆ alkoxy, C₁-C₆ alkoxycarbonylamino, C₁-C₆ alkyl, C₂-C₆ alkynyl, C₁-C₆ alkylamino, C₁-C₈ dialkylamino, C₁-C₆ alkylcarboxamide, C₁-C₆ alkylsulfonamide, C₁-C₆ alkylsulfinyl, C₁-C₆ alkylsulfonyl, C₁-C₆ alkylthio, C₁-C₆ alkylureyl, amino, carbo-C₁-C₆-alkoxy, carboxamide, carboxy, cyano, C₃-C₇ cycloalkyl, C₃-C₇ cycloalkyloxy, C₃-C₇ cycloalkylthio, C₃-C₇ cycloalkylsulfinyl, C₃-C₇ cycloalkylsulfonyl, C₂-C₆ dialkylcarboxamide, C₁-C₆ haloalkoxy, C₁-C₆ haloalkyl, C₁-C₆ haloalkylsulfinyl, C₁-C₆ haloalkylsulfonyl, halogen, heteroaryl, heterocyclyl, hydroxyl, nitro and sulfonamide, wherein C₁-C₆ alkoxy, C₁-C₆ alkylthio, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl;
R³ and R⁴ are each independently selected from the group consisting of H, C₁-C₂ alkyl, fluoro and chloro; and
R⁵ is H or C₁-C₆ alkyl.

Compounds of the invention encompass compounds which are S1P1 receptor agonists having at least immunosuppressive, anti-inflammatory and/or hemostatic activities, e.g. by virtue of modulating leukocyte trafficking, sequestering lymphocytes in secondary lymphoid tissues, and/or enhancing vascular integrity.

S1P1 receptor agonists are useful to treat or prevent conditions where suppression of the immune system or agonism of S1P1 receptor is in order, such as diseases and disorders mediated by lymphocytes, transplant rejection, autoimmune diseases and disorders, inflammatory diseases and disorders (e.g., acute and chronic inflammatory conditions), cancer, and conditions that have an underlying defect in vascular integrity or that are associated with angiogenesis such as may be pathologic (e.g., as may occur in inflammation, tumor development and atherosclerosis). Such conditions where suppression of the immune system or agonism of S1P1 receptor is in order include diseases and disorders mediated by lymphocytes, conditions that have an underlying defect in vascular integrity, autoimmune diseases and disorders, inflammatory diseases and disorders (e.g., acute and chronic inflammatory conditions), (acute or chronic) rejection of cells, tissue or solid organ grafts, arthritis including psoriatic arthritis and rheumatoid arthritis, diabetes including type I diabetes, demyelinating disease including multiple sclerosis, ischemia-reperfusion injury including renal and cardiac ischemia-reperfusion injury, inflammatory skin disease including psoriasis, atopic dermatitis and acne, hyperproliferative skin disease including acne, inflammatory bowel disease including Crohn's disease and ulcerative colitis, systemic lupus erythematosis, asthma, uveitis, myocarditis, allergy, atherosclerosis, brain inflammation including Alzheimer's disease and brain inflammatory reaction following traumatic brain injury, central nervous system disease including spinal cord injury or cerebral infarction, pathologic angiogenesis including as may occur in primary and metastatic tumor growth, rheumatoid arthritis, diabetic retinopathy and atherosclerosis, cancer, chronic pulmonary disease, acute lung injury, acute respiratory disease syndrome, sepsis and the like.

One aspect of the present invention pertains to pharmaceutical compositions comprising a compound of the present invention and a pharmaceutically acceptable carrier.

The present invention discloses methods for treating a disorder associated with the S1P1 receptor in an individual comprising administering to the individual in need thereof a therapeutically effective amount of a compound of the present invention or a pharmaceutical composition thereof.

The present invention discloses methods for treating a disorder associated with the S1P1 receptor in an individual comprising administering to the individual in need thereof a therapeutically effective amount of a compound of the present invention or a pharmaceutical composition thereof, wherein said disorder associated with the S1P1 receptor is selected from the group consisting of a disease or disorder mediated by lymphocytes, an autoimmune disease or disorder, an inflammatory disease or disorder, cancer, psoriasis, rheumatoid arthritis, Crohn's disease, transplant rejection, multiple sclerosis, systemic lupus erythematosus, ulcerative colitis, type I diabetes and acne.

The present invention discloses methods for treating a disorder in an individual comprising administering to said individual in need thereof a therapeutically effective amount of a compound of the present invention or a pharmaceutical composition thereof, wherein said disorder is selected from the group consisting of psoriasis, rheumatoid arthritis, Crohn's disease, transplant rejection, multiple sclerosis, systemic lupus erythematosus, ulcerative colitis, type I diabetes and acne.

The present invention discloses methods for treating a disease or disorder mediated by lymphocytes in an individual comprising administering to the individual in need thereof a therapeutically effective amount of a compound of the present invention or a pharmaceutical composition thereof.

The present invention discloses methods for treating an autoimmune disease or disorder in an individual comprising administering to the individual in need thereof a therapeutically effective amount of a compound of the present invention or a pharmaceutical composition thereof.

The present invention discloses methods for treating an inflammatory disease or disorder in an individual comprising administering to the individual in need thereof a therapeutically effective amount of a compound of the present invention or a pharmaceutical composition thereof.

The present invention discloses methods for treating cancer in an individual comprising administering to the individual in need thereof a therapeutically effective amount of a compound of the present invention or a pharmaceutical composition thereof. The present invention discloses methods for treating psoriasis in an individual comprising administering to the individual in need thereof a therapeutically effective amount of a compound of the present invention or a pharmaceutical composition thereof. The present invention discloses methods for treating rheumatoid arthritis in an individual comprising administering to the individual in need thereof a therapeutically effective amount of a compound of the present invention or a pharmaceutical composition thereof.

The present invention discloses methods for treating Crohn's disease in an individual comprising administering to the individual in need thereof a therapeutically effective amount of a compound of the present invention or a pharmaceutical composition thereof.

The present invention discloses methods for treating transplant rejection in an individual comprising administering to the individual in need thereof a therapeutically effective amount of a compound of the present invention or a pharmaceutical composition thereof.

The present invention discloses methods for treating multiple sclerosis in an individual comprising administering to the individual in need thereof a therapeutically effective amount of a compound of the present invention or a pharmaceutical composition thereof.

The present invention discloses methods for treating systemic lupus erythematosus in an individual comprising administering to the individual in need thereof a therapeutically effective amount of a compound of the present invention or a pharmaceutical composition thereof.

The present invention discloses methods for treating ulcerative colitis in an individual comprising administering to the individual in need thereof a therapeutically effective amount of a compound of the present invention or a pharmaceutical composition thereof.

The present invention discloses methods for treating type I diabetes in an individual comprising administering to the individual in need thereof a therapeutically effective amount of a compound of the present invention or a pharmaceutical composition thereof.

The present invention discloses methods for treating acne in an individual comprising administering to the individual in need thereof a therapeutically effective amount of a compound of the present invention or a pharmaceutical composition thereof. The present invention discloses methods for treating a disorder associated with the S1P1 receptor in an individual comprising administering to the individual in need thereof a therapeutically effective amount of a compound of the present invention or a pharmaceutical composition thereof, wherein said disorder associated with the S1P1 receptor is a microbial infection or disease or a viral infection or disease.

The present invention discloses methods for treating a disorder associated with the S1P1 receptor in an individual comprising administering to the individual in need thereof a therapeutically effective amount of a compound of the present invention or a pharmaceutical composition thereof, wherein said disorder associated with the S1P1 receptor is an intraocular inflammatory disease.

One aspect of the present invention pertains to the use of compounds of the present invention in the manufacture of a medicament for the treatment of a S1P1 receptor associated disorder.

One aspect of the present invention pertains to the use of compounds of the present invention in the manufacture of a medicament for the treatment of a S1P1 receptor associated disorder wherein said S1P1 receptor associated disorder is selected from the group consisting of: a disease or disorder mediated by lymphocytes, an autoimmune disease or disorder, an inflammatory disease or disorder, cancer, psoriasis, rheumatoid arthritis, Crohn's disease, transplant rejection, multiple sclerosis, systemic lupus erythematosus, ulcerative colitis, type I diabetes and acne.

One aspect of the present invention pertains to the use of compounds of the present invention in the manufacture of a medicament for the treatment of a S1P1 receptor associated disorder selected from the group consisting of psoriasis, rheumatoid arthritis, Crohn's disease, transplant rejection, multiple sclerosis, systemic lupus erythematosus, ulcerative colitis, type I diabetes and acne.

One aspect of the present invention pertains to the use of compounds of the present invention in the manufacture of a medicament for the treatment of a disease or disorder mediated by lymphocytes.

One aspect of the present invention pertains to the use of compounds of the present invention in the manufacture of a medicament for the treatment of an autoimmune disease or disorder.

One aspect of the present invention pertains to the use of compounds of the present invention in the manufacture of a medicament for the treatment of an inflammatory disease or disorder.

One aspect of the present invention pertains to the use of compounds of the present invention in the manufacture of a medicament for the treatment of cancer.

One aspect of the present invention pertains to the use of compounds of the present invention in the manufacture of a medicament for the treatment of psoriasis.

One aspect of the present invention pertains to the use of compounds of the present invention in the manufacture of a medicament for the treatment of rheumatoid arthritis.

One aspect of the present invention pertains to the use of compounds of the present invention in the manufacture of a medicament for the treatment of Crohn's disease.

One aspect of the present invention pertains to the use of compounds of the present invention in the manufacture of a medicament for the treatment of transplant rejection.

One aspect of the present invention pertains to the use of compounds of the present invention in the manufacture of a medicament for the treatment of multiple sclerosis.

One aspect of the present invention pertains to the use of compounds of the present invention in the manufacture of a medicament for the treatment of systemic lupus erythematosus.

One aspect of the present invention pertains to the use of compounds of the present invention in the manufacture of a medicament for the treatment of ulcerative colitis.

One aspect of the present invention pertains to the use of compounds of the present invention in the manufacture of a medicament for the treatment of type I diabetes.

One aspect of the present invention pertains to the use of compounds of the present invention in the manufacture of a medicament for the treatment of acne.

One aspect of the present invention pertains to the use of compounds of the present invention in the manufacture of a medicament for the treatment of a S1P1 receptor associated disorder wherein said S1P1 receptor associated disorder is a microbial infection or disease or a viral infection or disease.

One aspect of the present invention pertains to the use of compounds of the present invention in the manufacture of a medicament for the treatment of a S1P1 receptor associated disorder wherein said S1P1 receptor associated disorder is an intraocular inflammatory disease.

One aspect of the present invention pertains to compounds of the present invention for use in a method of treatment of the human or animal body by therapy.

One aspect of the present invention pertains to compounds of the present invention for use in a method for the treatment of a S1P1 receptor associated disorder.

One aspect of the present invention pertains to compounds of the present invention for use in a method for the treatment of a S1P1 receptor associated disorder wherein said S1P1 receptor associated disorder is selected from the group consisting of: a disease or disorder mediated by lymphocytes, an autoimmune disease or disorder, an inflammatory disease or disorder, cancer, psoriasis, rheumatoid arthritis, Crohn's disease, transplant rejection, multiple sclerosis, systemic lupus erythematosus, ulcerative colitis, type I diabetes and acne.

One aspect of the present invention pertains to compounds of the present invention for use in a method for the treatment of a S1P1 receptor associated disorder selected from the group consisting of psoriasis, rheumatoid arthritis, Crohn's disease, transplant rejection, multiple sclerosis, systemic lupus erythematosus, ulcerative colitis, type I diabetes and acne.

One aspect of the present invention pertains to compounds of the present invention for use in a method for the treatment of a disease or disorder mediated by lymphocytes.

One aspect of the present invention pertains to compounds of the present invention for use in a method for the treatment of an autoimmune disease or disorder.

One aspect of the present invention pertains to compounds of the present invention for use in a method for the treatment of an inflammatory disease or disorder.

One aspect of the present invention pertains to compounds of the present invention for use in a method for the treatment of cancer.

One aspect of the present invention pertains to compounds of the present invention for use in a method for the treatment of psoriasis.

One aspect of the present invention pertains to compounds of the present invention for use in a method for the treatment of rheumatoid arthritis.

One aspect of the present invention pertains to compounds of the present invention for use in a method for the treatment of Crohn's disease.

One aspect of the present invention pertains to compounds of the present invention for use in a method for the treatment of transplant rejection.

One aspect of the present invention pertains to compounds of the present invention for use in a method for the treatment of multiple sclerosis.

One aspect of the present invention pertains to compounds of the present invention for use in a method for the treatment of systemic lupus erythematosus.

One aspect of the present invention pertains to compounds of the present invention for use in a method for the treatment of ulcerative colitis.

One aspect of the present invention pertains to compounds of the present invention for use in a method for the treatment of type I diabetes.

One aspect of the present invention pertains to compounds of the present invention for use in a method for the treatment of acne.

One aspect of the present invention pertains to compounds of the present invention for use in a method for the treatment of a S1P1 receptor associated disorder wherein said S1P1 receptor associated disorder is a microbial infection or disease or a viral infection or disease.

One aspect of the present invention pertains to compounds of the present invention for use in a method for the treatment of a S1P1 receptor associated disorder wherein said S1P1 receptor associated disorder is an intraocular inflammatory disease.

One aspect of the present invention pertains to processes for preparing a composition comprising admixing a compound of the present invention and a pharmaceutically acceptable carrier.

These and other aspects of the invention disclosed herein will be set forth in greater detail as the patent disclosure proceeds.

### BRIEF DESCRIPTION OF THE DRAWINGS

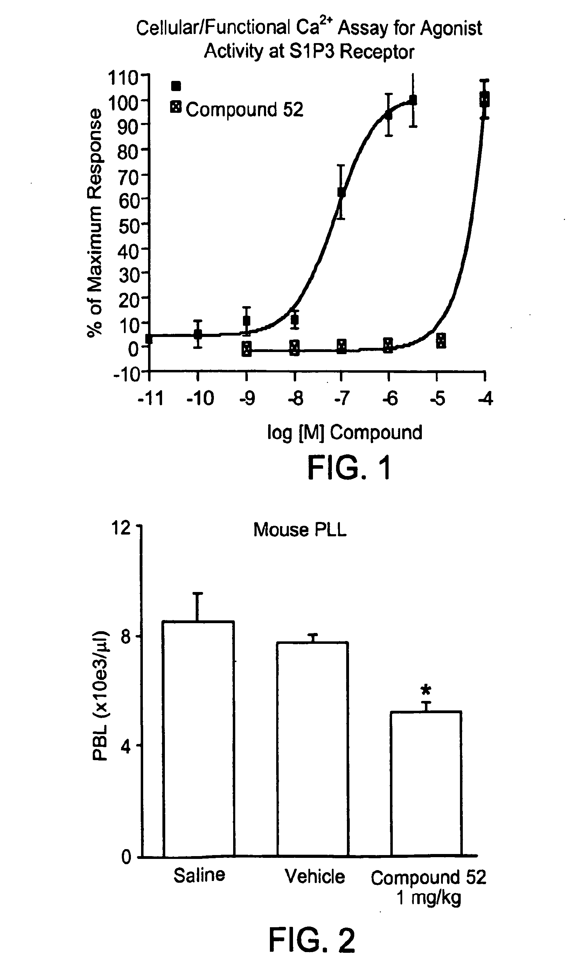

**Figure 1** shows the cellular/functional Ca²⁺ assay for agonist activity at the S1P3 receptor. The calcium ionophore A23187 shows pronounced agonist activity while Compound 52, a representative compound of the present invention, shows substantially little to no activity.
**Figure 2** shows the ability for Compound 52 to lower peripheral lymphocytes in the mouse compared to saline and vehicle.

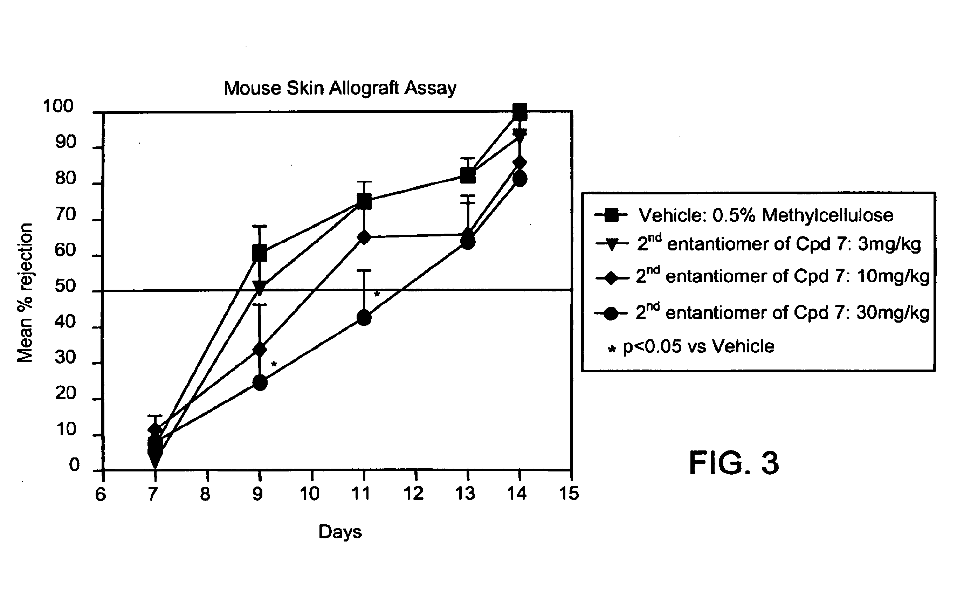

**Figure 3** shows the results of an experiment which measured the ability of three different doses of the 2^{nd} enantiomer of Compound 7 (isolated after resolution of compound 7 by HPLC, with a retention time of 24 min per the conditions reported in Example 1.22) to lower allograft rejection in mice compared to vehicle.

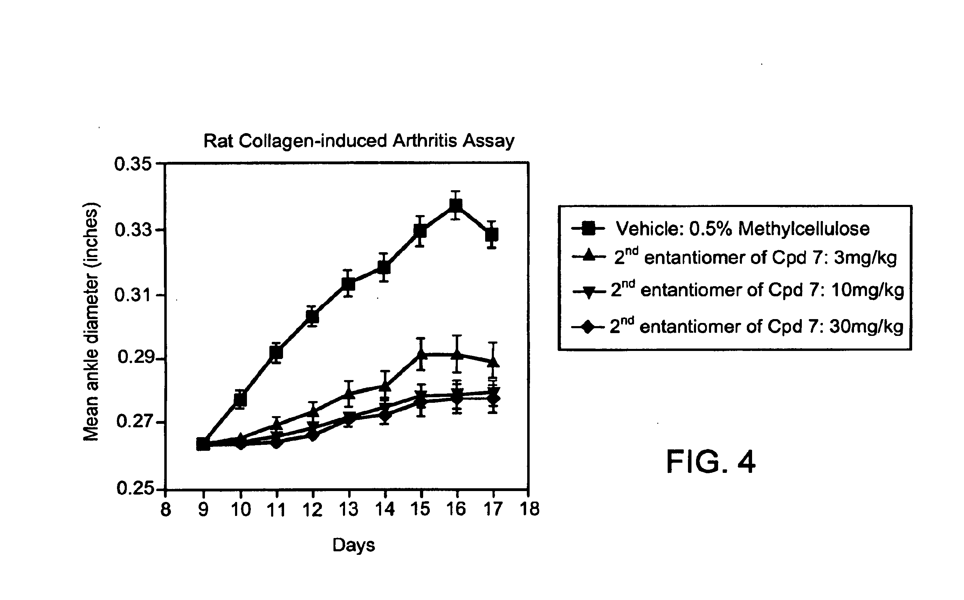

**Figure 4** shows the results of an experiment which measured the ability of three different doses of the 2^{nd} enantiomer of Compound 7 (isolated after resolution of compound 7 by HPLC, with a retention time of 24 min per the conditions reported in Example 1.22) to reduce the mean ankle diameter in rats compared to vehicle.

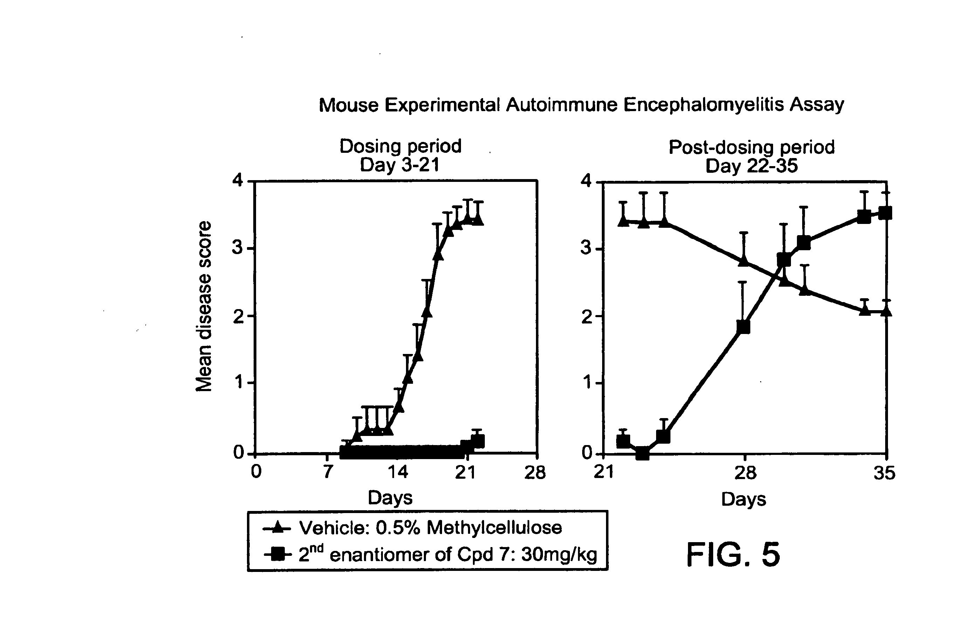

**Figure 5** shows the results of an experiment which measured the ability of the 2^{nd} enantiomer of Compound 7 (isolated after resolution of compound 7 by HPLC, with a retention time of 24 min per the conditions reported in Example 1.22) to have efficacy in experimental autoimmune encephalomyelitis (EAE) compared to vehicle.

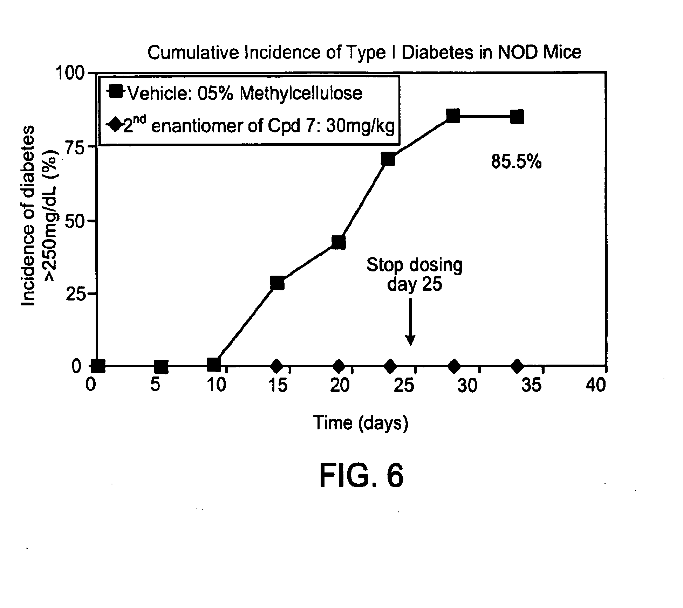

**Figure 6** shows the results of an experiment which measured the ability of the 2^{nd} enantiomer of Compound 7 (isolated after resolution of compound 7 by HPLC, with a retention time of 24 min per the conditions reported in Example 1.22) to lower the incidence of diabetes in NOD mice compared to vehicle.

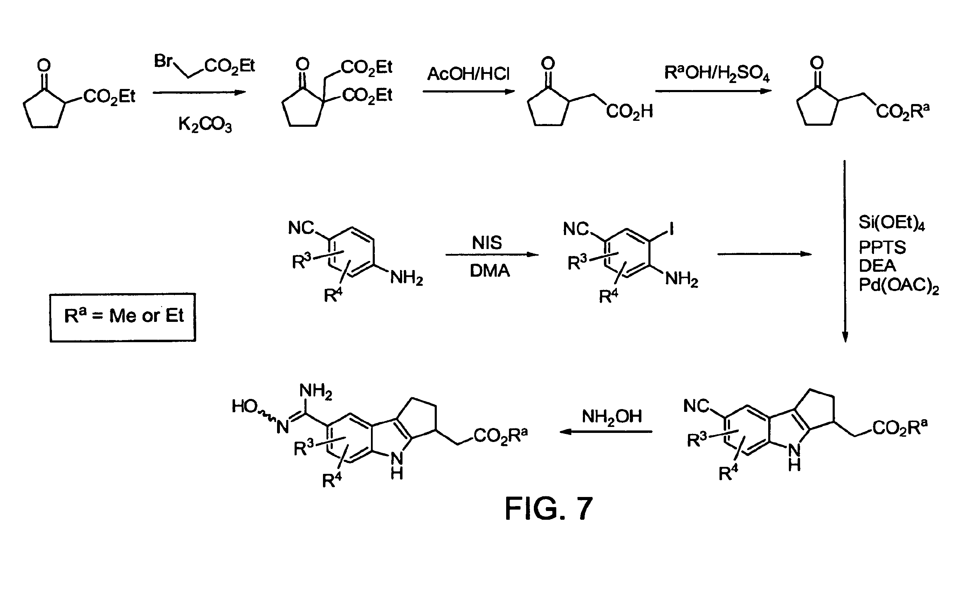

**Figure 7** shows a general synthetic scheme for the preparation of *N*-hydroxy-carbamimidoyl)-1,2,3,4-tetrahydrocyclopenta[b]indole intermediates useful in the preparation of Compound of Formula (**Ia**) wherein "n" is 1 and R⁵ is H.

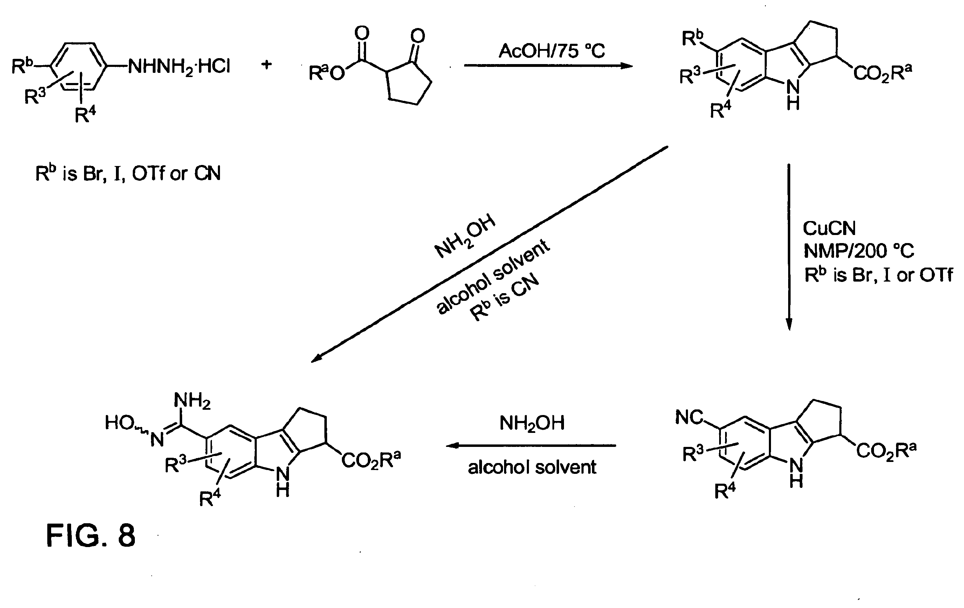

**Figure 8** shows a general synthetic scheme for the preparation of *N*-hydroxy-carbamimidoyl)-1,2,3,4-tetrahydrocyclopenta[b]indole intermediates useful in the preparation of compounds of Formula (**Ia**) wherein "n" is 0 and R⁵ is H.

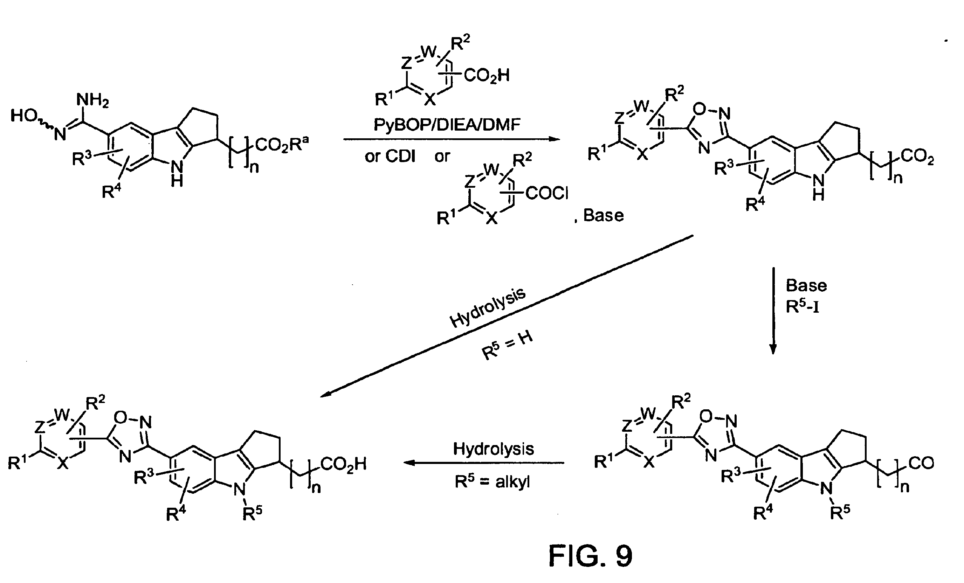

**Figure 9** shows a general synthetic scheme for the preparation of compounds of Formula (**Ia**). The synthetic scheme shows the coupling of carboxylic acids or acid chlorides with *N*-hydroxy-carbamimidoyl)-1,2,3,4-tetrahydrocyclopenta[b]indole intermediates and subsequent formation of the oxadiazole ring to provide ester intermediates. The resulting esters can be *N*-alkylated to introduce R⁵ alkyl groups or can be directly hydrolyzed to provide compounds of Formula (**Ia**) wherein R⁵ is alkyl or H respectively.

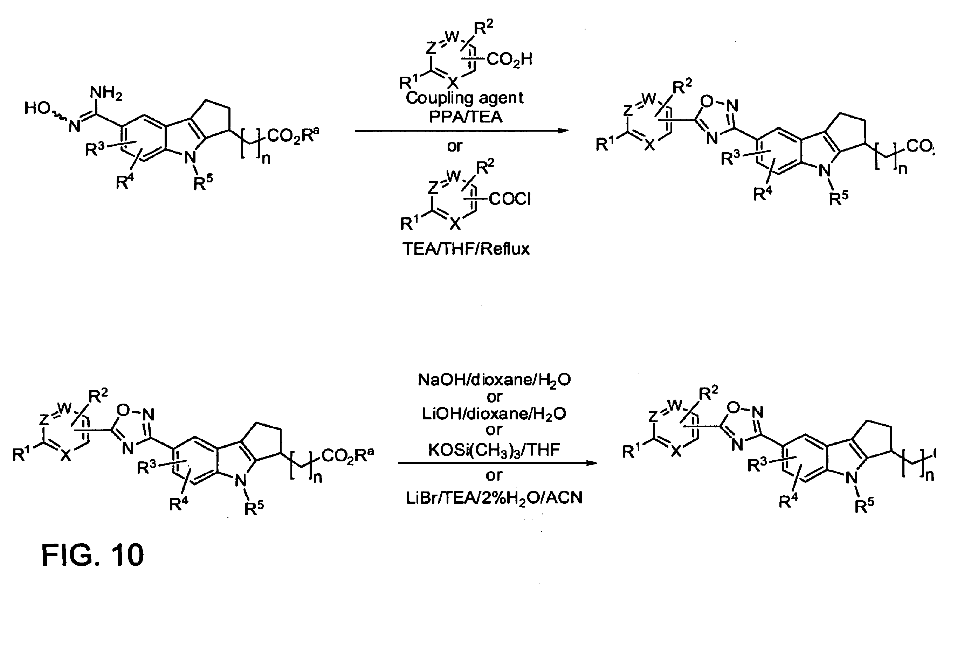

**Figure 10** shows the coupling of carboxylic acids or acid chlorides with *N*-hydroxy-carbamimidoyl)-1,2,3,4-tetrahydrocyclopenta[b]indole intermediates and subsequent formation of the oxadiazole ring to provide ester intermediates. **Figure 10** also shows general methods to hydrolyze the ester to provide compounds of Formula (**Ia**).

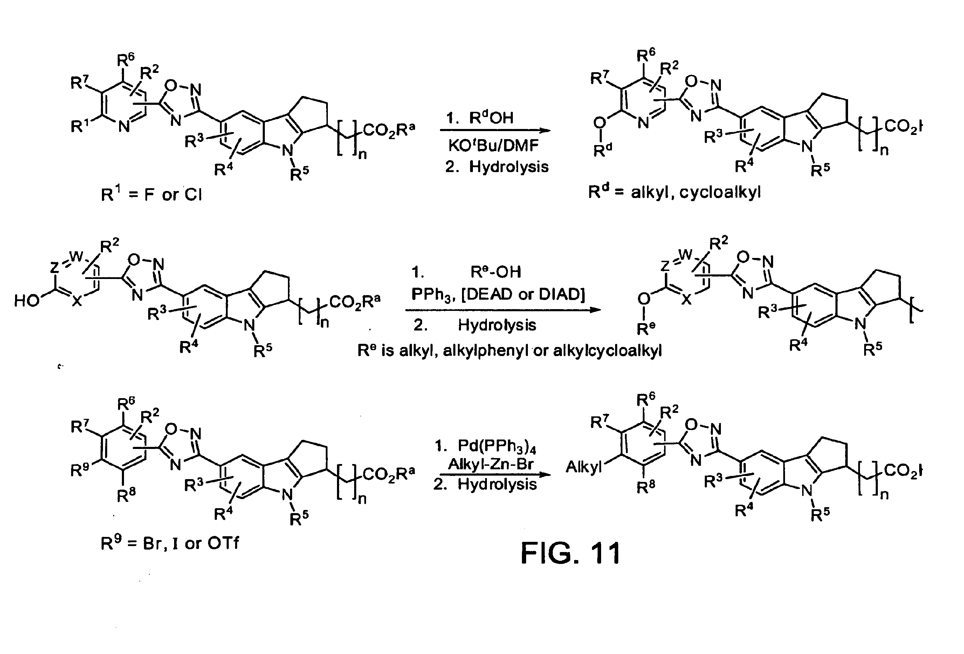

**Figure 11** shows three general routes for modifying the aryl and heteroaryl ring present in ester intermediates. The first route shows adding alcohols to fluoro or chloro pyridine to provide ethers. The second route shows the formation of ethers using PPh₃ and DEAD or DIAD. The third route shows Pd(0) catalyzed alkyl couplings. Each route provides esters that are subsequently hydrolyzed to provide compounds of Formula (**Ia**).

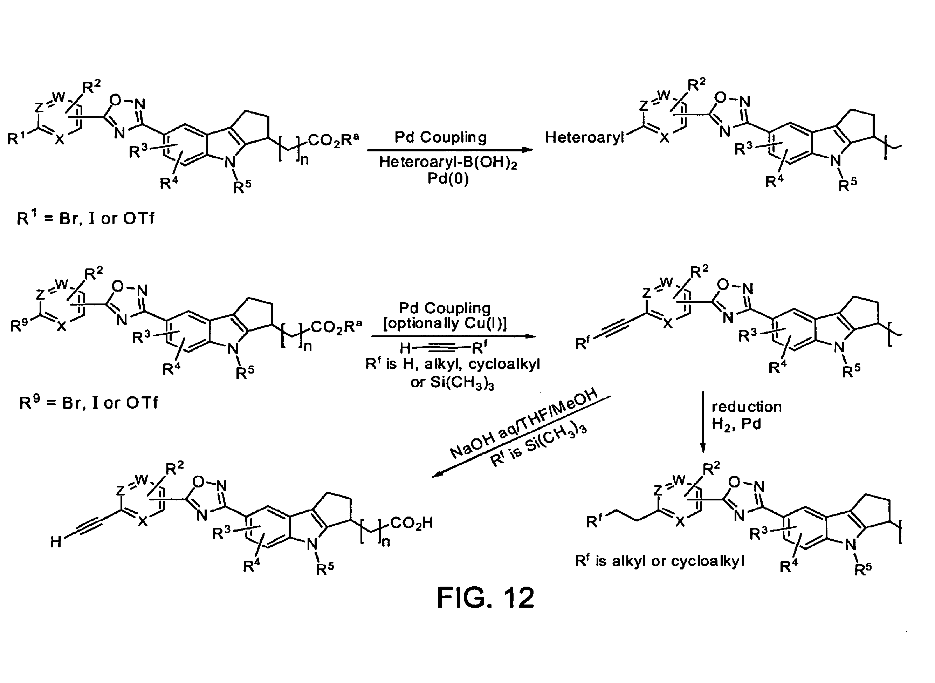

**Figure 12** shows two general routes for modifying the aryl and heteroaryl ring present in ester intermediates. The first route shows Pd(0) catalyzed heteroaryl coupling to provide ethers. The second route shows Pd(0) catalyzed couplings to give alkynes and can optionally be reduced to provide the corresponding alkyl group. Each route provides esters that are subsequently hydrolyzed to provide compounds of Formula (**Ia**).

### DETAILED DESCRIPTION OF THE INVENTION

### DEFINITIONS

For clarity and consistency, the following definitions will be used throughout this patent document.

The term "**agonist**" is intended to mean a moiety that interacts with and activates a G-protein-coupled receptor, such as the S1P1 receptor, such as can thereby initiate a physiological or pharmacological response characteristic of that receptor. For example, an agonist activites an intracellular response upon binding to the receptor, or enhances GTP binding to a membrane. In certain embodiments, an agonist of the invention is an S1P1 receptor agonist that is capable of facilitating sustained S1P1 receptor internalization (*see e.g.*, Matloubian *et al.,* Nature, 427, 355, 2004).

The term "**antagonist**" is intended to mean a moietiy that competitively binds to the receptor at the same site as an agonist (for example, the endogenous ligand), but which does not activate the intracellular response initiated by the active form of the receptor and can thereby inhibit the intracellular responses by an agonist or partial agonist. An antagonist does not diminish the baseline intracellular response in the absence of an agonist or partial agonist.

The term "**hydrate**" as used herein means a compound of the invention or a salt thereof, that further includes a stoichiometric or non-stoichiometric amount of water bound by noncovalent intermolecular forces.

The term "**solvate**" as used herein means a compound of the invention or a salt, thereof, that further includes a stoichiometric or non-stoichiometric amount of a solvent bound by noncovalent intermolecular forces. Preferred solvents are volatile, non-toxic, and/or acceptable for administration to humans in trace amounts.

The term "**in need of treatment"** and the term "**in need thereof**" when referring to treatment are used interchangeably to mean a judgment made by a caregiver (*e.g.* physician, nurse, nurse practitioner, *etc*. in the case of humans; veterinarian in the case of animals, including non-human mammals) that an individual or animal requires or will benefit from treatment. This judgment is made based on a variety of factors that are in the realm of a caregiver's expertise, but that includes the knowledge that the individual or animal is ill, or will become ill, as the result of a disease, condition or disorder that is treatable by the compounds of the invention. Accordingly, the compounds of the invention can be used in a protective or preventive manner; or compounds of the invention can be used to alleviate, inhibit or ameliorate the disease, condition or disorder.

The term "**individual**" is intended to mean any animal, including mammals, preferably mice, rats, other rodents, rabbits, dogs, cats, swine, cattle, sheep, horses, or primates and most preferably humans.

The term "**inverse agonist**" is intended to mean a moiety that binds to the endogenous form of the receptor or to the constitutively activated form of the receptor and which inhibits the baseline intracellular response initiated by the active form of the receptor below the normal base level of activity which is observed in the absence of an agonist or partial agonist, or decreases GTP binding to a membrane. Preferably, the baseline intracellular response is inhibited in the presence of the inverse agonist by at least 30%, more preferably by at least 50% and most preferably by at least 75%, as compared with the baseline response in the absence of the inverse agonist.

The term "**modulate or modulating**" is intended to mean an increase or decrease in the amount, quality, response or effect of a particular activity, function or molecule.

The term "**pharmaceutical composition**" is intended to mean a composition comprising at least one active ingredient; including but not limited to, salts, solvates and hydrates of compounds of the present invention, whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

The term "**therapeutically effective amount**" is intended to mean the amount of active compound or pharmaceutical agent that elicits the biological or medicinal response in a tissue, system, animal, individual or human that is being sought by a researcher, veterinarian, medical doctor or other clinician or caregiver or by an individual, which includes one or more of the following:
(1) Preventing the disease, for example, preventing a disease, condition or disorder in an individual that may be predisposed to the disease, condition or disorder but does not yet experience or display the pathology or symptomatology of the disease;
(2) Inhibiting the disease, for example, inhibiting a disease, condition or disorder in an individual that is experiencing or displaying the pathology or symptomatology of the disease, conditions or disorder (*i.e.*, arresting further development of the pathology and/or symptomatology); and
(3) Ameliorating the disease, for example, ameliorating a disease, condition or disorder in an individual that is experiencing or displaying the pathology or symptomatology of the disease, condition or disorder (*i.e.*, reversing the pathology and/or symptomatology).

### CHEMICAL GROUP, MOIETY OR RADICAL

The term "**C₁-C₆ acy**l" is intended to mean a C₁-C₆ alkyl radical attached to the carbon of a carbonyl group wherein the definition of alkyl has the same definition as described herein; some examples include, but are not limited to, acetyl, propionyl, *n*-butanoyl, *sec-*butanoyl, pivaloyl, pentanoyl and the like.

The term "**C₁-C₆ acyloxy**" is intended to mean an acyl radical attached to an oxygen atom wherein acyl has the same definition has described herein; some embodiments are when acyloxy is C₁-C₅ acyloxy, some embodiments are when acyloxy is C₁-C₆ acyloxy. Some examples include, but are not limited to, acetyloxy, propionyloxy, butanoyloxy, *iso-*butanoyloxy, pentanoyloxy, hexanoyloxy and the like.

The term "**C₁-C₆ alkoxy**" is intended to mean a C₁-C₆ alkyl radical, as defined herein, attached directly to an oxygen atom, some embodiments are 1 to 5 carbons, some embodiments are 1 to 4 carbons, some embodiments are 1 to 3 carbons and some embodiments are 1 or 2 carbons. Examples include methoxy, ethoxy, *n*-propoxy, *iso*-propoxy, *n*-butoxy, *t*-butoxy, *iso-*butoxy, *sec*-butoxy and the like.

The term "C₁-C₆ **alkoxycarbonylamino**" is intended to mean a single C₁-C₆ alkoxy group attached to the carbon of an amide group wherein alkoxy has the same definition as found herein. The alkoxycarbonylamino group may be represented by the following: 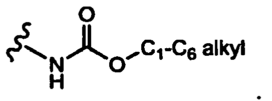 The term "**C₁-C₆ alkyl**" is intended to mean a straight or branched carbon radical containing 1 to 6 carbons, some embodiments are 1 to 5 carbons, some embodiments are 1 to 4 carbons, some embodiments are 1 to 3 carbons and some embodiments are 1 or 2 carbons. Examples of an alkyl include, but not limited to, methyl, ethyl, *n*-propyl, *iso*-propyl, *n*-butyl, *sec*-butyl, *iso*-butyl, *t*-butyl, pentyl, *iso*-pentyl, *t*-pentyl, *neo*-pentyl, 1-methylbutyl [*i.e.*, -CH(CH₃)CH₂CH₂CH₃], 2-methylbutyl [*i.e.*, -CH₂CH(CH₃)CH₂CH₃], *n*-hexyl and the like.

The term "**C₁₋C₆ alkylcarboxamido**" or "**C₁-C₆ alkylcarboxamide**" is intended to mean a single C₁-C₆ alkyl group attached to either the carbon or the nitrogen of an amide group, wherein alkyl has the same definition as found herein. The C₁-C₆ alkylcarboxamido group may be represented by the following:

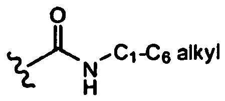

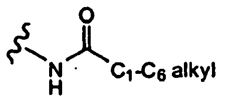

Examples include, but are not limited to, *N*-methylcarboxamide, *N*-ethylcarboxamide, *N*-*n*-propylcarboxamide, *N*-*iso*-propylcarboxamide, *N*-*n*-butylcarboxamide, *N-sec-*butylcarboxamide, *N*-*iso*-butylcarboxamide, *N*-*t*-butylcarboxamide and the like.

The term "**C₁-C₆alkylsulfinyl**" is intended to mean a C₁-C₆alkyl radical attached to the sulfur of a sulfoxide radical having the formula: -S(O)- wherein the alkyl radical has the same definition as described herein. Examples include, but are not limited to, methylsulfinyl, ethylsulfinyl, *n*-propylsulfinyl, *iso*-propylsulfinyl, *n*-butylsulfinyl, *sec*-butylsulfinyl, *iso-*butylsulfmyl, *t*-butylsulfinyl and the like.

The term "**C₁-C₆ alkylsulfonamide**" is intended to mean the groups shown below: 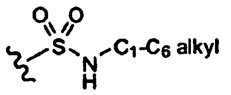 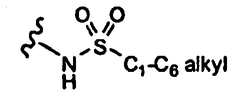 wherein C₁-C₆ alkyl has the same definition as described herein.

The term "**C₁-C₆ alkylsulfonyl**" is intended to mean a C₁-C₆ alkyl radical attached to the sulfur of a sulfone radical having the formula: -S(O)₂- wherein the alkyl radical has the same definition as described herein. Examples include, but are not limited to, methylsulfonyl, ethylsulfonyl, *n*-propylsulfonyl, *iso*-propylsulfonyl, *n*-butylsulfonyl, *sec*-butylsulfonyl, *iso-*butylsulfonyl, *t*-butylsulfonyl and the like.

The term "**C₁-C₆ alkylthio**" is intended to mean a C₁-C₆ alkyl radical attached to a sulfur atom (i.e., -S-) wherein the alkyl radical has the same definition as described herein. Examples include, but are not limited to, methylsulfanyl (i.e., CH₃S), ethylsulfanyl, *n-*propylsulfanyl, *iso*-propylsulfanyl, *n*-butylsulfanyl, *sec*-butylsulfanyl, *iso*-butylsulfanyl, *t-*butylsulfanyl, and the like.

The term "**C₁-C₆ alkylureyl**" is intended to mean the group of the formula: -NC(O)N-wherein one or both of the nitrogens are substituted with the same or different C₁-C₆ alkyl group wherein alkyl has the same definition as described herein. Examples of an alkylureyl include, but are not limited to, CH₃NHC(O)NH-, NH₂C(O)NCH₃-, (CH₃)₂NC(O)NH-, (CH₃)₂NC(O)NCH₃-, CH₃CH₂NHC(O)NH-, CH₃CH₂NHC(O)NCH₃- and the like.

The term "**amino**" is intended to mean the group -NH₂.

The term "**C₁-C₆ alkylamino**" is intended to mean one alkyl radical attached to a -NH-radical wherein the alkyl radical has the same meaning as described herein. Some examples include, but are not limited to, methylamino, ethylamino, *n*-propylamino, *iso*-propylamino, *n-*butylamino, *sec*-butylamino, *iso*-butylamino, *t*-butylamino and the like. Some embodiments are "**C₁-C₂alkylamino**."

The term "**aryl**" is intended to mean an aromatic ring radical containing 6 to 10 ring carbons. Examples include phenyl and naphthyl.

The term **"carbo-C₁-C₆-alkoxy**" is intended to mean a C1-C6 alkyl ester of a carboxylic acid, wherein the alkyl group is as defined herein. Examples include, but are not limited to, carbomethoxy [-C(=O)OCH3], carboethoxy, carbopropoxy, carboisopropoxy, carbobutoxy, carbo-sec-butoxy, carbo-iso-butoxy, carbo-t-butoxy, carbo-n-pentoxy, carbo-iso-pentoxy, carbo-t-pentoxy, carbo-neo-pentoxy, carbo-n-hexyloxy and the like.

The term "**C₂-C₆ alkynyl**" is intended to mean a radical containing at least one carbon-carbon triple bond and 2 to 6 carbons. Some examples include, but are not limited to, ethynyl, 1-propynyl, 2-propynyl, 1-butynyl, 2-butynyl, and the like.

The term "**carboxamide**" is intended to mean the group -CONH₂.

The term "**carboxy**" or "**carboxyl**" is intended to mean the group -CO₂H; also referred to as a carboxylic acid group.

The term "**cyano**" is intended to mean the group -CN.

The term "**C₃-C₇ cycloalkyl**" is intended to mean a saturated ring radical containing 3 to 7 carbons; some embodiments contain 3 to 6 carbons; some embodiments contain 3 to 5 carbons; some embodiments contain 5 to 7 carbons; some embodiments contain 3 to 4 carbons. Examples include cyclopropyl, cyclobutyl, cyclopentyl, cyclohexyl, cycloheptyl and the like.

The term "**C₃-C₇ cycloalkyloxy**" is intended to mean a saturated ring radical containing 3 to 7 carbons directly bonded to an oxygen atom. Some examples include cyclopropyl-O-, cyclobutyl-O-, cyclopentyl-O-, cyclohexyl-O-, and the like.

The term "**C₃-C₇ cycloalkylthio**" is intended to mean a saturated ring radical containing 3 to 7 carbons directly bonded to a sulfur atom. Some examples include cyclopropyl-S-, cyclobutyl-S-, cyclopentyl-S-, cyclohexyl-S-, and the like.

The term "**C₃-C₇ cycloalkylsulfinyl**" is intended to mean a saturated ring radical containing 3 to 7 carbons directly bonded to a sulfoxide group. Some examples include cyclopropyl-S(O)-, cyclobutyl-S(O)-, cyclopentyl-S(O)-, cyclohexyl-S(O)-, and the like.

The term "**C₃-C₇ cycloalkylsulfonyl**" is intended to mean a saturated ring radical containing 3 to 7 carbons directly bonded to a sulfoxide group. Some examples include cyclopropyl-S(O)₂-, cyclobutyl-S(O)₂-, cyclopentyl-S(O)₂-, cyclohexyl-S(O)₂-, and the like.

The term "**C₂-C₈ dialkylamino**" is intended to mean an amino substituted with two of the same or different C₁-C₄ alkyl radicals wherein alkyl radical has the same definition as described herein. Some examples include, but are not limited to, dimethylamino, methylethylamino, diethylamino, methylpropylamino, methylisopropylamino, ethylpropylamino, ethylisopropylamino, dipropylamino, propylisopropylamino and the like. Some embodiments are "**C₂-C₄ dialkylamino**."

The term "**C₂-C₆ dialkylcarboxamido**" or "**C₂-C₆ dialkylcarboxamide**" is intended to mean two alkyl radicals, that are the same or different, attached to an amide group, wherein alkyl has the same definition as described herein. A C₂-C₆ dialkylcarboxamido may be represented by the following groups: 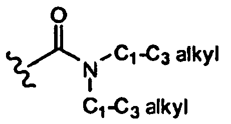 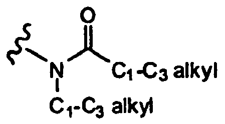 wherein C₁-C₃ has the same definition as described herein. Examples of a dialkylcarboxamide include, but are not limited to, *N*,*N*-dimethylcarboxamide, *N*-methyl-*N*-ethylcarboxamide, *N,N-*diethylcarboxamide, *N*-methyl-*N*-isopropylcarboxamide and the like.

The term "**C₁₋C₆ haloalkoxy**" is intended to mean a C₁-C₆ haloalkyl, as defined herein, which is directly attached to an oxygen atom. Examples include, but are not limited to, difluoromethoxy, trifluoromethoxy, 2,2,2-trifluoroethoxy, pentafluoroethoxy and the like.

The term "**C₁-C₆ haloalkyl**" is intended to mean an C₁-C₆ alkyl group, defined herein, wherein the alkyl is substituted with one halogen up to fully substituted and a fully substituted C₁-C₆ haloalkyl can be represented by the formula CₙL₂ₙ₊₁ wherein L is a halogen and "n" is 1, 2, 3, 4, 5 or 6; when more than one halogen is present then they may be the same or different and selected from the group consisting of F, Cl, Br and **I**, preferably **F**, some embodiments are 1 to 5 carbons, some embodiments are 1 to 4 carbons, some embodiments are 1 to 3 carbons and some embodiments are 1 or 2 carbons. Examples of haloalkyl groups include, but are not limited to, fluoromethyl, difluoromethyl, trifluoromethyl, chlorodifluoromethyl, 2,2,2-trifluoroethyl, pentafluoroethyl and the like.

The term "**C₁-C₆ haloalkylsulfinyl**" is intended to mean a C₁-C₆ haloalkyl radical attached to the sulfur atom of a sulfoxide group having the formula: -S(O)- wherein the haloalkyl radical has the same definition as described herein. Examples include, but are not limited to, trifluoromethylsulfinyl, 2,2,2-trifluoroethylsulfinyl, 2,2-difluoroethylsulfinyl and the like.

The term "**C₁-C₆ haloalkylsulfonyl**" is intended to mean a C₁-C₆ haloalkyl radical attached to the sulfur atom of a sulfone group having the formula: -S(O)₂- wherein haloalkyl has the same definition as described herein. Examples include, but are not limited to, trifluoromethylsulfonyl, 2,2,2-trifluoroethylsulfonyl, 2,2-difluoroethylsulfonyl and the like.

The term "**halogen**" or "**halo**" is intended to mean to a fluoro, chloro, bromo or iodo group.

The term "**heteroaryl**" is intended to mean an aromatic ring system containing 5 to 14 aromatic ring atoms that may be a single ring, two fused rings or three fused rings wherein at least one aromatic ring atom is a heteroatom selected from, for example, but not limited to, the group consisting of O, S and N wherein the N can be optionally substituted with H, C₁-C₄ acyl or C₁-C₄ alkyl. Some embodiments contain 5 to 6 ring atoms for example furanyl, thienyl, pyrrolyl, imidazolyl, oxazolyl, thiazolyl, isoxazolyl, pyrazolyl, isothiazolyl, oxadiazolyl, triazolyl, thiadiazolyl, pyridinyl, pyrazinyl, pyrimidinyl, pyridazinyl and triazinyl and the like. Some embodiments contain 8 to 14 ring atoms for example quinolizinyl, quinolinyl, isoquinolinyl, cinnolinyl, phthalazinyl, quinazolinyl, quinoxalinyl, triazinyl, indolyl, isoindolyl, indazolyl, indolizinyl, purinyl, naphthyridinyl, pteridinyl, carbazolyl, acridinyl. phenazinyl, phenothiazinyl, phenoxazinyl, benzoxazolyl, benzothiazolyl, 1*H*-benzimidazolyl, imidazopyridinyl, benzothienyl, benzofuranyl, and isobenzofuran and the like.

The term "heterocyclic" or "heterocyclyl" is intended to mean a non-aromatic carbon ring containing 3 to 8 ring atoms wherein one, two or three ring carbons are replaced by a heteroatom selected from, for example, the group consisting of O, S, S(=O), S(=O)₂ and NH, wherein the N is optionally substituted as described herein. In some embodiments, the nitrogen is optionally substituted with C₁-C₄ acyl or C₁-C₄ alkyl and ring carbon atoms are optionally substituted with oxo or a thiooxo thus forming a carbonyl or thiocarbonyl group. The heterocyclic group can be attached/bonded to any available ring atom, for example, ring carbon, ring nitrogen and the like. In some embodiments the heterocyclic group is a 3-, 4-, 5-, 6- or 7-membered ring. Examples of a heterocyclic group include, but are not limited to, aziridin-1-yl, aziridin-2-yl, azetidin-1-yl, azetidin-2-yl, azetidin-3-yl, piperidin-1-yl, piperidin-2-yl, piperidin-3-yl, piperidin4-yl, morpholin-2-yl, morpholin-3-yl, morpholin-4-yl, piperzin-1-yl, piperzin-2-yl, piperzin-3-yl, piperzin-4-yl, pyrrolidin-1-yl, pyrrolidin-2-yl, pyrrolidin-3-yl, [1,3]-dioxolan-2-yl, thiomorpholin-C₄-yl, [1,4]oxazepan-4-yl, 1,1-dioxothiomorpholin-4-yl, azepan-1-yl, azepan-2-yl, azepan-3-yl, azepan-4-yl, tetrahydrofuran-2-yl, tetrahydrofuran-3-yl and the like.

The term "**hydroxyl**" is intended to mean the group -OH.

The term "**nitro**" is intended to mean the group -NO₂.

The term "**phenyl**" is intended to mean the group -C₆H₅.

The term "**sulfonamide**" is intended to mean the group -SO₂NH₂.

### COMPOUNDS OF THE INVENTION:

One aspect of the present invention pertains to certain compounds of Formula (**Ia**) and pharmaceutically acceptable salts, solvates and hydrates thereof: 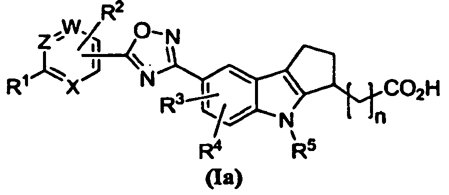 wherein:
n, R¹, R², R³, R⁴, R⁵, W, Z and X have the same definitions as described herein , *supra* and *infra.*

It is understood that the present invention embraces compounds, solvates and/or hydrates of compounds, pharmaceutically acceptable salts of compounds, and solvates and/or hydrates of pharmaceutically acceptable salts of compounds, wherein the compounds are as described herein.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination. All combinations of the embodiments pertaining to the chemical groups represented by the variables (*e.g*., n, **R¹, R², R³, R⁴, R⁵, W, Z** and **X**) contained within the generic chemical formulae described herein, for example, **(Ia), (Ic), (Ie), (Ig), (Ii), (Ik), (Im)**, **(Io), (Iq), (Ir), (It), (Iv), (Ix), (Iz), (IIa), (IIc), (Ile), (IIg), (IIi), (IIk), (IIm), (IIo), (IIq)** and **(IIIa)** are specifically embraced by the present invention just as if each and every combination was individually explicitly recited, to the extent that such combinations embrace compounds that result in stable compounds (*i.e*., compounds that can be isolated, characterized and tested for biological activity). In addition, all subcombinations of the chemical groups listed in the embodiments describing such variables, as well as all subcombinations of uses and medical indications described herein, are also specifically embraced by the present invention just as if each and every subcombination of chemical groups and subcombination of uses and medical indications was individually and explicitly recited herein.

As used herein, "substituted" indicates that at least one hydrogen atom of the chemical group is replaced by a non-hydrogen substituent or group, the non-hydrogen substituent or group can be monovalent or divalent. When the substituent or group is divalent, then it is understood that this group is further substituted with another substituent or group. When a chemical group herein is "substituted" it may have up to the full valance of substitution; for example, a methyl group can be substituted by 1, 2, or 3 substituents, a methylene group can be substituted by 1 or 2 substituents, a phenyl group can be substituted by 1, 2, 3, 4, or 5 substituents, a naphthyl group can be substituted by 1, 2, 3, 4, 5, 6, or 7 substituents and the like. Likewise, "substituted with one or more substituents" refers to the substitution of a group with one substituent up to the total number of substituents physically allowed by the group. Further, when a group is substituted with more than one group they can be identical or they can be different.

Compounds of the invention can also include tautomeric forms, such as keto-enol tautomers and the like. Tautomeric forms can be in equilibrium or sterically locked into one form by appropriate substitution. It is understood that the various tautomeric forms are within the scope of the compounds of the present invention.

Compounds of the invention can also include all isotopes of atoms occurring in the intermediates and/or final compounds. Isotopes include those atoms having the same atomic number but different mass numbers. For example, isotopes of hydrogen include deuterium and tritium.

It is understood and appreciated that compounds of Formula (Ia) and formulae related thereto may have one or more chiral centers and therefore can exist as enantiomers and/or diastereomers. The invention is understood to extend to and embrace all such enantiomers, diastereomers and mixtures thereof, including but not limited to racemates. It is understood that compounds of Formula (**Ia**) and formulae used throughout this disclosure are intended to represent all individual enantiomers and mixtures thereof, unless stated or shown otherwise.

### The Variable "n"

In some embodiments, n is 0.

In some embodiments, compounds of the present invention are represented by Formula (**Ic**) as illustrated below: 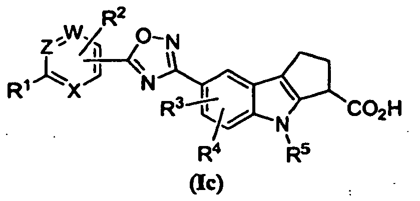 wherein each variable in Formula (Ic) has the same meaning as described herein, *supra* and *infra.*

In some embodiments, n is 1.

In some embodiments, compounds of the present invention are represented by Formula **(Ie)** as illustrated below: 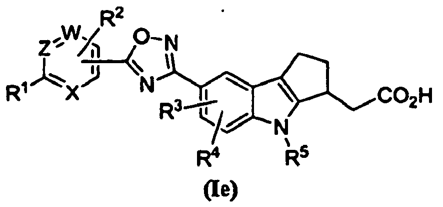 wherein each variable in Formula (**Ie**) has the same meaning as described herein, *supra* and *infra.*

### The Group R¹

In some embodiments, R¹ is selected from the group consisting of H, C₁-C₆ alkoxy, C₁-C₆ alkyl, C₁-C₆ alkylamino, C₂-C₈ dialkylamino, C₁-C₆ alkylsulfonyl, carboxamide, cyano, C₂-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents each selected independently from the group consisting of C₁-C₆ alkoxy, carbo-C₁-C₆-alkoxy, C₁-C₆ alkyl, cyano, halogen, C₃-C₇ cycloalkyl and phenyl.

In some embodiments, R¹ is selected from the group consisting of H, C₁-C₆ alkoxy, C₁-C₆ alkyl, C₁-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl, and hydroxyl, wherein C₁-C₆ alkoxy, C₁-C₆ alkyl, C₂-C₆ alkynyl, and heteroaryl are optionally substituted with 1 substituent selected from C₁-C₆ alkoxy, carbo-C₁-C₆-alkoxy, cyano, C₃-C₇ cycloalkyl, halogen, and phenyl.

In some embodiments, R¹ is selected from the group consisting of H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from the group consisting of C₁-C₆ alkoxy, carbo-C₁-C₆-alkoxy and C₃-C₇ cycloalkyl.

In some embodiments, R¹ is selected from the group consisting of H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl, and hydroxyl, wherein C₁-C₆ alkoxy and heteroaryl are optionally substituted with 1 substituent selected from C₁-C₆ alkoxy, carbo-C₁-C₆-alkoxy.

In some embodiments, R¹ is selected from the group consisting of H, C₁-C₆ alkoxy, C₁-C₆ alkyl, C₂-C₈ dialkylamino, carboxamide, cyano, C₃-C₇ cycloalkyloxy, C₁-C₆ haloalkoxy, C₁-C₆ haloalkyl and hydroxyl, wherein C₁-C₆ alkoxy is optionally substituted with C₃-C₇ cycloalkyl.

In some embodiments, R¹ is selected from the group consisting of H, *sec*-butoxy, isopropoxy, methoxy, ethoxy, methyl, ethyl, propyl, *tert*-butyl, diethylamino, methylsulfonyl, carboxamide, cyano, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, pyridin-3-yl, pyrrolidin-1-yl, morpholino, hydroxyl, cyclopropylmethoxy, benzyloxy, 6-methoxypyridin-3-yl, 1-(*tert*-butoxycarbonyl)-1*H*-pyrrol-2-yl, pyridin-4-yl, cyclopropylethynyl, 6-fluoropyridin-3-yl, 2-fluoropyridin4-yl, 2-fluoropyridin-3-yl, 2-methoxypyrimidin-5-yl, propylamino, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol4-yl, 2-methylpyridin-4-yl, 3-methylpyridin-4-yl, 1,3,5-trimethyl-1*H*-pyrazol-4-yl, 1-isobutyl-1*H*-pyrazol-4-yl, difluoromethoxy, ethynyl, cyclobutyl, cyclopropyl, propoxy, 2-cyanoethyl, 1,2,4-triazol-4-yl and pyrimidin-5-yl.

In some embodiments, R¹ is selected from the group consisting of H, *sec*-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(tert-butoxycarbonyl)-1*H*-pyrrol-2-yl, 2-fluoropyridin-4-yl, and 2-fluoropyridin-3-yl.

In some embodiments, R¹ is selected from the group consisting of H, *sec*-butoxy, isopropoxy, methoxy, ethoxy, methyl, ethyl, propyl, butyl, diethylamino, methylsulfonyl, carboxamide, cyano, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, pyridin-3-yl, pyrrolidin-1-yl, morpholino, hydroxyl, cyclopropylmethoxy; benzyloxy, 6-methoxypyridin-3-yl and 1-(*tert*-butoxycarbonyl)-1H-pyrrol-2-yl.

In some embodiments, R¹ is selected from the group consisting of H, *sec*-butoxy, isopropoxy, methoxy, ethoxy, butyl, diethylamino, carboxamide, cyano, cyclopentyloxy, trifluoromethoxy, 1,1,1,3,3,3-hexafluoropropan-2-yloxy, 2,2,2-trifluoroethoxy, trifluoromethyl, chloro, hydroxyl and cyclopropylmethoxy.

### The Group R²

In some embodiments, R² is H.

### The Groups R³, R⁴ and R⁵

In some embodiments, **R³** and **R⁴** are each independently selected from the group consisting of H, CH₃ and F.

It is understood that R³ and R⁴ can be bonded to any of the three available positions on the phenyl ring of the 1,2,3,4-tetrahydrocyclo-penta[b]indole ring system, specifically these positions are C(5), C(6) and C(8) as illustrated in the formula below:

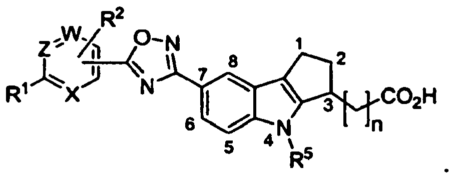

In some embodiments, R³ is H.

In some embodiments, R⁴ is H.

In some embodiments, R³ and R⁴ are both H.

In some embodiments, compounds of the present invention are represented by Formula **(Ig)** as illustrated below: 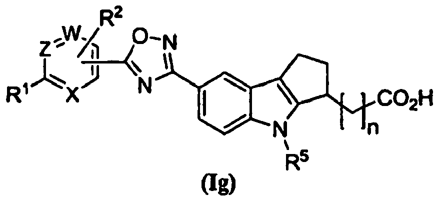 wherein each variable in Formula **(Ig)** has the same meaning as described herein, *supra* and *infra.*

In some embodiments, R⁵ is H or methyl.

In some embodiments, R⁵ is H.

In some embodiments, compounds of the present invention are represented by Formula **(Ii)** as illustrated below: 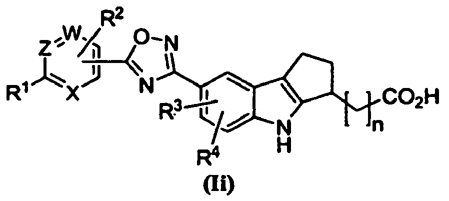 wherein each variable in Formula **(Ii)** has the same meaning as described herein, *supra* and *infra.*

In some embodiments, R³, R⁴ and R⁵ are each H.

In some embodiments, compounds of the present invention are represented by Formula **(Ik)** as illustrated below: 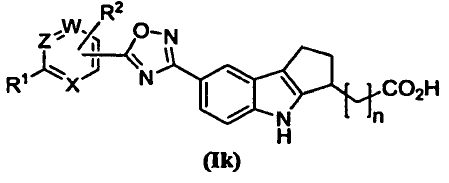 wherein each variable in Formula **(Ik)** has the same meaning as described herein, *supra* and *infra.*

### The Group R⁶

In some embodiments, R⁶ is selected from the group consisting of H, C₁-C₆ alkoxy, C₁-C₆ alkyl, C₁-C₆ alkylsulfonyl, carboxamide, cyano, C₂-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents each selected independently from the group consisting of C₁-C₆ alkoxy, carbo-C₁-C₆-alkoxy, C₁-C₆ alkyl, cyano, halogen, C₃-C₇ cycloalkyl, and phenyl.

In some embodiments, R⁶ is selected from the group consisting of H, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl, and hydroxyl, wherein C₁-C₆ alkoxy, C₁-C₆ aryl, C₂-C₆ alkynyl, and heteroaryl are optionally substituted with 1 substituent selected from C₁-C₆ alkoxy, carbo-C₁-C₆-alkoxy, cyano, C₃-C₇ cycloalkyl, halogen, and phenyl.

In some embodiments, R⁶ is selected from the group consisting of H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from the group consisting of C₁-C₆ alkoxy, carbo-C₁-C₆-alkoxy, C₃-C₇ cycloalkyl and phenyl.

In some embodiments, R⁶ is selected from the group consisting of H, isopropoxy, methoxy, ethoxy, methyl, ethyl, propyl, tert-butyl, methylsulfonyl, carboxamide, cyano, cyclopentyloxy, trifluoromethoxy, 2-fluoroethoxy, 2,2,2-trifluoroethoxy, trifluoromethyl, fluoro, chloro, bromo, 1,2,4-triazol-1-yl, pyridin-3-y1, pyrrolidin-1-yl, morpholino, hydroxyl, cyclopropylmethoxy, benzyloxy, 6-methoxypyridin-3-yl, 1-(tert-butoxycarbonyl)-1H-pyrrol-2-yl, pyridin-4-yl, pyrimidin-5-yl, cyclopropylethynyl, 6-fluoropyridin-3-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl, 1-isobutyl-1*H-*pyrazol-4-yl, ethynyl, cyclobutyl, cyclopropyl, propoxy, 2-cyanoethyl and 1,2,4-triazol-4-yl.

In some embodiments, **R⁶** is selected from the group consisting of H, sec-butoxy, isopropoxy, methoxy, ethoxy, propoxy, methyl, ethyl, propyl, butyl, tert-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(tert-butoxycarbonyl)-1*H*-pyrrol-2-yl, 2-fluoropyridin-4-yl, and 2-fluoropyridin-3-yl.

In some embodiments, **R⁶** is selected from the group consisting of H, *sec*-butoxy, isopropoxy, methoxy, ethoxy, methyl, ethyl, propyl, butyl, diethylamino, methylsulfonyl, carboxamide, cyano, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, pyridin-3-yl, pyrrolidin-1-yl, morpholino, hydroxyl, cyclopropylmethoxy, benzyloxy, 6-methoxypyridin-3-yl and 1-(*tert*-butoxycarbonyl)-1*H*-pyrrol-2-yl.

In some embodiments, R⁶ is H.

### The Group R⁷

In some embodiments, R⁷ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents each selected independently from C₁-C₆ alkoxy, C₁-C₆ alkyl, cyano, carbo-C₁-C₆-alkoxy, C₃-C₇ cycloalkyl, halogen and phenyl.

In some embodiments, R¹ is selected from the group consisting of H, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl, and hydroxyl, wherein C₁-C₆ alkoxy, C₁-C₆ alkyl, C₂-C₆ alkynyl, and heteroaryl are optionally substituted with 1 substituent selected from C₁-C₆ alkoxy, carbo-C₁-C₆-alkoxy, cyano, C₃-C₇ cycloalkyl, halogen, and phenyl.

In some embodiments, R⁷ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

In some embodiments, R⁷ is selected from the group consisting of H, C₁-C₆ alkoxy, C₁-C₆ alkyl, C₁-C₆ alkylsulfonyl, cyano, C₃-C₇ cycloalkyloxy, C₁-C₆ haloalkoxy, C₁-C₆ haloalkyl, halogen, heteroaryl, and heterocyclyl, wherein C₁-C₆ alkoxy and heteroaryl are optionally substituted with 1 substituent selected from C₁-C₆ alkoxy, carbo-C₁-C₆-alkoxy and phenyl.

In some embodiments, R⁷ is selected from the group consisting ofH, isopropoxy, methoxy, ethoxy, propoxy, methyl, ethyl, propyl, butyl, tert-butyl, ethynyl, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1H-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl, 1-isobutyl-1*H*-pyrazol-4-yl, hexyl and difluoromethoxy.

In some embodiments, **R⁷** is selected from the group consisting of H, *sec*-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(tert-butoxycarbonyl)-1*H*-pyrrol-2-yl, 2-fluoropyridin-4-yl, and 2-fluoropyridin-3-yl.

In some embodiments, **R⁷** is selected from the group consisting of H, *sec*-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl and 2-fluoropyridin-3-yl.

In some embodiments, **R⁷** is selected from the group consisting of H, isopropoxy, methoxy, ethoxy, propoxy, methyl, ethyl, propyl, butyl, *tert-*butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl and 2-fluoropyridin-3-yl.

In some embodiments, **R⁷** is selected from the group consisting of **H**, isopropoxy, methoxy, ethoxy, methyl, ethyl, propyl, methylsulfonyl, cyano, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyrimidin-5-yl, pyrrolidin-1-yl, morpholino, benzyloxy, 6-methoxypyridin-3-yl and 1-(*tert*-butoxycarbonyl)-1*H*-pyrrol-2-yl.

### The Group R⁸

In some embodiments, wherein **R⁸** is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents each selected independently from C₁-C₆ alkoxy, C₁-C₆ alkyl, carbo-C₁-C₆-alkoxy, cyano, C₃-C₇ cycloalkyl, halogen and phenyl.

In some embodiments, **R⁸** is selected from the group consisting of **H**, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl, and hydroxyl, wherein C₁-C₆ alkoxy, C₁-C₆ alkyl, C₂-C₆ alkynyl, and heteroaryl are optionally substituted with 1 substituent selected from C₁-C₆ alkoxy, carbo-C₁-C₆-alkoxy, cyano, C₃-C₇ cycloalkyl, halogen, and phenyl.

In some embodiments, **R⁸** is selected from the group consisting of **H**, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

In some embodiments, **R⁸** is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₁-C₆ haloalkoxy, C₁-C₆ haloalkyl, halogen, heteroaryl and hydroxyl, wherein C₁-C₆ alkoxy, C₁-C₆ alkyl, C₂-C₆ alkynyl and heteroaryl are optionally substituted with 1 substituent selected from cyano, C₃-C₇ cycloalkyl, halogen and phenyl.

In some embodiments, **R⁸** is selected from the group consisting of **H**, isopropoxy, methoxy, ethoxy, propoxy, methyl, propyl, *tert*-butyl, ethynyl, methylsulfonyl, carboxamide, cyano, cyclobutyl, cyclopropyl, cyclopentyloxy, trifluoromethoxy, 2-fluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(tert-butoxycarbonyl)-1*H*-pyrrol-2-yl, 2-fluoropyridin-4-yl 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-yl.

In some embodiments, **R⁸** is selected from the group consisting of **H,** *sec*-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(tert-butoxycarbonyl)-1H-pyrrol-2-yl, 2-fluoropyridin-4-yl, and 2-fluoropyridin-3-yl.

In some embodiments, **R⁸** is selected from the group consisting of **H**, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1H-pyrrol-2-yl, 2-fluoropyridin-4-yl and 2-fluoropyridin-3-yl.

In some embodiments, R⁸ is selected from the group consisting of H, isopropoxy, methoxy, ethoxy, propoxy, methyl, tert-butyl, ethynyl, methylsulfonyl, carboxamide, cyano, cyclobutyl, cyclopropyl, trifluoromethoxy, trifluoromethyl, fluoro, chloro, bromo, pyridin-4-yl, hydroxyl, cyclopropylmethoxy, benzyloxy, 2-cyanoethyl, cyclopropylethynyl, 6-fluoropyridin-3-yl, 2-fluoropyridin-4-yl and 2-fluoropyridin-3-yl.

### Certain Combinations for Groups R¹, R², R⁶, R⁷ and R⁸

In some embodiments, R¹, R², R⁶, R⁷ and R⁸ are each independently selected from the group consisting of H, C₁-C₆ acyl, C₁-C₆ acyloxy, C₁-C₆ alkoxy, C₁-C₆ alkoxycarbonylamino, C₁-C₆ alkyl, C₂-C₆ alkynyl, C₁-C₆ alkylamino, C₂-C₈ dialkylamino, C₁-C₆ alkylcarboxamide, C₁-C₆ alkylsulfonamide, C₁-C₆ alkylsulfinyl, C₁-C₆ alkylsulfonyl, C₁-C₆ alkylthio, C₁-C₆ alkylureyl, amino, carbo-C₁-C₆-alkoxy, carboxamide, carboxy, cyano, C₃-C₇ cycloalkyl, C₃-C₇ cycloalkyloxy, C₃-C₇ cycloalkylthio, C₃-C₇ cycloalkylsulfinyl, C₃-C₇ cycloalkylsulfonyl, C₂-C₆ dialkylcarboxamide, C₁-C₆ haloalkoxy, C₁-C₆ haloalkyl, C₁-C₆ haloalkylsulfinyl, C₁-C₆ haloalkylsulfonyl, halogen, heteroaryl, heterocyclyl, hydroxyl, nitro and sulfonamide, wherein C₁-C₆ alkoxy, C₁-C₆ alkylthio, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

In some embodiments, R¹, R², R⁶, R⁷ and R⁸ are each independently selected from the group consisting of H, C₁-C₆ alkoxy, C₁-C₆ alkyl, C₂-C₆ alkynyl, C₁-C₆ alkylamino, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, carbo-C₁-C₆-alkoxy, C₁-C₆ alkyl, cyano, C₃-C₇ cycloalkyl, halogen and phenyl.

In some embodiments, R¹, R², R⁶, R⁷ and R⁸ are each independently selected from the group consisting of H, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

In some embodiments, R¹, R², R⁶, R⁷ and R⁸ are each independently selected from the group consisting of H, sec-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl, 2-fluoropyridin-3-yl, 2-methoxypyrimidin-5-yl, propylamino, 3-fluoropyridin-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl, 1-isobutyl-1*H*-pyrazol-4-yl, difluoromethoxy and hexyl.

In some embodiments, R¹, R², R⁶, R⁷ and R⁸ are each independently selected from the group consisting of H, *sec*-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1H-pyrrol-2-yl, 2-fluoropyridin-4-yl and 2-fluoropyridin-3-yl.

In some embodiments,
R¹ is selected from the group consisting of H, C₁-C₆ alkoxy, C₁-C₆ alkyl, C₁-C₆ alkylamino, C₂-C₈ dialkylamino, C₁-C₆ alkylsulfonyl, carboxamide, cyano, C₂-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from the group consisting of C₁-C₆ alkoxy, carbo-C₁-C₆-alkoxy, C₁-C₆ alkyl, cyano, halogen, C₃-C₇ cycloalkyl and phenyl;
R² is H;
R⁶ is selected from the group consisting H, C₁-C₆ alkoxy, C₁-C₆ alkyl, C₁-C₆ alkylsulfonyl, carboxamide, cyano, C₁-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from the group consisting of C₁-C₆ alkoxy, carbo-C₁-C₆-alkoxy, C₁-C₆ alkyl, cyano, halogen, C₃-C₇ cycloalkyl, and phenyl;
R⁷ is selected from the group consisting of H, C₁-C₆ alkoxy, C₁-C₆ alkyl, C₁-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl; and
R⁸ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, carbo-C₁-C₆-alkoxy, cyano, C₃-C₇ cycloalkyl, halogen and phenyl.

In some embodiments,
R¹ is selected from the group consisting of H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from the group consisting of C₁-C₆ alkoxy, carbo-C₁-C₆-alkoxy and C₃-C₇ cycloalkyl;
R² is H;
R⁶ is selected from the group consisting of H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from the group consisting of C₁-C₆ alkoxy, carbo-C₁-C₆-alkoxy, C₃-C₇ cycloalkyl and phenyl;
R⁷ is selected from the group consisting of H, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, cyano, carbo-C₁-C₆-alkoxy, C₃-C₇ cycloalkyl, halogen and phenyl; and
R⁸ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₁-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

In some embodiments,
R¹ is selected from the group consisting of H, *sec*-butoxy, isopropoxy, methoxy, ethoxy, methyl, ethyl, propyl, *tert*-butyl, diethylamino, methylsulfonyl, carboxamide, cyano, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, pyridin-3-yl, pyrrolidin-1-y1, morpholino, hydroxyl, cyclopropylmethoxy, benzyloxy, 6-methoxypyridin-3-yl, 1-(tert-butoxycarbonyl)-1*H*-pyrrol-2-yl, pyridin-4-yl, cyclopropylethynyl, 6-fluoropyridin-3-yl, 2-fluoropyridin-4-yl, 2-fluoropyridin-3-yl, 2-methoxypyrimidin-5-yl, propylamino, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol4-yl, 2-methylpyridin4-yl, 3-methylpyridin-4-yl, 1,3,5-trimethyl-1*H*-pyrazol-4-yl, 1-isobutyl-1*H*-pyrazol-4-yl, difluoromethoxy, ethynyl, cyclobutyl, cyclopropyl, propoxy, 2-cyanoethyl, 1,2,4-triazol-4-yl and pyrimidin-5-yl;
R² is H;
R⁶ is selected from the group consisting H, isopropoxy, methoxy, ethoxy, methyl, ethyl, propyl, *tert*-butyl, methylsulfonyl, carboxamide, cyano, cyclopentyloxy, trifluoromethoxy, 2-fluoroethoxy, 2,2,2-trifluoroethoxy, trifluoromethyl, fluoro, chloro, bromo, 1,2,4-triazol-1-yl, pyridin-3-yl, pyrrolidin-1-yl, morpholino, hydroxyl, cyclopropylmethoxy, benzyloxy, 6-methoxypyridin-3-yl, 1-(*tert*-butoxycarbonyl)-1H-pyrrol-2-yl, pyridin-4-yl, pyrimidin-5-yl, cyclopropylethynyl, 6-fluoropyridin-3-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin4-yl, 1,3,5-trimethyl-1*H*-pyrazol-4-yl, 1-isobutyl-1*H*-pyrazol-4-yl, ethynyl, cyclobutyl, cyclopropyl, propoxy, 2-cyanoethyl and 1,2,4-triazol-4-yl;
R⁷ is selected from the group consisting of H, isopropoxy, methoxy, ethoxy, propoxy, methyl, ethyl, propyl, butyl, *tert*-butyl, ethynyl, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H* pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H-*pyrazol-4-yl, 2-methylpyridin-4-yl, 3-methylpyridin-4-yl, 1,3,5-trimethyl-1*H*-pyrazol-4-yl, 1-isobutyl-1*H*-pyrazol-4-yl, hexyl and difluoromethoxy; and
R⁸ is selected from the group consisting of H, isopropoxy, methoxy, ethoxy, propoxy, methyl, propyl, *tert*-butyl, ethynyl, methylsulfonyl, carboxamide, cyano, cyclobutyl, cyclopropyl, cyclopentyloxy, trifluoromethoxy, 2-fluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1H-pyrrol-2-yl, 2-fluoropyridin-4-yl 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-4-yl.

In some embodiments,
R¹ is selected from the group consisting of H, *sec*-butoxy, isopropoxy, methoxy, ethoxy, methyl, ethyl, propyl, butyl, diethylamino, methylsulfonyl, carboxamide, cyano, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, pyridin-3-yl, pyrrolidin-1-yl, morpholino, hydroxyl, cyclopropylmethoxy, benzyloxy, 6-methoxypyridin-3-yl and 1-(*tert-*butoxycarbonyl)-1H-pyrrol-2-yl;
R² is H;
R⁶ is H;
R⁷ is selected from the group consisting of H, isopropoxy, methoxy, ethoxy, propoxy, methyl, ethyl, propyl, butyl, *tert*-butyl, ethynyl, methylsulfonyl, carboxamide, cyano, cyclobutyl, cyclopropyl, cyclopentyloxy, trifluoromethoxy; 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-*(tert*-butoxycarbonyl)-1H-pyrrol-2-yl, 2-fluoropyridin-4-yl and 2-fluoropyridin-3-yl; and
R⁸ is selected from the group consisting of H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1H-pyrrol-2-yl, 2-fluoropyridin-4-yl and 2-fluoropyridin-3-yl.

In some embodiments,
R¹ is selected from the group consisting of H, *sec*-butoxy, isopropoxy, methoxy, ethoxy, butyl, diethylamino, carboxamide, cyano, cyclopentyloxy, trifluoromethoxy, 1,1,1,3,3,3-hexafluoropropan-2-yloxy, 2,2,2-trifluoroethoxy, trifluoromethyl, chloro, hydroxyl and cyclopropylmethoxy;
R² is H;
R⁶ is H;
R⁷ is selected from the group consisting of H, isopropoxy, methoxy, ethoxy, methyl, ethyl, propyl, methylsulfonyl, cyano, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyrimidin-5-yl, pyrrolidin-1-yl, morpholino, benzyloxy, 6-methoxypyridin-3-yl and 1-(*tert*-butoxycarbonyl)-1H-pyrrol-2-yl; and
R⁸ is selected from the group consisting of H, isopropoxy, methoxy, ethoxy, propoxy, methyl, *tert*-butyl, ethynyl, methylsulfonyl, carboxamide, cyano, cyclobutyl, cyclopropyl, trifluoromethoxy, trifluoromethyl, fluoro, chloro, bromo, pyridin-4-yl, hydroxyl, cyclopropylmethoxy, benzyloxy, 2-cyanoethyl, cyclopropylethynyl, 6-fluoropyridin-3-yl, 2-fluoropyridin-4-yl and 2-fluoropyridin-3-yl.

### The Variables W, Z and X

In some embodiments, W is CR⁶; Z is CR⁷; and X is CR⁸.

In some embodiments, compounds of the present invention are represented by Formula (**Im**) as illustrated below: 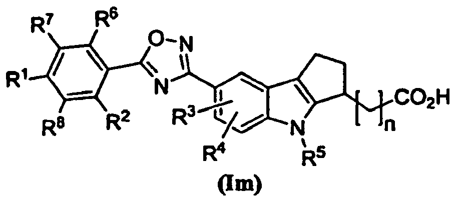 wherein each variable in Formula (**Im**) has the same meaning as described herein, *supra* and *infra.*

In some embodiments, W is CR⁶; Z is CR⁷; and X is N.

In some embodiments, compounds of the present invention are represented by Formula (**Io**) as illustrated below: 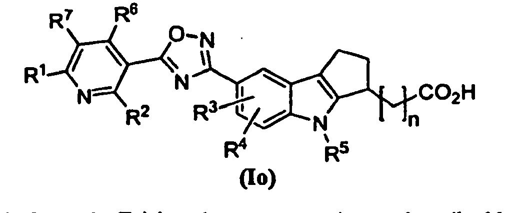 wherein each variable in Formula (**Io**) has the same meaning as described herein, *supra* and *infra.*

In some embodiments, compounds of the present invention are represented by Formula (**Iq**) as illustrated below: 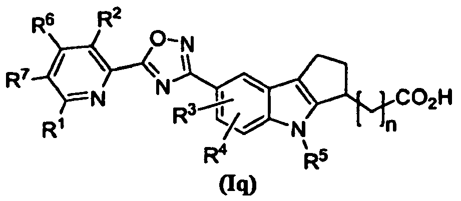 wherein each variable in Formula (**Iq**) has the same meaning as described herein, *supra* and *infra.*

In some embodiments, W is N; Z is N; and X is CR⁸.

In some embodiments, compounds of the present invention are represented by Formula (**Ir**) as illustrated below: 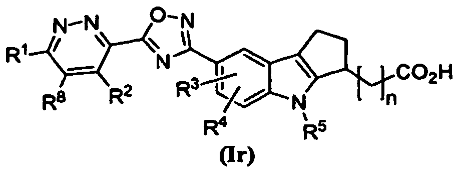 wherein each variable in Formula (**Ir**) has the same meaning as described herein, *supra* and
*infra.*

In some embodiments, compounds of the present invention are represented by Formula (**It**) as illustrated below: 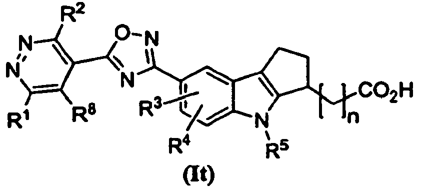 wherein each variable in Formula (**It**) has the same meaning as described herein, *supra* and *infra.*

In some embodiments, W is CR⁶; Z is N; and X is N.

In some embodiments, compounds of the present invention are represented by Formula (**Iv**) as illustrated below: 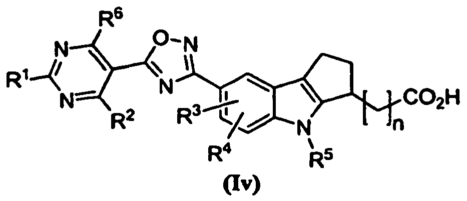 wherein each variable in Formula (**Iv**) has the same meaning as described herein, *supra* and *infra.*

In some embodiments, W is N; Z is CR⁷; and X is N.

In some embodiments, compounds of the present invention are represented by Formula (**Ix**) as illustrated below: 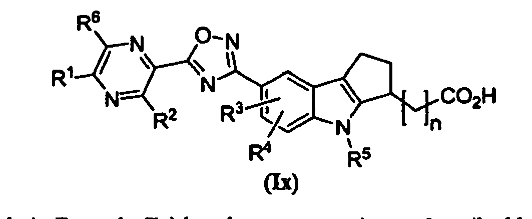 wherein each variable in Formula (**Ix**) has the same meaning as described herein, *supra* and *infra.*

In some embodiments, W is CR⁶; Z is N; and X is CR⁸.

In some embodiments, compounds of the present invention are represented by Formula (**Iz**) as illustrated below: 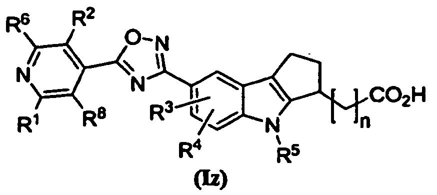 wherein each variable in Formula (**Iz**) has the same meaning as described herein, *supra* and *infra.*

### Certain Combinations

Some embodiments of the present invention pertain to compounds of Formula (**Ig**) and pharmaceutically acceptable salts, solvates and hydrates thereof: 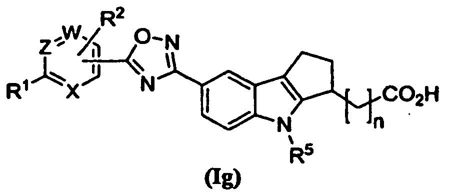
n is 0 or 1;
W is N or CR⁶;
Z is N or CR⁷;
X is N or CR⁸; provided that W, Z and X are not all N;
R¹ is selected from the group consisting of H, C₁-C₆ alkoxy, C₁-C₆ alkyl, C₁-C₆ alkylamino, C₂-C₈ dialkylamino, C₁-C₆ alkylsulfonyl, carboxamide, cyano, C₁-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from the group consisting of C₁-C₆ alkoxy, carbo-C₁-C₆-alkoxy, C₁-C₆ alkyl, cyano, halogen, C₁-C₇ cycloalkyl and phenyl;
R² is H;
R⁵ is H or C₁-C₆ alkyl;
R⁶ is selected from the group consisting of H, C₁-C₆ alkoxy, C₁-C₆ alkyl, C₁-C₆ alkylsulfonyl, carboxamide, cyano, C₁-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from the group consisting of C₁-C₆ alkoxy, carbo-C₁-C₆-alkoxy, C₁-C₆ alkyl, cyano, halogen, C₃-C₇ cycloalkyl, and phenyl;
R⁷ is selected from the group consisting of H, C₁-C₆ alkoxy, C₁-C₆ alkyl, C₁-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl; and
R⁸ is selected from the group consisting of H, C₁-C₆ alkoxy, C₁-C₆ alkyl, C₁-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, carbo-C₁-C₆-alkoxy, cyano, C₃-C₇ cycloalkyl, halogen and phenyl.

Some embodiments of the present invention pertain to compounds of Formula (**Ig**) and pharmaceutically acceptable salts, solvates and hydrates thereof: 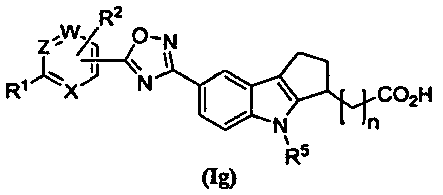
n is 0 or 1;
W is N or CR⁶;
Z is N or CR⁷;
X is N or CR⁸; provided that W, Z and X are not all N;
R¹ is selected from the group consisting of H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from the group consisting of C₁-C₆ alkoxy, carbo-C₁-C₆-alkoxy and C₃-C₇ cycloalkyl;
R² is H;
R⁵ is H or C₁-C₆ alkyl;
R⁶ is selected from the group consisting of H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from the group consisting of C₁-C₆ alkoxy, carbo-C₁-C₆-alkoxy, C₃-C₇ cycloalkyl and phenyl;
R⁷ is selected from the group consisting of H, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, cyano, carbo-C₁-C₆-alkoxy, C₃-C₇ cycloalkyl, halogen and phenyl; and
R⁸ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

Some embodiments of the present invention pertain to compounds of Formula **(IIa)** and pharmaceutically acceptable salts, solvates and hydrates thereof: 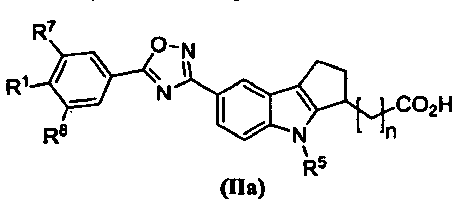
n is 0 or 1;
R¹ is selected from the group consisting of H, C₁-C₄ alkoxy, cyano, C₃-C₇ cycloalkyloxy, C₁-C₆ haloalkoxy, halogen and hydroxyl, wherein C₁-C₆ alkoxy, is optionally substituted with 1, 2 or 3 substituents selected from halogen and C₃-C₇ cycloalkyl;
R⁵ is H or C₁-C₆ alkyl;
R⁷ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl; and
R⁸ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl.

Some embodiments of the present invention pertain to compounds of Formula **(IIa)** and pharmaceutically acceptable salts, solvates and hydrates thereof: 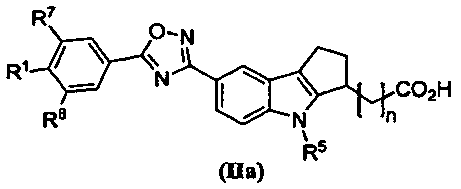
n is 0 or 1;
R¹ is selected from the group consisting of H, C₁-C₆ alkoxy, cyano, C₃-C₇ cycloalkyloxy, C₁-C₆ haloalkoxy and hydroxyl, wherein C₁-C₆ alkoxy, is optionally substituted with 1 substituent selected from C₁-C₆ alkoxy;
R⁵ is H or C₁-C₆ alkyl;
R⁷ is selected from the group consisting of H, C₁-C₆ alkoxy, C₁-C₆ alkyl, C₁-C₆ alkylsulfonyl, cyano, C₃-C₇ cycloalkyloxy, C₁-C₆ haloalkoxy, C₁-C₆ haloalkyl, halogen, heteroaryl, heterocyclyl, wherein C₁-C₆ alkoxy and heteroaryl are optionally substituted with 1 substituent selected from C₁-C₆ alkoxy, carbo-C₁-C₆-alkoxy and phenyl; and
R⁸ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₁-C₆ haloalkoxy, C₁-C₆ haloalkyl, halogen, heteroaryl and hydroxyl, wherein C₁-C₆ alkoxy, C₁-C₆ alkyl, C₂-C₆ alkynyl and heteroaryl are optionally substituted with 1 substituent selected from cyano, C₃-C₇ cycloalkyl, halogen and phenyl.

Some embodiments of the present invention pertain to compounds of Formula (**IIa**) and pharmaceutically acceptable salts, solvates and hydrates thereof: 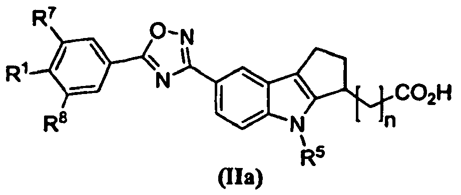
n is 0 or 1;
R¹ is selected from the group consisting of H, isopropoxy, methoxy, ethoxy, cyano, cyclopentyloxy, trifluoromethoxy, 1,1,1,3,3,3-hexafluoropropan-2-yloxy, fluoro, hydroxyl, cyclopropylmethoxy, and difluoromethoxy;
R⁵ is H or methyl;
R⁷ is selected from the group consisting of H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(tert-butoxycarbonyl)-1*H*-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-yl and 1-isobutyl-1*H*-pyrazol-yl; and
R⁸ is selected from the group consisting of H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1H-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-4-yl.

Some embodiments of the present invention pertain to compounds of Formula (**IIa**) and pharmaceutically acceptable salts, solvates and hydrates thereof: 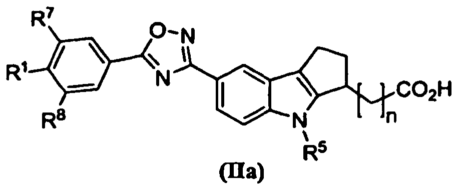
n is 0 or 1;
R¹ is selected from the group consisting of H, isopropoxy, methoxy, ethoxy, cyano; cyclopentyloxy, trifluoromethoxy, 1,1,1,3,3,3-hexafluoropropan-2-yloxy, hydroxyl and cyclopropylmethoxy;
R⁵ is H or methyl;
R⁷ is selected from the group consisting of H, isopropoxy, methoxy,ethoxy, methyl, ethyl, propyl, methylsulfonyl, cyano, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyrimidin-5-yl, pyrrolidin-1-yl, morpholino, benzyloxy, 6-methoxypyridin-3-yl and 1-(*tert*-butoxycarbonyl)-1H-pyrrol-2-yl; and
R⁸ is selected from the group consisting of H, isopropoxy, methoxy, ethoxy, propoxy, methyl, *tert*-butyl, ethynyl, methylsulfonyl, carboxamide, cyano, cyclobutyl, cyclopropyl, trifluoromethoxy, trifluoromethyl, fluoro, chloro, bromo, pyridin-4-yl, hydroxyl, cyclopropylmethoxy, benzyloxy, 2-cyanoethyl, cyclopropylethynyl, 6-fluoropyridin-3-yl, 2-fluoropyridin-4-yl and 2-fluoropyridin-3-yl.

Some embodiments of the present invention pertain to compounds of Formula **(IIc)** and pharmaceutically acceptable salts, solvates and hydrates thereof: 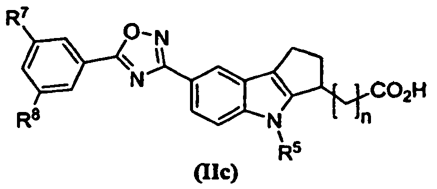
n is 0 or 1;
R⁵ is H or methyl;
R⁷ is selected from the group consisting of H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-4-yl; and
R⁸ is selected from the group consisting of H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-4-yl.

Some embodiments of the present invention pertain to compounds of Formula **(IIc)** and pharmaceutically acceptable salts, solvates and hydrates thereof: 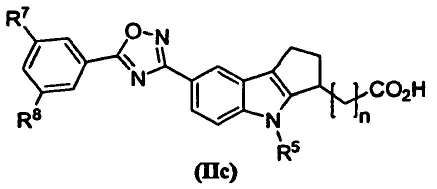
n is 0 or 1;
R⁵ is H or C₁-C₆ alkyl;
R⁷ is selected from the group consisting of H, C₁-C₆ alkoxy, C₁-C₆ alkyl, C₁-C₆ alkylsulfonyl, cyano, C₃-C₇ cycloalkyloxy, C₁-C₆ haloalkoxy, C₁-C₆ haloalkyl, halogen, heteroaryl, heterocyclyl, wherein C₁-C₆ alkoxy and heteroaryl are optionally substituted with 1 substituent selected from C₁-C₆ alkoxy, carbo-C₁-C₆-alkoxy and phenyl; and
R⁸ is selected from the group consisting of H, C₁-C₆ alkoxy, C₁-C₆ alkyl, C₂-C₆ alkynyl, C₁-C₆ alkylsulfonyl, carboxamide, cyano, C₁-C₇ cycloalkyl, C₁-C₆ haloalkoxy, C₁-C₆ haloalkyl, halogen, heteroaryl and hydroxyl, wherein C₁-C₆ alkoxy, C₁-C₆ alkyl, C₂-C₆ alkynyl and heteroaryl are optionally substituted with 1 substituent selected from cyano, C₁-C₇ cycloalkyl, halogen and phenyl.

Some embodiments of the present invention pertain to compounds of Formula **(IIe)** and pharmaceutically acceptable salts, solvates and hydrates thereof: 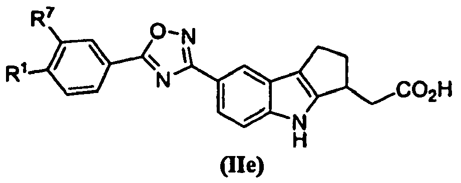
R¹ is selected from the group consisting ofH, C₁-C₆ alkoxy, cyano, C₃-C₇ cycloalkyloxy, C₁-C₆ haloalkoxy and hydroxyl, wherein C₁-C₆ alkoxy is optionally substituted with 1, 2 or 3 substituents selected from halogen or C₃-C₇ cycloalkyl; and
R⁷ is selected from the group consisting of H, C₁-C₆ alkoxy, C₁-C₆ alkyl, C₁-C₆ alkylsulfonyl, cyano, trifluoromethoxy, trifluoromethyl halogen and hydroxyl, wherein C₁-C₆ alkoxy is optionally substituted with one phenyl group.

Some embodiments of the present invention pertain to compounds of Formula **(IIe)** and pharmaceutically acceptable salts, solvates and hydrates thereof: 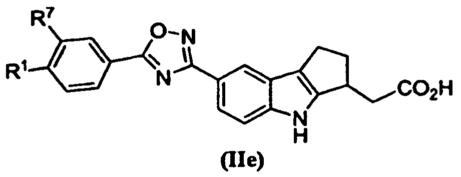
R¹ is selected from the group consisting of C₁-C₆ alkoxy, cyano, C₃-C₇ cycloalkyloxy, C₁-C₆ haloalkoxy and hydroxyl, wherein C₁-C₆ alkoxy, is optionally substituted with 1 substituent selected from C₃-C₇ cycloalkyl; and
R⁷ is selected from the group consisting of C₁-C₆ alkoxy, C₁-C₆ alkyl, C₁-C₆ alkylsulfonyl, cyano, C₁-C₆ haloalkoxy, C₁-C₆ haloalkyl and halogen.

Some embodiments of the present invention pertain to compounds of Formula **(IIe)** and pharmaceutically acceptable salts, solvates and hydrates thereof: 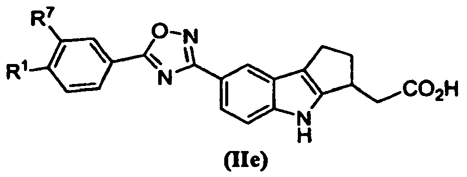
R¹ is selected from the group consisting of H, isopropoxy, methoxy, ethoxy, cyano, cyclopentyloxy, trifluoromethoxy, 1,1,1,3,3,3-hexafluoropropan-2-yloxy, hydroxyl, cyclopropylmethoxy and difluoromethoxy; and
R⁷ is selected from the group consisting of H, methoxy, ethoxy, methyl, methylsulfonyl, cyano, trifluoromethoxy, trifluoromethyl, fluoro, chloro, hydroxyl and benzyloxy.

Some embodiments of the present invention pertain to compounds of Formula **(IIe)** and pharmaceutically acceptable salts, solvates and hydrates thereof: 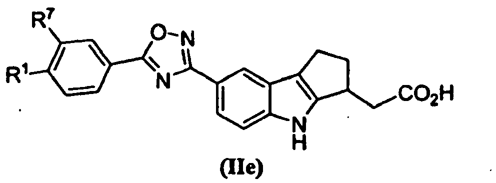
R¹ is selected from the group consisting of isopropoxy, methoxy, ethoxy, cyano, cyclopentyloxy, 1,1,1,3,3,3-hexafluoropropan-2-yloxy, hydroxyl and cyclopropylmethoxy; and
R⁷ is selected from the group consisting of methoxy, methyl, methylsulfonyl, cyano, trifluoromethoxy, trifluoromethyl, fluoro and chloro.

Some embodiments of the present invention pertain to compounds of Formula **(IIg)** and pharmaceutically acceptable salts, solvates and hydrates thereof: 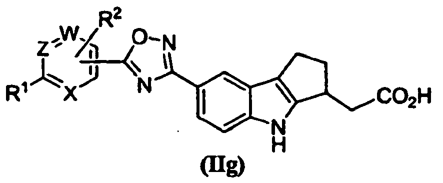
W is N or CR⁶;
Z is N or CR⁷;
X is N or CR⁸; provided that at least one W, Z and X is N;
R¹ is selected from the group consisting of H, C₁-C₆ alkoxy, C₁-C₆ alkyl, C₁-C₆ alkylamino, C₂-C₈ dialkylamino, C₁-C₆ alkylsulfonyl, carboxamide, cyano, C₂-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from the group consisting of C₁-C₆ alkoxy, carbo-C₁-C₆-alkoxy, C₁-C₆ alkyl, cyano, halogen, C₃-C₇ cycloalkyl and phenyl;
R² is H;
R⁶ is selected from the group consisting of H, C₁-C₆ alkoxy, C₁-C₆ alkyl, C₁-C₆ alkylsulfonyl, carboxamide, cyano, C₂-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from the group consisting of C₁-C₆ alkoxy, carbo-C₁-C₆-alkoxy, C₁-C₆ alkyl, cyano, halogen, C₃-C₇ cycloalkyl, and phenyl;
R⁷ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl; and
R⁸ is selected from the group consisting of H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, carbo-C₁-C₆-alkoxy, cyano, C₃-C₇ cycloalkyl, halogen and phenyl.

Some embodiments of the present invention pertain to compounds of Formula **(IIg)** and pharmaceutically acceptable salts, solvates and hydrates thereof: 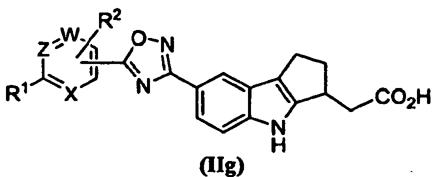 wherein:
W is N or CR⁶;
Z is N or CR⁷;
X is N or CR⁸; provided that at least one W, Z and X is N;
R¹ is selected from the group consisting of H, C₁-C₆ alkoxy, C₂-C₈ dialkylamino, carboxamide, C₁-C₆ haloalkoxy, C₁-C₆ haloalkyl and halogen,
R² is H;
R⁶ is H or C₁-C₆ alkyl;
R⁷ is selected from the group consisting of H, C₁-C₆ alkoxy and C₁-C₆ alkyl; and
R⁸ is H.

Some embodiments of the present invention pertain to compounds of Formula **(IIi)** and pharmaceutically acceptable salts, solvates and hydrates thereof:

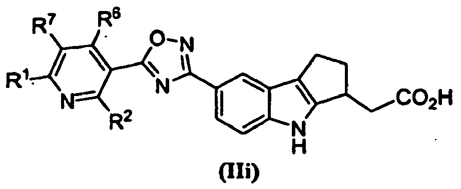

R¹ is selected from the group consisting of H, sec-butoxy, isopropoxy, methoxy, ethoxy, carboxamide, 2,2,2-trifluoroethoxy and trifluoromethyl;
R² is H;
R⁶ is H; and
R⁷ is selected from the group consisting of H, methyl, chloro, bromo, 1-methyl-1*H-*pyrazol-4-yl and hexyl.

Some embodiments of the present invention pertain to compounds of Formula **(IIi)** and pharmaceutically acceptable salts, solvates and hydrates thereof: 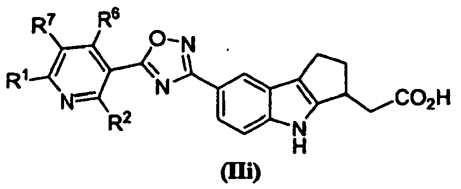 wherein:
R¹ is selected from the group consisting of H, sec-butoxy, isopropoxy, methoxy, ethoxy, carboxamide, 2,2,2-trifluoroethoxy and trifluoromethyl;
R² is H;
R⁶ is H; and
R⁷ is selected from the group consisting of H, methyl, chloro and bromo.

### C(3) Ring Carbon Stereochemistry

Compounds of the present invention contain the three ring fused system named 1,2,3,4-tetrahydrocyclopenta[b]indole. Present on one of the rings is either a carboxylic acid (n = 0) or an acetic acid group (n = 1). The ring carbon to which the carboxylic acid or acetic acid group is bonded is labeled as the C(3) Ring Carbon by convention. It is understood that the stereochemistry for the C(3) Ring Carbon contained in the 1,2,3,4-tetrahydrocyclopenta[b]indole ring system can be either *R* or *S*.

### A. C(3) Ring Carbon "R" Stereochemistry

In some embodiments, the stereochemistry for the ring carbon [e.g., C(3)] is R.

Some embodiments of the present invention pertain to compounds of Formula **(IIk)** and pharmaceutically acceptable salts, solvates and hydrates thereof: 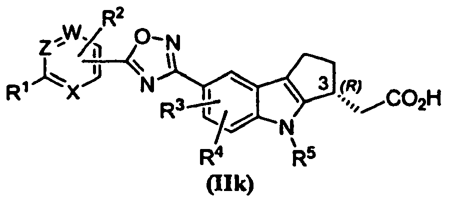 wherein each variable in Formula **(IIk)** has the same meaning as described herein, *supra* and *infra.*

Some embodiments of the present invention pertain to compounds of Formula **(IIm)** and pharmaceutically acceptable salts, solvates and hydrates thereof: 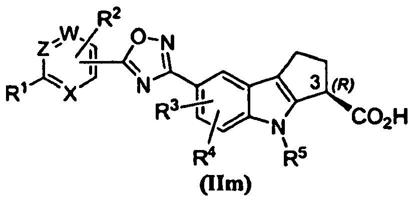 wherein each variable in Formula **(IIm)** has the same meaning as described herein, *supra* and *infra.*

### B. C(3) Ring Carbon "S" Stereochemistry

In some embodiments, the stereochemistry for the ring carbon [e.g., C(3)] is S.

Some embodiments of the present invention pertain to compounds of Formula **(IIo)** and pharmaceutically acceptable salts, solvates and hydrates thereof: 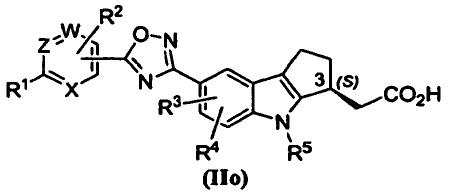 wherein each variable in Formula (IIo) has the same meaning as described herein, *supra* and *infra.*

Some embodiments of the present invention pertain to compounds of Formula **(IIq)** and pharmaceutically acceptable salts, solvates and hydrates thereof: 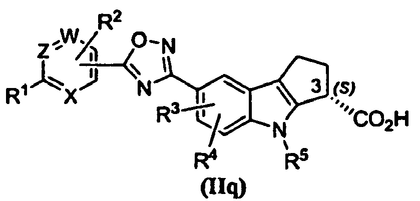 wherein each variable in Formula **(IIq)** has the same meaning as described herein, *supra* and *infra.*

### Esters and Prodrugs

One aspect of the present invention pertains to compounds of Formula **(IIIa)** as synthetic intermediates useful in the preparation of compounds of Formula **(Ia)** and/or prodrugs useful for the delivery of compounds of Formula **(Ia):** 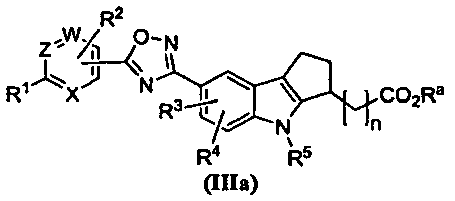 wherein:
n, R¹, R², R³, R⁴, R⁵, R⁶, R⁷, R⁸, W, X, and Z have the same definitions as described herein, *supra* and *infra,* and R⁸ is C₁-C₆ alkyl.

One aspect of the present invention pertains to compounds of Formula **(IIIa).**

In some embodiments, R⁸ is ethyl.

In some embodiments, R^{a} is *tert*-butyl.

In some embodiments, R^{a} is methyl.

In some embodiments, R⁸ is isopropyl.

For brevity, it is appreciated that all of the embodiments described herein, *supra* and *infra,* that relate to the common variables shared between Compounds of Formula **(Ia)** and **(IIIa)** namely, n, R¹, R², R³, R⁴, R⁵, W, X, and Z, apply to Compounds of Formula **(IIIa)** just as if they were each individually disclosed herewith with specific reference to Formula **(IIIa).**

One aspect of the present invention pertains to compounds of Formula **(IIIa)** as synthetic intermediates useful in the preparation of compounds of Formula **(Ia).**

One aspect of the present invention pertains to compounds of Formula **(IIIa)** as esters of compounds, described and shown herein, such as compounds in Table A, where R^{a} is ethyl.

One aspect of the present invention pertains to compounds of Formula **(IIIa)** as esters of compounds, described and shown herein, such as compounds in Table A, where R⁸ is *tert*-butyl.

One aspect of the present invention pertains to compounds of Formula **(IIIa)** as esters of compounds, described and shown herein, such as compounds in Table A, where R⁸ is methyl.

One aspect of the present invention pertains to compounds of Formula **(IIIa)** as esters of compounds, described and shown herein, such as compounds in Table A, where R^{a} is isopropyl.

One aspect of the present invention pertains to compounds of Formula **(IIIa)** as prodrugs useful for the delivery of compounds of Formula **(Ia).**

One aspect of the present invention pertains to compounds of Formula **(IIIa)** useful as prodrugs of compounds of Formula **(Ia).**

Some embodiments of the present invention include every combination of one or more compounds selected from the following group shown in **Table A.**

**TABLE A**

| Cmpd No. | Chemical Structure | Chemical Name |
|---|---|---|
| 1 | 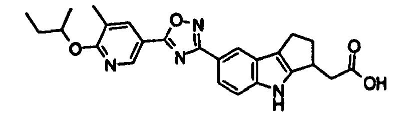 | 2-(7-(5-(6-*sec*-butoxy-5-methylpyridin-3-yl)-1,2,4-oxadiazol-3-yl)-1,2,3,4-tetrahydrocyclopenta[b]indol-3-yl)acetic acid |
| 2 | 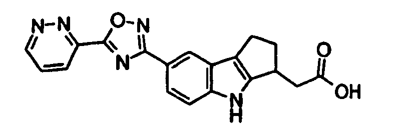 | 2-(7-(5-(pyridazin-3-yl)-1,2,4-oxadiazol-3-yl)-1,2,3,4-tetrahydrocyclopenta[b]indol-3-yl)acetic acid |
| 3 | 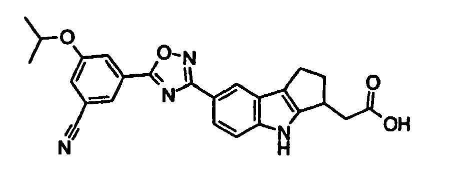 | 2-(7-(5-(3-cyano-5-isopropoxyphenyl)-1,2,4-oxadiazol-3-yl)-1,2,3,4-tetrahydrocyclopenta[b]indol-3-yl)acetic acid |
| 4 | 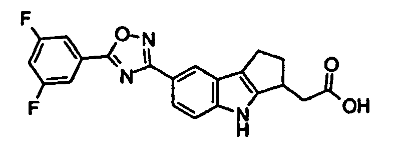 | 2-(7-(5-(3,5-difluorophenyl)-1,2,4-oxadiazol-3-yl)-1,2,3,4-tetrahydrocyclopenta[b]indol-3-yl)acetic acid |
| 5 | 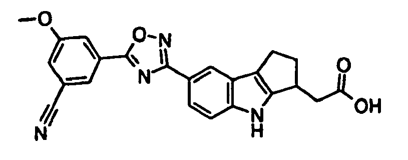 | 2-(7-(5-(3-cyano-5-methoxyphenyl)-1,2,4-oxadiazol-3-yl)-1,2,3,4-tetrahydrocyclopenta[b]indol-3-yl)acetic acid |
| 6 | 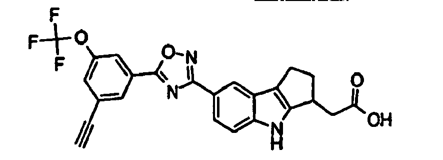 | 2-(7-(5-(3-ethynyl-5-(trifluoromethoxy)phenyl)-1,2,4-oxadiazol-3-yl)-1,2,3,4-tetrahydrocyclopenta[b]indol-3-yl)acetic acid |
| 7 | 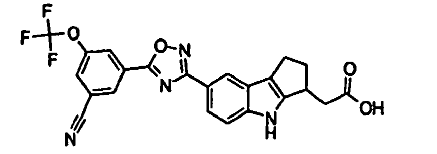 | 2-(7-(5-(3-cyano-5-(trifluoromethoxy)phenyl)-1,2,4-oxadiazol-3-yl)-1,2,3,4-tetrahydrocyclopenta[b]indol-3-yl)acetic acid |
| 8 | 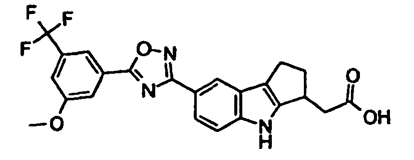 | 2-(7-(5-(3-methoxy-5-(trifluoromethyl)phenyl)-1,2,4-oxadiazol-3-yl)-1,2,3,4-tetrahydrocyclopenta[b]indol-3-yl)acetic acid |
| 9 | 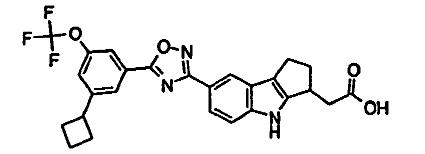 | 2-(7-(5-(3-cyclobutyl-5-(trifluoromethoxy)phenyl)-1,2,4-oxadiazol-3-yl)-1,2,3,4-tetrahydrocyclopenta[b]indol-3-yl)acetic acid |
| 10 | 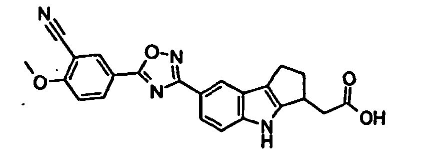 | 2-(7-(5-(3-cyano-4-methoxyphenyl)-1,2,4-oxadiazol-3-yl)-1,2,3,4-tetrahydrocyclopenta[b]indol-3-yl)acetic acid |
| 11 | 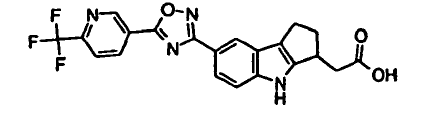 | 2-(7-(5-(6-(trifluoromethylyridin-3-yl)-1,2,4-oxadiazol-3-yl)-1,2,3,4-tetrahydrocyclopenta[b]indol-3-yl)acetic acid |
| 12 | 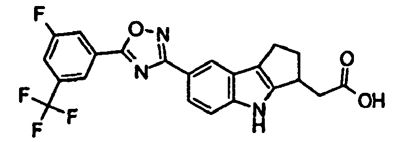 | 2-(7-(5-(3-fluoro-5-(trifluoromethyl)phenyl)-1,2,4-oxadiazol-3-yl)-1,2,3,4-tetrahydrocyclopenta[b]indol-3-yl)acetic acid |
| 13 | 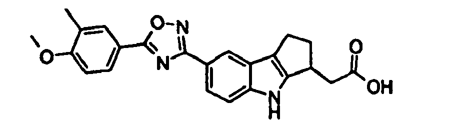 | 2-(7-(5-(4-methoxy-3-methylphenyl)-1,2,4-oxadiazol-3-yl)-1,2,3,4-tetrahydrocyclopenta[b]indol-3-yl)acetic acid |
| 14 | 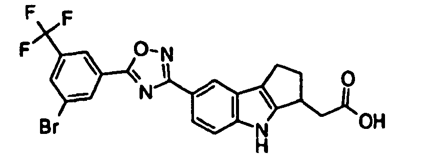 | 2-(7-(5-(3-bromo-5-(trifluoromethyl)phenyl)-1,2,4-oxadiaxol-3-yl)-1,2,3,4-tetrahydrocyclopenta[b]indol-3-yl)acetic acid |
| 15 | 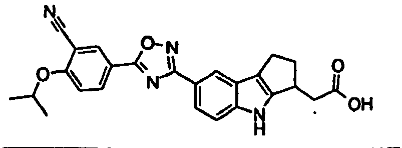 | 2-(7-(5-(3-cyano-4-isopropoxyphenyl)-1,2,4-oxadiazol-3-yl)-1,2,3,4-tetrahydrocyclopenta[b]indol-3-yl)acetic acid |
| 16 | 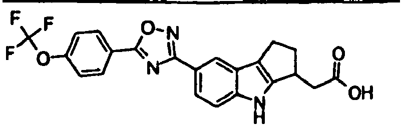 | 2-(7-(5-(4-(trifluoromethoxy)phenyl)-1,2,4-oxadiazol-3-yl)-1,2,3,4-tetrahydrocyclopenta[b]indol-3-yl)acetic acid |
| 17 | 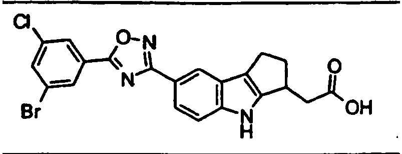 | 2-(7-(5-(3-bromo-5-chlorophenyl)-1,2,4-oxadiazol-3-yl)-1,2,3,4-tetrahydrocyclopenta[b]indol-3-yl)acetic acid |
| 18 | 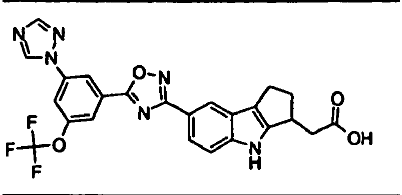 | 2-(7-(5-(3-(1H-1,2,4-triazol-1-yl)-5-(trifluoromethoxy)phenyl)-1,2,4-oxadiazol-3-yl)-1,2,3,4-tetrahydrocyclopenta[b]indol-3-yl)acetic acid |
| 19 | 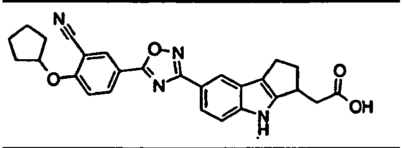 | 2-(7-(5-(3-cyano-4-(cyclopentyloxy)phenyl)-1,2,4-oxadiazol-3-yl)-1,2,3,4-tetrahydrocyclopenta[b]indol-3-yl)acetic acid |
| 20 | 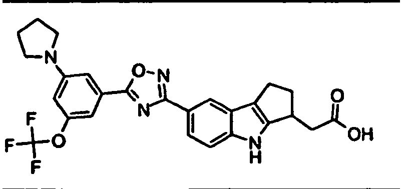 | 2-(7-(5-(3-(pyrrolidin-1-yl)-5-(trifluoromethoxy)phenyl)-1,2,4-oxadiazol-3-yl)-1,2,3,4-tetrahydrocyclopenta[b]indol-3-yl)acetic acid |
| 21 | 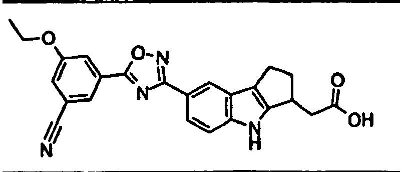 | 2-(7-(5-(3-cyano-5-ethoxyphenyl)-1,2,4-oxadiazol-3-yl)-1,2,3,4-tetrahydrocyclopenta[b]indol-3-yl)acetic acid |
| 22 | 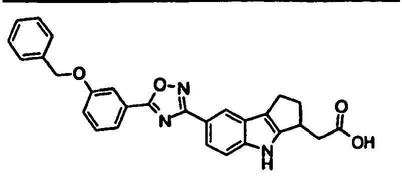 | 2-(7-(5-(3-(benzyloxy)phenyl)-1,2,4-oxadiazol-3-yl)-1,2,3,4-tetrahydrocyclopenta[b]indol-3-yl)acetic acid |
| 23 | 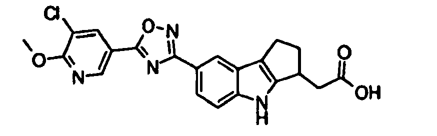 | 2-(7-(5-(5-chloro-6-methoxypyridin-3-yl)-1,2,4-oxadiazol-3-yl)-1,2,3,4-tetrahydrocyclopenta[b]indol-3-yl)acetic acid |
| 24 | 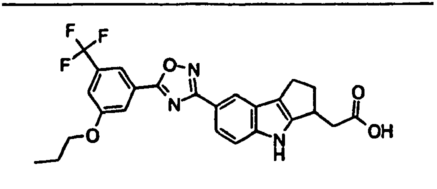 | 2-(7-(5-(3-propoxy-5-(trifluoromethyl)phenyl)-1,2,4-oxadiazol-3-yl)-1,2,3,4-tetrahydrocyclopenta[b]indol-3-yl)acetic acid |
| 25 | 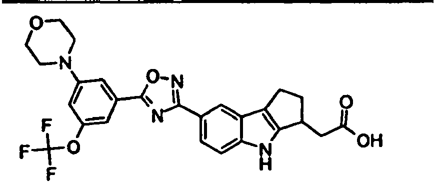 | 2-(7-(5-(3-morpholino-5-(trifluoromethoxy)phenyl)-1,2,4-oxadiazol-3-yl)-1,2,3,4-tetrahydrocyclopenta[b]indol-3-yl)acetic acid |
| 26 | 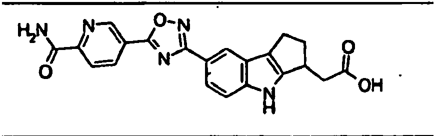 | 2-(7-(5-(6-carbamoylpyridin-3-yl)-1,2,4-oxadiazol-3-yl)-1,2,3,4-tetrahydrocyclopenta[b]indol-3-yl)acetic acid |
| 27 | 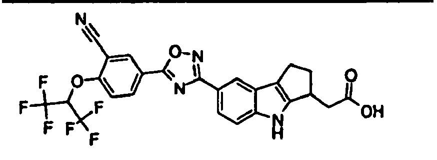 | 2-(7-(5-(3-cyano4-(1,1,1,3,3,3-hexafluoropropan-2-yloxy)phenyl)-1,2,4-oxadiazol-3-yl)-1,2,3,4-tetrahydrocyclopenta[b]indol-3-yl)acetic acid |
| 28 | 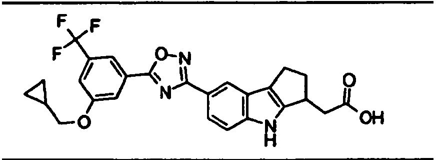 | 2-(7-(5-(3-(cyclopropylmethoxy)-5-(trifluoromethyl)phenyl)-1,2,4-oxadiazol-3-yl)-1,2,3,4-tetrahydrocyclopenta[b]indol-3-yl)acetic acid |
| 29 | 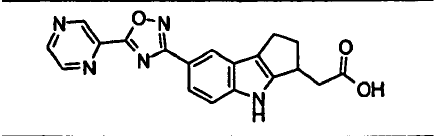 | 2-(7-(5-(pyrazin-2-yl)-1,2,4-oxadiazol-3-yl)-1,2,3,4-tetrahydrocyclopenta[b]indol-3-yl)acetic acid |
| 30 | 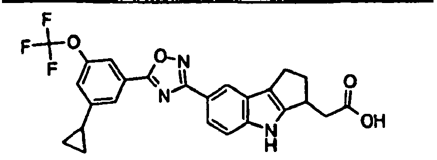 | 2-(7-(5-(3-cyclopropyl-5-(trifluoromethoxy)phenyl)-1,2,4-oxadiazol-3-yl)-1,2,3,4-tetrahydrocyclopenta[b]indol-3-yl)acetic acid |
| 31 | 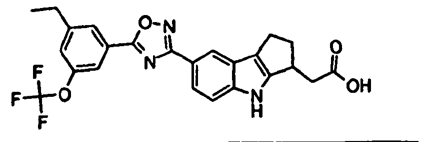 | 2-(7-(5-(3-ethyl-5-(trifluoromethoxy)phenyl)-1,2,4-oxadiazol-3-yl)-1,2,3,4-tetrahydrocyclopenta[b]indol-3-yl)acetic acid |
| 32 | 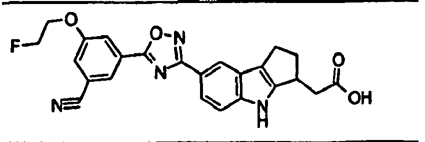 | 2-(7-(5-(3-cyano-5-(2-fluoroethoxy)phenyl)-1,2,4-oxadiazol-3-yl)-1,2,3,4-tetrahydrocyclopenta[b]indol-3-yl)acetic acid |
| 33 | 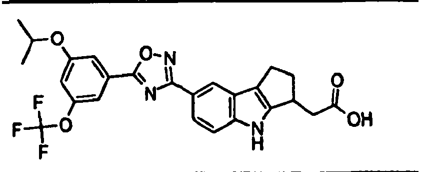 | 2-(7-(5-(3-isopropoxy-5-(trifluoromethoxy)phenyl)-1,2,4-oxadiazol-3-yl)-1,2,3,4-tetrahydrocyclopenta[b]indol-3-yl)acetic acid |
| 34 | 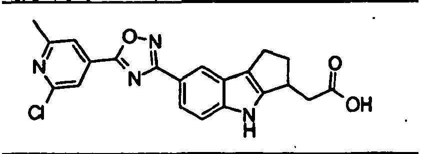 | 2-(7-(5-(2-chloro-6-methylpyridin-4-yl)-1,2,4-oxadiazol-3-yl)-1,2,3,4-tetrahydrocyclopenta[b]indol-3-yl)acetic acid |
| 35 | 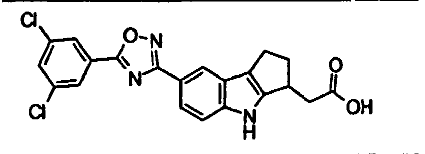 | 2-(7-(5-(3,5-dichlorophenyl)-1,2,4-oxadiazol-3-yl)-1,2,3,4-tetrahydrocyclopenta[b]indol-3-yl)acetic acid |
| 36 | 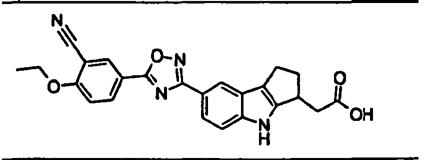 | 2-(7-(5-(3-cyano-4-ethoxyphenyl)-1,2,4-oxadiazol-3-yl)-1,2,3,4-tetrahydrocyclopenta[b]indol-3-yl)acetic acid |
| 37 | 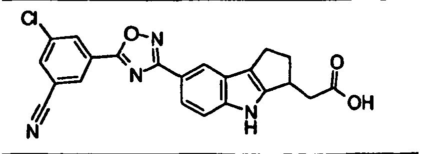 | 2-(7-(5-(3-chloro-5-cyanophenyl)-1,2,4-oxadiazol-3-yl)-1,2,3,4-tetrahydrocyclopenta[b]indol-3-yl)acetic acid |
| 38 | 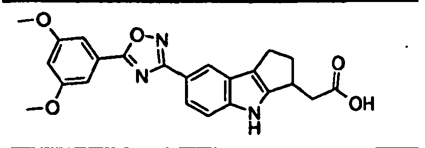 | 2-(7-(5-(3,5-dimethoxyphenyl)-1,2,4-oxadiazol-3-yl)-1,2,3,4-tetrahydrocyclopenta[b]indol-3-yl)acetic acid |
| 39 | 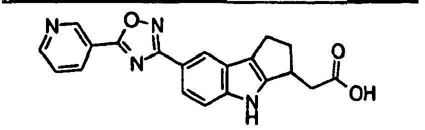 | 2-(7-(5-(pyridin-3-yl)-1,2,4-oxadiazol-3-yl)-1,2,3,4-tetrahydrocyclopenta[b]indol-3-yl)acetic acid |
| 40 | 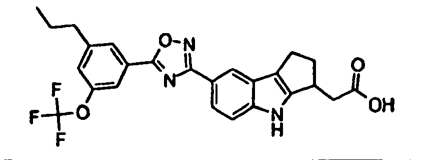 | 2-(7-(5-(3-propyl-5-(trifluoromethoxy)phenyl)-1,2,4-oxadiazol-3-yl)-1,2,3,4-tetrahydrocyclopenta[b]indol-3-yl)acetic acid |
| 41 | 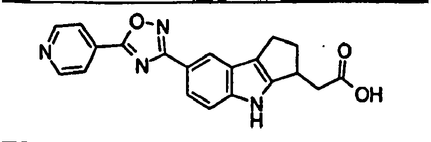 | 2-(7-(5-(pyridin-4-yl)-1,2,4-oxadiazol-3-yl)-1,2,3,4-tetrahydrocyclopenta[b]indol-3-yl)acetic acid |
| 42 | 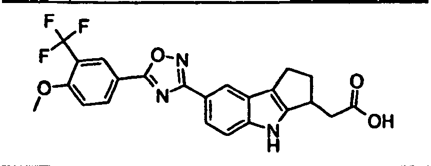 | 2-(7-(5-(4-methoxy-3-(trifluoromethyl)phenyl)-1,2,4-oxadiazol-3-yl)-1,2,3,4-tetrahydrocyclopenta[b]indol-3-yl)acetic acid |
| 43 | 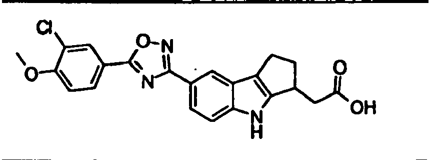 | 2-(7-(5-(3-chloro-4-methoxyphenyl)-1,2,4-oxadiazol-3-yl)-1,2,3,4-tetrahydrocyclopenta[b]indol-3-yl)acetic acid |
| 44 | 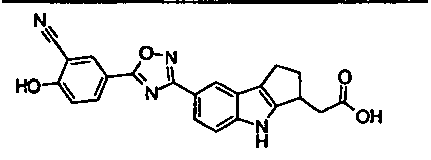 | 2-(7-(5-(3-cyano-4-hydroxyphenyl)-1,2,4-oxadiazol-3-yl)-1,2,3,4-tetrahydrocyclopenta[b]indol-3-yl)acetic acid |
| 45 | 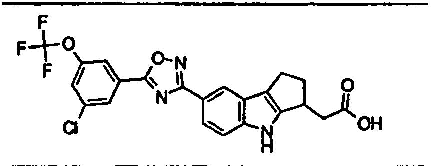 | 2-(7-(5-(3-chloro-5-(trifluoromethoxy)phenyl)-1,2,4-oxadiazol-3-yl)-1,2,3,4-tetrahydrocyclopenta[b]indol-3-yl)acetic acid |
| 46 | 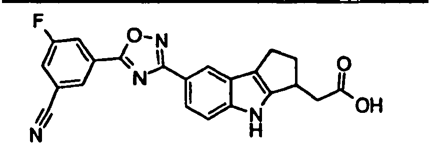 | 2-(7-(5-(3-cyano-5-fluorophenyl)-1,2,4-oxadiazol-3-yl)-1,2,3,4-tetrahydrocyclopenta[b]indol-3-yl)acetic acid |
| 47 | 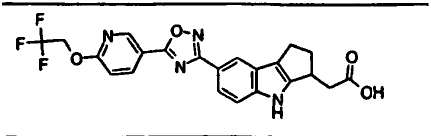 | 2-(7-(5-(6-(2,2,2-trifluoroethoxy)pyridin-3-yl)-1,2,4-oxadiazol-3-yl)-1,2,3,4-tetrahydrocyclopenta[b]indol-3-yl)acetic acid |
| 48 | 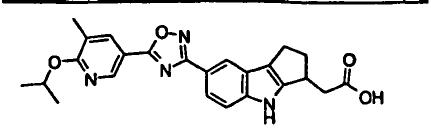 | 2-(7-(5-(6-isopropoxy-5-methylpyridin-3-yl)-1,2,4-oxadiazol-3-yl)-1,2,3,4-tetrahydrocyclopenta[b]indol-3-yl)acetic acid |
| 49 | 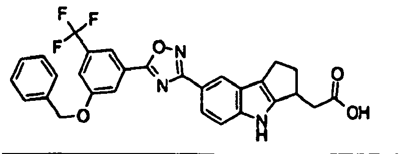 | 2-(7-(5-(3-(benzyloxy)-5-(trifluoromethyl)phenyl)-1,2,4-oxadiazol-3-yl)-1,2,3,4-tetrahydrocyclopenta[b]indol-3-yl)acetic acid |
| 50 | 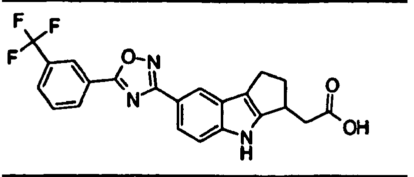 | 2-(7-(5-(3-(trifluoromethyl)phenyl)-1,2,4-oxadiazol-3-yl)-1,2,3,4-tetrahydrocyclopenta[b]indol-3-yl)acetic acid |
| 51 | 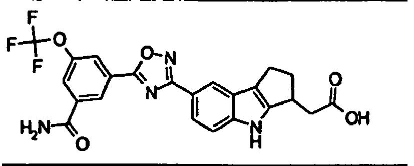 | 2-(7-(5-(3-carbamoyl-5-(trifluoromethoxy)phenyl)-1,2,4-oxadiazol-3-yl)-1,2,3,4-tetrahydrocyclopenta[b]indol-3-yl)acetic acid |
| 52 | 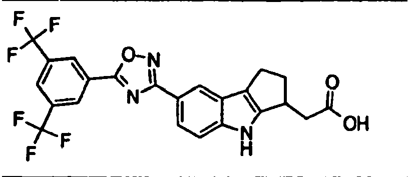 | 2-(7-(5-(3,5-bis(trifluoromethyl)phenyl)-1,2,4-oxadiazol-3-yl)-1,2,3,4-tetrahydrocyclopenta[b]indol-3-yl)acetic acid |
| 53 | 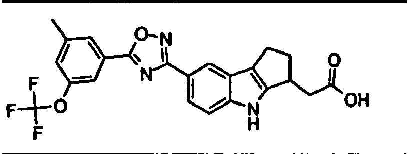 | 2-(7-(5-(3-methyl-5-(trifluoromethoxy)phenyl)-1,2,4-oxadiazol-3-yl)-1,2,3,4-tetrahydrocyclopenta[b]indol-3-yl)acetic acid |
| 54 | 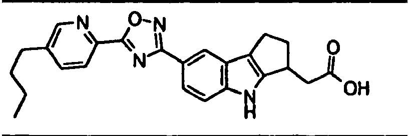 | 2-(7-(5-(5-butylpyridin-2-yl)-1,2,4-oxadiazol-3-yl)-1,2,3,4-tetrahydrocyclopenta[b]indol-3-yl)acetic acid |
| 55 | 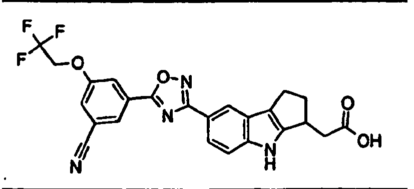 | 2-(7-(5-(3-cyano-5-(2,2,2-trifluoroethoxy)phenyl)-1,2,4-oxadiazol-3-yl)-1,2,3,4-tetrahydrocyclopenta[b]indol-3-yl)acetic acid |
| 56 | 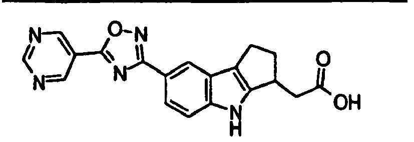 | 2-(7-(5-(pyrimidin-5-yl)-1,2,4-oxadiazol-3-yl)-1,2,3,4-tetrahydrocyclopenta[b]indol-3-yl)acetic acid |
| 57 | 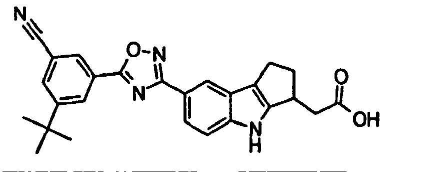 | 2-(7-(5-(3-*tert*-butyl-5-cyanophenyl)-1,2,4-oxadiazol-3-yl)-1,2,3,4-tetrahydrocyclopenta[b]indol-3-yl)acetic acid |
| 58 | 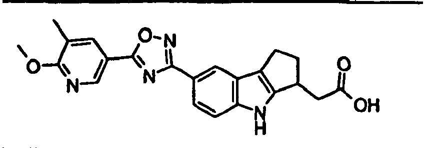 | 2-(7-(5-(6-methoxy-5-methylpyridin-3-yl)-1,2,4-oxadiazol-3-yl)-1,2,3,4-tetrahydrocyclopenta[b]indol-3-yl)acetic acid |
| 59 | 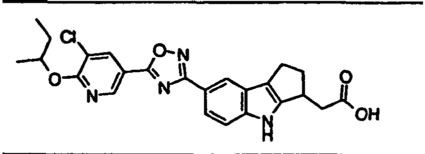 | 2-(7-(5-(6-*sec-*butoxy-5-chloropyridin-3-yl)-1,2,4-oxadiazol-3-yl)-1,2,3,4-tetrahydrocyclopenta[b]indol-3-yl)acetic acid |
| 60 | 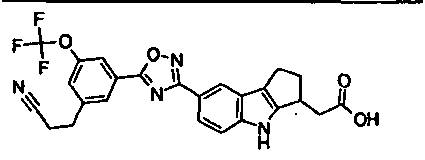 | 2-(7-(5-(3-(2-cyanoethyl)-5-(trifluoromethoxy)phenyl)-1,2,4-oxadiazol-3-yl)-1,2,3,4-tetrahydrocyclopenta[b]indol-3-yl)acetic acid |
| 61 | 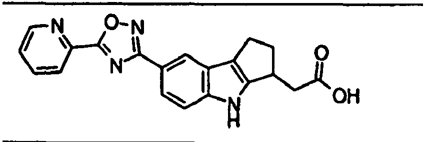 | 2-(7-(5-(pyridin-2-yl)-1,2,4-oxadiazol-3-yl)-1,2,3,4-tetrahydrocyclopenta[b]indol-3-yl)acetic acid |
| 62 | 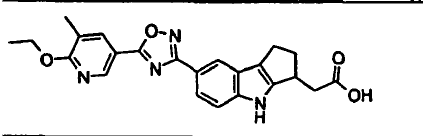 | 2-(7-(5-(6-ethoxy-5-methylpyridin-3-yl)-1,2,4-oxadiazol-3-yl)-1,2,3,4-tetrahydrocyclopenta[b]indol-3-yl)acetic acid |
| 63 | 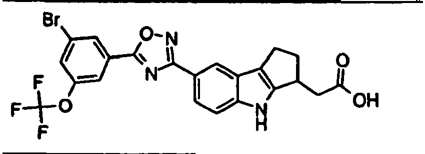 | 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid |
| 64 | 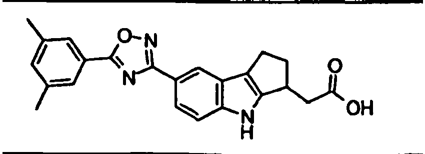 | 2-(7-(5-(3,5-dimethylphenyl)-1,2,4-oxadiazol-3-yl)-1,2,3,4-tetrahydrocyclopenta[b]-indol-3-yl)acetic acid |
| 65 | 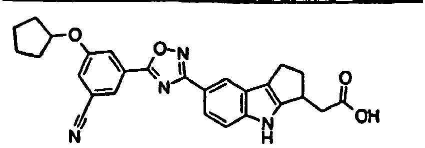 | 2-(7-(5-(3-cyano-5-(cyclopentyloxy)phenyl)-1,2,4-oxadiazol-3-yl)-1,2,3,4-tetrahydrocyclopenta[b]indol-3-yl)acetic acid |
| 66 | 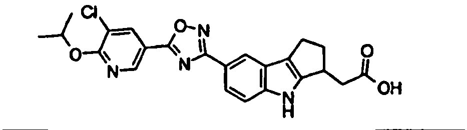 | 2-(7-(5-(5-chloro-6-isopropoxypyridin-3-yl)-1,2,4-oxadiazol-3-yl)-1,2,3,4-tetrahydrocyclopenta[b]indol-3-yl)acetic acid |
| 67 | 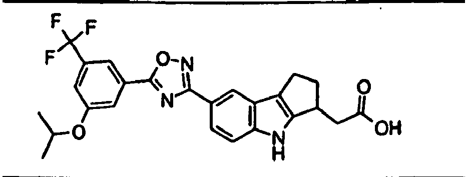 | 2-(7-(5-(3-isopropoxy-5-(trifluoromethyl)phenyl)-1,2,4-oxadiazol-3-yl)-1,2,3,4-tetrahydrocyclopenta[b]indol-3-yl)acetic acid |
| 68 | 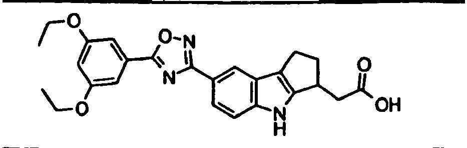 | 2-(7-(5-(3,5-diethoxyphenyl)-1,2,4-oxadiazol-3-yl)-1,2,3,4-tetrahydrocyclopenta[b]indol-3-yl)acetic acid |
| 69 | 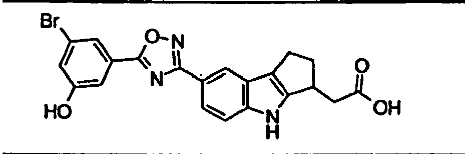 | 2-(7-(5-(3-bromo-5-hydroxyphenyl)-1,2,4-oxadiazol-3-yl)-1,2,3,4-tetrahydrocyclopenta[b]indol-3-yl)acetic acid |
| 70 | 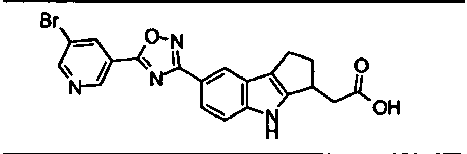 | 2-(7-(5-(5-bromopyridin-3-yl)-1,2,4-oxadiazol-3-yl)-1,2,3,4-tetrahydrocyclopenta[b]indol-3-yl)acetic acid |
| 71 | 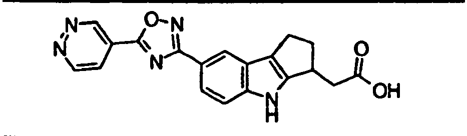 | 2-(7-(5-(pyridazin-4-yl)-1,2,4-oxadiazol-3-yl)-1,2,3,4-tetrahydrocyclopenta[b]indol-3-yl)acetic acid |
| 72 | 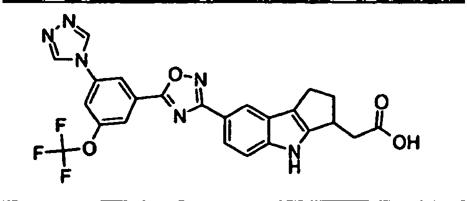 | 2-(7-(5-(3-(4H-1,2,4-triazol-4-yl)-5-(trifluoromethoxy)phenyl)-1,2,4-oxadiazol-3-yl)-1,2,3,4-tetrahydrocyclopenta[b]indol-3-yl)acetic acid |
| 73 | 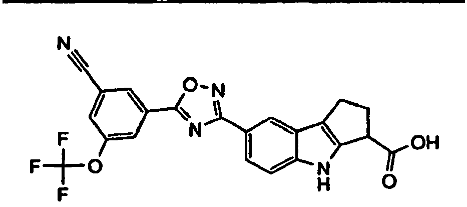 | 7-(5-(3-cyano-5-(trifluoromethoxy)phenyl)-1,2,4-oxadiazol-3-yl)-1,2,3,4-tetrahydrocyclopenta[*b*]indole-3-carboxylic acid |
| 74 | 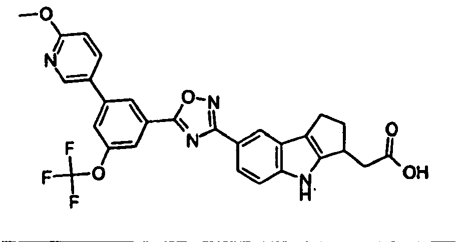 | 2-(7-(5-(3-(6-methoxypyridin-3-yl)-5-(trifluoromethoxy)phenyl)-1,2,4-oxadiazol-3-yl)-1,2,3,4-tetrahydrocyclopenta[b]indol-3-yl)acetic acid |
| 75 | 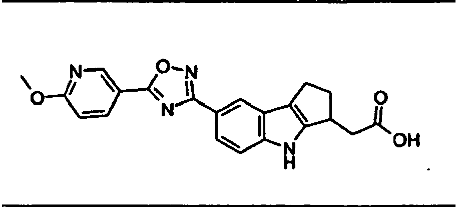 | 2-(7-(5-(6-methoxypyridin-3-yl)-1,2,4-oxadiazol-3-yl)-1,2,3,4-tetrahydrocyclopenta[b]indol-3-yl)acetic acid |
| 76 | 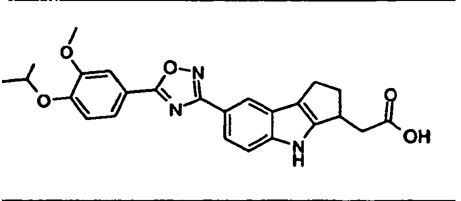 | 2-(7-(5-(4-isopropoxy-3-methoxyphenyl)-1,2,4-oxadiazol-3-yl)-1,2,3,4-tetrahydrocyclopenta[b]indol-3-yl)acetic acid |
| 77 | 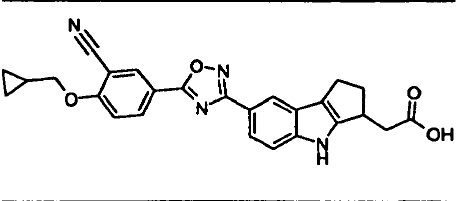 | 2-(7-(5-(3-cyano-4-(cyclopropylmethoxy)phenyl)-1,2,4-oxadiazol-3-yl)-1,2,3,4-tetrahydrocyclopenta[b]indol-3-yl)acetic acid |
| 78 | 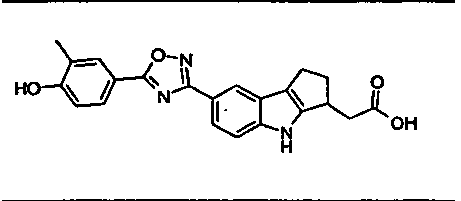 | 2-(7-(5-(4-hydroxy-3-methylphenyl)-1,2,4-oxadiazol-3-yl)-1,2,3,4-tetrahydrocyclopenta[b]indol-3-yl)acetic acid |
| 79 | 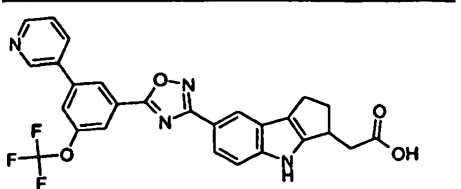 | 2-(7-(5-(3-(pyridin-3-yl)-5-(trifluoromethoxy)phenyl)-1,2,4-oxadiazol-3-yl)-1,2,3,4-tetrahydrocyclopenta[b]indol-3-yl)acetic acid |
| 80 | 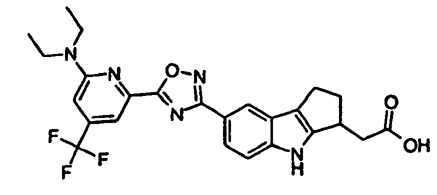 | 2-(7-(5-(6-(diethytamino)-4-(trifluoromethyl)pyridin-2-yl)-1,2,4-oxadiazol-3-yl)-1,2,3,4-tetrahydrocyclopenta[b]indol-3-yl)acetic acid |
| 81 | 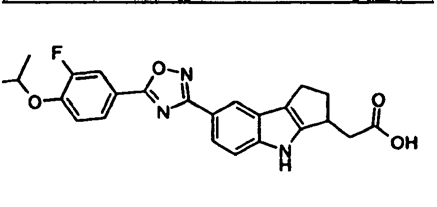 | 2-(7-(5-(3-fluoro-4-isopropoxyphenyl)-1,2,4-oxadiazol-3-yl)-1,2,3,4-tetrahydrocyclopenta[b]indol-3-yl)acetic acid |
| 82 | 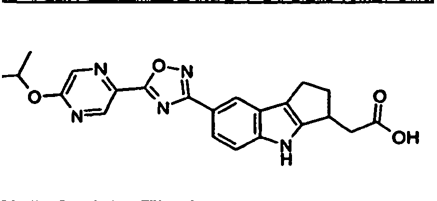 | 2-(7-(5-(5-isopropoxypyrazin-2-yl)-1,2,4-oxadiazol-3-yl)-1,2,3,4-tetrahydrocyclopenta[b]indol-3-yl)acetic acid |
| 83 | 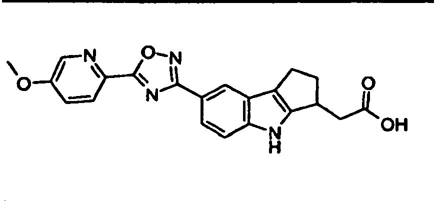 | 2-(7-(5-(5-methoxypyridin-2-yl)-1,2,4-oxadiazol-3-yl)-1,2,3,4-tetrahydrocyclopenta[b]indol-3-yl)acetic acid |
| 84 | 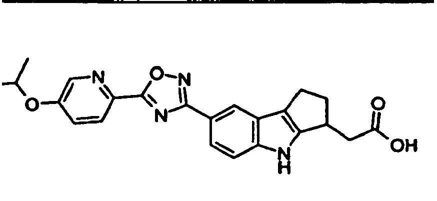 | 2-(7-(5-(5-isopropoxypyridin-2-yl)-1,2,4-oxadiazol-3-yl)-1,2,3,4-tetrahydrocyclopenta[b]indol-3-yl)acetic acid |
| 85 | 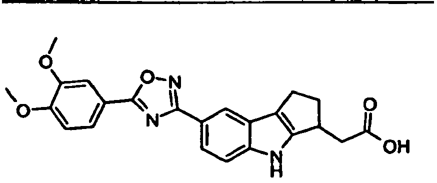 | 2-(7-(5-(3,4-dimethoxyphenyl)-1,2,4-oxadiazol-3-yl)-1,2,3,4-tetrahydrocyclopenta[b]indol-3-yl)acetic acid |
| 86 | 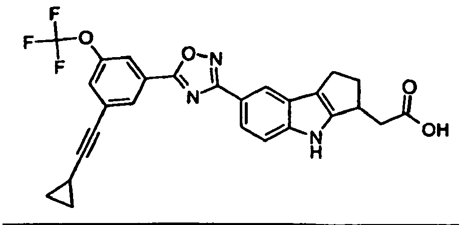 | 2-(7-(5-(3-(cyclopropylethynyl)-5-(trifluoromethoxy)phenyl)-1,2,4-oxadiazol-3-yl)-1,2,3,4-tetrahydrocyclopenta[b]indol-3-yl)acetic acid |
| 87 | 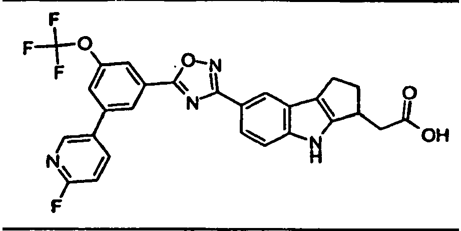 | 2-(7-(5-(3-(6-fluoropyridin-3-yl)-5-(trifluoromethoxy)phenyl)-1,2,4-oxadiazol-3-yl)-1,2,3,4-tetrahydrocyclopenta[b]indol-3-yl)acetic acid |
| 88 | 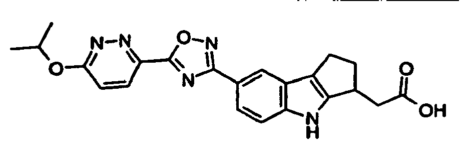 | 2-(7-(5-(6-isopropoxypyridazin-3-yl)-1,2,4-oxadiazol-3-yl)-1,2,3,4-tetrahydrocyclopenta[b]indol-3-yl)acetic acid |
| 89 | 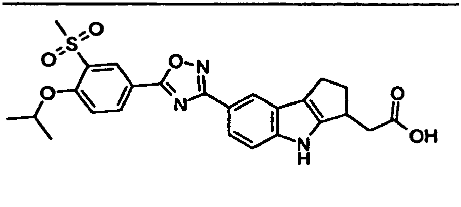 | 2-(7-(5-(4-isopropoxy-3-(methylsulfonyl)phenyl)-1,2,4-oxadiazol-3-yl)-1,2,3,4-tetrahydrocyclopenta[b]indol-3-yl)acetic acid |
| 90 | 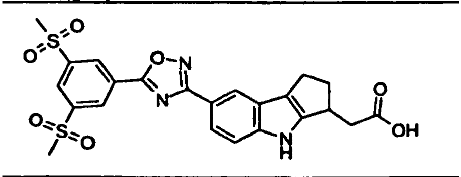 | 2-(7-(5-(3,5-bis(methylsulfonyl)phenyl)-1,2,4-oxadiazol-3-yl)-1,2,3,4-tetrahydrocyclopenta[b]indol-3-yl)acetic acid |
| 91 | 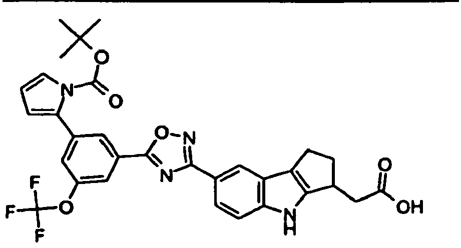 | 2-(7-(5-(3-(1-(*tert*-butoxycarbonyl)-1H-pyrrol-2-yl)-5-(trifluoromethoxy)phenyl)-1,2,4-oxadiazol-3-yl)-1,2,3,4-tetrahydrocyclopenta[b]indol-3-yl)acetic acid |
| 92 | 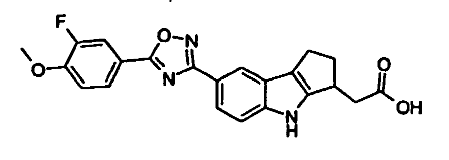 | 2-(7-(5-(3-fluoro-4-methoxyphenyl)-1,2,4-oxadiazol-3-yl)-1,2,3,4-tetrahydrocyclopenta[b]indol-3-yl)acetic acid |
| 93 | 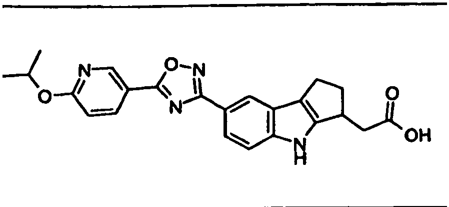 | 2-(7-(5-(6-isopropoxypyridin-3-yl)-1,2,4-oxadiazol-3-yl)-1,2,3,4-tetrahydrocyclopenta[b] indol-3-yl)acetic acid |
| 94 | 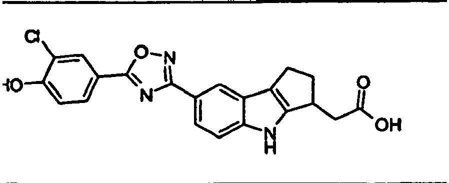 | 2-(7-(5-(3-chloro-4-hydroxyphenyl)-1,2,4-oxadiazol-3-yl)-1,2,3,4-tetrahydrocyclopenta[b]indol-3-yl)acetic acid |
| 95 | 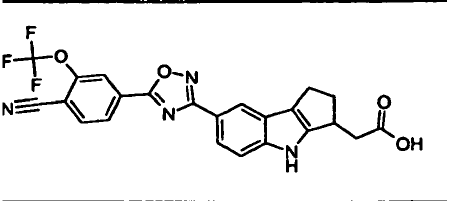 | 2-(7-(5-(4-cyano-3-(trifluoromethoxy)phenyl)-1,2,4-oxadiazol-3-yl)-1,2,3,4-tetrahydrocyclopenta[b]indol-3-yl)acetic acid |
| 96 | 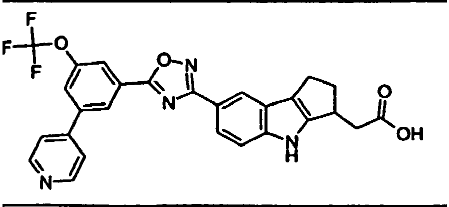 | 2-(7-(5-(3-(pyridin4-yl)-5-(trifluoromethoxy)phenyl)-1,2,4-oxadiazol-3-yl)-1,2,3,4-tetrahydrocyclopenta[b]indol-3-yl)acetic acid |
| 97 | 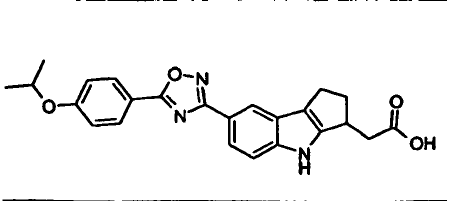 | 2-(7-(5-(4-isopropoxyphenyl)-1,2,4-oxadiazol-3-yl)-1,2,3,4-tetrahydrocyclopenta[b]indol-3-yl)acetic acid |
| 98 | 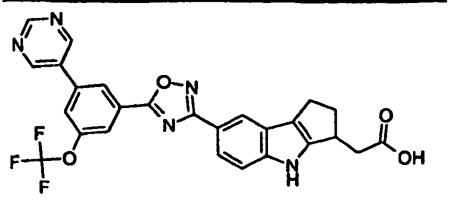 | 2-(7-(5-(3-(pyrimidin-5-yl)-5-(trifluoromethoxy)phenyl)-1,2,4-oxadiazol-3-yl)-1,2,3,4-tetrahydrocyclopenta[b]indol-3-yl)acetic acid |
| 99 | 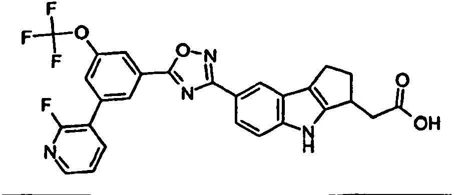 | 2-(7-(5-(3-(2-fluoropyridin-3-yl)-5-(trifluoromethoxy)phenyl)-1,2,4-oxadiazol-3-yl)-1,2,3,4-tetrahydrocyclopenta[b]indol-3-yl)acetic acid |
| 100 | 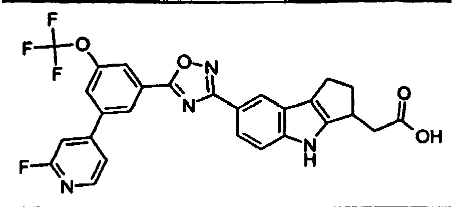 | 2-(7-(5-(3-(2-fluoropyridin-4-yl)-5-(trifluoromethoxy)phenyl)-1,2,4-oxadiazol-3-yl)-1,2,3,4-tetrahydrocyclopenta[b]indol-3-yl)acetic acid |
| 101 | 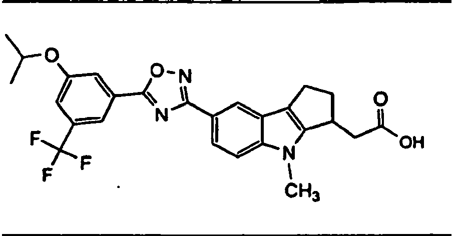 | 2-(7-(5-(3-isopropoxy-5-(trifluoromethyl)phenyl)-1,2,4-oxadiazol-3-yl)-4-methyl-1,2,3,4-tetrahydrocyclopenta[b]indol-3-yl)acetic acid |
| 102 | 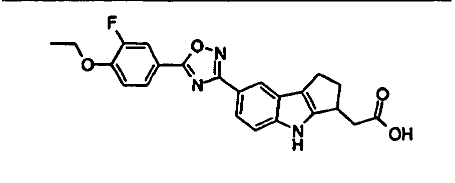 | 2-(7-(5-(4-ethoxy-3-fluorophenyl)-1,2,4-oxadiazol-3-yl)-1,2,3,4-tetrahydrocyclopenta[b]indol-3-yl)acetic acid |
| 103 | 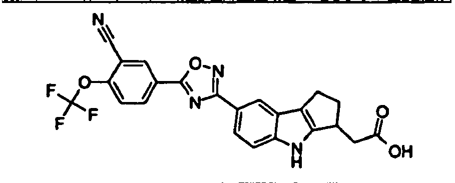 | 2-(7-(5-(3-cyano-4-(trifluoromethoxy)phenyl)-1,2,4-oxadiazol-3-yl)-1,2,3,4-tetrahydrocyclopenta[b]indol-3-yl)acetic acid |
| 104 | 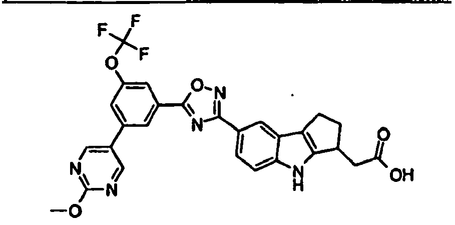 | 2-(7-(5-(3-(2-methoxypyrimidin-5-yl)-5-(trifluoromethoxy)phenyl)-1,2,4-oxadiazol-3-yl)-1,2,3,4-tetrahydrocyclopenta[b]indol-3-yl)acetic acid |
| 105 | 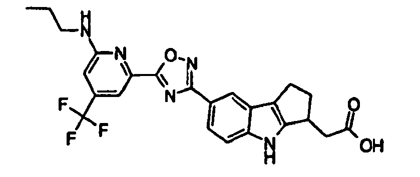 | 2-(7-(5-(6-(propylamino)-4-(trifluoromethyl)pyridin-2-yl)-1,2,4-oxadiazol-3-yl)-1,2,3,4-tetrahydrocyclopenta[b]indol-3-yl)acetic acid |
| 106 | 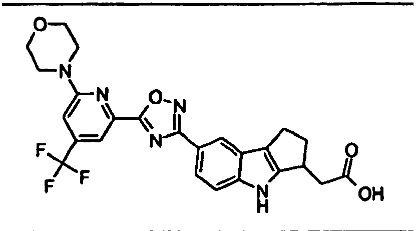 | 2-(7-(5-(6-morpholino-4-(trifluoromethyl)pyridin-2-yl)-1,2,4-oxadiazol-3-yl)-1,2,3,4-tetrahydrocyclopenta[b]indol-3-yl)acetic acid |
| 107 | 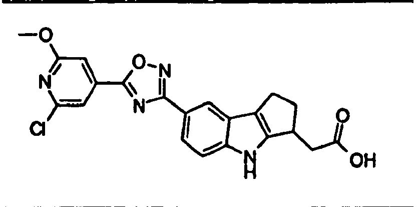 | 2-(7-(5-(2-chloro-6-methoxypyridin-4-yl)-1,2,4-oxadiazol-3-yl)-1,2,3,4-tetrahydrocyclopenta[b]indol-3-yl)acetic acid |
| 108 | 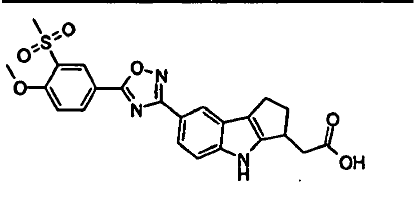 | 2-(7-(5-(4-methoxy-3-(methylsulfonyl)phenyl)-1,2,4-oxadiazol-3-yl)-1,2,3,4-tetrahydrocyclopenta[b] indol-3-yl)acetic acid |
| 109 | 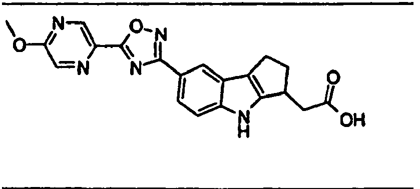 | 2-(7-(5-(5-methoxypyrazin-2-yl)-1,2,4-oxadiazol-3-yl)-1,2,3,4-tetrahydrocyclopenta[b]indol-3-yl)acetic acid |
| 110 | 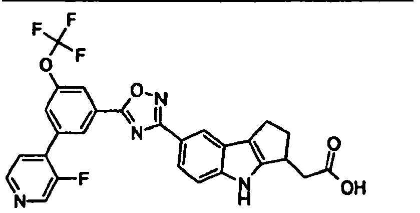 | 2-(7-(5-(3-(3-fluoropyridin-4-yl)-5-(trifluoromethoxy)phenyl)-1,2,4-oxadiazol-3-yl)-1,2,3,4-tetrahydrocyclopenta[b]indol-3-yl)acetic acid |
| 111 | 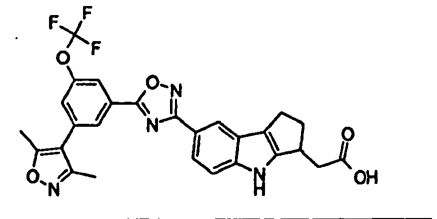 | 2-(7-(5-(3-(3,5-dimethylisoxazol-4-yl)-5-(trifluoromethoxy)phenyl)-1,2,4-oxadiazol-3-yl)-1,2,3,4-tetrahydrocyclopenta[b]indol-3-yl)acetic acid |
| 112 | 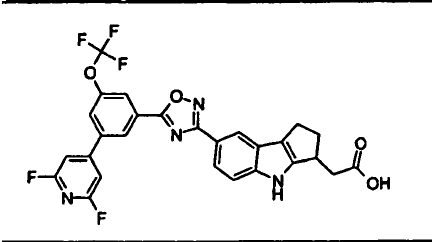 | 2-(7-(5-(3-(2,6-difluoropyridin-4-yl)-5-(trifluoromethoxy)phenyl)-1,2,4-oxadiazol-3-yl)-1,2,3,4-tetrahydrocyclopenta[b]indol-3-yl)acetic acid |
| 113 | 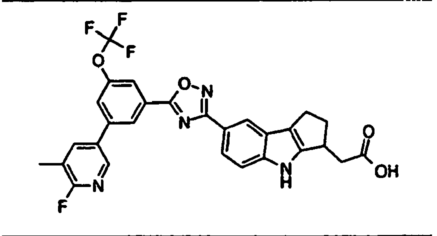 | 2-(7-(5-(3-(6-fluoro-5-methylpyridin-3-yl)-5-(trifluoromethoxy)phenyl)-1,2,4-oxadiazol-3-yl)-1,2,3,4-tetrahydrocyclopenta[b]indol-3-yl)acetic acid |
| 114 | 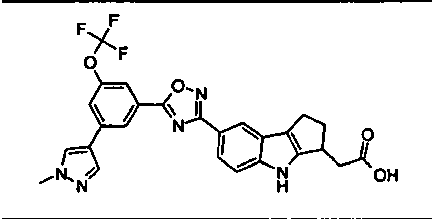 | 2-(7-(5-(3-(1-methyl-1H-pyrazol-4-yl)-5-(trifluoromethoxy)phenyl)-1,2,4-oxadiazol-3-yl)-1,2,3,4-tetrahydrocyclopenta[b]indol-3-yl)acetic acid |
| 115 | 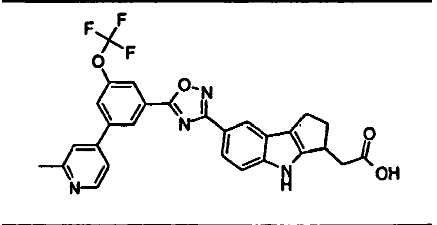 | 2-(7-(5-(3-(2-methylpyridin-4-yl)-5-(trifluoromethoxy)phenyl)-1,2,4-oxadiazol-3-yl)-1,2,3,4-tetrahydrocyclopenta[b]indol-3-yl)acetic acid |
| 116 | 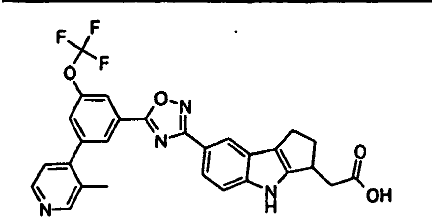 | 2-(7-(5-(3-(3-methylpyridin-4-yl)-5-(trifluoromethoxy)phenyl)-1,2,4-oxadiazol-3-yl)-1,2,3,4-tetrahydrocyclopenta[b]indol-3-yl)acetic acid |
| 117 | 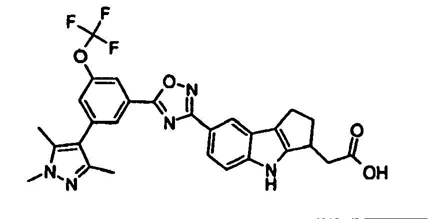 | 2-(7-(5-(3-(trifluoromethoxy)-5-(1,3,5-trimethyl-1*H*-pyrazol-4-yl)phenyl)-1,2,4-oxadiazol-3-yl)-1,2,3,4-tetrahydrocyclopenta[b]indol-3-yl)acetic acid |
| 118 | 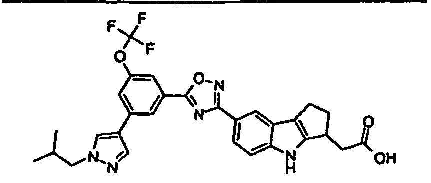 | 2-(7-(5-(3-(1-isobutyl-1*H* pyrazol-4-yl)-5-(trifluoromethoxy)phenyl)-1,2,4-oxadiazol-3-yl)-1,2,3,4-tetrahydrocyclopenta[b]indol-3-yl)acetic acid |
| 119 | 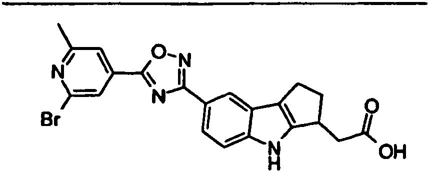 | 2-(7-(5-(2-bromo-6-methylpyridin4-yl)-1,2,4-oxadiazol-3-yl)-1,2,3,4-tetrahydrocyclopenta[b]indol-3-yl)acetic acid |
| 120 | 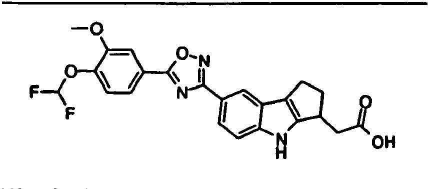 | 2-(7-(5-(4-(difluoromethoxy)-3-methoxyphenyl)-1,2,4-oxadiazol-3-yl)-1,2,3,4-tetrahydrocyclopenta[b]indol-3-yl)acetic acid |
| 121 | 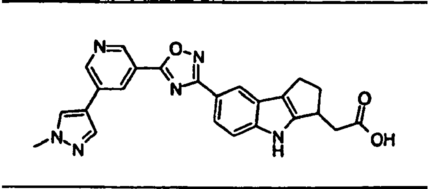 | 2-(7-(5-(5-(1-methyl-1*H*-pyrazol-4-yl)pyridin-3-yl)-1,2,4-oxadiazol-3-yl)-1,2,3,4-tetrahydrocyclopenta[b]indol-3-yl)acetic acid |
| 122 | 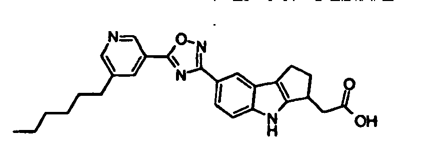 | 2-(7-(5-(5-hexylpyridin-3-yl)-1,2,4-oxadiazol-3-yl)-1,2,3,4-tetrahydrocyclopenta[b]indol-3-yl)acetic acid |

Additionally, individual compounds and chemical genera of the present invention, for example those compounds found in TABLE A including diastereomers and enantiomers thereof, encompass all pharmaceutically acceptable salts, solvates and particularly hydrates, thereof.

It is understood that the present invention embraces each diastereomer, each enantiomer and mixtures thereof of each compound and generic formulae disclosed herein just as if they were each individually disclosed with the specific stereochemical designation for each chiral carbon. Separation of the individual isomers (such as, by chiral HPLC, recrystallization of diastereomeric mixtures and the like) or selective synthesis (such as, by enantiomeric selective syntheses and the like) of the individual isomers is accomplished by application of various methods which are well known to practitioners in the art.

The compounds of the Formula **(Ia)** of the present invention may be prepared according to relevant published literature procedures that are used by one skilled in the art. Exemplary reagents and procedures for these reactions appear hereinafter in the working Examples. Protection and deprotection may be carried out by procedures generally known in the art (see, for example, Greene, T. W. and Wuts, P. G. M., Protecting Groups in Organic Synthesis, 3rd Edition, 1999 [Wiley]; incorporated herein by reference in its entirety).

### PHARMACEUTICAL COMPOSITIONS

A further aspect of the present invention pertains to pharmaceutical compositions comprising one or more compounds as described herein and one or more pharmaceutically acceptable carriers. Some embodiments pertain to pharmaceutical compositions comprising a compound of the present invention and a pharmaceutically acceptable carrier.

Some embodiments of the present invention include a method of producing a pharmaceutical composition comprising admixing at least one compound according to any of the compound embodiments disclosed herein and a pharmaceutically acceptable carrier.

Formulations may be prepared by any suitable method, typically by uniformly mixing the active compound(s) with liquids or finely divided solid carriers, or both, in the required proportions and then, if necessary, forming the resulting mixture into a desired shape.

Conventional excipients, such as binding agents, fillers, acceptable wetting agents, tabletting lubricants and disintegrants may be used in tablets and capsules for oral administration. Liquid preparations for oral administration may be in the form of solutions, emulsions, aqueous or oily suspensions and syrups. Alternatively, the oral preparations may be in the form of dry powder that can be reconstituted with water or another suitable liquid vehicle before use. Additional additives such as suspending or emulsifying agents, non-aqueous vehicles (including edible oils), preservatives and flavorings and colorants may be added to the liquid preparations. Parenteral dosage forms may be prepared by dissolving the compound of the invention in a suitable liquid vehicle and filter sterilizing the solution before filling and sealing an appropriate vial or ampule. These are just a few examples of the many appropriate methods well known in the art for preparing dosage forms.

A compound of the present invention can be formulated into pharmaceutical compositions using techniques well known to those in the art. Suitable pharmaceutically-acceptable carriers, outside those mentioned herein, are known in the art; for example, see Remington, The Science and Practice of Pharmacy, 20th Edition, 2000, Lippincott Williams & Wilkins, (Editors: Gennaro *et al*.)

While it is possible that, for use in the prophylaxis or treatment, a compound of the invention may, in an alternative use, be administered as a raw or pure chemical, it is preferable however to present the compound or active ingredient as a pharmaceutical formulation or composition further comprising a pharmaceutically acceptable carrier.

The invention thus further provides pharmaceutical formulations comprising a compound of the invention or a pharmaceutically acceptable salt, solvate, hydrate or derivative thereof together with one or more pharmaceutically acceptable carriers thereof and/or prophylactic ingredients. The carriers) must be "acceptable" in the sense of being compatible with the other ingredients of the formulation and not overly deleterious to the recipient thereof.

Pharmaceutical formulations include those suitable for oral, rectal, nasal, topical (including buccal and sub-lingual), vaginal or parenteral (including intramuscular, subcutaneous and intravenous) administration or in a form suitable for administration by inhalation, insufflation or by a transdermal patch. Transdermal patches dispense a drug at a controlled rate by presenting the drug for absorption in an efficient manner with a minimum of degradation of the drug. Typically, transdermal patches comprise an impermeable backing layer, a single pressure sensitive adhesive and a removable protective layer with a release liner. One of ordinary skill in the art will understand and appreciate the techniques appropriate for manufacturing a desired efficacious transdermal patch based upon the needs of the artisan.

The compounds of the invention, together with a conventional adjuvant, carrier, or diluent, may thus be placed into the form of pharmaceutical formulations and unit dosages thereof and in such form may be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles and such unit dosage forms may contain any suitable effective amount of the active ingredient commensurate with the intended daily dosage range to be employed.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable pharmaceutically acceptable carrier.

Compounds of the present invention or a salt, solvate, hydrate or physiologically functional derivative thereof can be used as active ingredients in pharmaceutical compositions, specifically as S1P1 receptor modulators. The term "active ingredient" is defined in the context of a "pharmaceutical composition" and is intended to mean a component of a pharmaceutical composition that provides the primary pharmacological effect, as opposed to an "inactive ingredient" which would generally be recognized as providing no pharmaceutical benefit.

The dose when using the compounds of the present invention can vary within wide limits and as is customary and is known to the physician, it is to be tailored to the individual conditions in each individual case. It depends, for example, on the nature and severity of the illness to be treated, on the condition of the patient, on the compound employed or on whether an acute or chronic disease state is treated or prophylaxis is conducted or on whether further active compounds are administered in addition to the compounds of the present invention. Representative doses of the present invention include, but not limited to, about 0.001 mg to about 5000 mg, about 0.001 mg to about 2500 mg, about 0.001 mg to about 1000 mg, 0.001 mg to about 500 mg, 0.001 mg to about 250 mg, about 0.001 mg to 100 mg, about 0.001 mg to about 50 mg and about 0.001 mg to about 25 mg. Multiple doses may be administered during the day, especially when relatively large amounts are deemed to be needed, for example 2, 3 or 4 doses. Depending on the individual and as deemed appropriate from the patient's physician or caregiver it may be necessary to deviate upward or downward from the doses described herein.

The amount of active ingredient, or an active salt, solvate or hydrate or derivative thereof, required for use in treatment will vary not only with the particular salt selected but also with the route of administration, the nature of the condition being treated and the age and condition of the patient and will ultimately be at the discretion of the attendant physician or clinician. In general, one skilled in the art understands how to extrapolate *in vivo* data obtained in a model system, typically an animal model, to another, such as a human. In some circumstances, these extrapolations may merely be based on the weight of the animal model in comparison to another, such as a mammal, preferably a human, however, more often, these extrapolations are not simply based on weights, but rather incorporate a variety of factors. Representative factors include the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized, whether an acute or chronic disease state is being treated or prophylaxis is conducted or whether further active compounds are administered in addition to the compounds of the present invention and as part of a drug combination. The dosage regimen for treating a disease condition with the compounds and/or compositions of this invention is selected in accordance with a variety factors as cited above. Thus, the actual dosage regimen employed may vary widely and therefore may deviate from a preferred dosage regimen and one skilled in the art will recognize that dosage and dosage regimen outside these typical ranges can be tested and, where appropriate, may be used in the methods of this invention.

The desired dose may conveniently be presented in a single dose or as divided doses administered at appropriate intervals, for example, as two, three, four or more sub-doses per day. The sub-dose itself may be further divided, *e.g.*, into a number of discrete loosely spaced administrations. The daily dose can be divided, especially when relatively large amounts are administered as deemed appropriate, into several, for example 2, 3 or 4 part administrations. If appropriate, depending on individual behavior, it may be necessary to deviate upward or downward from the daily dose indicated.

For preparing pharmaceutical compositions from the compounds of the present invention, the suitable pharmaceutically acceptable carrier can be either solid, liquid or a mixture of both. Solid form preparations include powders, tablets, pills, capsules, cachets, suppositories and dispersible granules. A solid carrier can be one or more substances which may also act as diluents, flavoring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable proportions and compacted to the desired shape and size.

The powders and tablets may contain varying percentage amounts of the active compound. A representative amount in a powder or tablet may contain from 0.5 to about 90 percent of the active compound; however, an artisan would known when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethylcellulose, a low melting wax, cocoa butter and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds according to the present invention may thus be formulated for parenteral administration (*e.g.* by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, *e.g.* sterile, pyrogen-free water, before use.

Aqueous formulations suitable for oral use can be prepared by dissolving or suspending the active component in water and adding suitable colorants, flavors, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well-known suspending agents.

Also included are solid form preparations which are intended to be converted, shortly before use, to liquid form preparations for oral administration. Such liquid forms include solutions, suspensions and emulsions. These preparations may contain, in addition to the active component, colorants, flavors, stabilizers, buffers, artificial and natural sweeteners, dispersants, thickeners, solubilizing agents and the like.

For topical administration to the epidermis the compounds according to the invention may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant. If the compounds of the present invention or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds of the present invention as an aerosol can be prepared by processes well known to the person skilled in the art. For their preparation, for example, solutions or dispersions of the compounds of the present invention or a pharmaceutically acceptable salt, solvate, hydrate or derivative thereof in water, water/alcohol mixtures or suitable saline solutions can be employed using customary additives, for example benzyl alcohol or other suitable preservatives, absorption enhancers for increasing the bioavailability, solubilizers, dispersants and others and, if appropriate, customary propellants, for example include carbon dioxide, CFCs, such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively the active ingredients may be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, *e.g.*, gelatin, or blister packs from which the powder may be administered by means of an inhaler.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

The compounds according to the invention may optionally exist as pharmaceutically acceptable salts including pharmaceutically acceptable acid addition salts prepared from pharmaceutically acceptable non-toxic acids including inorganic and organic acids. Representative acids include, but are not limited to, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfiric, tartaric, oxalic, p-toluenesulfonic and the like, such as those pharmaceutically acceptable salts listed *in* Journal of Pharmaceutical Sciences, 66:1-19 (1977), incorporated herein by reference in its entirety.

The acid addition salts may be obtained as the direct products of compound synthesis. In the alternative, the free base may be dissolved in a suitable solvent containing the appropriate acid and the salt isolated by evaporating the solvent or otherwise separating the salt and solvent. The compounds of this invention may form solvates with standard low molecular weight solvents using methods known to the skilled artisan.

Compounds of the present invention can be converted to "pro-drugs." The term "pro-drugs" refers to compounds that have been modified with specific chemical groups known in the art and when administered into an individual undergo biotransformation to give the parent compound. Pro-drugs can thus be viewed as compounds of the invention containing one or more specialized non-toxic protective groups used in a transient manner to alter or to eliminate a property of the compound. In one general aspect, the "pro-drug" approach is utilized to facilitate oral absorption. A thorough discussion is provided in T. Higuchi and V. Stella, Pro-drugs as Novel Delivery Systems Vol. 14 of the A.C.S. Symposium Series; and in *Bioreversible* Carriers in Drug Design, ed. Edward B. Roche, American Pharmaceutical Association and Pergamon Press, 1987, both of which are hereby incorporated by reference in their entirety.

Some embodiments of the present invention include a method of producing a pharmaceutical composition for "combination-therapy" comprising admixing at least one compound according to any of the compound embodiments disclosed herein, together with at least one known pharmaceutical agent as described herein and a pharmaceutically acceptable carrier.

It is noted that when the S1P1 receptor agonists are utilized as active ingredients in a pharmaceutical composition, these are not intended for use only in humans, but in other non-human mammals as well. Indeed, recent advances in the area of animal health-care mandate that consideration be given for the use of active agents, such as S1P1 receptor agonists, for the treatment of a S1P1 receptor associated disease or disorder in companionship animals (*e.g.*, cats, dogs, *etc.*) and in livestock animals (*e.g.*, cows, chickens, fish, *etc.*) Those of ordinary skill in the art are readily credited with understanding the utility of such compounds in such settings.

### Hydrates and Solvates

It is understood that when the phrase "pharmaceutically acceptable salts, solvates and hydrates" is used when referring to a particular formula herein, it is intended to embrace solvates and/or hydrates of compounds of the particular formula, pharmaceutically acceptable salts of compounds of the particular formula as well as solvates and/or hydrates of pharmaceutically acceptable salts of compounds of the particular formula.

The compounds of the present invention can be administrated in a wide variety of oral and parenteral dosage forms. It will be apparent to those skilled in the art that the following dosage forms may comprise, as the active component, either a compound of the invention or a pharmaceutically acceptable salt or as a solvate or hydrate thereof. Moreover, various hydrates and solvates of the compounds of the invention and their salts will find use as intermediates in the manufacture of pharmaceutical compositions. Typical procedures for making and identifying suitable hydrates and solvates, outside those mentioned herein, are well known to those in the art; see for example, pages 202-209 of K.J. Guillory, "Generation of Polymorphs, Hydrates, Solvates, and Amorphous Solids," in: Polymorphism in Pharmaceutical Solids, ed. Harry G. Brittan, Vol. 95, Marcel Dekker, Inc., New York, 1999, incorporated herein by reference in its entirety. Accordingly, one aspect of the present invention pertains to hydrates and solvates of compounds of Formula **(Ia)** or Formula **(IIa)** and/or their pharmaceutical acceptable salts, as described herein, that can be isolated and characterized by methods known in the art, such as, thermogravimetric analysis (TGA), TGA-mass spectroscopy, TGA-Infrared spectroscopy, powder X-ray diffraction (XRPD), Karl Fisher titration, high resolution X-ray diffraction, and the like. There are several commercial entities that provide quick and efficient services for identifying solvates and hydrates on a routine basis. Example companies offering these services include Wilmington PharmaTech (Wilmington, DE), Avantium Technologies (Amsterdam) and Aptuit (Greenwich, CT).

### OTHER UTILITIES

Another object of the present invention relates to radio-labeled compounds of the present invention that would be useful not only in radio-imaging but also in assays, both *in vitro* and *in vivo*, for localizing and quantitating the S1P1 receptor in tissue samples, including human and for identifying S1P1 receptor ligands by inhibition binding of a radio-labeled compound. It is a further object of this invention to develop novel S1P1 receptor assays of which comprise such radio-labeled compounds.

The present invention embraces isotopically-labeled compounds of the present invention. Isotopically or radio-labeled compounds are those which are identical to compounds disclosed herein, but for the fact that one or more atoms are replaced or substituted by an atom having an atomic mass or mass number different from the atomic mass or mass number most commonly found in nature. Suitable radionuclides that may be incorporated in compounds of the present invention include but are not limited to ²H (also written as D for deuterium), ³H (also written as T for tritium) ¹¹C, ¹³C, ¹⁴C, ¹³N, ¹⁵N, ¹⁵O, ¹⁷O, ¹⁸O, ¹⁸F, ³⁵S, ³⁶Cl, ⁷⁵Br, ⁷⁶Br, ⁷⁷Br, ⁸²Br, ¹²³I, ¹²⁴I, ¹²⁵I and ¹³¹I. The radionuclide that is incorporated in the instant radio-labeled compounds will depend on the specific application of that radio-labeled compound. For example, for *in vitro* S1P1 receptor labeling and competition assays, compounds that incorporate ³H, ¹⁴C, ⁸²Br, ¹²⁵I, ¹³¹I or ³⁵S will generally be most useful. For radio-imaging applications ¹¹C, ¹⁸F, ¹²⁵I, ¹²³I, ¹²⁴I, ¹³¹I, ⁷⁵Br, ⁷⁶Br or ⁷⁷Br will generally be most useful.

It is understood that a "radio-labeled " or "labeled compound" is a compound of Formula **(Ia), (Ic), (Ie), (Ig), (Ii), (Ik), (Im), (Io), (Iq), (Ir), (It), (Iv), (Ix), (Iz), (IIa), (IIc), (IIe), (IIg), (IIi), (IIk), (IIm), (IIo)** and **(IIIa)** that has incorporated at least one radionuclide; in some embodiments the radionuclide is selected from the group consisting of ³H, ¹⁴C, ¹²⁵I, ³⁵S and ⁸²Br.

Certain isotopically-labeled compounds of the present invention are useful in compound and/or substrate tissue distribution assays. In some embodiments the radionuclide ³H and/or ¹⁴C isotopes are useful in these studies. Further, substitution with heavier isotopes such as deuterium (*i.e.*, ²H) may afford certain therapeutic advantages resulting from greater metabolic stability (*e.g.*, increased *in vivo* half-life or reduced dosage requirements) and hence may be preferred in some circumstances. Isotopically labeled compounds of the present invention can generally be prepared by following procedures analogous to those disclosed in the Drawings and Examples *infra*, by substituting an isotopically labeled reagent for a non-isotopically labeled reagent. Other synthetic methods that are useful are discussed *infra*. Moreover, it should be understood that all of the atoms represented in the compounds of the invention can be either the most commonly occurring isotope of such atoms or the scarcer radio-isotope or nonradioactive isotope.

Synthetic methods for incorporating radio-isotopes into organic compounds are applicable to compounds of the invention and are well known in the art. These synthetic methods, for example, incorporating activity levels of tritium into target molecules, are as follows:
A. Catalytic Reduction with Tritium Gas: This procedure normally yields high specific activity products and requires halogenated or unsaturated precursors.
B. Reduction with Sodium Borohydride [³H]: This procedure is rather inexpensive and requires precursors containing reducible functional groups such as aldehydes, ketones, lactones, esters and the like.
C. Reduction with Lithium Aluminum Hydride [³H]: This procedure offers products at almost theoretical specific activities. It also requires precursors containing reducible functional groups such as aldehydes, ketones, lactones, esters and the like.
D. Tritium Gas Exposure Labeling: This procedure involves exposing precursors containing exchangeable protons to tritium gas in the presence of a suitable catalyst.
E. *N*-Methylation using Methyl Iodide [³H]: This procedure is usually employed to prepare *O*-methyl or *N*-methyl (³H) products by treating appropriate precursors with high specific activity methyl iodide (³H). This method in general allows for higher specific activity, such as for example, about 70-90 Ci/mmol.

Synthetic methods for incorporating activity levels of ¹²⁵I into target molecules include:
A. Sandmeyer and like reactions: This procedure transforms an aryl amine or a heteroaryl amine into a diazonium salt, such as a diazonium tetrafluoroborate salt and subsequently to ¹²⁵I labeled compound using Na ¹²⁵I. A represented procedure was reported by Zhu, G-D. and co-workers in J. Org. Chem., 2002, 67, 943-948.
B. Ortho ¹²⁵Iodination of phenols: This procedure allows for the incorporation of ¹²⁵I at the ortho position of a phenol as reported by Collier, T. L. and co-workers in J. Labelled Compd. Radiopharm., 1999, 42, S264-S266.
C. Aryl and heteroaryl bromide exchange with ¹²⁵I: This method is generally a two step process. The first step is the conversion of the aryl or heteroaryl bromide to the corresponding tri-alkyltin intermediate using for example, a Pd catalyzed reaction [*i.e.* Pd(Ph₃P)₄] or through an aryl or heteroaryl lithium, in the presence of a tri-alkyltinhalide or hexaalkylditin [*e.g.*, (CH₃)₃SnSn(CH₃)₃]. A representative procedure was reported by Le Bas, M.-D. and co-workers in J. Labelled Compd. Radiopharm. 2001, 44, S280-S282.

A radiolabeled S1P1 receptor compound of Formula (Ia) can be used in a screening assay to identify/evaluate compounds. In general terms, a newly synthesized or identified compound (*i.e.*, test compound) can be evaluated for its ability to reduce binding of the "radio-labeled compound of Formula (**Ia**)" to the S1P1 receptor. Accordingly, the ability of a test compound to compete with the "radio-labeled compound of Formula (**Ia**)" for the binding to the S1P1 receptor directly correlates to its binding affinity.

The labeled compounds of the present invention bind to the S1P1 receptor. In one embodiment the labeled compound has an IC₅₀ less than about 500 µM, in another embodiment the labeled compound has an IC₅₀ less than about 100 µM, in yet another embodiment the labeled compound has an IC₅₀ less than about 10 µM, in yet another embodiment the labeled compound has an IC₅₀ less than about 1 µM and in still yet another embodiment the labeled inhibitor has an IC₅₀ less than about 0.1 µM.

Other uses of the disclosed receptors and methods will become apparent to those in the art based upon, *inter alia*, a review of this disclosure.

As will be recognized, the steps of the methods of the present invention need not be performed any particular number of times or in any particular sequence. Additional objects, advantages and novel features of this invention will become apparent to those skilled in the art upon examination of the following examples thereof, which are intended to be illustrative and not intended to be limiting.

### EXAMPLES

### Example 1: Syntheses of compounds of the present invention.

Illustrated syntheses for compounds of the present invention are shown in Figures 7 through 12 where the symbols have the same definitions as used throughout this disclosure.

The compounds of the invention and their syntheses are further illustrated by the following examples. The following examples are provided to further define the invention without, however, limiting the invention to the particulars of these examples. The compounds described herein, *supra* and *infra*, are named according to the AutoNom version 2.2, or CS ChemDraw Ultra Version 9.0.7. In certain instances common names are used and it is understood that these common names would be recognized by those skilled in the art.

**Chemistry:** Proton nuclear magnetic resonance (¹H NMR) spectra were recorded on a Bruker Avance-400 equipped with a QNP (Quad Nucleus Probe) or a BBI (Broad Band Inverse) and z-gradient. Chemical shifts are given in parts per million (ppm) with the residual solvent signal used as reference. NMR abbreviations are used as follows: s = singlet, d = doublet, dd = doublet of doublets, ddd = doublet of doublet of doublets, dt = doublet of triplets, t = triplet, td = triplet of doublets, tt = triplet of triplets, q = quartet, m = multiplet, bs = broad singlet, bt = broad triplet. Microwave irradiations were carried out using a Smith Synthesizer™ or an Emrys Optimizer™ (Biotage). Thin-layer chromatography (TLC) was performed on silica gel 60 F₂₅₄ (Merck), preparatory thin-layer chromatography (prep TLC) was preformed on PK6F silica gel 60 A 1 mm plates (Whatman) and column chromatography was carried out on a silica gel column using Kieselgel 60, 0.063-0.200 mm (Merck). Evaporation was done under reduced pressure on a Büchi rotary evaporator.

LCMS spec: HPLC-pumps: LC-10AD *VP*, Shimadzu Inc.; HPLC system controller: SCL-10A *VP*, Shimadzu Inc; UV-Detector: SPD-10A *VP*, Shimadzu Inc; Autosampler: CTC HTS, PAL, Leap Scientific; Mass spectrometer: API 150EX with Turbo Ion Spray source, AB/MDS Sciex; Software: Analyst 1.2.

### Example 1.1: Preparation of Ethyl 2-(7-(N-Hydroxycarbamimidoyl)-1,2,3,4-tetrahydro-cyclopenta[b]indol-3-yl)acetate.

### Step A: Preparation of 4-Amino-3-iodobenzonitrile.

To a solution of 4-aminobenzonitrile (50.0 g, 423 mmol) in DMA (250 mL) at room temperature was added 1-iodopyrrolidine-2,5-dione (99.0 g, 440 mmol). The resulting mixture was warmed to 45 °C and stirred for 36 h. The reaction mixture was poured into ice-water (3.5 L) with vigorous stirring. The product precipitated out. After the ice melted, the precipitate was collected by filtration. The filter cake was rinsed with water (3 x 150 mL) and dried on the funnel. The dark brown solid was suspended in saturated solution of sodium sulfite (1.5 L) and the mixture was refluxed for 1 h. The suspension was filtered while still hot. The filter cake was washed with warm water (3 x 150 mL) and dried under reduced pressure at 40 °C overnight to afford the title compound as a light brown powder (94.92 g). LCMS *m*/*z* = 245.1 [M + H]⁺; ¹H NMR (400 MHz, CDCl₃) δ ppm 4.58 (s, 2H), 6.64 (d, *J* = 8.37 Hz, 1H), 7.33 (dd, *J* = 8.37, 1.74 Hz, 1H), 7.83 (d, *J* = 1.74 Hz, 1H).

### Step B: Preparation of Ethyl 1-(2-Ethoxy-2-oxoethyl)-2-oxocyclopentanecarboxylate.

To a solution of ethyl 2-oxocyclopentanecarboxylate (93.27 g, 597 mmol) and ethyl 2-bromoacetate (144.64 g, 866 mmol) in acetone (1.2 L) was added K₂CO₃ (165 g, 1194 mmol). The mixture was heated at 56 °C for 24 h. The solid was filtered off and the filter cake was washed with acetone (3 x 100 mL). The filtrate was concentrated and the resulting crude liquid was passed through a silica gel plug to afford the title compound as a slightly yellowish liquid (54.7 g). LCMS *m*/*z* = 243.3 [M + H]⁺; ¹H NMR (400 MHz, CDCl₃) δ ppm 1.23 (t, *J* = 7.14 Hz, 3H), 1.24 (t, *J* = 7.14 Hz, 3H), 1.95- 2.03 (m, 1H), 2.06-2.15 (m, 2H), 2.35-2.50 (m, 2H), 2.55-2.60 (m, 1H), 2.80 (dd, *J* = 15.2, 2.09 Hz, 1H), 2.95 (dd, *J* = 15.2, 2.09 Hz, 1H), 4.09 (q, *J* = 7.14 Hz, 2H), 4.12 (q, *J* = 7.14 Hz, 2H).

### Step C: Preparation of 2-(2-Oxocyclopentyl)acetic Acid.

A solution of ethyl 1-(2-ethoxy-2-oxoethyl)-2-oxocyclopentanecarboxylate (50.0 g, 206 mmol) in AcOH (500 mL) and 6 M HCl (250 mL) was heated at 100 °C for 6 h. The solvent was removed under reduced pressure and the residue was partitioned between EtOAc (500 mL) and H₂O (200 mL). The aqueous layer was separated and extracted with EtOAc (2 x 250 mL). The combined organic layers were washed with H₂O (300 mL) and brine (300 mL), dried over Na₂SO₄, decanted, and concentrated to yield the title compound as a white solid (22 g). ¹H NMR (400 MHz, CDCl₃) δ ppm 1.59-1.72 (m, 1H), 1.75-1.90 (m, 1H), 2.03-2.10 (m, 1H), 2.10-2.25 (dd, *J* = 10.98, 8.9 Hz, 1H), 2.30-2.40 (m, 2H), 2.40-2.50 (m, 2H), 2.80 (dd, *J* = 15.7, 7.2 Hz, 1H), 11.5 (s, 1H).

### Step D: Preparation of Ethyl 2-(2-Oxocyclopentyl)acetate.

To a solution of 2-(2-oxocyclopentyl)acetic acid (23.6 g, 166 mmol) in absolute ethanol (400 mL) was added H₂SO₄ (16.28 g, 166 mmol). The resulting solution was refluxed overnight. The reaction mixture was concentrated and ice-water (200 mL) was added. The aqueous mixture was extracted with DCM (3 x 200 mL). The combined organic layers were washed with H₂O (300 mL) and brine (300 mL), dried over Na₂SO₄, decanted, concentrated, and dried under reduced pressure to afford the title compound as a slightly yellowish liquid (27.2 g). LCMS *m*/*z* = 171.3 [M + H]⁺; ¹H NMR (400 MHz, CDCl₃) δ ppm 1.19 (t, *J* = 7.14 Hz, 3H), 1.50-1.62 (m, 1H), 1.65-1.80 (m, 1H), 1.92-2.02 (m, 1H), 2.08-2.17 (dd, *J* = 16.7, 8.86 Hz, 1H), 2.19-2.29 (m, 2H), 2.30-2.44 (m, 2H), 2.65 (dd, *J* = 15.12, 2.6 Hz, 1H), 4.07 (q, *J* = 7.14 Hz, 2H).

### Step E: Preparation of Ethyl 2-(7-Cyano-1,2,3,4-tetrahydrocyclopenta[b]indol-3-yl)acetate.

To a 500 mL three-necked round bottom flask containing DMF (195 mL) (deoxygenated with N₂ and slight vacuum) were sequentially added 4-amino-3-iodobenzonitrile (12.2 g, 50.0 mmol), pyridine 4-methylbenzenesulfonate (0.477 g, 1.9 mmol), tetraethoxysilane (13.23 g, 63.5 mmol), and ethyl 2-(2-oxocyclopentyl)acetate (12.76 g, 75.0 mmol) under N₂. The reaction mixture was heated at 135 °C in the dark overnight. To the mixture were added *N-*ethyl-*N*-isopropylpropan-2-amine (26.2 mL, 150 mmol) and palladium (**II**) acetate (0.337 g, 1.5 mmol) under N₂. The resulting mixture was cooled to 120 °C and stirred for additional 16 h. The reaction mixture was cooled to room temperature and acidified to pH ~ 5 with 1 N aq. HCl. The solvent was evaporated under reduced pressure at 60 °C. To the resulting liquid residue was added water (300 mL) and EtOAc (500 mL). The organic layer was separated and the aqueous layer was extracted with EtOAc (2 x 400 mL). The combined organic layers were dried over Na₂SO₄, decanted and concentrated under reduced pressure to afford a dark brown liquid (22.1 g). The crude liquid was passed through a silica gel plug and eluted with 10%-40% EtOAc in hexanes. Fractions were concentrated and further purified by precipitation in DCM/hexanes to yield the title compound as a light orange solid (4.65 g). LCMS *m*/*z*= 269.2 [M + H]⁺; ¹H NMR (400 MHz, DMSO-*d₆*) δ ppm 1.19 (t, *J* = 7.12 Hz, 3H), 2.05-2.18 (m, 1H), 2.54 (dd, *J* = 16.44, 8.45 Hz, 1H), 2.64-2.85 (m, 4H), 3.52-3.63 (m, 1H), 4.12 (q, *J* = 7.12 Hz, 2H), 7.34 (dd, *J* = 8.45, 1.58 Hz, 1H), 7.49 (d, *J* = 8.45, 1H), 7.85 (d, *J* = 0.81, 1H), 11.30 (s, 1H).

### Step F: Preparation of Ethyl 2-(7-(N-Hydroxycarbamimidoyl)-1,2,3,4-tetrahydrocyclopenta[b]indol-3-yl)acetate.

To a suspension of ethyl 2-(7-cyano-1,2,3,4-tetrahydrocyclopenta[b]indol-3-yl)acetate (4.6 g, 17.1 mmol) in ethanol (117 mL) in a 200 mL round bottom flask was added 50% aqueous solution of hydroxylamine (10.5 mL, 171 mmol). The resulting mixture was stirred in a closed flask at 70 °C for 4 h. The mixture was cooled in an ice-bath and then poured into 250 mL of ice-water. The resulting solution was carefully concentrated at water bath temperature (< 22 °C) to ~ 120 mL. The aqueous suspension was extracted with DCM/IPA (3/1, 3 x 250 mL), dried with Na₂SO₄, decanted, and concentrated to give the title compound as a light orange solid (5.02 g). LCMS *m*/*z* = 302.4 [M + H]⁺; ¹H NMR (400 MHz, DMSO-*d₆*) δ ppm 1.20 (t, *J* = 7.08 Hz, 3H), 2.08-2.11 (m, 1H), 2.47 (dd, *J* = 8.8, 14.7 Hz, 1H), 2.64-2.85 (m, 4H), 3.45-3.58 (m, 1H), 4.12 (q, *J* = 7.08 Hz, 2H), 5.67 (s, 2H), 7.27 *(d, J =* 8.56 Hz, 1H), 7.37 (dd, *J* = 1.56, 8.56 Hz, 1H), 7.65 (s, 1H), 9.32 (s, 1H), 10.74 (s, 1H).

### Example 1.2: Preparation of 2-(7-(5-(6-Ethoxy-5-methylpyridin-3-yl)-1,2,4-oxadiazol-3-yl)-1,2,3,4-tetrahydrocyclopenta[b]indol-3-yl)acetic Acid (Compound 62).

### Step A: Preparation of 6-Fluoro-5-methylnicotinoyl Chloride.

To a cooled (0 °C) mixture of 6-fluoro-5-methylnicotinic acid (0.776 g, 5.00 mmol) in DCM (10.00 mL) was added oxalyl chloride (0.481 mL, 5.50 mmol) followed by the addition of one drop of DMF. The reaction mixture was stirred for 2 h while warming to room temperature. It was concentrated under reduced pressure to give the title compound as a clear oil (0.821 g) without further purification.

### Step B: Preparation of Ethyl 2-(7-(5-(6-fluoro-5-methylpyridin-3-yl)-1,2,4-oxadiazol-3-yl)-1,2,3,4-tetrahydrocyclopenta[b]indol-3-yl)acetate.

To a solution of ethyl 2-(7-(*N*-hydroxycarbamimidoyl)-1,2,3,4-tetrahydrocyclopenta[b]indol-3-yl)acetate (0.360 g, 1.195 mmol) in THF (7.20 mL) was added 6-fluoro-5-methylnicotinoyl chloride (0.207 g, 1.195 mmol) followed by the addition of TEA (0.58 mL, 4.18 mmol). The resulting mixture was stirred for 30 min, heated to reflux for 18 h, and concentrated under reduced pressure. The residue was taken up in EtOAc, washed with water, NaHCO₃, and brine. The organics were dried over MgSO₄ and concentrated. The residue was purified via column chromatography (1:3 EtOAc/hexanes) to afford the title compound as a white solid (0.313 g). LCMS *m*/*z* = 421.2 [M + H]⁺; ¹H NMR (400 MHz, CDCl₃) δ ppm 1.31 (t, *J* = 7.20 Hz, 3H), 2.08-2.20 (m, 1H), 2.42 (s, 3H), 2.54 (dd, *J* = 16.93, 11.62 Hz, 1H), 2.72-3.01 (m, 4H), 3.53-3.65 (m, 1H), 4.15-4.31 (m, 2H), 7.41 (d, *J* = 8.34 Hz, 1H), 7.91 (dd, *J* = 8.59, 1.52 Hz, 1H), 8.29 (s, 1H), 8.43 (dd, *J* = 8.59, 1.77 Hz, 1H), 8.84 (s, 1H), 8.92 (s, 1H).

### Step C: Preparation of Ethyl 2-(7-(5-(6-Ethoxy-5-methylpyridin-3-yl)-1,2,4-oxadiazol-3-yl)-1,2,3,4-tetrahydrocyclopenta[b]indol-3-yl)acetate.

NaH (6.60 mg, 0.165 mmol) was added to ethanol (0.50 mL). The resulting solution was added to a solution of ethyl 2-(7-(5-(6-fluoro-5-methylpyridin-3-yl)-1,2,4-oxadiazol-3-yl)-1,2,3,4-tetrahydrocyclopenta[b]indol-3-yl)acetate (0.063 g, 0.15 mmol) in DMF (1.0 mL) and heated to 120 °C under microwave irradiation for 30 min. The mixture was taken up in EtOAc, washed with water (x 3) and brine. The organic layer was dried over MgSO₄ and concentrated to afford the title compound as a yellow solid (61.8 mg). LCMS *m*/*z* = 447.2 [M + H]⁺.

### Step D: Preparation of 2-(7-(5-(6-Ethoxy-5-methylpyridin-3-yl)-1,2,4-oxadiazol-3-yl)-1,2,3,4-tetrahydrocyclopenta[b]indol-3-yl)acetic Acid (Compound 62).

To a solution of ethyl 2-(7-(5-(6-ethoxy-5-methylpyridin-3-yl)-1,2,4-oxadiazol-3-yl)-1,2,3,4-tetrahydrocyclopenta[b]indol-3-yl)acetate (62 mg, 0.139 mmol) in dioxane (309 µL)/water (154 µL) was added NaOH (139 µL, 0.139 mmol). The mixture was stirred for 1 h, heated to 70 °C, and stirred overnight. Additional NaOH (139 µL, 0.139 mmol) was added the mixture was stirred for 3 h. The mixture was purified by preparative LCMS to afford the title compound as a white solid (16.7 mg). LCMS *m*/*z* = 419.5 [M + H]⁺; ¹H NMR (400 MHz, DMSO-*d₆*) δ ppm 1.38 (t, *J* = 7.07 Hz, 3H), 2.06-2.21 (m, 1H), 2.27 (s, 3H), 2.37-2.45 (m, 1H), 2.68-2.90 (m, 4H), 3.54 (bs, 1H), 4.47 (q, *J* = 6.91 Hz, 2H), 7.49 (d, *J* = 8.59 Hz, 1H), 7.74 (dd, *J* = 8.59, 1.52 Hz, 1H), 8.12 (s, 1H), 8.31 (d, *J* = 1.26 Hz, 1H), 8.82 (d, *J* = 2.27 Hz, 1H), 11.08 (s, 1H), 12.27 (s, 1H).

### Example 1.3: Preparation of 2-(7-(5-(6-Isopropoxy-5-methylpyridin-3-yl)-1,2,4-oxadiazol-3-yl)-1,2,3,4-tetrahydrocyclopenta[b]indol-3-yl)acetic Acid (Compound 48).

To a solution of ethyl 2-(7-(5-(6-fluoro-5-methylpyridin-3-yl)-1,2,4-oxadiazol-3-yl)-1,2,3,4-tetrahydrocyclopenta[b]indol-3-yl)acetate (63.1 mg, 0.15 mmol) in DMF (1500 µL) was added IPA (116 µL, 1.500 mmol) followed by KO*^{t}*Bu (33.7 mg, 0.300 mmol). The reaction mixture was stirred for 18 h. Water (100 µL) was added, and let stir for 24 h. The mixture was purified by preparative LCMS to afford the title compound as a white solid (2.2 mg). LCMS *m*/*z* = 433.4 [M + H]⁺; ¹H NMR (400 MHz, CDCl₃) δ ppm 1.40 (d, *J* = 6.06 Hz, 6H), 2.11-2.20 (m, *J* = 5.81 Hz, 1H), 2.26 (s, 3H), 2.57-2.65 (m, 2H), 2.76-3.00 (m, 4H), 3.62 (bs, 1H), 5.40-5.50 (m, 1H), 7.40 (d, *J* = 8.59Hz, 1H), 7.92 (dd, *J* = 8.46, 1.64 Hz, 1H), 8.16 (d, *J* = 1.26 Hz, 1H), 8.30 (s, 1H), 8.68 (s, 1H), 8.85 (d, *J* = 2.27 Hz, 1H).

### Example 1.4: Preparation of 2-(7-(5-(6-Methoxy-5-methylpyridin-3-yl)-1,2,4-oxadiazol-3-yl)-1,2,3,4-tetrahydrocyclopenta[b]indol-3-yl)acetic Acid (Compound 58).

### Step A: Preparation of Ethyl 2-(7-(5-(6-methoxy-5-methylpyridin-3-yl)-1,2,4-oxadiazol-3-yl)-1,2,3,4-tetrahydrocyclopenta[b]indol-3-yl)acetate.

To a solution of ethyl 2-(7-(5-(6-fluoro-5-methylpyridin-3-yl)-1,2,4-oxadiazol-3-yl)-1,2,3,4-tetrahydrocyclopenta[b]indol-3-yl)acetate (0.063 g, 0.15 mmol) in DMF (1.500 mL) was added MeOH (0.061 mL, 1.500 mmol) followed by KO*^{t}*Bu (0.034 g, 0.300 mmol). The reaction mixture was stirred for 30 min and concentrated under reduced pressure. The residue was taken up in EtOAc and washed with water (x 3) and brine. The organics were dried over MgSO₄ and concentrated to afford the title compound as a white solid (55.3 mg). LCMS *m*/*z* = 433.5 [M + H]⁺.

### Step B: Preparation of 2-(7-(5-(6-Methoxy-5-methylpyridin-3-yl)-1,2,4-oxadiazol-3-yl)-1,2,3,4-tetrahydrocyclopenta[b]indol-3-yl)acetic Acid (Compound 58).

From ethyl 2-(7-(5-(6-methoxy-5-methylpyridin-3-yl)-1,2,4-oxadiazol-3-yl)-1,2,3,4-tetrahydrocyclopenta[b]indol-3-yl)acetate, using a similar method to the one described in **Example 1.2, Step D,** the title compound was obtained as a white solid. LCMS *m*/*z* = 405.5 [M + H]⁺; ¹H NMR (400 MHz, DMSO-*d₆*) δ ppm 2.09-2.20 (m, 1H), 2.28 (s, 3H), 2.38-2.47 (m, 1H), 2.68-2.93 (m, 4H), 3.54 (bs, 1H), 4.01 (s, 3H), 7.49 (d, *J* = 8.59 Hz, 1H), 7.74 (dd, *J* = 8.59, 1.52 Hz, 1H), 8.11 (s, 1H), 8.32 (d, *J* = 1.52 Hz, 1H), 8.84 (d, *J* = 2.27 Hz, 1H), 11.08 (s, 1H), 12.27 (s, 1H).

### Example 1.5: Preparation of 2-(7-(5-(6-sec-Butoxy-5-methylpyridin-3-yl)-1,2,4-oxadiazol-3-yl)-1,2,3,4-tetrahydrocyclopenta[b]indol-3-yl)acetic Acid (Compound 1).

NaH (18.00 mg, 0.450 mmol) was added to a solution of (*S*)-(+)-sec-butanol (139 µL, 1.500 mmol) in DMF (500 µL). The resulting solution was added to a solution of ethyl 2-(7-(5-(6-fluoro-5-methylpyridin-3-yl)-1,2,4-oxadiazol-3-yl)-1,2,3,4-tetrahydrocyclopenta[b]indol-3-yl)acetate (63.1 mg, 0.15 mmol) in DMF (1000 µL). The reaction mixture was stirred overnight, diluted with EtOAc, and washed with water (x 3) and brine. The organic layer was dried over MgSO₄ and concentrated to afford the title compound as a light grey solid (4.8 mg). LCMS *m*/*z* = 447.4 [M + H]⁺; ¹H NMR (400 MHz, CDCl₃) δ ppm 0.99 (t, *J* = 7.45 Hz, 3H), 1.36 (d, *J* = 6.32 Hz, 2H), 1.66-1.87 (m, 3H), 2.13-2.20 (m, 1H), 2.27 (s, 3H), 2.57-2.66 (m, 1H), 2.76-3.01 (m, 4H), 3.62 (bs, 1H), 5.24-5.33 (m, 1H), 7.40 (d, *J* = 8.34 Hz, 1H), 7.93 (dd, *J* = 8.59, 1.77 Hz, 1H), 8.17 (d, *J* = 2.27 Hz, 1H), 8.30 (s, 1H), 8.66 (s, 1H), 8.85 (d, *J* = 2.27 Hz, 1H).

### Example 1.6: Preparation of 2-(7-(5-(3-Cyano-4-(1,1,1,3,3,3-hexafluoropropan-2-yloxy)phenyl)-1,2,4-oxadiazol-3-yl)-1,2,3,4-tetrahydrocyclopenta[b]indol-3-yl)acetic Acid (Compound 27).

### Step A: Preparation of 5-Formyl-2-(1,1,1,3,3,3-hexafluoropropan-2-yloxy)benzonitrile.

To a solution of 1,1,1,3,3,3-hexafluoropropan-2-ol (2.82 g, 16.76 mmol) in DMA (8 mL) at 0 °C sodium hydride (0.660 g, 27.5 mmol) was added slowly and the reaction mixture was warmed to room temperature and stirred for 30 min. 2-Fluoro-5-formylbenzonitrile (1.0 g, 6.71 mmol) was added and the mixture was stirred at room temperature overnight. The mixture was purified by silica flash chromatography using 5%-25% EtOAc/hexanes to give the title compound as a yellow oil (0.860 g). LCMS *m*/*z* = 298.3 [M + H]⁺.

### Step B: Preparation of 3-Cyano-4-(1,1,1,3,3,3-hexafluoropropan-2-yloxy)benzoic Acid.

5-Formyl-2-(1,1,1,3,3,3-hexafluoropropan-2-yloxy)benzonitrile (0.440 g, 1.481 mmol) was dissolved in acetone (5 mL). To this mixture at 0 °C was added Jones reagent which was prepared by dissolving chromium (VI) oxide (0.270 g, 2.221 mmol) in H₂SO₄ (250 µL) and diluting with water (2 mL) at 0 °C. The mixture was removed from the ice-bath and allowed to stir at room temperature overnight. After removal of the solvent under reduced pressure, the residue was dissolved in EtOAc (20 mL), washed with brine (10 mL), and water (3 x 10 mL), and brine (10 mL). The organics were dried over magnesium sulfate, filtered and concentrated to afford the title compound as a white solid (0.401 g). LCMS *m*/*z* = 314.0 [M + H]⁺.

### Step C: Preparation of 3-Cyano-4-(1,1,1,3,3,3-hexafluoropropan-2-yloxy)benzoyl Chloride.

3-Cyano-4-(1,1,1,3,3,3-hexafluoropropan-2-yl)benzoic acid was dissolved in DCM and DMF (one drop) was added followed by oxalyl chloride (3.0 equivalent). The mixture was stirred at room temperature for 1 h. The yellow solution was concentrated, and the resulting residue was used in the next step without further purification.

### Step D: Preparation of 2-(7-(5-(3-Cyano-4-(1,1,1,3,3,3-hexafluoropropan-2-yloxy)phenyl)-1,2,4-oxadiazol-3-yl)-1,2,3,4-tetrahydrocyclopenta[b]indol-3-yl)acetic Acid (Compound 27).

To a solution of 2-(7-(*N*-hydroxycarbamimidoyl)-1,2,3,4-tetrahydrocyclopenta[b]indol-3-yl)acetic acid (0.045 g, 0.165 mmol) and triethylamine (0.091 mL, 0.659 mmol) in dioxane (4 mL) was added a solution of 3-cyano-4-(1,1,1,3,3,3-hexafluoropropan-2-yloxy)benzoyl chloride (0.164 g, 0.494 mmol) in dioxane (2 mL) at 0 °C. The mixture was removed from ice and allowed to stir at room temperature for 20 min. The solvent was removed under reduced pressure. The residue was taken up in DMA (4 mL) and heated at 100 °C for 1 h. DMA was evaporated and the residue was purified by preparative HPLC. Fractions with pure material were combined and the product extracted with EtOAc and brine. The organic layers were combined, dried over sodium sulfate, filtered and concentrated to give the title compound as a purple solid (57 mg). LCMS *m*/*z* = 551.5 [M + H]⁺; ¹H NMR (400 MHz, DMSO-*d₆*) δ ppm 2.14-2.19 (m, 1H), 2.42-2.50 (m, 2H), 3.72-3.88 (m, 4H), 7.03-7.11 (m, 1H), 7.54 (d, *J* = 8.59 Hz, 1H), 7.75 (dd, *J* = 8.59 Hz, 1.52 Hz, 1H), 7.92 (d, *J* = 8.59 Hz, 1H), 8.13 (s, 1H), 8.63 (dd, *J* = 8.84 Hz, 2.02 Hz, 1H), 8.72 (d, *J* = 2.02 Hz, 1H), 11.12 (s, 1H), 12.28 (bs, 1H).

### Example 1.7: Preparation of 2-(7-(5-(3-Cyano-4-methoxyphenyl)-1,2,4-oxadiazol-3-yl)-1,2,3,4-tetrahydrocyclopenta[b]indol-3-yl)acetic Acid (Compound 10).

### Step A: Preparation of Methyl 3-Bromo-4-methoxybenzoate.

To a solution of 3-bromo-4-hydroxybenzoic acid (2.50 g, 11.52 mmol), Cs₂CO₃ (8.26 g, 25.3 mmol) in DMF (25 mL) was added iodomethane (2.87 mL, 46.1 mmol). The mixture was heated to 50 °C for 16 h. After removal of the solvent under reduced pressure, the residue was taken up in EtOAc and poured into 1 M HCl. After extraction with EtOAc (3 x 50 mL), the combined organic layers were washed with saturated NaCl (50 mL), dried over MgSO₄, and concentrated to give the title compound (2.70 g) without further purification. LCMS *m*/*z* = 245.1 [M + H]⁺.

### Step B: Preparation of 3-Bromo-4-methoxybenzoic Acid.

To a solution of 3-bromo-4-methoxybenzoate (2.70 g, 11.02 mmol) in 1:1 THF:MeOH was added LiOH_{(aq)} (1.0 M, 11.52 mmol). The reaction mixture was stirred for 16 h at 40 °C, poured into 1 M HCl and extracted with EtOAc (3 x 40 mL). The organics were washed with saturated NaCl (40 mL), dried over Na₂SO₄, and concentrated under reduced pressure to give the title compound as a white solid (2.52 g) without further purification. LCMS *m*/*z* = 231.3.

### Step C: Preparation of 3-Cyano-4-methoxybenzoic Acid.

3-Bromo-4-methoxybenzoic acid (2.52 g, 10.91 mmol), CuCN (1.270 g, 14.18 mmol) and anhydrous NMP (20 mL) were heated to 200 °C for 3 h in a heavy walled sealed tube under microwave irradiation. The mixture was poured into 1 M HCl and extracted with EtOAc (3 x 25 mL). The organics were washed with saturated NaCl (25 mL), dried over MgSO₄, and concentrated under reduced pressure. The residue was purified by preparative HPLC to give the title compound as a brown solid (1.34 g). LCMS *m*/*z* = 178.1 [M + H]⁺.

### Step D: Preparation of Ethyl 2-(7-(5-(3-Cyano-4-methoxyphenyl)-1,2,4-oxadiazol-3-yl)-1,2,3,4-tetrahydrocyclopenta[b]indol-3-yl)acetate.

To a solution of ethyl 2-(7-(*N*-hydroxycarbamimidoyl)-1,2,3,4-tetrahydrocyclopenta[b]indol-3-yl)acetate (0.099 g, 0.33 mmol) in EtOAc (2.0 mL) and 3-cyano-4-methoxybenzoic acid (0.058 g, 0.330 mmol) was added TEA (0.460 mL, 3.30 mmol) followed by 1-propylphosphonic acid cyclic anhydride (0.097 mL, 0.330 mmol). The reaction mixture was heated to reflux for 3 h. After cooling to room temperature, it was diluted with EtOAc, washed with water, NaHCO₃ and brine. The organics were dried over MgSO₄, filtered and concentrated. The residue was purified *via* silica column chromatography (1:3 EtOAc/hexanes) to afford the title compound as a white solid (54 mg). ¹H NMR (400 MHz, CDCl₃) δ ppm 1.27-1.34 (m, 3H), 2.07-2.21 (m, 1H), 2.46-2.60 (m, 1H), 2.70-2.89 (m, 4H), 3.48-3.67 (m, 1H), 4.06 (s, 3H), 4.16-4.28 (m, 2H), 7.15 (d, *J* = 9.09 Hz, 1H), 7.41 (d, *J* = 8.59 Hz, 1H), 7.91 (dd, *J* = 8.46, 1.64 Hz, 1H), 8.29 (s, 1H), 8.41 (dd, *J* = 8.84, 2.02 Hz, 1H), 8.48 (d, *J* = 2.02 Hz, 1H), 8.83 (s, 1H).

### Step E: Preparation of 2-(7-(5-(3-Cyano-4-methoxyphenyl)-1,2,4-oxadiazol-3-yl)-1,2,3,4-tetrahydrocyclopenta[b]indol-3-yl)acetic Acid (Compound 10).

From ethyl 2-(7-(5-(3-cyano-4-methoxyphenyl)-1,2,4-oxadiazol-3-yl)-1,2,3,4-tetrahydrocyclopenta[b]indol-3-yl)acetate, using a similar method to the one described in **Example 1.2, Step D,** the title compound was obtained as a white solid. LCMS *m*/*z* = 415.1 [M + H]⁺; ¹H NMR (400 MHz, DMSO-*d*₆) δ ppm 2.09-2.23 (m, 1H), 2.43 (dd, *J* = 16.17, 8.84 Hz, 1H), 2.70-2.89 (m, 4H), 3.54 (bs, 1H), 4.06 (s, 3H), 7.51 (dd, *J* = 11.24, 8.72 Hz, 2H), 7.74 (dd, *J =* 8.59, 1.52 Hz, 1H), 8.12 (d, *J* = 1.26 Hz, 1H), 8.47 (dd, *J* = 9.09, 2.27 Hz, 1H), 8.53 (d, *J* = 2.27 Hz, 1H), 11.08 (s, 1H), 12.27 (s, 1H).

### Example 1.8: Preparation of 2-(7-(5-(Pyridin-3-yl)-1,2,4-oxadiazol-3-yl)-1,2,3,4-tetrahydrocyclopenta[b]indol-3-yl)acetic Acid (Compound 39).

### Step A: Preparation of Ethyl 2-(7-(5-(pyridin-3-yl)-1,2,4-oxadiazol-3-yl)-1,2,3,4-tetrahydrocyclopenta[b]indol-3-yl)acetate.

From ethyl 2-(7-(*N*-hydroxycarbamimidoyl)-1,2,3,4-tetrahydrocyclopenta[b]indol-3-yl)acetate and nicotinic acid, using a similar method to the one described in **Example 1.7, Step D,** the title compound was obtained as a white solid. LCMS *m*/*z* = 389.4 [M + H]⁺.

### Step B: Preparation of 2-(7-(5-(Pyridin-3-yl)-1,2,4-oxadiazol-3-yl)-1,2,3,4-tetrahydrocyclopenta[b]indol-3-yl)acetic Acid (Compound 39).

From ethyl 2-(7-(5-(pyridin-3-yl)-1,2,4-oxadiazol-3-yl)-1,2,3,4-tetrahydrocyclopenta[b]indol-3-yl)acetate, using a similar method to the one described in **Example 1.2, Step D,** the title compound was obtained as a white solid. LCMS *m*/*z* = 361.3 [M + H]⁺; ¹H NMR (400 MHz, DMSO-*d*₆) δ ppm 2.08-2.21 (m, 1H), 2.43 (dd, *J* = 16.04, 8.97 Hz, 1H), 2.69-2.92 (m, 4H), 3.54 (bs, 1H), 7.51 (d, *J* = 8.59 Hz, 1H), 7.68-7.73 (m, 1H), 7.77 (dd, *J* = 8.46, 1.64 Hz, 1H), 8.14 (d, *J* = 1.26 Hz, 1H), 8.57 (dt, *J* = 8.08, 1.89 Hz, 1H), 8.89 (dd, *J* = 4.80, 1.52 Hz, 1H), 9.36 (d, *J* = 1.52 Hz, 1H), 11.09 (s, 1H), 12.26 (s, 1H).

### Example 1.9: Preparation of 2-(7-(5-(Pyridin-4-yl)-1,2,4-oxadiazol-3-yl)-1,2,3,4-tetrahydrocyclopenta[b]indol-3-yl)acetic Acid (Compound 41).

### Step A: Preparation of Ethyl 2-(7-(5-(Pyridin-4-yl)-1,2,4-oxadiazol-3-yl)-1,2,3,4-tetrahydrocyclopenta[b]indol-3-yl)acetate.

From ethyl 2-(7-(*N*-hydroxycarbamimidoyl)-1,2,3,4-tetrahydrocyclopenta[b]indol-3-yl)acetate and isonicotinic acid, using a similar method to the one described in **Example 1.7, Step D,** the title compound was obtained as a white solid. LCMS *m*/*z* = 389.3 [M + **H]⁺.**

### Step B: Preparation of 2-(7-(5-(Pyridin-4-yl)-1,2,4-oxadiazol-3-yl)-1,2,3,4-tetrahydrocyclopenta[b]indol-3-yl)acetic Acid (Compound 41).

From ethyl 2-(7-(5-(pyridin-4-yl)-1,2,4-oxadiazol-3-yl)-1,2,3,4-tetrahydrocyclopenta[b]indol-3-yl)acetate, using a similar method to the one described in **Example 1.2, Step D** (alternate purification: the mixture was neutralized with 1 M HCl and the resulting precipitate was collected), the title compound was obtained as an orange solid. LCMS *m*/*z* = 361.3 [M + H]⁺; ¹H NMR (400 MHz, DMSO-*d*₆) δ ppm 2.08-2.22 (m, 1H), 2.44 (dd, *J* = 16.17, 8.84 Hz, 1H), 2.69-2.90 (m, 4H), 3.55 (s, 1H), 7.51 (d, *J* = 8.59 Hz, 1H), 7.77 (dd, *J* = 8.46, 1.64 Hz, 1H), 8.08-8.18 (m, 3H), 8.92 (d, *J* = 5.56 Hz, 2H), 11.10 (s, 1H), 12.26 (s, 1H).

### Example 1.10: Preparation of 2-(7-(5-(pyrimidin-5-yl)-1,2,4-oxadiazol-3-yl)-1,2,3,4-tetrahydrocyclopenta[b]indol-3-yl)acetic Acid (Compound 56).

From ethyl 2-(7-(*N* hydroxycarbamimidoyl)-1,2,3,4-tetrahydrocyclopenta[b]indol-3-yl)acetate and pyrimidine-5-carboxylic acid, using a similar method to the one described in **Example 1.9,** the title compound was obtained as an orange solid. LCMS *m*/*z* = 362.4 [M + H]⁺; ¹H NMR (400 MHz, DMSO-*d*₆) δ ppm 2.08-2.21 (m, 1H), 2.43 (dd, *J* = 15.92, 8.84 Hz, 1H), 2.69-2.93 (m, 4H), 3.55 (s, 1H), 7.52 (d, *J* = 8.59 Hz, 1H), 7.77 (dd, *J* = 8.59, 1.52 Hz, 1H), 8.15 (d, *J* = 1.52 Hz, 1H), 9.49 (s, 1H), 9.55 (s, 2H), 11.11 (s, 1H).

### Example 1.11: Preparation of 2-(7-(5-(Pyridin-2-yl)-1,2,4-oxadiazol-3-yl)-1,2,3,4-tetrahydrocyclopenta[b]indol-3-yl)acetic Acid (Compound 61).

From ethyl 2-(7-(*N*-hydroxycarbamimidoyl)-1,2,3,4-tetrahydrocyclopenta[b]indol-3-yl)acetate and picolinic acid, using a similar method to the one described in **Example 1.8,** the title compound was obtained as a light yellow solid. LCMS *m*/*z* = 361.4 [M + H]⁺; ¹H NMR (400 MHz, DMSO-*d*₆) δ ppm 2.09-2.23 (m, 1H), 2.44 (dd, *J* = 16.04, 8.97 Hz, 1H), 2.69-2.92 (m, 4H), 3.55 (s, 1H), 7.51 (d, *J* = 8.59 Hz, 1H), 7.71-7.75 (m, 1H), 7.77 (dd, *J* = 8.46, 1.64 Hz, 1H), 8.10-8.14 (m, 1H), 8.14-8.16 (m, 1H), 8.37 (d, *J* = 7.83 Hz, 1H), 8.86 (d, *J* = 4.04 Hz, 1H), 11.08 (s, 1H), 12.27 (s, 1H).

### Example 1.12: Preparation of 2-(7-(5-(Pyridazin-4-yl)-1,2,4-oxadiazol-3-yl)-1,2,3,4-tetrahydrocyclopenta[b]indol-3-yl)acetic Acid (Compound 71).

From ethyl 2-(7-(*N*-hydroxycarbamimidoyl)-1,2,3,4-tetrahydrocyclopenta[b]indol-3-yl)acetate and pyrazine-4-carboxylic acid, using a similar method to the one described in Example 1.9, the title compound was obtained as an orange solid. LCMS *m*/*z* = 362.5 [M + H]⁺; ¹H NMR (400 MHz, DMSO-*d*₆) δ ppm 2.07-2.20 (m, 1H), 2.44 (dd, *J* = 16.17, 8.59 Hz, 1H), 2.63-2.94 (m, 4H), 3.55 (s, 1H), 7.52 (d, *J* = 8.59 Hz, 1H), 7.77 (dd, *J* = 8.46, 1.64 Hz, 1H), 8.15 (d, *J* = 1.26 Hz, 1H), 8.39 (dd, *J* = 5.31, 2.27 Hz, 1H), 9.60 (dd, *J* = 5.43, 1.14 Hz, 1H), 9.85-9.93 (m, 1H), 11.14 (s, 1H).

### Example 1.13: Preparation of 2-(7-(5-(pyridazin-3-yl)-1,2,4-oxadiazol-3-yl)-1,2,3,4-tetrahydrocyclopenta[b]indol-3-yl)acetic Acid (Compound 2).

From ethyl 2-(7-(*N*-hydroxycarbamimidoyl)-1,2,3,4-tetrahydrocyclopenta[b]indol-3-yl)acetate and pyradazine-3-carboxylic acid, using a similar method to the one described in **Example 1.8,** the title compound was obtained as an orange solid. LCMS *m*/*z* = 362.5 [M + H]⁺; ¹H NMR (400 MHz, DMSO-*d*₆) δ ppm 2.10-2.21 (m, 1H), 2.44 (dd, *J* = 16.17, 8.84 Hz, 1H), 2.71-2.90 (m, 4H), 3.55 (s, 1H), 7.53 (d, *J* = 8.59 Hz, 1H), 7.79 (dd, *J* = 8.59,1.52 Hz, 1H), 8.04 (dd, *J* = 8.59,5.05 Hz, 1H), 8.17 (d, *J* = 1.26 Hz, 1H), 8.57 (dd, *J* = 8.46, 1.64 Hz, 1H), 9.52 (dd, *J* = 5.18, 1.64 Hz, 1H), 11.15 (s, 1H), 12.26 (s, 1H).

### Example 1.14: Preparation of 2-(7-(5-(5-Bromopyridin-3-yl)-1,2,4-oxadiazol-3-yl)-1,2,3,4-tetrahydrocyclopenta[b]indol-3-yl)acetic Acid (Compound 70).

From ethyl 2-(7-(*N*-hydroxycarbamimidoyl)-1,2,3,4-tetrahydrocyclopenta[b]indol-3-yl)acetate and 5-bromonicotinic acid, using a similar method to the one described in **Example 1.8,** the title compound was obtained as a yellow solid. LCMS *m*/*z* = 439.2 [M + H]⁺; ¹H NMR (400 MHz, DMSO-*d*₆) δ ppm 2.10-2.20 (m, 1H), 2.43 (dd, *J* = 16.17, 9.09 Hz, 1H), 2.69-2.92 (m, 4H), 3.56 (s, 1H), 7.51 (d, *J* = 8.59 Hz, 1H), 7.77 (dd, *J* = 8.46, 1.64 Hz, 1H), 8.15 (d, *J* = 1.26 Hz, 1H), 8.78 (t, *J* = 2.15 Hz, 1H), 9.05 (d, *J* = 2.27 Hz, 1H), 9.33 (d, *J* = 1.77 Hz, 1H), 11.10 (s, 1H), 12.27 (s, 1H).

### Example 1.15: Preparation of 2-(7-(5-(Pyrazin-2-yl)-1,2,4-oxadiazol-3-yl)-1,2,3,4-tetrahydrocyclopenta[b]indol-3-yl)acetic Acid (Compound 29).

From ethyl 2-(7-(*N*-hydroxycarbamimidoyl)-1,2,3,4-tetrahydrocyclopenta[b]indol-3-yl)acetate and pyrazine-2-carboxylic acid, using a similar method to the one described in **Example 1.8,** the title compound was obtained as a yellow solid. LCMS *m*/*z* = 362.4 [M + H]⁺; ¹H NMR (400 MHz, DMSO-*d*₆) δ ppm 2.08-2.22 (m, 1H), 2.44 (dd, *J* = 16.17, 8.84 Hz, 1H), 2.70-2.90 (m, 4H), 3.55 (s, 1H), 7.52 *(d, J =* 8.34 Hz, 1H), 7.78 (dd, *J* = 8.46, 1.64 Hz, 1H), 8.16 (d, *J* = 1.52 Hz, 1H), 8.95 (dd, *J* = 2.53, 1.52 Hz, 1H), 8.98 (d, *J* = 2.53 Hz, 1H), 9.53 (d, *J* = 1.52 Hz, 1H), 11.11 (s, 1H), 12.27 (s, 1H).

### Example 1.16: Preparation of 2-(7-(5-(6-(2,2,2-Trifluoroethoxy)pyridin-3-yl)-1,2,4-oxadiazol-3-yl)-1,2,3,4-tetrahydrocyclopenta[b]indol-3-yl)acetic Acid (Compound 47).

From ethyl 2-(7-(*N*-hydroxycarbamimidoyl)-1,2,3,4-tetrahydrocyclopenta[b]indol-3-yl)acetate and 6-(2,2,2-trifluoroethoxy)nicotinic acid, using a similar method to the one described in **Example 1.8,** the title compound was obtained as a white solid. LCMS *m*/*z* = 459.4 [M + H]⁺; ¹H NMR (400 MHz, DMSO-*d*₆) δ ppm 2.10-2.20 (m, 1H), 2.43 (dd, *J* = 16.17, 8.84 Hz, 1H), 2.69-2.93 (m, 4H), 3.54 (s, 1H), 5.15 (q, *J* = 8.84 Hz, 2H), 7.27 (d, *J* = 8.84 Hz, 1H), 7.50 (d, *J* = 8.59 Hz, 1H), 7.75 (dd, *J* = 8.46, 1.64 Hz, 1H), 8.12 (d, *J* = 1.01 Hz, 1H), 8.54 (dd, *J* = 8.72, 2.40 Hz, 1H), 9.04 (d, *J* = 2.53 Hz, 1H), 11.08 (s, 1H), 12.27 (s, 1H).

### Example 1.17: Preparation of 2-(7-(5-(5-Butylpyridin-2-yl)-1,2,4-oxadiazol-3-yl)-1,2,3,4-tetrahydrocyclopenta[b]indol-3-yl)acetic Acid (Compound 54).

From ethyl 2-(7-(*N*-hydroxycarbamimidoyl)-1,2,3,4-tetrahydrocyclopenta[b]indol-3-yl)acetate and 5-butylpicolinic acid, using a similar method to the one described in **Example 1.8,** the title compound was obtained as a light yellow solid. LCMS *m*/*z* = 417.6 [M + H]⁺; ¹H NMR (400 MHz, DMSO-*d*₆) δ ppm 0.93 (t, *J* = 7.33 Hz, 3H), 1.28-1.42 (m, 2H), 1.59-1.68 (m, 2H), 2.07-2.21 (m, 1H), 2.43 (dd, *J* = 16.17, 9.09 Hz, 1H), 2.70-2.91 (m, 6H), 3.54 (s, 1H), 7.50 (d, *J* = 8.34 Hz, 1H), 7.76 (dd, *J* = 8.46, 1.64 Hz, 1H), 7.96 (dd, *J* = 8.21, 2.15 Hz, 1H), 8.14 (d, *J* = 1.26 Hz, 1H), 8.28 (d, *J* = 8.08 Hz, 1H), 8.71 (d, *J* = 2.02 Hz, 1H), 11.08 (s, 1H), 12.28 (s, 1H).

### Example 1.18: Preparation of 2-(7-(5-(6-(Tritluoromethyl)pyridin-3-yl)-1,2,4-oxadiazol-3-yl)-1,2,3,4-tetrahydrocyclopenta[b]indol-3-yl)acetic Acid (Compound 11).

### Step A: Preparation of Ethyl 2-(7-(5-(6-(Trifluoromethyl)pyridin-3-yl)-1,2,4-oxadiazol-3-yl)-1,2,3,4-tetrahydrocyclopenta[b]indol-3-yl)acetate.

From ethyl 2-(7-(*N*-hydroxycarbamimidoyl)-1,2,3,4-tetrahydrocyclopenta[b]indol-3-yl)acetate and 6-(trifluoromethyl)nicotinyl chloride, using a similar method to the one described in **Example 1.2, Step B,** the title compound was obtained as a light green solid. LCMS *m*/*z* = 457.1 [M + H]⁺.

### Step B: Preparation of 2-(7-(5-(6-(Trifluoromethyl)pyridin-3-yl)-1,2,4-oxadiazol-3-yl)-1,2,3,4-tetrahydrocyclopenta[b]indol-3-yl)acetic Acid (Compound 11).

From ethyl 2-(7-(5-(6-(trifluoromethyl)pyridin-3-yl)-1,2,4-oxadiazol-3-yl)-1,2,3,4-tetrahydrocyclopenta[b]indol-3-yl)acetate, using a similar method to the one described in **Example 1.2, Step D,** the title compound was obtained as a white solid. LCMS *m*/*z* = 429.1 [M + H]⁺; ¹H NMR (400 MHz, DMSO-*d*₆) δ ppm 2.07-2.22 (m, 1H), 2.44 (dd, *J* = 16.04, 8.97 Hz, 1H), 2.67-2.92 (m, 4H), 3.55 (s, 1H), 7.52 (d, *J* = 8.34 Hz, 1H), 7.78 (dd, *J* = 8.46, 1.39 Hz, 1H), 8.15 (s, 1H), 8.20 (d, *J* = 8.34 Hz, 1H), 8.84 (dd, *J* = 8.08, 1.52 Hz, 1H), 9.52 (s, 1H), 11.11 (s, 1H), 12.21 (s, 1H).

### Example 1.19: Preparation of 2-(7-(5-(6-Carbamoylpyridin-3-yl)-1,2,4-oxadiazol-3-yl)-1,2,3,4-tetrahydrocyclopenta[b]indol-3-yl)acetic Acid (Compound 26).

From ethyl 2-(7-(*N*-hydroxycarbamimidoyl)-1,2,3,4-tetrahydrocyclopenta[b]indol-3-yl)acetate and 6-cyanonicotinic acid, using a similar method to the one described in **Example 1.8,** the title compound was obtained as a white solid. LCMS *m*/*z* = 404.2 [M + H]⁺; ¹H NMR (400 MHz, DMSO-*d*₆) δ ppm 2.09-2.20 (m, 1H), 2.44 (dd, *J* = 16.29, 8.72 Hz, 1H), 2.68-2.91 (m, 4H), 3.55 (s, 1H), 7.52 (d, *J* = 8.59 Hz, 1H), 7.78 (dd, *J* = 8.59, 1.52 Hz, 1H), 7.89 (s, 1H), 8.15 (s, 1H), 8.28 (d, *J* = 8.08 Hz, 1H), 8.34 (s, 1H), 8.75 (dd, *J* = 8.21, 2.15 Hz, 1H), 9.37 (d, *J* = 1.52 Hz, 1H), 11.10 (s, 1H), 12.27 (s, 1H).

### Example 1.20: Preparation of 2-(7-(5-(3-Cyano-4-(cyclopentyloxy)phenyl)-1,2,4-oxadiazol-3-yl)-1,2,3,4-tetrahydrocyclopenta[b]indol-3-yl)acetic Acid (Compound 19).

### Step A: Preparation of Cyclopentyl 3-Bromo-4-(cyclopentyloxy)benzoate.

A mixture of 3-bromo-4-hydroxybenzoic acid (1.00 g, 4.61 mmol), cesium carbonate (3.30 g, 10.1 mmol), and bromocyclopentane (1.98 mL, 18.4 mmol) in DMF (20 mL) was stirred at room temperature for 2 days. The solvent was removed under reduced pressure and the residue was taken up in EtOAc and washed with water (2 x 20 mL). The ethyl acetate solution was dried over MgSO₄, filtered, and concentrated under reduced pressure to give the title compound (1.62 g). LCMS *m*/*z* = 353.4 [M + H]⁺.

### Step B: Preparation of 3-Bromo-4-(cyclopentyloxy)benzoic Acid.

To a solution of crude cyclopentyl 3-bromo-4-(cyclopentyloxy)benzoate (1.62 g, 4.58 mmol) in THF (20 mL), 1.0 M LiOH (27.6 mL, 27.6 mmol) was added and the reaction mixture was stirred at 40 °C for 72 h. The reaction mixture was poured into water and washed with EtOAc (2 x 25 mL). The aqueous layer was then acidified to pH = 4 with 1.0 M HCl and extracted into EtOAc (3 x 25 mL). The combined extracts were dried over MgSO₄, filtered, and concentrated under reduced pressure to give the title compound (1.31 g). LCMS *m*/*z* = 285.4.

### Step C: Preparation of 3-Cyano-4-(cyclopentyloxy)benzoic Acid.

3-Bromo4-(cyclopentyloxy)benzoic acid (1.31 g, 4.61 mmol), CuCN (0.537 g, 5.99 mmol), and anhydrous NMP (10.0 mL) were heated to 200 °C for 3 h in a heavy walled sealed tube under microwave irradiation. The reaction mixture was poured into 1 M HCl and extracted with EtOAc (3 x 25 mL). The combined extracts were washed with brine (25 mL), dried over MgSO₄, filtered, and concentrated under reduced pressure. The residue was purified by preparative HPLC to afford the title compound (663 mg). LCMS *m*/*z* = 232.3 [M + H]⁺.

### Step D: Preparation of Ethyl 2-(7-(5-(3-Cyano-4-(cyclopentyloxy)phenyl)-1,2,4-oxadiazol-3-yl)-1,2,3,4-tetrahydrocyclopenta[b]indol-3-yl)acetate.

Oxalyl chloride (2.0 M in DCM, 1.65 mL, 3.3 mmol) was added to neat 3-cyano-4-(cyclopentyloxy)benzoic acid (254 mg, 1.10 mmol) and the resulting solution was stirred for 5 min. The solution was cooled to 0 °C and anhydrous DMF (1 drop) was added. The solution was warmed to room temperature and stirred for 2 h. The reaction mixture was concentrated under reduced pressure and dioxane (2.0 mL) was added. Triethylamine (0.14 mL, 1.0 mmol) and ethyl 2-(7-(*N*-hydroxycarbamimidoyl)-1,2,3,4-tetrahydrocyclopenta[b]indol-3-yl)acetate (300 mg, 1.0 mmol) were added and the solution was stirred at room temperature for 1 h. The reaction mixture was then warmed to 70 °C and stirred for 16 h. After cooling to room temperature, the reaction mixture was diluted with water and extracted three times with ethyl acetate. The combined extracts were dried over Na₂SO₄, filtered, and concentrated under reduced pressure. LCMS *m*/*z* = 497.2 [M + H]⁺.

### Step E: Preparation of 2-(7-(5-(3-Cyano-4-(cyclopentyloxy)phenyl)-1,2,4-oxadiazol-3-yl)-1,2,3,4-tetrahydrocyclopenta[b]indol-3-yl)acetic Acid (Compound 19).

The crude ethyl 2-(7-(5-(3-cyano-5-(trifluoromethoxy)phenyl)-1,2,4-oxadiazol-3-yl)-1,2,3,4-tetrahydrocyclopenta[b]indol-3-yl)acetate was dissolved in THF (15 mL) and potassium trimethylsilanolate (383 mg, 3.0 mmol) was added. The reaction mixture was stirred at room temperature for 25 min. and then acidified to pH 4 with 10% aqueous HCl. The aqueous mixture was extracted three times with EtOAc, dried over Na₂SO₄, and concentrated under reduced pressure. The residue was purified by HPLC and then by column chromatography (SiO₂, 0%-5% MeOH in DCM) to afford the title compound (19.8 mg). LCMS *m*/*z* = 469.5 [M + H]⁺. ¹H NMR (400 MHz, CDCl₃) δ ppm 1.64-1.75 (m, 3H), 1.84-2.06 (m, 6H), 2.12-2.24 (m, 1H), 2.65 (dd, *J* = 17.4, 11.3 Hz, 1H), 2.78-3.03 (m, 3H), 3.59-3.68 (m, 1H), 4.94-5.03 (m, 1H), 7.12 (d, *J* = 9.1 Hz, 1H), 7.40 (d, *J* = 8.6 Hz, 1H), 7.91 (d, *J* = 8.6 Hz, 1H), 8.29 (s, 1H), 8.35 (dd, *J* = 8.8, 2.0 Hz, 1H), 8.45 (d, *J* = 2.0 Hz, 1H), 8.66 (s, 1H).

Racemic 2-(7-(5-(3-cyano-4-(cyclopentyloxy)phenyl)-1,2,4-oxadiazol-3-yl)-1,2,3,4-tetrahydrocyclopenta[b]indol-3-yl)acetic acid was resolved to give two enantiomers by normal phase preparative chiral HPLC under the following conditions:
Column: normal phase preparative ChiralPak AD-H, 250 x 20 mm ID, 5 µm particle size.
Eluent: 90% Hexanes/10% Isopropanol, with 0.05% trifluoroacetic acid.
Gradient: Isocratic.
Flow: 10 mL/min.
Detector: 254 nm.
Retention Times: 1^{st} enantiomer: 25 min.; 2^{nd} enantiomer: 30 min.

### Example 1.21: Preparation of Ethyl 2-(7-(5-(3-Cyano-5-(trifluoromethoxy)phenyl)-1,2,4-oxadiazol-3-yl)-1,2,3,4-tetrahydrocyclopenta[b]indol-3-yl)acetate.

From 3-cyano-5-(trifluoromethoxy)benzoic acid and ethyl 2-(7-(*N* hydroxycarbamimidoyl)-1,2,3,4-tetrahydrocyclopenta[b]indol-3-yl)acetate, using a similar method to the one described in **Example 1.20, Step D,** the title compound was obtained. LCMS *m*/*z* = 497.2 [M + H]⁺. ¹H NMR (400 MHz, CDCl₃) δ ppm 1.32 (t, *J* = 7.1 Hz, 3H), 2.11-2.22 (m, 1H), 2.54 (dd, *J*= 17.2, 11.6 Hz, 1H), 2.76-2.99 (m, 4H), 3.54-3.65 (m, 1H), 4.17-4.30 (m, 2H), 7.42 (d, *J* = 8.6 Hz, 1H), 7.72 (s, 1H), 7.91 (dd, *J* = 8.3, 1.5 Hz, 1H), 8.29 (s, 1H), 8.32 (s, 1H), 8.47-8.49 (m, 1H), 8.87 (s, 1H).

### Example 1.22: Preparation of 2-(7-(5-(3-Cyano-5-(trifluoromethoxy)phenyl)-1,2,4-oxadiazol-3-yl)-1,2,3,4-tetrahydrocyclopenta[b]indol-3-yl)acetic Acid (Compound 7).

Ethyl 2-(7-(5-(3-cyano-5-(trifluoromethoxy)phenyl)-1,2,4-oxadiazol-3-yl)-1,2,3,4-tetrahydrocyclopenta[b]indol-3-yl)acetate (0.147g, 2.96 mmol) was dissolved in a solution of 2% water in acetonitrile (1.5 mL) and triethylamine (0.124 mL, 0.89 mmol) and lithium bromide (0.257g, 2.96 mmol) were added. The reaction mixture was warmed to 75 °C and stirred for 24 h. The reaction mixture was acidified to pH4 with 1.0 M HCl and then extracted with ethyl acetate. The combined extracts were dried over Na₂SO₄, filtered, and concentrated under reduced pressure. The residue was purified by column chromatography (SiO₂, 2-5% MeOH in DCM) and then by preparative HPLC to afford the title compound (61 mg). LCMS *m*/*z* = 469.4 [M + H]⁺. ¹H NMR (400 MHz, DMSO-*d*₆) δ ppm 2.10-2.21 (m, 1H), 2.43 (dd, *J*= 16.2, 8.8 Hz, 1H), 2.70-2.91 (m, 4H), 3.49-3.59 (m, 1H), 7.50 (d, *J* = 8.6 Hz, 1H), 7.76 (dd, *J* = 8.3, 1.5 Hz, 1H), 8.14 (s, 1H), 8.43 (s, 1H), 8.45 (s, 1H), 8.68 -8.71 (m, 1H), 11.1 (s, 1H), 12.2 (s, 1H).

Racemic 2-(7-(5-(3-cyano-5-(trifluoromethoxy)phenyl)-1,2,4-oxadiazol-3-yl)-1,2,3,4-tetrahydrocyclopenta[b]indol-3-yl)acetic acid was resolved to give two enantiomers using a normal phase preparative chiral HPLC as following:

### Resolution via Chiral HPLC

Column: normal phase preparative ChiralCel OD, 20 x 250mm ID, 10 µm particle size
Eluent: 75% Hexanes/25% Isopropanol, with 0.05% trifluoroacetic acid
Gradient: Isocratic
Flow: 13 mL/minute
Detector. 254 nm
Retention Times: 1^{st} enantiomer: 13 min.; 2^{nd} enantiomer: 24 min.

### Example 1.23: Preparation of 2-(7-(5-(3-Carbamoyl-5-(trifluoromethoxy)phenyl)-1,2,4-oxadiazol-3-yl)-1,2,3,4-tetrahydrocyclopenta[b]indol-3-yl)acetic Acid (Compound 51).

From ethyl 2-(7-(5-(3-cyano-5-(trifluoromethoxy)phenyl)-1,2,4-oxadiazol-3-yl)-1,2,3,4-tetrahydrocyclopenta[b]indol-3-yl)acetate, using a similar method to the one described in **Example 1.22,** the title compound was obtained. LCMS *m*/*z* = 487.0 [M + H]⁺. ¹H NMR (400 MHz, DMSO-*d*₆) δ ppm 2.08-2.20 (m, 1H), 2.44 (dd, *J* = 16.2, 9.1 Hz, 1H), 2.70-2.91 (m, 4H), 3.40-3.65 (m, 1H), 7.51 (d,*J* = 8.6 Hz, 1H), 7.77 (dd, *J* = 8.6, 1.5 Hz, 1H), 7.88 (s, 1H), 8.16 (s, 1H), 8.18 (s, 1H), 8.28 (s, 1H), 8.49 (s, 1H), 11.1 (s, 1H), 12.2-12.5 (bs, 1H).

### Example 1.24: Preparation of 2-(7-(5-(3,5-Bis(trifluoromethyl)phenyl)-1,2,4-oxadiazol-3-yl)-1,2,3,4-tetrahydrocyclopenta[b]indol-3-yl)acetic Acid (Compound 52).

### Step A: Preparation of 2-(7-(N-Hydroxycarbamimidoyl)-1,2,3,4-tetrahydrocyclopenta[b]indol-3-yl)acetic Acid.

Ethyl 2-(7-cyano-1,2,3,4-tetrahydrocyclopenta[b]indol-3-yl)acetate (1.43 g, 5.33 mmol) was dissolved in THF (50 mL) and water was added (10 mL) followed by 5 pellets of NaOH. The solution was warmed to 50 °C and stirred for 8 h. The reaction mixture was acidified to pH 4 with 10% HCl and concentrated under reduced pressure. The concentrate was dissolved in EtOH (20 mL) and hydroxylamine (0.327 mL, 5.33 mmol) was added. The reaction mixture was warmed to 65 °C and stirred for 16 h. An additional equivalent of hydroxylamine (0.327 mL, 5.33 mmol) was added and the mixture was warmed to 80 °C and stirred for 4 h. The reaction mixture was cooled to room temperature and then passed through a strong cation exchange column (Strata SCX) rinsing with MeOH and then 3.5 M ammonia in MeOH. The ammonia/methanol fraction was concentrated to dryness to afford the title compound. LCMS *m*/*z* = 274.4 [M + **H]⁺.**

### Step B: Preparation of 2-(7-(5-(3,5-Bis(trifluoromethyl)phenyl)-1,2,4-oxadiazol-3-yl)-1,2,3,4-tetrahydrocyclopenta[b]indol-3-yl)acetic Acid (Compound 52).

To a solution of 2-(7-(*N*-hydroxycarbamimidoyl)-1,2,3,4-tetrahydrocyclopenta[b]indol-3-yl)acetic acid (0.205 g, 0.750 mmol) in THF (2 mL), triethylamine (0.228 g, 2.25 mmol), 3,5-bis(trifluoromethyl)benzoyl chloride (0.207 g, 0.750 mmol), and 3 A mol sieves (20) were added. The reaction mixture was stirred at room temperature for 1 h, warmed to 45 °C and stirred for 2 days. The solids were removed by filtration and the filtrate was concentrated under reduced pressure. The residue was then dissolved in a minimal amount of DMF and diluted with MeOH until a precipitate had formed. The precipitate was removed by filtration and the remaining solution was concentrated under reduced pressure. The crude residue was purified by column chromatography (SiO₂, 0%-10% MeOH/DCM) to afford the title compound (40 mg, 11%). LCMS *m*/*z* = 496.3 [M + H]⁺; ¹H NMR (400 MHz, CDCl₃) δ ppm 2.12-2.23 (m, 1H), 2.57 (dd, *J* = 16.9, 11.1 Hz, 1H), 2.76-3.00 (m, 4H), 3.56-3.67 (m, 1H), 7.42 (d, *J* = 8.6 Hz, 1H), 7.93 (dd, *J* = 8.6, 1.8 Hz, 1H), 8.11 (s, 1H), 8.32 (d, *J* = 1.3 Hz, 1H), 8.70 (s, 2H).

### Example 1.25: Preparation of 2-(7-(5-(5-Chloro-6-methoxypyridin-3-yl)-1,2,4-oxadiazol-3-yl)-1,2,3,4-tetrahydrocyclopenta[b]indol-3-yl)acetic Acid (Compound 23).

### Step A: Preparation of Ethyl 2-(7-(5-(5,6-Dichloropyridin-3-yl)-1,2,4-oxadiazol-3-yl)-1,2,3,4-tetrahydrocyclopenta[b]indol-3-yl)acetate.

5,6-Dichloronicotinoyl chloride was prepared by reaction of 5,6-dichloronicotinic acid in DCM and one drop DMF with 1.1 equivalents oxalyl chloride at room temperature for 2 h. The orange solution was concentrated, and the resulting solid was used without further purification. To a solution of ethyl 2-(7-(*N*-hydroxycarbamimidoyl)-1,2,3,4-tetrahydrocyclopenta[b]indol-3-yl)acetate (0.20 g, 0.664 mmol) in dioxane (4 mL) was added 5,6-dichloronicotinoyl chloride (0.140 g, 0.664 mmol) then triethylamine (0.324 mL, 2.323 mmol). The resulting suspension was stirred at 70 °C for 2.5 h. The solvent was removed under reduced pressure. The residue was purified by silica gel flash chromatography (15 to 100% EtOAc/hexanes) to give the title compound as a beige solid (159 mg). LCMS *m*/*z* = 457.2 [M + H]⁺.

### Step B: Preparation of Methyl 2-(7-(5-(5-Chloro-6-methoxypyridin-3-yl)-1,2,4-oxadiazol-3-yl)-1,2,3,4-tetrahydrocyclopenta[b]indol-3-yl)acetate.

To a solution of ethyl 2-(7-(5-(5,6-dichloropyridin-3-yl)-1,2,4-oxadiazol-3-yl)-1,2,3,4-tetrahydrocyclopenta[b]indol-3-yl)acetate (50 mg, 0.109 mmol) and methanol (0.044 mL, 1.093 mmol) in DMF (1 mL) was added a 1.0 M THF solution of potassium 2-methylpropan-2-olate (0.219 mL, 0.219 mmol). The mixture was stirred at 23 °C for 1 h, concentrated, and purified by preparative HPLC to give the title compound as a white solid (24 mg). LCMS *m*/*z* = 439.4 [M + H]⁺.

### Step C: Preparation of 2-(7-(5-(5-Chloro-6-methoxypyridin-3-yl)-1,2,4-oxadiazol-3-yl)-1,2,3,4-tetrahydrocyclopenta[b]indol-3-yl)acetic Acid (Compound 23).

To a solution of methyl 2-(7-(5-(5-chloro-6-methoxypyridin-3-yl)-1,2,4-oxadiazol-3-yl)-1,2,3,4-tetrahydrocyclopenta[b]indol-3-yl)acetate (24 mg, 0.055 mmol) in 2% aqueous acetonitrile (1.0 mL) was added lithium bromide (47.5 mg, 0.547 mmol) and triethylamine (0.023 mL, 0.164 mmol). The mixture was stirred at 80 °C for 17 h and then 0.5 M HCl (10 mL) was added. The resulting precipitate was collected by filtration, rinsed with water, dried under reduced pressure, and purified by preparative TLC (75% EtOAc/hexanes) to give the title compound as a pale yellow solid (11 mg). LCMS *m*/*z* = 425.2 [M + H]⁺; ¹H NMR (400 MHz, DMSO-*d*₆) δ ppm 2.12-2.18 (m, 1H), 2.33-2.42 (m, 1H), 2.68-2.87 (m, 4H), 3.51-3.56 (m, 1H), 4.07 (s, 3H), 7.46 (d, *J* = 8.3 Hz, 1H), 7.68 (d, *J* = 8.3 Hz, 1H), 8.08 (s, 1H), 8.56 (d, *J* = 1.8 Hz, 1H),8.93 (d,*J* = 1.8 Hz, 1H), 11.16(s, 1H), 12.28 (bs, 1H).

### Example 1.26: Preparation of 2-(7-(5-(5-Chloro-6-isopropoxypyridin-3-yl)-1,2,4-oxadiazol-3-yl)-1,2,3,4-tetrahydrocyclopenta[b]indol-3-yl)acetic Acid (Compound 66).

### Step A: Preparation of Isopropyl 2-(7-(5-(5-Chloro-6-isopropoxypyridin-3-yl-1,2,4-oxadiazol-3-yl)-1,2,3,4-tetrahydrocyclopenta[b]indol-3-yl)acetate.

To a solution of ethyl 2-(7-(5-(5,6-dichloropyridin-3-yl)-1,2,4-oxadiazol-3-yl)-1,2,3,4-tetrahydrocyclopenta[b]indol-3-yl)acetate (50 mg, 0.109 mmol) and propan-2-ol (0.084 mL, 1.093 mmol) in DMF (1 mL) was added a 1.0 M THF solution of potassium 2-methylpropan-2-olate (0.219 mL, 0.219 mmol). The mixture was stirred at 23 °C for 45 min. After adding 0.5 M HCl_{(aq)} (2 mL) and water (10 mL), it was extracted with DCM (3 x 25 mL). The combined organic extracts were dried over MgSO₄, filtered, and concentrated under reduced pressure to afford an orange residue (52 mg) as a mixture of ethyl 2-(7-(5-(5-chloro-6-isopropoxypyridin-3-yl)-1,2,4-oxadiazol-3-yl)-1,2,3,4-tetrahydrocyclopenta[b]indol-3-yl)acetate and isopropyl 2-(7-(5-(5-chloro-6-isopropoxypyridin-3-yl)-1,2,4-oxadiazol-3-yl)-1,2,3,4-tetrahydrocyclopenta[b]indol-3-yl)acetate, which was carried on without further purification. LCMS *m*/*z* = 495.3 [M + H]⁺.

### Step B: 2-(7-(5-(5-Chloro-6-isopropoxypyridin-3-yl)1,2,4-oxadiazol-3-yl)-1,2,3,4-tetrahydrocyclopenta[b]indol-3-yl)acetic Acid (Compound 66).

From isopropyl 2-(7-(5-(5-chloro-6-isopropoxypyridin-3-yl)-1,2,4-oxadiazol-3-yl)-1,2,3,4-tetrahydrocyclopenta[b]indol-3-yl)acetate, using a similar method to the one described in **Example 1.25, Step C,** the title compound was obtained as a white solid. LCMS *m*/*z* = 453.2 [M + H]⁺; ¹H NMR (400 MHz, DMSO-*d*₆) δ ppm 1.39 (d, *J* = 6.3 Hz, 6H), 2.12-2.18 (m, 1H), 2.43 (dd, *J* = 15.9, 9.1 Hz, 1H), 2.71-2.87 (m, 4H), 3.51-3.56 (m, 1H), 5.45 (sep, *J* = 6.3 Hz, 1H), 7.49 (d, *J* = 8.3 Hz, 1H), 7.74 (d, *J* = 8.3 Hz, 1H), 8.11 (s, 1H), 8.55 (d, *J* = 1.8 Hz, 1H), 8.91 (d, J = 1.8 Hz, 1H), 11.08 (s, 1H), 12.28 (bs, 1H).

### Example 1.27: Preparation of 2-(7-(5-(6-sec-Butoxy-5-chloropyridin-3-yl)-1,2,4-oxadiazol-3-yl)-1,2,3,4-tetrahydrocyclopenta[b]indol-3-yl)acetic Acid (Compound 59).

To an ice-cooled solution of ethyl 2-(7-(5-(5,6-dichloropyridin-3-yl)-1,2,4-oxadiazol-3-yl)-1,2,3,4-tetrahydrocyclopenta[b]indol-3-yl)acetate (50 mg, 0.109 mmol) and (*S*)-butan-2-ol (81 mg, 1.093 mmol) in DMF (1.0 mL) was added a 1.0 M THF solution of potassium 2-methylpropan-2-olate (0.219 mL, 0.219 mmol). The mixture was stirred at 0 °C for 2 h. Saturated aqueous citric acid (5 mL) was added and the mixture sonicated for 1 min. The resulting precipitate was collected by filtration, rinsed with water, and dried under reduced pressure to afford a tan solid (as a mixture of ethyl and *sec*-butyl esters). To this was added LiBr (88 mg), triethylamine (42 µL), and 2% H₂O/MeCN (1 mL). The mixture was stirred at 75 °C for 22 h. Saturated aqueous citric acid (1 mL) and water (5 mL) were added and the mixture was sonicated for 1 min. The resulting precipitate was collected by filtration, rinsed with water, and dried under reduced pressure. It was purified by preparative HPLC to give the title compound as a white solid (12 mg, epimeric mixture). LCMS *m*/*z* = 467.4 [M + H]⁺; ¹H NMR (400 MHz, DMSO-d₆) δ ppm 0.95 (t,*J*= 6.4 Hz, 3H), 1.36 (d, *J* = 6.1 Hz, 3H), 1.66-1.83 (m, 2H), 2.11-2.18 (m, 1H), 2.43 (dd, *J* = 16.2, 8.8 Hz, 1H), 2.71-2.87 (m, 4H), 3.51-3.57 (m, 1H), 5.45 (sep, *J* = 6.1 Hz, 1H), 7.50 (d, *J* = 8.3 Hz, 1H), 7.75 (dd,*J* = 8.3, 1.5 Hz, 1H), 8.12 (d, *J* = 1.5 Hz, 1H), 8.57 (d, *J* = 2.0 Hz, 1H), 8.92 (d, *J* = 2.0 Hz, 1H), 11.08 (s, 1H), 12.24 (bs, 1H).

### Example 1.28: Preparation of 2-(7-(5-(3-Cyano-4-isopropoxyphenyl)-1,2,4-oxadiazol-3-yl)-1,2,3,4-tetrahydrocyclopenta[b]indol-3-yl)acetic Acid (Compound 15).

### Step A: Preparation of Ethyl 2-(7-(5-(3-Cyano-4-fluorophenyl)-1,2,4-oxadiazol-3-yl; 1,2,3,4-tetrahydrocyclopenta[b]indol-3-yl)acetate.

From ethyl 2-(7-(N'-hydroxycarbamimidoyl)-1,2,3,4-tetrahydrocyclopenta[b]indol-3-yl)acetate, and 3-cyano-4-fluorobenzoyl chloride, using a similar method to the one described in **Example 1.25, Step A,** the title compound was obtained as a beige solid. LCMS *m*/*z* = 431.3 [M+H]⁺.

### Step B: Preparation of 2-(7-(5-(3-cyano-4-isopropoxyphenyl)-1,2,4-oxadiazol-3-yl)-1,2,3,4-tetrahydrocyclopenta[b]indol-3-yl)acetic Acid (Compound 15).

To a solution of propan-2-ol (0.036 mL, 0.465 mmol) in THF (1.0 mL) was added a 60% oil dispersion of sodium hydride (18.58 mg, 0.465 mmol) resulting in vigorous gas evolution. After stirring at room temperature for 10 min, ethyl 2-(7-(5-(3-cyano-4-fluorophenyl)-1,2,4-oxadiazol-3-yl)-1,2,3,4-tetrahydrocyclopenta[b]indol-3-yl)acetate (50 mg, 0.116 mmol) was added. The resulting mixture was heated under microwave irradiation in a sealed, thick-walled glass tube at 110 °C for 60 min. Saturated aqueous citric acid (5 mL) was added. The mixture was diluted with water (15 mL) and extracted thrice with 25% iPrOH/CH₂Cl₂ (3x25 mL). The combined organic extracts were dried over MgSO₄, filtered, and concentrated under reduced pressure. The residue was Purified by preparative TLC (10% MeOH/CH₂Cl₂) followed by HPLC to give the title compound as a white solid (11 mag). LCMS *m*/*z*=443.5 [M+H]⁺; ¹H NMR (400 MHz, DMSO-*d*₆) δ ppm 1.39 (d, *J* = 6.1 Hz, 6H), 2.11-2.18 (m, 1H), 2.43 (dd, *J* = 16.2, 9.1 Hz, 1H), 2.71-2.87 (m, 4H), 3.50-3.56 (m, 1H),4.97 (sep, *J* = 6.1 Hz, 1H), 7.49 (d, *J* = 8.6 Hz, 1H), 7.53 (d, *J*=9.1 Hz, 1H), 7.74 (d, *J* = 8.6 Hz, 1H), 8.11 (s, 1H), 8.40 (dd, *J* = 8.8, 1.8 Hz, 1H), 8.50 (d, *J* = 2.0 Hz, 1H), 11.08 (s, 1H), 12.28 (bs, 1H).

### Resolution via Chiral HPLC

Column: normal phase preparative Chiralcel OD, 20 x 250mm ID, 10 µm particle size
Eluent: 25%IPA/Hexanes, with 0.05% trifluoroacetic acid
Gradient: Isocratic
Flow: 60 mL/minute
Detector: 280 nm
Retention Times: 1^{st} enantiomer: 35 min.; 2^{nd} enantiomer: 55 min.

### Example 1.29: Preparation of 2-(7-(5-(3-(Trifluoromethyl)phenyl-1,2,4-oxadiazol-3-yl)-1,2,3,4-tetrahydrocyclopenta[b]indol-3-yl)acetic Acid (Compound 50).

### Step A: Preparation of Ethyl 2-(7-(5-(3-(Trifluoromethyl)phenyl)-1,2,4-oxadiazol-3-yl-1,2,3,4-tetrahydrocyclopenta[b]indol-3-yl)acetate.

From ethyl 2-(7-(*N-*hydroxycarbamimidoyl)-1,2,3,4-tetrahydrocyclopenta[b]indol-3-yl)acetate and 3-(trifluoromethyl)benzoyl chloride, using a similar method to the one described in **Example 1.25, Step A,** the title compound was obtained as a beige solid. LCMS *m*/*z* = 456.3 [M + H]⁺.

### Step B: Preparation of 2-(7-(5-(3-(Trifluoromethyl)phenyl)-1,2,4-oxadiazol-3-yl)-1,2,3,4-tetrahydrocyclopenta[b]indol-3-yl)acetic Acid (Compound 50).

To a solution of ethyl 2-(7-(5-(3-(trifluoromethyl)phenyl)-1,2,4-oxadiazol-3-yl)-1,2,3,4-tetrahydrocyclopenta[b]indol-3-yl)acetate (60 mg, 0.132 mmol) in methanol (1 mL) and THF (1 mL) was added a 2.0 M aqueous solution of sodium hydroxide (0.329 mL, 0.659 mmol). The resulting solution was stirred at 23 °C for 16 h. The solvents were removed under reduced pressure. The remaining residue was diluted with water (10 mL). The solution was filtered and acidified to pH 1 with 1 M HCl. The resulting precipitate was collected and purified by preparative HPLC to give the title compound as an off-white solid (30 mg). LCMS *m*/*z* = 428.3 [M + H]⁺; ¹H NMR (400 MHz, DMSO-*d*₆) δ ppm 2.10-2.20 (m, 1H), 2.44 (dd, *J* = 16.2, 8.8 Hz, 1H), 2.72-2.89 (m, 4H), 3.51-3.58 (m, 1H), 7.51 (d, *J*= 8.6 Hz, 1H), 7.77 (dd, *J*= 8.6, 1.5 Hz, 1H), 7.92 (t, *J* = 8.1 Hz, 1H), 8.12 (d, *J* = 7.8 Hz, 1H), 8.15 (d, *J* = 1.3 Hz, 1H), 8.45 (s, 1H), 8.50 (d, *J* = 8.1 Hz, 1H), 11.09 (s, 1H), 12.28 (bs, 1H).

### Example 1.30: Preparation of 2-(7-(5-(3-(Benzyloxy)phenyl)-1,2,4-oxadiazol-3-yl)-1,2,3,4-tetrahydrocyclopenta[b]indol-3-yl)acetic Acid (Compound 22).

From ethyl 2-(7-(*N*-hydroxycarbamimidoyl)-1,2,3,4-tetrahydrocyclopenta[b]indol-3-yl)acetate and 3-(benzyloxy)benzoyl chloride, using a similar method to the one described in **Example 1.29,** the title compound was obtained as a beige solid. LCMS *m*/*z* = 466.4 [M + H]⁺; ¹H NMR (400 MHz, DMSO-*d*₆) δ ppm 2.11-2.18 (m, 1H), 2.43 (dd, *J* = 16.2, 8.8 Hz, 1H), 2.72-2.88 (m, 4H), 3.51-3.57 (m, 1H), 5.27 (s, 2H), 7.35-7.45 (m, 4H), 7.49-7.53 (m, 3H), 7.51 (t, *J* = 7.8 Hz, 1H), 7.76 (dd, *J* = 8.3, 1.5 Hz, 1H), 7.78-7.81 (m, 2H), 8.13 (d, *J* = 1.5 Hz, 1H), 11.08 (s, 1H), 12.27 (bs, 1H).

### Example 1.31: Preparation of 2-(7-(5-(3-Cyano-5-fluorophenyl)-1,2,4-oxadiazol-3-yl)-1,2,3,4-tetrahydrocyclopenta[b]indol-3-yl)acetic Acid (Compound 46).

### Step A: Preparation of Ethyl 2-(7-(5-(3-(Trifluoromethyl)phenyl)-1,2,4-oxadiazol-3-yl)-1,2,3,4-tetrahydrocyclopenta[b]indol-3-yl)acetate.

To a solution of ethyl 2-(7-(*N*-hydroxycarbamimidoyl)-1,2,3,4-tetrahydrocyclopenta[b]indol-3-yl)acetate (50 mg, 0.166 mmol) and 3-cyano-5-fluorobenzoic acid (27.4 mg, 0.166 mmol) in dioxane (2 mL) was added *N*¹-((ethylimino)methylene)-*N*³*,N*³-dimethylpropane-1,3-diamine hydrochloride (39.8 mg, 0.207 mmol) and triethylamine (0.069 mL, 0.498 mmol). The resulting suspension was stirred at 23 °C for 1 h and 75 °C for 4 h. The mixture was concentrated under reduced pressure. The residue was purified by preparative TLC (35% EtOAc/hexanes) to give the title compound as a beige solid (30 mg). LCMS *m*/*z* = 431.3 [M + H]⁺.

### Step B: Preparation of 2-(7-(5-(3-Cyano-5-fluorophenyl)-1,2,4-oxadiazol-3-yl)-1,2,3,4-tetrahydrocyclopenta[b]indol-3-yl)acetic Acid (Compound 46).

From ethyl 2-(7-(5-(3-cyano-5-fluorophenyl)-1,2,4-oxadiazol-3-yl)-1,2,3,4-tetrahydrocyclopenta[b]indol-3-yl)acetate, using a similar method to the one described in **Example 1.25, Step C,** the title compound was obtained as a white solid. LCMS *m*/*z* = 403.4 [M + H]⁺; ¹H NMR (400 MHz, DMSO-d6) δ ppm 2.11-2.18 (m, 1H), 2.44 (dd, *J* = 16.2, 9.1 Hz, 1H), 2.71-2.87 (m, 4H), 3.51.3.58 (m, 1H), 7.49 (d,*J* = 8.6 Hz, 1H), 7.74 (dd,*J* = 8.6, 1.5 Hz, 1H), 8.11 (s, 1H), 8.22 (dt,*J* = 8.6, 1.3 Hz, 1H), 8.32 (dt,*J* = 8.6, 1.5 Hz, 1H), 8.45 (s, 1H), 11.10 (s, 1H), 12.30 (bs, 1H).

### Example 1.32: Preparation of 2-(7-(5-(3-(1H-1,2,4-Triazol-1-yl)-5-(trifluoromethoxy)phenyl)-1,2,4-oxadiazol-3-yl)-1,2,3,4-tetrahydrocyclopenta[b]indol-3-yl)acetic Acid (Compound 18).

### Step A: Preparation of 3-(1H-1,2,4-Triazol-1-yl)-5-(trifluoromethoxy)benzoic Acid.

To a solution of 3-bromo-5-(trifluoromethoxy)benzoic acid (0.50 g, 1.754 mmol), 1H-1,2,4-triazole (0.33 g, 4.387 mmol) and *N¹,N²*-dimethylethane-1,2-diamine (0.062 g, 0.702 mmol) in DMF (5 mL) was added copper(I) iodide (0.067 g, 0.351 mmol) and tripotassium phosphate (0.819 g, 3.86 mmol). The reaction mixture was stirred at 130 °C for 15 h. The mixture was diluted with ethyl acetate (20 mL), Celite® was added, and the mixture was filtered, and concentrated. The residue was purified by preparative HPLC to give the title compound as a white solid (0.13 g). LCMS *m*/*z* = 274.2 [M + H]⁺; ¹H NMR (400 MHz, DMSO-*d*₆) δ ppm 7.83 (s, 1H), 8.23 (s, 1H), 8.33 (s, 1H), 8.45 (s, 1H), 9.54 (s, 1H), 13.88 (bs, 1H).

### Step B: Preparation of Ethyl 2-(7-(5-(3-(1H-1,2,4-Triazol-1-yl)-5-(trifluoromethoxy)phenyl)-1,2,4-oxadiazol-3-yl)-1,2,3,4-tetrahydrocyclopenta[b]indol-3-yl)acetate.

From ethyl 2-(7-(*N*-hydroxycarbamimidoyl)-1,2,3,4-tetrahydrocyclopenta[b]indol-3-yl)acetate and 3-(1*H*-1,2,4-triazol-1-yl)-5-(trifluoromethoxy)benzoic acid, using a similar method to the one described in **Example 1.31, Step A,** the title compound was obtained as a pale yellow solid. LCMS *m*/*z* = 539.4 [M + H]⁺.

### Step C: Preparation of 2-(7-(5-(3-(1H-1,2,4-Triazol-1-yl)-5-(trifluoromethoxy)phenyl)-1,2,4-oxadiazol-3-yl)-1,2,3,4-tetrahydrocyclopenta[b]indol-3-yl)acetic Acid (Compound 18).

From ethyl 2-(7-(5-(3-(1*H*-1,2,4-triazol-1-yl)-5-(trifluoromethoxy)phenyl)-1,2,4-oxadiazol-3-yl)-1,2,3,4-tetrahydrocyclopenta[b]indol-3-yl)acetate, using a similar method to the one described in **Example 1.29, Step B,** the title compound was obtained as a white solid. LCMS *m*/*z* = 511.2 [M + H]⁺; ¹H NMR (400 MHz, DMSO-*d*₆) δ ppm 2.10-2.18 (m, 1H), 2.44 (dd, *J* = 16.4, 8.8 Hz, 1H), 2.71-2.89 (m, 4H), 3.51-3.56 (m, 1H), 7.51 (d, *J* = 8.3 Hz, 1H), 7.78 (d, *J* = 8.6, 1H), 8.16 (s, 2H), 8.32 (s, 1H), 8.39 (s, 1H), 8.72 (s, 1H), 9.64 (s, 1H), 11.14 (s, 1H), 12.30 (bs, 1H).

### Example 1.33: Preparation of 2-(7-(5-(3-(4H-1,2,4-Triazol-4-yl)-5-(tritluoromethoxy)phenyl)-1,2,4-oxadiazol-3-yl)-1,2,3,4-tetrahydrocyclopenta[b]indol-3-yl)acetic Acid (Compound 72).

### Step A: Preparation of 3-Amino-5-(trifluoromethoxy)benzoic Acid.

To a solution of 3-bromo-5-(trifluoromethoxy)benzoic acid (0.50 g, 1.754 mmol), sodium azide (0.303 g, 4.66 mmol) and *N*¹,*N*²-dimethylethane-1,2-diamine (0.062 g, 0.702 mmol) in DMF (5 mL) was added copper(I) iodide (0.067 g, 0.351 mmol) and tripotassium phosphate (0.819 g, 3.86 mmol). The resulting brown mixture was stirred in a sealed vial at 135 °C for 15 h (caution-gas evolution). The mixture was diluted with ethyl acetate (20 mL), Celite® was added, and the mixture was filtered, and concentrated. The residue was purified by preparative HPLC to give the title compound as an orange solid (0.23 g). LCMS *m*/*z* = 222.2 [M +H]⁺.

### Step B: Preparation of 3-(4H-1,2,4-Triazol4-yl)-5-(trifluoromethoxy)benzoic Acid.

3-Amino-5-(trifluoromethoxy)benzoic acid (100 mg, 0.452 mmol) and *N'-*formylformohydrazide (43.8 mg, 0.497 mmol) were dissolved in methanol (4 mL) and the solution was concentrated. The resulting solid was heated at 200 °C in open atmosphere for 1 h., It was purified by preparative HPLC to give the title compound as a white solid (18 mg). LCMS *m*/*z* = 274.1 [M + H]⁺.

### Step C: Preparation of Ethyl 2-(7-(5-(3-(4H-1,2,4-Triazol4-yl)-5-(trifluoromethoxy)phenyl)-1,2,4-oxadiazol-3-yl)-1,2,3,4-tetrahydrocyclopenta[b]indol-3-yl)acetate.

From ethyl 2-(7-(*N*-hydroxycarbamimidoyl)-1,2,3,4-tetrahydrocyclopenta[b]indol-3-yl)acetate and 3-(4*H*-1,2,4-triazol-4-yl)-5-(trifluoromethoxy)benzoic acid, using a similar method to the one described in **Example 1.31, Step A,** the title compound was obtained as a pale-yellow solid. LCMS *m*/*z* = 539.4 [M + H]⁺.

### Step D: Preparation of 2-(7-(5-(3-(4H-1,2,4-triazol-4-yl)-5-(trifluoromethoxy)phenyl)-1,2,4-oxadiazol-3yl)-1,2,3,4-tetrahydrocyclopenta[b]indol-3-yl)acetic Acid (Compound 72).

From ethyl 2-(7-(5-(3-(4*H*-1,2,4-triazol-4-yl)-5-(trifluoromethoxy)phenyl)-1,2,4-oxadiazol-3-yl)-1,2,3,4-tetrahydrocyclopenta[b]indol-3-yl)acetate, using a similar method to the one described in **Example 1.29, Step B,** the title compound was obtained as a white solid. LCMS *m*/*z* = 511.2 [M + H]⁺.

### Example 1.34: Preparation of 2-(7-(5-(3-Bromo-5-(trifluoromethoxy)phenyl)-1,2,4-oxadiazol-3-yl)-1,2,3,4-tetrahydrocyclopenta[b]indol-3-yl)acetic Acid (Compound 63).

From ethyl 2-(7-(*N*-hydroxycarbamimidoyl)-1,2,3,4-tetrahydrocyclopenta[b]indol-3-yl)acetate and 3-bromo-5-(trifluoromethoxy)benzoyl chloride, using a similar method to the one described in **Example 1.29,** the title compound was obtained as a white solid. LCMS *m*/*z* = 522.3 [M + H]⁺; ¹H NMR (400 MHz, DMSO-*d*₆) δ ppm 2.11-2.19 (m, 1H), 2.43 (dd, *J* = 16.2, 9.1. Hz, 1H), 2.73-2.88 (m, 4H), 3.52-3.57 (m, 1H), 7.50 (d,*J* = 8.6 Hz, 1H), 7.75 (dd, *J* = 8.6, 1.5 Hz, 1H), 8.10 (s, 1H), 8.13 (s, 2H), 8.38 (t, *J* = 1.5 Hz, 1H), 11.09 (s, 1H), 12.27 (bs, 1H).

### Example 1.35: Preparation of 2-(7-(5-(3-Methyl-5-(trifluoromethoxy)phenyl)-1,2,4-oxadiazol-3-yl)-1,2,3,4-tetrahydrocyclopenta[b]indol-3-yl)acetic Acid (Compound 53).

### Step A: Preparation of 3-Methyl-5-(trifluoromethoxy)benzoic Acid.

3-Bromo-5-(trifluoromethoxy)benzoic acid (1.0 g, 3.51 mmol) was dissolved in THF (10 mL). Nitrogen was bubbled through the solution for 10 min. Palladium tetrakis(triphenylphosphine) (0.182 g, 0.158 mmol) was added followed by a 2.0 M hexanes solution of trimethylaluminium (5.26 mL, 10.53 mmol). The resulting orange solution was heated under microwave irradiation in a sealed thick-walled glass tube with stirring at 100 °C for 2 h. The orange solution was carefully added to 1 M HCl (75 mL). The mixture was extracted with DCM (2 x 50 mL). The combined organic extracts were back extracted with 2 M NaOH (50 mL). The basic aqueous solution was filtered and acidified to pH 2 with 6 M HCl. The resulting precipitate was collected by filtration, and dried under reduced pressure to afford the title compound as a white solid (0.63 g). LCMS *m*/*z* = 221.1 [M + H]⁺; ¹H NMR (400 MHz, DMSO-*d*₆) δ ppm 2.42 (s, 3H), 7.49 (s, 1H), 7.61 (s, 1H), 7.80 (s, 1H), 13.40 (bs, 1H).

### Step B: Preparation of Ethyl 2-(7-(5-(3-Methyl-5-(trifluoromethoxy)phenyl)-1,2,4-oxadiazol-3-yl)-1,2,3,4-tetrahydrocyclopenta[b]indol-3-yl)acetate.

From 3-methyl-5-(trifluoromethoxy)benzoyl chloride and (Z)-ethyl **2-(7-(*N-***hydroxycarbamimidoyl)-1,2,3,4-tetrahydrocyclopenta[b]indol-3-yl)acetate, using a similar method to the one described in **Example 1.25, Step A,** the title compound was obtained as a pale yellow solid. LCMS *m*/*z* = 486.3 [M + H]⁺; NMR (400 MHz, CDCl₃) δ ppm 1.31 (t, *J* = 7.1 Hz, 3H), 2.11-2.18 (m, 1H), 2.50 (s, 3H), 2.54 (dd, *J*= 16.9, 11.4 Hz, 1H), 2.75-2.96 (m, 4H), 3.54-3.62 (m, 1H), 4.18-4.27 (m, 2H), 7.25 (s, 1H), 7.40 (d, *J* = 8.6 Hz, 1H), 7.90 (s, 1H), 7.92 (dd, *J* = 8.6, 1.5 Hz, 1H), 8.00 (s, 1H), 8.30 (s, 1H), 8.83 (s, 1H).

### Step C: Preparation of 2-(7-(5-(3-Methyl-5-(trifluoromethoxy)phenyl)-1,2,4-oxadiazol-3-yl)-1,2,3,4-tetrahydrocyclopenta[b]indol-3-yl)acetic Acid (Compound 53).

From ethyl 2-(7-(5-(3-methyl-5-(thfluoromethoxy)phenyl)-1,2,4-oxadiazol-3-yl)-1,2,3,4-tetrahydrocyclopenta[b]indol-3-yl)acetate, using a similar method to the one described in **Example 1.29, Step B,** the title compound was obtained as a white solid. LCMS *m*/*z* = 458.2 [M + H]⁺; ¹H NMR (400 MHz, DMSO-*d*₆) δ ppm 2.11-2.18 (m, 1H), 2.43 (dd, *J* = 16.2, 9.1 Hz, 1H),2.51 (s, 3H), 2.74-2.88 (m, 4H), 3.51-3.57 (m, 1H), 7.50 (d, *J* = 8.6 Hz, 1H), 7.60 (s, 1H), 7.75 (dd, *J* = 8.6, 1.8 Hz, 1H), 7.91 (s, 1H), 8.09 (s, 1H), 8.13 (s, 1H), 11.08 (s, 1H), 12.26 (bs, 1H).

### Example 1.36: Preparation of 2-(7-(5-(3-tert-Butyl-5-cyanophenyl)-1,2,4-oxadiazol-3-yl)-1,2,3,4-tetrahydrocyclopenta[b]indol-3-yl)acetic Acid (Compound 57).

### Step A: Preparation of Ethyl 2-(7-(5-(3-Methyl-5-(trifluoromethoxy)phenyl)-1,2,4-oxadiazol-3-yl)-1,2,3,4-tetrahydrocyclopenta[b]indol-3-yl)acetate.

From 3-*tert*-butyl-5-cyanobenzoyl chloride and (Z)-ethyl 2-(7-(*N* hydroxycarbamimidoyl)-1,2,3,4-tetrahydrocyclopenta[b]indol-3-yl)acetate, using a similar method to the one described in **Example 1.25, Step A,** the title compound was obtained as a yellow solid. LCMS *m*/*z* = 469.4 [M + H]⁺; NMR (400 MHz, CDCl₃) δ ppm 1.32 (t, *J* = 7.1 Hz, 3H), 1.43 (s, 9H), 2.11-2.21 (m, 1H), 2.54 (dd, *J* = 17.2, 11.4 Hz, 1H), 2.77-2.98 (m, 4H), 3.54-3.62 (m, 1H), 4.17-4.29 (m, 2H), 7.42 (d, *J* = 8.6 Hz, 1H), 7.88 (t, *J* = 1.5 Hz, 1H), 7.93 (dd, *J* = 8.6, 1.5 Hz, 1H), 8.31 (s, 1H), 8.36 (t, *J* = 1.3 Hz, 1H), 8.46 (t, *J* = 1.5 Hz, 1H), 8.86 (s, 1H).

### Step B: Preparation of 2-(7-(5-(3-tert-Butyl-5-cyanophenyl)-1,2,4-oxadiazol-3-yl)-1,2,3,4-tetrahydrocyclopenta[b]indol-3-yl)acetic Acid (Compound 57).

From ethyl 2-(7-(5-(3-*tert*-butyl-5-cyanophenyl)-1,2,4-oxadiazol-3-yl)-1,2,3,4-tetrahydrocyclopenta[b]indol-3-yl)acetate, using a similar method to the one described in **Example 1.25, Step C,** the title compound was obtained as a white solid. LCMS *m*/*z* = 441.3 [M + H]⁺; ¹H NMR (400 MHz, DMSO-d6) δ ppm 1.33 (s, 9H), 2.11-2.18 (m, 1H), 2.43 (dd, *J* = 16.2,9.1 Hz, 1H), 2.74-2.88 (m, 4H), 3.51-3.57 (m, 1H), 7.43 (d,*J* = 8.6 Hz, 1H), 7.76 (dd,*J*= 8.6, 1.5 Hz, 1H), 7.99 (t, *J* = 1.5 Hz, 1H), 8.15 (s, 1H), 8.29 (s, 1H), 8.41 (s, 1H), 9.0 (s, 1H), 12.26 (bs, 1H).

### Example 1.37: Preparation of 2-(7-(5-(3-Propyl-5-(trifluoromethoxy)phenyl)-1,2,4-oxadiazol-3-yl)-1,2,3,4-tetrahydrocyclopenta[b]indol-3-yl)acetic Acid (Compound 40).

### Step A: Preparation of Ethyl 2-(7-(5-(3-propyl-5-(trifluoromethoxy)phenyl)-1,2,4-oxadiazol-3-yl)-1,2,3,4-tetrahydrocyclopenta[b]indol-3-yl)acetate.

To a solution of ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetate (50 mg, 0.091 mmol) in THF (0.5 mL) was added palladium tetrakis(triphenylphosphine) (10.50 mg, 9.09 µmol). Nitrogen was bubbled through the solution for 10 min. A 0.5 M THF solution of propylzinc(II) bromide (0.545 mL, 0.273 mmol) was added and the resulting solution was heated under microwave irradiation with stirring in a sealed thick-walled glass tube at 50 °C for 1 h. The mixture was concentrated and purified by preparative TLC (25% EtOAc/hexanes) to afford the title compound as a yellow solid (20 mg). LCMS *mlz* = 514.4 [M + H]⁺.

### Step B: Preparation of 2-(7-(5-(3-Propyl-5-(trifluoromethoxy)phenyl)-1,2,4-oxadiazol-3-yl)-1,2,3,4-tetrahydrocyclopenta[b]indol-3-yl)acetic Acid (Compound 40).

From ethyl 2-(7-(5-(3-propyl-5-(trifluoromethoxy)phenyl)-1,2,4-oxadiazol-3-yl)-1,2,3,4-tetrahydrocyclopenta[b]indol-3-yl)acetate, using a similar method to the one described in **Example 1.29, Step B,** the title compound was obtained as a white solid. LCMS *m*/*z* = 486.2 [M + H]⁺; ¹H NMR (400 MHz, Acetonitrile-*d*₃) δ ppm 0.97 (t, *J* = 7.3 Hz, 3H), 1.71 (m, 2H), 2.13-2.22 (m, 1H), 2.57-2.93 (m, 7H), 3.55-3.61 (m, 1H), 7.43 (s, 1H), 7.50 (d, *J* = 8.6 Hz, 1H), 7.81 (dd, *J* = 8.6, 1.3 Hz, 1H), 7.91 (s, 1H), 8.04 (s, 1H), 8.19 (s, 1H), 9.46 (s, 1H).

### Example 1.38: Preparation of 2-(7-(5-(3-Ethynyl-5-(trifluoromethoxy)pheny)-1,2,4-oxadiazol-3-yl)-1,2,3,4-tetrahydrocyclopenta[b]indol-3-yl)acetic Acid (Compound 6).

### Step A: Preparation of Ethyl 2-(7-(5-(3-(Trifluoromethoxy)-5-((trimethylsilyl)ethynyl)phenyl)-1,2,4-oxadiazol-3-yl)-1,2,3,4-tetrahydrocyclopenta[b]indol-3-yl)acetate.

To a solution of ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetate (75 mg, 0.136 mmol) in THF (0.5 mL) was added ethynyltrimethylsilane (0.096 mL, 0.681 mmol), copper(I) iodide (5.19 mg, 0.027 mmol), and triethylamine (0.095 mL, 0.681 mmol). Nitrogen was bubbled through the solution for 10 min. Palladium tetrakis(triphenylphosphine) (15.75 mg, 0.014 mmol) was added and the resulting brown solution was heated under microwave irradiation in a sealed, thick-walled glass tube with stirring at 75°C for 2 h. The mixture was purified by preparative TLC (35% EtOAc/hexanes) to afford the title compound as a yellow solid (33 mg). LCMS *m*/*z* = 568.3 [M +H]⁺.

### Step B: Preparation of 2-(7-(5-(3-Ethynyl-5-(trifluoromethoxy)pheny)-1,2,4-oxadiazol-3-yl)-1,2,3,4-tetrahydrocyclopenta[b]indol-3-yl)acetic Acid (Compound 6).

To a solution of ethyl 2-(7-(5-(3-(trifluoromethoxy)-5-((trimethylsilyl)ethynyl)phenyl)-1,2,4-oxadiazol-3-yl)-1,2,3,4-tetrahydrocyclopenta[b]indol-3-yl)acetate (33 mg, 0.058 mmol) in THF (0.4mL) and methanol (0.4 mL) was added a 2.0 M aqueous solution of sodium hydroxide (0.174 mL, 0.349 mmol). The reaction was stirred at 23°C for 2 h and concentrated. The mixture was diluted with water (8 mL). A small amount of insoluble material was removed by filtration. The aqueous solution was acidified to pH 2, and the resulting precipitate was collected by filtration, and dried under reduced pressure to afford the title compound (19 mg) as an orange solid. LCMS *m*/*z* = 468.4 [M + H)⁺; ¹H NMR (400 MHz, Acetonitrile-*d*₃) δ ppm 2.13-2.22 (m, 1H), 2.65 (d, *J* = 7.3 Hz, 2H), 2.75-2.94 (m, 3H), 3.55-3.62 (m, 1H), 3.67 (s,1H), 7.51 (d, *J* = 8.6 Hz, 1H), 7.70 (s, 1H), 7.84 (dd, *J* = 8.6,1.3 Hz, 1H), 8.11 (s, 1H), 8.22 (s, 1H), 8.28 (s, 1H), 9.15 (s, 1H), 9.31 (s, 1H).

### Example 1.39: Preparation of 2-(7-(5-(3-Ethyl-5-(trifluoromethoxy)phenyl)-1,2,4-oxadiazol-3-yl)-1,2,3,4-tetrahydrocyclopenta[b]indol-3-yl)acetic Acid (Compound 31).

To a solution of 2-(7-(5-(3-ethynyl-5-(trifluoromethoxy)phenyl)-1,2,4-oxadiazol-3-yl)-1,2,3,4-tetrahydrocyclopenta[b]indol-3-yl)acetic acid (19 mg, 0.041 mmol) in ethyl acetate (1 mL) was added wet 10 wt% Pd/C (4.33 mg, 4.07 µmol). The mixture was stirred under 1 atm. of hydrogen at 23°C for 3 h. The reaction was filtered and concentrated. The residue was purified by preparative HPLC to afford the title compound as a white solid (7 mg). LCMS *m*/*z* = 472.2 [M + H]⁺; ¹H NMR (400 MHz, Acetonitrile-*d*₃) δ ppm 1.30 (t, *J* = 7.6 Hz, 3H), 2.14-2.22 (m, 1H), 2.64-2.66 (m, 2H), 2.74-2.94 (m, 5H), 3.54-3.62 (m, 1H), 7.46 (s, 1H), 7.50 (d, *J* = 8.6 Hz, 1H), 7.83 (dd, *J* = 8.6, 1.8 Hz, 1H), 7.91 (s, 1H), 8.07 (s, 1H), 8.20 (s, 1H), 9.35 (s, 1H).

### Example 1.40: Preparation of 2-(7-(5-(3-Cyclopropyl-5-(trifluoromethoxy)phenyl)-1,2,4-oxadiazol-3-yl)-1,2,3,4-tetrahydrocyclopenta[b]indot-3-yl)acetic Add (Compound 30).

From ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetate and cyclopropylzinc(II) bromide, using a similar method to the one described in **Example 1.37,** the title compound was obtained as a white solid. LCMS *m*/*z* = 484.2 [M+H]⁺; ¹H NMR (400 MHz, Acetonitrile-*d*₃) δ ppm 0.83-0.87 (m,2H), 1.09-1.14 (m, 2H), 2.07-2.22 (m, 2H), 2.63-2.65 (m, 2H), 2.74-2.94 (m, 3H), 3.54-3.61 (m, 1H), 7.27 (s, 1H), 7.50 (d, *J* = 8.6 Hz, 1H), 7.82 (dd, *J* = 8.6, 1.8 Hz, 1H), 7.84 (t, *J* = 1.3 Hz, 1H), 7.90 (t, *J*= 1.5 Hz, 1H), 8.19 (s, 1H), 9.39 (s, 1H).

### Example 1.41: Preparation of 2-(7-(5-(3-Cyclobutyl-5-(tritluoromethoxy)phenyl)1,2,4-oxadiazol-3-yl)-1,2,3,4-tetrahydrocyclopenta [b]indol-3-yl)acetic Acid (Compound 9).

From ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetate and cyclobutylzinc(II) bromide, using a similar method to the one described in **Example 1.37,** the title compound was obtained as a white solid. LCMS *m*/*z* = 498.4 [M + H]⁺; ¹H NMR (400 MHz, Acetonitrile-*d*₃) δ ppm 1.86-1.95 (m, 1H), 2.03-2.26 (m, 4H), 2.39-2.46 (m, 2H), 2.63-2.65 (m, 2H), 2.74-2.94 (m, 3H), 3.54-3.61 (m, 1H), 3.71 (pentet, *J* = 8.8 Hz, 1H), 7.43 (s, 1H), 7.50 (d, *J* = 8.6 Hz, 1H), 7.82 (dd, *J* = 8.6, 1.5 Hz, 1H), 7.89 (s, 1H), 8.03 (s, 1H), 8.19 (s, 1H), 9.36 (s, 1H).

### Example 1.42: Preparation of 2-(7-(5-(3-(2-Cyanoethyl)-5-(trifluoromethoxy)phenyl)-1,2,4-oxadiazol-3-yl)-1,2,3,4-tetrahydrocyclopenta[b]indol-3-yl)acetic Acid (Compound 60).

From ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetate and (2-cyanoethyl)zinc(II) bromide, using a similar method to the one described in **Example 1.37,** the title compound was obtained as a white solid. LCMS *m*/*z* = 497.4 [M + H]⁺; ¹H NMR (400 MHz, Acetonitrile-*d*₃) δ ppm 2.13-2.22 (m, 1H), 2.63-2.65 (m, 2H), 2.74-2.94 (m, 5H), 3.10 (t, *J* = 7.3 Hz, 2H), 3.54-3.61 (m, 1H). 7.50 (d, *J* = 8.6 Hz, 1H), 7.52 (s, 1H), 7.82 (dd, *J* = 8.6,1.5 Hz, 1H), 7.99 (s, 1H), 8.13 (s, 1H), 8.19 (s, 1H), 9.46 (s, 1H).

**Example 1.43: Preparation of 2-(7-(5-(3-Bromo-5-(trifluoromethyl)phenyl)-1,2,4-oxadiazol-3-yl)-1,2,3,4-tetrahydrocyclopenta[b]indol-3-yl)acetic Acid (Compound 14).**

### Step A: Preparation of Ethyl 2-(7-(5-(3-Bromo-5-(trifluoromethyl)phenyl)-1,2,4-oxadiazol-3-yl)-1,2,3,4-tetrahydrocyctopenta[b]indol-3-yl)acetate.

To a solution of 3-bromo-5-(trifluoromethyl)benzoic acid (26.8 mg, 0.100 mmol) in DCM was added a 2.0 M DCM solution of oxalyl chloride (0.100 mL, 0.200 mmol), a catalytic amount of DMF and the mixture was stirred at room temperature for 1 h. The solvent was removed under reduced pressure to give 3-bromo-5-(trifluoromethyl)benzoyl chloride. To a solution of ethyl 2-(7-(*N*-hydroxycarbamimidoyl)-1,2,3,4-tetrahydrocyclopenta[b]indol-3-yl)acetate (30 mg, 0.100 mmol) and TEA (0.014 mL, 0.100 mmol) in THF (2 mL) was added 3-bromo-5-(trifluoromethyl)benzoyl chloride (28.7 mg, 0.100 mmol). After 1 h, the solvent was removed under reduced pressure. The reaction mixture was reconstituted with DMA (2 mL) and heated to 100 °C for 2 h. The solvent was then removed under reduced pressure. Water was added and product was extracted into EtOAc (3 x 5 mL). The organic layers were combined and washed with saturated NaCl (10 mL), dried over MgSO₄, and concentrated. The residue was purified by preparative TLC (30% EtOAc/hexanes) to give the title compound as a solid (27:3 mg). LCMS *m*/*z* = 534.3 [M + H]⁺.

### Step B: Preparation of 2-(7-(5-(3-Bromo-5-(trifluoromethyl)phenyl)-1,2,4-oxadiazol-3-yl)-1,2,3,4-tetrahydrocyclopenta[b]indol-3-yl)acetic Acid.

Ethyl 2-(7-(5-(3-bromo-5-(trifluoromethyl)phenyl)-1,2,4-oxadiazol-3-yl)-1,2,3,4-tetrahydrocyclopenta[b]indol-3-yl)acetate (27.3 mg, 0.051 mmol), lithium bromide (44.4 mg, 0.511 mmol) and triethylamine (15.2 mg, 0.153 mmol) were dissolved in a solution of acetonitrile containing 2% water (1mL) and the reaction was stirred at 70°C for 16 h. The reaction was concentrated and the residue was taken up in ethyl acetate. The solution was washed with 1M HCl and brine, dried over Na₂SO₄, and concentrated. The residue was purified by preparative TLC (50% EtOAc/hexanes containing 1% acetic acid) to give the title compound as a solid (5.5 mg). LCMS *m*/*z* = 506.2 [M + H]⁺.

### Example 1.44: Preparation of 2-(7-(5-(3-Bromo-5-chlorophenyl)-1,2,4-oxadiazol-3-yl)-1,2,3,4-tetrahydrocyclopenta[b]indol-3-yl)acetic Acid (Compound 17).

### From 3-bromo-5-chlorobenzoic acid, using a similar method to the one described in Example 1.43, the title compound was obtained as solid. LCMS m/z = 472.1 [M + H]⁺. Example 1.45: Preparation of 2-(7-(5-(3-Chloro-5-(trifluoromethoxy)phenyl)-1,2,4-oxadiazol-3-yl)-1,2,3,4-tetrahydrocyclopenta[b]indol-3-yl)acetic Acid (Compound 45).

From 3-chloro-5-(trifluoromethoxy)benzoic acid, using a similar method to the one described in **Example 1.43,** the title compound was obtained as a solid. LCMS ***m*/*z*** = 478.2 [M + H]⁺.

### Example 1.46: Preparation of 2-(7-(5-(3-Bromo-5-bydroxypbenyl)-1,2,4-oxadiazol-3-yl)-1,2,3,4-tetrahydrocyclopenta[b]indol-3-yl)acetic Acid (Compound 69).

From 3-bromo-5-hydroxybenzoic acid, using a similar method to the one described in **Example 1.43,** the title compound was obtained as a solid. LCMS *m*/*z* = 454.2 [M + H]⁺**.**

### Example 1.47: Preparation of 2-(7-(5-(3-Isopropoxy-5-(trifluoromethoxy)phenyl)-1,2,4-oxadiazol-3-yl)-1,2,3,4-tetrahydrocyclopenta[b]indol-3-yl)acetic Acid (Compound 33).

### Step A: Preparation of 3-Hydroxy-5-(trifluoromethoxy)benzoic Acid.

3-Bromo-5-(trifluoromethoxy)benzoic acid (0.500 g, 1.754 mmol), di-*tert-*butyl(2',4',6'-triisopropylbiphenyl-2-yl) phosphine (0.060 g, 0.080 mmole), tris(dibenzylideneacetone) dipalladium(0) (0.032 g, 0.035 mmol) and potassium hydroxide (0.394 g, 7.02 mmol) were dissolved in dioxane (5.00 mL) and water (5.00 mL) in a 50 mL round bottomed flask under N₂. The reaction was heated to 100°C for 2 h. The product was poured into 1 M HCl and extracted into EtOAc (2 x 20 mL), filtered by vacuum filtration through Elite®, then washed with saturated NaCl (20mL), dried over MgSO₄ and concentrated under reduced pressure. The residue was purified by preparative HPLC to give the title compound as a white solid (0.300 g). LCMS *mlz* = 223.2 [M + H]⁺.

### Step B: Preparation of Isopropyl 3-Isopropoxy-5-(tritluoromethoxy)benzoate.

3-Hydroxy-5-(trifluoromethoxy)benzoic acid (0.223 g, 1.01 mmol), cesium carbonate (0.655 g, 2.01 mmol), 2-bromopropane (0.189 mL, 2.01 mmol), and DMF (10 mL) were stirred at room temperature for 16 h in a 20mL sealed scintillation vial. After 16 h at room temperature, the reaction was heated to 50°C for 2 h. The reaction mixture was taken up in EtOAc and washed with water (2 x 20 mL), dried over MgSO₄, filtered, and concentrated under reduced pressure to yield the title compound as a solid (0.309 g). LCMS *m*/*z* = 307.2 [M + H]⁺.

### Step C: Preparation of 3-Isopropoxy-5-(trifluoromethoxy)benzoic Acid.

To a solution of isopropyl 3-isopropoxy-5-(trifluoromethoxy)benzoate (0.309 g, 1.01 mmol) in THF (10 mL) was added 1.0 M LiOH_{(aq)} (6.036 mL, 6.036 mmol) and the mixture was stirred at 40°C for 48 h. The reaction mixture was poured into water and washed with EtOAc (25 mL). The aqueous layer was then acidified to pH 4 with 1 M HCl and extracted into EtOAc (3 x 25 mL), dried over MgSO₄. filtered, and concentrated under reduced pressure to give the title compound as a white solid (0.190 g). LCMS *m*/*z* = 263.0 [M-H]⁻.

### Step D: Preparation of 2-(7-(5-(3-Isopropoxy-5-(trifluoromethoxy)phenyl)-1,2,4-oxadiazol-3-yl)-1,2,3,4-tetrahydrocyclopenta[b]indol-3-yl)acetic Acid (Compound 33).

From 3-isopropoxy-5-(trifluoromethoxy)benzoic acid, using a similar method to the one described in **Example 1.43,** the title compound was obtained as solid. LCMS *m*/*z* = 502.5 [M + H]⁺.

### Example 1.48: Preparation of 2-(7-(5-(3-Cyano-5-methoxyphenyl)-1,2,4-oxadiazol-3-yl)-1,2,3,4-tetrahydrocyclopenta[b]indol-3-yl)acetic Acid (Compound 5).

From 3-cyano-5-methoxybenzoic acid, using a similar method to the one described in **Example 1.43,** the title compound was obtained as a solid. LCMS *m*/*z* = 415.2 [M + **H]⁺.**

### Example 1.49: Preparation of 2-(7-(5-(3-Chloro-5-cyanophenyl)-1,2,4-oxadiazol-3-yl)-1,2,3,4-tetrahydrocyclopenta[b]indol-3-yl)acetic Acid (Compound 37).

From 3-chloro-5-cyanobenzoic acid, using a similar method to the one described in **Example 1.43,** the title compound was obtained as a solid. LCMS *m*/*z* = 419.4 [M + **H]⁺.**

### Example 1.50 : Preparation of 2-(7-(5-(3-Cyano-5-(2,2,2-trifluoroethoxy)phenyl)-1,2,4-oxadiazol-3-yl)-1,2,3,4-tetrahydrocyclopenta[b]indol-3-yl)acetic Acid (Compound 55).

### Step A: Preparation of 3-Cyano-5-hydroxybenzoic Acid.

3-Bromo-5-hydroxybenzoic acid (2.17 g, 10.00 mmol), CuCN (0.896 g, 10.00 mmol) and anhydrous NMP (20.00 mL) were heated to 200°C for 2 h in a 20 mL heavy walled sealed tube under microwave irradiation. The mixture was poured into 1 M **HCl** and extracted into EtOAc (3 x 25 mL). The organic layer was then washed with saturated NaCl (25 mL), dried over MgSO₄, filtered, and concentrated under reduced pressure. The residue was purified by preparative HPLC to give the title compound as a solid (1.132 g). LCMS *m*/*z* = 164.1 [M + H]⁺.

### Step B: Preparation of Methyl 3-Cyano-5-hydroxybenzoate

To a solution of 3-cyano-5-hydroxybenzoic acid (1.12 g, 6.87 mmol) in MeOH (15 mL) was added sulfuric acid (0.150 µL, 2.75 mmol). The reaction was heated to 70°C for 24 h and the solvent was removed under reduced pressure. To the residue was added saturated NaHCO₃. The organics were extracted into EtOAc (3 x 100 mL), washed with saturated NaCl (100 mL), dried over MgSO₄, filtered, and concentrated under reduced pressure to give the title compound as a light yellow solid (1.09 g). LCMS *m*/*z* = 178.1 [M + H]⁺.

### Step C: Preparation of Methyl 3-Cyano-5-(2,2,2-trifluoroethoxy)benzoate).

Methyl 3-cyano-5-hydroxybenzoate (0.200 g, 1.129 mmol), 2,2,2-trifluoroethyl 4-methylbenzenesulfonate (0.316 g, 1.242 mmol), cesium carbonate (0.405 g, 1.242 mmol) in DMF (3 mL) were heated to 80°C for 16 h in a 20 mL sealed scintillation vial. After 16 h the reaction was not complete. Extra 2,2,2-trifluoroethyl 4-methylbenzenesulfonate (0.316 g, 1.242 mmol) was added. After 6 h reaction was still not complete. The reaction was allowed to continue for an additional 16 h at 80°C. The solvent was then removed under reduced pressure. The residue was then diluted with 10 mL of water and extracted into EtOAc (3 x 10 mL). The organic layers were combined and washed with saturated NaCl (10 mL), dried over MgSO₄. filtered, and concentrated under reduced pressure. The crude was purified *via* column chromatography (0%-20% EtOAc/hexanes, silica) to give the title compound as a white solid (0.202 g). LCMS *m*/*z* = 260.1 [M + H]⁺.

### Step D: Preparation of 3-Cyano-5-(2,2,2-trifluoroethoxy)benzoic Acid

Methyl 3-cyano-5-(2,2,2-trifluoroethoxy)benzoate (0.202 g, 0.756 mmol), lithium bromide (0.980 g, 11.29 mmol), triethylamine (0.479 mL, 3.39 mmol) in acetonitrile (5mL) with 2% H₂O was heated to 40°C for 1 h in a 20 mL sealed scintillation vial. The reaction mixture was then poured into 1 M HCl and extracted into EtOAc (3x10 mL), washed with saturated NaCl (10 mL), dried over MgSO₄, filtered, and concentrated under reduced pressure. The residue was purified by preparative HPLC to give the title compound (0.109 g) as a white solid. LCMS *m*/*z* = 246.1 [M + H]⁺.

### Step E: Preparation of 2-(7-(5-(3-Cyano-5-(2,2,2-trifluoroethoxy)phenyl)-1,2,4-oxadiazol-3-yl)-1,2,3,4-tetrahydrocyclopenta[b]indol-3-yl)acetic Acid (Compound 55).

From 3-cyano-5-(2,2,2-trifluoroethoxy)benzoic acid, using a similar method to the one described in **Example 1.43,** the title compound was obtained as a solid. LCMS *m*/*z* = 483.2 [M +H]⁺.

### Example 1.51: Preparation of 2-(7-(5-(3-Cyano-5-ethoxyphenyl)-1,2,4-oxadiazol-3-yl)-1,2,3,4-tetrahydrocyclopenta[b]indol-3-yl)acetic Acid (Compound 21).

From 3-cyano-5-ethoxybenzoic acid, using a similar method to the one described in **Example 1.43,** the title compound was obtained as a solid. LCMS *m*/*z* = 429.2 [M + H]⁺.

### Example 1.52 : Preparation of 2-(7-(5-(3-Cyano-5-(2-fluoroethoxy)phenyl)-1,2,4-oxadiazol-3-yl)-1,2,3,4-tetrahydrocyclopenta[b]indol-3-yl)acetic Acid (Compound 32).

### Step A: Preparation of Methyl 3-Cyano-5-(2-fluoroethoxy)benzoate.

Methyl 3-cyano-5-hydroxybenzoate (0.200 g, 1.129 mmol), 2-fluoroethanol (0.108 g, 1.693 mmol) and triphenylphosphine (0.444 g, 1.693 mmol) in DCM (3 mL) were stirred at room temperature for 1 h in a 20 mL sealed scintillation vial. After 1 h, the reaction mixture was cooled to 0°C and DIAD (0.329 mL, 1.693 mmol) was added slowly. After all the DIAD was added, the reaction mixture was warmed to room temperature. After 2 h the solvent was removed under reduced pressure. The triphenylphosphine was precipitated out with 20 mL of 10% EtOAc/hexanes. The mixture was filtered and the solid was washed with 10% EtOAc/hexanes (2 x 20 mL). The EtOAc/hexanes solutions were combined and concentrated under reduced pressure. The residue was purified via column chromatography (0%-20% EtOAc/hexanes, silica) to give the title compound as a white solid (0.205 g). LCMS m/z = 224.3 [M + H]⁺.

### Step B: Preparation of 3-Cyano-5-(2-fluoroethoxy)benzoic Acid

From methyl 3-cyano-5-(2-fluoroethoxy)benzoate, using a similar method to the one described in **Example 1.50, Step D,** the title compound was obtained as a solid. LCMS *m*/*z* = 210.6 [M + H]⁺.

### Step C: Preparation of 2-(7-(5-(3-Cyano-5-(2-fluoroethoxy)phenyl)-1,2,4-oxadiazol-3-yl)-1,2,3,4-tetrahydrocyclopenta[b]indol-3-yl)acetic Acid (Compound 32).

From 3-cyano-5-(2-fluoroethoxy)benzoic acid, using a similar method to the one described in Example 1.43, the title compound was obtained as a solid. LCMS *m*/*z* = 447.4 [M + H]⁺.

### Example 1.53: Preparation of 2-(7-(5-(3-Cyano-5-(cyclopentyloxy)phenyl)-1,2,4-oxadiazol-3-yl)-1,2,3,4-tetrahydrocylopenta[b]indol-3-yl)acetic Acid (Compound 65).

### Step A: Preparation of Methyl 3-Cyano-5-(cyclopentyloxy)benzoate.

Methyl 3-cyano-5-hydroxybenzoate (0.200 g, 1.129 mmol), cesium carbonate (0.405 g, 1.242 mmol), and bromocyclopentane (0.133 mL, 1.242 mmol) in DMF (5mL) were stirred at 80°C for 16 h in a 20 mL sealed scintillation vial. The solvent was removed under reduced pressure. The residue was taken up in EtOAc and washed with water (2 x 20 mL), dried over MgSO₄, filtered, and concentrated under reduced pressure. The residue was purified *via* column chromatography (0%-20% EtOAc/hexanes, silica) to give the title compound (0.254 g) as a colorless oil. LCMS *m*/*z* = 246.0 [M + H]⁺.

### Step B: Preparation of 3-Cyano-5-(cyclopentyloxy)benzoic Acid.

From methyl 3-cyano-5-(cyclopentyloxy)benzoate, using a similar method to the one described in Example 1.50, Step D, the title compound was obtained as a white solid. LCMS *m*/*z* = 232.0 [M + H]⁺.

**Step C: Preparation of 2-(7-(5-(3-Cyano-5-(cyctopentyloxy)phenyl)-1,2,4-oxadiazol-3-yl)-1,2,3,4-tetrahydrocyclopenta[b]indol-3-yl)acetic Acid (Compound 65).**

From 3-cyano-5-(cyclopentyloxy)benzoic acid, using a similar method to the one described in Example 1.43, the title compound was obtained as a solid. LCMS *m*/*z* = 469.3 [M +H]⁺.

### Example 1.54: Preparation of 2-(7-(5-(3,5-Dimethylphenyl)-1,2,4-oxadiazol-3-yl)-1,2,3,4-tetrahydrocyclopenta[b]indol-3-yl)acetic Acid (Compound 64).

### Step A: Preparation of ethyl 2-(7-(5-(3,5-dimethylphenyl)-1,2,4-oxadiazol-3-yl)-1,2,3,4-tetrahydrocyclopenta[b]indol-3-yl)acetate.

To a solution of ethyl 2-(7-(*N*-hydroxycarbamimidoyl)-1,2,3,4-tetrahydrocyclopenta[b]indol-3-yl)acetate (30 mg, 0.100 mmol) and triethylamine (10.1 mg, 0.100 mmol) in anhydrous dioxane (1mL) was added 3,5-dimethylbenzoyl chloride (17 mg, 0.1 mmol). The reaction was stirred at 50°C for 30 min and then 100°C for 1.5 h. The mixture was concentrated and the residue was taken up in DCM. The organics were washed with 1 M HCl and brine, dried over Na₂SO₄, filtered and concentrated. The residue was purified by preparative LCMS (50%-95% MeCN/H₂O) to give the title compound as a solid (6.2 mg). LCMS *m*/*z* = 416.5 [M + M+.

### Step B: Preparation of 2-(7-(5-(3,5-Dimethylphenyl)-1,2,4-oxadiazol-3-yl)-1,2,3,4-tetrahydrocyclopenta[b]indol-3-yl)acetic Acid (Compound 64).

To a mixture of ethyl 2-(7-(5-(3,5-dimethylphenyl)-1,2,4-oxadiazol-3-yl)-1,2,3,4-tetrahydrocyclopenta[b]indol-3-yl)acetate(6.2 mg, 0.015 mmol) in THF/MeOH (1:1, 1 mL) was added 1 M aqueous LiOH (0.06 mL). After stirring overnight, the mixture was concentrated. The residue was taken up in EtOAc and washed with 1 M aqueous HCl. The organic phase was dried over MgSO₄, filtered, and concentrated to yield the title compound as a solid (4.6 mg). LCMS m/z = 388.3 [M + H]⁺. ¹H NMR (400 MHz, Methanol-*d*₄) δ ppm 2.22 (m, 1H), 2.42 (s, 6H), 2.54 (dd, *J* = 16.02, 7.70 Hz, 1H), 2.73 (dd, *J* = 16.05, 7.03 Hz, 1H), 2.80-2.92 (m, 3H), 3.61 (m, 1H), 7.30 (s, 1H), 7.41 (d, *J* = 8.51 Hz, 1H), 7.78 (dd, *J* = 8.55, 1.56 Hz, 1H), 7.82 (s, 2H), 8.15 (s, 1H).

### Example 1.55: Preparation of 2-(7-(5-(3,5-Difluorophenyl)-1,2,4-oxadiazol-3-yl)-1,2,3,4-tetrahydrocyclopenta[b]indol-3-yl)acetic Acid (Compound 4).

From 3,5-difluorobenzoyl chloride, using a similar method to the one described in Example 1.54, the title compound was obtained as a solid. LCMS *m*/*z* = 396.1 [M + H]⁺. ¹H NMR (400 MHz, Methanol-*d*₄) δ ppm 2.19 (m, 1H), 2.50 (dd, *J* = 15.42, 7.42 Hz, 1H), 2.64 (dd, *J* = 15.50,7.60 Hz, 1H), 2.76-2.87 (m, 3H), 3.60 (m, 1H), 7.28 (m, 1H), 7.36 (d, *J* = 8.51 Hz, 1H), 7.72-7.78 (m, 3H), 8.09 (s, 1H).

### Example 1.56: Preparation of 2-(7-(5-(3,5-Dimethoxyphenyl)-1,2,4-oxadiazol-3-yl)-1,2,3,4-tetrahydrocyclopenta[b]indol-3-yl)acetic Acid (Compound 38).

From 3,5-dimethoxybenzoyl chloride, using a similar method to the one described in **Example 1.54,** the title compound was obtained as a solid. LCMS *m*/*z* = 420.2 [M + H]⁺. ¹H NMR (400 MHz, Methanol-*d*₄) δ ppm 2.22 (m, 1H), 2.56 (dd, *J* = 16.22, 7.75 Hz, 1H), 2.75 (dd, *J* = 16.26, 7.00 Hz, 1H), 2.81-2.92 (m, 3H), 3.62 (m, 1H), 3.90 (s, 6H), 7.28 (t, *J* = 2.28 Hz, 1H), 7.35 (d, *J* = 2.27 Hz, 2H), 7.42 (d, *J* = 8.53 Hz, 1H), 7.79 (dd, *J* = 8.49,1.53 Hz, 1H), 8.16 (s, 1H).

### Example 1.57: Preparation of 2-(7-(5-(3,5-Dichlorophenyl)-1,2,4-oxadiazol-3-yl)-1,2,3,4-tetrahydrocyclopenta[b]indol-3-yl)acetic Acid (Compound 35).

From 3,5-dichlorobenzoyl chloride, using a similar method to the one described in **Example 1.54,** the title compound was obtained as a solid. LCMS *m*/*z* = 428.1 [M + H]⁺. ¹H NMR (400 MHz, Methanol-*d*₄) δ ppm 2.21 (m, 1H), 2.55 (dd, *J* = 16.13, 7.77 Hz, 1H), 2.72-2.92 (m, 4H), 3.61 (t, *J =* 6.88 Hz, 1H), 7.39 (d, *J* = 8.53 Hz, 1H), 7.71 (m, 1H), 7.76 (dd, *J* = 8.49 Hz, 1.56 Hz, 1H), 8.07 (d, *J* = 1.87 Hz, 2H), 8.10 (s, 1H).

### Example 1.58: Preparation of 2-(7-(5-(3-Fluoro-5-(trifluoromethyl)phenyl)-1,2,4-oxadiazol-3-yl)-1,2,3,4-tetrahydrocyclopenta[b]indol-3-yl)acetic Acid (Compound 12).

From 3-fluoro-5-(trifluoromethyl)benzoyl chloride, using a similar method to the one described in Example 1.54, the title compound was obtained as a solid. LCMS *m*/*z* = 446.4 [M + H]⁺. ¹H NMR (400 MHz, Methanol-*d*₄) δ ppm 2.22 (m, 1H), 2.55 (dd, *J* = 16.14, 7.78 Hz, 1H), 2.71-2.93 (m, 4H), 3.60 (t, *J* = 6.72, 1H), 7.40 (d, *J* = 8.52 Hz, 1H), 7.78 (m, 2H), 8.15 (m, 2H), 8.28 (s, 1H).

### Example 1.59: Preparation of 2-(7-(5-(3,5-Diethoxyphenyl)-1,2,4-oxadiazol-3-yl)-1,2,3,4-tetrahydrocyclopenta[b]indol-3-yl)acetic Acid (Compound 68).

From 3,5-diethoxybenzoyl chloride, using a similar method to the one described in Example 1.54, the title compound was obtained as a solid. LCMS *m*/*z* = 448.5 [M + H]⁺. ¹H NMR (400 MHz, Methanol-*d*₄) δ ppm 1.41 (t, *J* = 7.67, 6H), 2.19 (m, 1H), 2.54 (dd, *J* = 16.87, 7.86 Hz, 1H), 2.70-2.89 (m, 4H), 3.58 (m, 1H), 4.08 (m, 4H), 6.66 (m, 1H), 7.24 (m, 2H), 7.39 (m, 1H), 7.75 (m, 1H), 8.09 (s, 1H).

### Example 1.60: Preparation of Ethyl 2-(7-(5-(4-(Trifluoromethoxy)phenyl)-1,2,4-oxadiazol-3-yl)-1,2,3,4-tetrahydrocyclopenta[b]indol-3-yl)acetate.

To a solution of ethyl 2-(7-(*N*-hydroxycarbamimidoyl)-1,2,3,4-tetrahydrocyclopenta[b]indol-3-yl)acetate (0.100 g, 0.332 mmol) and triethylamine (0.046 mL, 0.332 mmol) in THF (2 mL) was added 4-(trifluoromethoxy)benzoyl chloride (0.052 mL, 0.332 mmol). The reaction was stirred overnight at room temperature. The mixture was concentrated under reduced pressure. The residue was dissolved in DMF (2mL) and heated to 100°C for 4 h. The reaction was cooled to room temperature and the solvent was removed under reduced pressure. The residue was purified on silica gel eluting with 5% and 10% EtOAc/hexanes to yield the title compound as a white solid (0.112 g). LCMS *mlz* = 472.3 [M + H]⁺.

### Example 1.61: Preparation of 2-(7-(5-(4-(Trifluoromethoxy)phenyl)-1,2,4-oxadiazol-3-yl)-1,2,3,4-tetrahydrocyclopenta[bjindol-3-yl)acetic Acid (Compound 16).

To a solution of ethyl 2-(7-(5-(4-(trifluoromethoxy)phenyl)-1,2,4-oxadiazol-3-yl)-1,2,3,4-tetrahydrocyclopenta[b]indol-3-yl)acetate (0.0458 g, 0.097 mmol) in 2% H₂O/ACN (1.5 mL) was added triethylamine (0.041 mL, 0.291 mmol) and lithium bromide (0.084 g, 0.972 mmol). The mixture was warmed to 75°C and stirred overnight. Additional lithium bromide (0.084 g, 0.972 mmol) was added and the reaction was again stirred overnight. The mixture was concentrated and the residue was purified by preparative HPLC to give the title compound as a white solid (0.0086 g). LCMS *m*/*z* = 444.4 [M + H]⁺.

### Example 1.62: Preparation of 2-(7-(5-(2-Chloro-6-methylpyridin-4-yl)-1,2,4-oxadiazol-3-yl)-1,2,3,4-tetrahydrocyclopenta[b]indol-3-yl)acetic Acid (Compound 34).

### Step A: Preparation of Ethyl 2-(7-(5-(2-Chloro-6-methylpyridin-4-yl)-1,2,4-oxadiazol-3-yl)-1,2,3,4-tetrahydrocyclopenta[b]indol-3-yl)acetate.

From 2-chloro-6-methylisonicotinoyl chloride, the title compound was obtained using a similar method to the one described in **Example 1.60,** except that it was purified by triturating with DCM instead of column chromatography. LCMS *m*/*z* = 437.4 [M + H]⁺.

### Step B: Preparation of 2-(7-(5-(2-Chloro-6-methylpyridin-4-yl)-1,2,4-oxadiazol-3-yl)-1,2,3,4-tetrahydrocyclopenta[b]indol-3-yl)acetic Acid (Compound 34).

From ethyl 2-(7-(5-(2-chloro-6-methylpyridin-4-yl)-1,2,4-oxadiazol-3-yl)-1,2,3,4-tetrahydrocyclopenta[b]indol-3-yl)acetate, using a similar method to the one described in **Example 1.54,** the title compound was obtained as a solid. LCMS m/z = 409.3 [M + H]⁺.

### Example 1.63: Preparation of Ethyl 2-(7-(5-(4-Methoxy-3-(trifluoromethyl)phenyl)-1,2,4-oxadiazol-3-yl)-1,2,3,4-tetrahydrocyclopenta[b]indol-3-yl)acetate.

From 4-methoxy-3-(trifluoromethyl)benzoyl chloride, using a similar method to the one described in Example 1.60, the title compound was obtained as white solid. LCMS m/z = 486.4 [M + H]⁺. ¹H NMR (400 MHz, CDCl₃) δ ppm 1.31 (t, *J* = 7.16 Hz, 3H), 2.15 (m, 1H), 2.54 (dd, *J* = 16.94, 11.5 Hz, 1H), 2.76-2.98 (m, 4H), 3.58 (m, 1H), 4.19, (s, 3H), 4.23 (m, 2H), 7.17 (d, *J* = 8.80 Hz, 1H), 7.41 (d, *J* = 8.97 Hz, 1H), 7.92 (dd, *J* = 8.53, 1.62 Hz, 1H), 8.30 (m, 1H), 8.38 (dd, *J* = 8.69, 2.13 Hz, 1H), 8.48 (d, *J* = 1.93 Hz, 1H), 8.82 (s, 1H).

### Example 1.64: Preparation of 2-(7-(5-(4-Methoxy-3-(trifluoromethyl)phenyl)-1,2,4-oxadiazol-3-yl)-1,2,3,4-tetrahydrocyclopenta[b]indol-3-yl)acetic Acid (Compound 42).

From ethyl 2-(7-(5-(4-methoxy-3-(trifluoromethyl)phenyl)-1,2,4-oxadiazol-3-yl)-1,2,3,4-tetrahydrocyclopenta[b]indol-3-yl)acetate, using a similar method to the one described in **Example 1.61,** the title compound was obtained as a solid. LCMS *m*/*z* = 458.2 [M + H]⁺. ¹H NMR (400 MHz, Methanol-*d₄*) δ ppm 2.23 (m, 1H), 2.56 (dd, *J* = 16.17, 7.81 Hz, 1H), 2.75 (dd, *J* = 16.34, 6.93 Hz, 1H), 2.81-2.92 (m, 3H), 3.62 (m, 1H), 4.05 (s, 3H), 7.44 (t, *J* = 9.15 Hz, 2H), 7.81 (dd, *J* = 8.28, 1.58 Hz, 1H), 8.17 (s, 1H), 8.43 (m,2H).

### Example 1.65: Preparation of Ethyl 2-(7-(5-(4-Methoxy-3-methylphenyl)-1,2,4-oxadiazol-3-yl)-1,2,3,4-tetrahydrocyclopenta[b]indol-3-yl)acetate.

To a solution of 4-methoxy-3-methylbenzoic acid (0.026 g, 0.158 mmol), and oxalyl chloride (0.158 mL, 0.315 mmol) in DCM (1.00 mL) was added a catalytic amount of DMF (0.976 µl, 0.013 mmol). The reaction was stirred for 1 h to form 4-methoxy-3-methylbenzoyl chloride. The solvent was removed under reduced pressure and the residue was taken up in THF (5.0 mL). To this solution of the acid chloride was added ethyl 2-(7-(*N*-hydroxycarbamimidoyl)-1,2,3,4-tetrahydrocyclopenta[b]indol-3-yl)ethaneperoxoate (0.050 g, 0.158 mmol) and triethylamine (0.022 mL, 0.158 mmol). The reaction was stirred for 1 h. The solvent was removed and the residue was dissolved in DMF (5.00 mL) and the solution was stirred at 80 °C overnight. The mixture was diluted with EtOAc and washed twice with water. The organic phase was dried over MgSO₄ filtered and concentrated. The residue was purified by flash chromatography (0%-25% EtOAc/hexanes) to give the title compound as a solid (0.0295 g). LCMS *m*/*z* = 432.1 [M + H]⁺.

### Example 1.66: Preparation of 2-(7-(5-(4-Methoxy-3-methylphenyl)-1,2,4-oxadiazol-3-yl)-1,2,3,4-tetrahydrocyclopenta[b]indol-3-yl)acetic Acid (Compound 13).

From ethyl 2-(7-(5-(4-methoxy-3-methylphenyl)-1,2,4-oxadiazol-3-yl)-1,2,3,4-tetrahydrocyclopenta[b]indol-3-yl)acetate, using a similar method to the one described in **Example 1.61,** the title compound was obtained as a solid. LCMS *m*/*z* = 404.3 [M + H]⁺.

### Example 1.67: Preparation of Ethyl 2-(7-(5-(3-Chloro-4-methoxyphenyl-1,2,4-oxadiazol-3-yl)-1,2,3,4-tetrahydrocyctopenta[b]indol-3-yl)acetate.

From 3-chloro-4-methoxybenzoic acid, using a similar method to the one described in Example 1.65, the title compound was obtained as a solid. LCMS *m*/*z* = 452.2 [M + H)⁺.

### Example 1.68: Preparation of 2-(7-(5-(3-Chloro-4-methoxyphenyl)-1,2,4-oxadiazol-3-yl)-1,2,3,4-tetrahydrocyclopenta[b]indol-3-yl)acetic Acid (Compound 43).

From ethyl 2-(7-(5-(3-chloro-4-methoxyphenyl)-1,2,4-oxadiazol-3-yl)-1,2,3,4-tetrahydrocyclopenta[b]indol-3-yl)acetate, using a similar method to the one described in **Example 1.61,** the title compound was obtained as a solid. LCMS *m*/*z* = 424.3 [M + H]⁺.

### Example 1.69: Preparation of 2-(7-(5-(3-Cyano-5-isopropoxyphenyl)-1,2,4-oxadiazol-3-yl)-1,2,3,4-tetrahydrocyclopenta[b]indol-3-yl)acetic Acid (Compound 3).

From methyl 3-cyano-5-hydroxybenzoate and 2-bromopropane, using a similar method to the one described in **Example 1.53,** the title compound was obtained as a solid. LCMS *m*/*z* = 443.3 [M + H]⁺.

### Example 1.70: Preparation of 2-(7-(5-(3-(Pyrrolidin-1-yl)-5-(trifluoromethoxy)phenyl)-1,2,4-oxadiazol-3-yl)-1,2,3,4-tetrahydrocyclopenta[b]indol-3-yl)acetic Acid (Compound 20).

### Step A: Preparation of 3-(Pyrrolidin-1-yl)-5-(trifluoromethoxy)benzoic Acid.

3-Bromo-5-(trifluoromethoxy)benzoic acid (1.0 g, 3.51mmol), pyrrolidine (0.499 g, 7.02 mmol), copper(I) iodide (0.100 g, 0.526 mmol), cesium carbonate (2.51 g, 7.72 mmol), and 1,10-phenanthroline (0.158 g, 0.877 mmol) were suspended in DMSO (10 mL) in a 20 mL vial. The reaction mixture was heated under microwave irradiation at 150°C for 6 h. The reaction mixture was added to chilled water, brought to pH 3 using aqueous 6 N HCl and extracted with EtOAc (2 x 100 mL). The organic layer was washed with 1 M aqueous citric acid (3 x 50 mL). The organic layer was washed with brine, and concentrated. The residue was purified by preparative HPLC to give the title compound as a yellow solid (0.230 g). LCMS *m*/*z* = 276.1 [M + H]⁺; ¹H NMR (400 MHz, DMSO*d*₆) δ ppm 1.95-1.98 (m, 4H), 3.25-328 (m, 4H), 6.63 (s, 1H), 6.98 (s, 1H), 7.06 (s, 1H).

### Step B: Preparation of 3-(Pyrrolidin-1-yl)-5-(trifluoromethoxy)benzoyl Chloride.

3-(Pyrrolidin-1-yl)-5-(trifluoromethoxy)benzoyl chloride was prepared by reaction of 3-(pyrrolidin-1-yl)-5-(trifluoromethoxy)benzoic acid in DCM and one drop of DMF with 3.0 equivalents oxalyl chloride at room temperature for 1 h. The yellow solution was concentrated, and the resulting residue was used in the next step without further purification.

### Step C: Preparation of Ethyl 2-(7-(5-(3-(Pyrrolidin-1-yl-5-(trifluoromethoxy)phenyl)1,2,4-oxadiazol-3-yl)-1,2,3,4-tetrahydrocyclopenta[b]indol-3-yl)acetate.

To a solution of ethyl 2-(7-(*N*-hydroxycarbamimidoyl)-1,2,3,4-tetrahydrocyclopenta[b]indol-3-yl)acetate (0.050 g, 0.166 mmol) and triethylamine (0.093 mL, 0.664 **mmol).in** dioxane (2 mL) was added 3-(pyrrolidin-1-yl)-5-(trifluoromethoxy)benzoyl chloride (0.097 g, 0.332 mmol) in dioxane (2 mL) at 0°C. The resulting suspension was stirred at room temperature for 1 h. The solvent was removed under reduced pressure. The residue was taken up in DMA (4 mL) and heated at 100°C for 30 min. DMA was evaporated and the residue was purified by preparative HPLC to give the title compound. LCMS *m*/*z* = 541.3 [M + H]⁺.

### Step D: Preparation of 2-(7-(5-(3-(pyrrolidin-1-yl)-5-(trifluoromethoxy)phenyl)-1,2,4-oxadiazol-3-yl)-1,2,3,4-tetrahydrocyclopenta[b]indol-3-yl)acetic Acid (Compound 20).

Ethyl 2-(7-(5-(3-(pyrrolidin-1-yl)-5-(trifluoromethoxy)phenyl)-1,2,4-oxadiazol-3-yl)-1,2,3,4-tetrahydrocyclopenta[b]indol-3-yl)acetate from the above step was dissolved in THF (800 µL) and methanol (800 µL), and to this was added 2 M NaOH (0.139 mL, 0.278 mmol). The mixture was stirred at room temperature for 4 h. The volatiles were evaporated under reduced pressure. Water (10 mL) was added, the mixture was acidified to pH 3 using 6 N HCl, extracted with EtOAc, and the organic phase was concentrated. The residue was purified by preparative HPLC to give the title compound as a tan solid (0.008 g). LCMS *m*/*z* = 513.4 [M + H]⁺; ¹H NMR (400 MHz, Methanol-*d*₄) δ ppm 1.97-2.04 (m, 4H), 2.08-2.15 (m, 1H), 2.41-2.46 (m, 1H), 2.62-2.73 (m, 4H), 3.20-3.28 (m, 4H), 3.48-3.53 (m, 1H), 6.52 (s, 1H), 7.17 (m, 2H), 7.32 (d, *J* = 8.59 Hz, 1H), 7.69 (dd, *J* = 8.59Hz, 1.77 Hz, 1H), 8.05 (s, 1H).

### Example 1.71: Preparation of 2-(7-(5-(3-Morpholino-5-(trifluoromethoxy)phenyl)-1,2,4-oxadiazol-3-yl)-1,2,3,4-tetrahydrocyclopenta[b]indol-3-yl)acetic Acid (Compound 25).

### Step A: Preparation of 3-Morpholino-5-(trifluoromethoxy)benzoic Acid.

3-Bromo-5-(trifluoromethoxy)benzoic acid (1g, 3.51 mmol), morpholine (0.957g, 10.5 mmol), copper(I) iodide (0.134 g, 0.702 mmol), cesium carbonate (2.51 g, 7.72 mmol), and 1,10-phenanthroline (0.221 g, 1.228mmol) were suspended in DMSO (10 mL) in a 20L vial. The reaction mixture was heated under microwave irradiation at 175°C for 6 h. The reaction mixture was added to chilled water, acidified to pH 3 using aqueous 6 N HCl and extracted with EtOAc (3 x 100 mL). The organic layers were combined, washed with 1 M aqueous citric acid (3 x 50 mL) and brine, and concentrated. The residue was purified by preparative HPLC to give the title compound as a brown oil (0.350 g). LCMS *m*/*z* = 292.3. [M + H]⁺.

### Step B: Preparation of 3-Morpholino-5-(trifluoromethoxy)benzoyl Chloride

3-Morpholino-5-(trifluoromethoxy)benzoyl chloride was prepared by reaction of 3-morpholino-5-(trifluoromethoxy)benzoic acid in DCM and one drop of DMF with 3.0 equivalents oxalyl chloride at room temperature for 1h. The yellow solution was concentrated, and the resulting residue was used in the next step without further purification.

### Step C: Preparation of Ethyl 2-(7-(5-(3-Morpholino-5-(trifluoromethoxy)phenyl)-1,2,4-oxadiazol-3-yl)-1,2,3,4-tetrahydrocyclopenta[b]indol-3-yl)acetate.

To a solution of ethyl 2-(7-(*N*-hydroxycarbamimidoyl)-1,2,3,4-tetrahydrocyclopenta[b]indol-3-yl)acetate (0.050 g, 0.166 mmol) and triethylamine (0.093 mL, 0.64 mmol) in dioxane (2 mL) was added 3-morpholino-5-(trifluoromethoxy)benzoyl chloride (0.103 g, 0.332 mmol) in dioxane (2 mL) at 0°C. The resulting suspension was stirred at room temperature for 3 h. The solvent was removed under reduced pressure. The residue was taken up in DMA (4 mL) and heated at 100°C for 1h. DMA was evaporated and the residue was purified by preparative HPLC to give the title compound. LCMS *m*/*z* = 557.5 [M + H]⁺.

### Step D: Preparation of 2-(7-(5-(3-Morpholino-5-(trifluoromethoxy)phenyl-1,2,4-oxadiazol-3-yl)-1,2,3,4-tetrahydrocyclopenta[b]indol-3-yl)acetic Acid (Compound 25).

Ethyl 2-(7-(5-(3-morpholino-5-(trifluoromethoxy)phenyl)-1,2,4-oxadiazol-3-yl)-1,2,3,4-tetrahydrocyclopenta[b]indol-3-yl)acetate from **Step C** was dissolved in THF (800 µL) and methanol (800 pL), and to this was added 2 M NaOH (0.135 mL, 0.270 mmol). The mixture was stirred at room temperature for 4 h. The organic layers were combined and concentrated under reduced pressure. Water (10 mL) was added, the mixture was acidified to pH 3 using aqueous 6 N HCl, extracted with ethyl acetate, and the organic phase was concentrated. The residue was purified by preparative HPLC to give the title compound as a tan solid (0.004 g). LCMS *m*/*z* = 529.2 [M + H]⁺; ¹H NMR (400 MHz, CDCl₃) δ ppm 2.04-2.15 (m, 1H), 2.58-2.61 (m, 1H), 2.73-2.81 (m, 4H), 3.22-3.24 (m, 4H), 3.50-3.60 (m, 1H), 3.83-3.85 (m, 4H), 6.82 (s, 1H), 7.32 (d, *J* = 8.59 Hz, 1H), 7.49 (s, 1H), 7.59 (s, 1H), 7.84 (dd, *J* = 8.59Hz, 1.26 Hz, 1H), 8.21 (s, 1H), 8.56 (s, 1H).

### Example 1.72: Preparation of 2-(7-(5-(3-Methoxy-5-(trifluoromethyl)phenyl)-1,2,4-oxadiazol-3-yl)-1,2,3,4-tetrahydrocyclopenta[b]indol-3-yl)acetic Acid (Compound 8).

### Step A: Preparation of Ethyl 2-(7-(5-(3-Hydroxy-5-(trifluoromethyl)phenyl)-1,2,4-oxadiazol-3-yl-1,2,3,4-tetrahydrocyclopenta[b]indol-3-yl)acetate.

3-Hydroxy-5-(trifluoromethyl)benzoic acid (0.206 g, 1.00 mmol) was dissolved in anhydrous DMF (2.00 mL). PyBOP (0.572 g, 1.10 mmol) and DIEA (0.384 mL, 2.20 mmol) were added and the reaction mixture was stirred at 25°C for 3 min. Ethyl 2-(7-(*N-*hydroxycarbamimidoyl)-1,2,3,4-tetrahydrocyclopenta[b]indol-3-yl)acetate(0.301 g, 1.00mmol) was then added at 25°C to give a dark amber solution which was stirred at 25°C for 2 h. The reaction was diluted with anhydrous DMF (3 mL) and heated at 90°C for 1h after which the solvent was evaporated under reduced pressure to a volume of 2 mL. The mixture was then diluted with MTBE (50 mL), washed with water twice, washed with brine, dried over MgSO₄ and the solvent was evaporated under reduced pressure to give the crude product which was purified by silica flash chromatography to afford the title compound as a white solid (0.217 g). LCMS m/z = 472.2 [M + H]⁺; ¹H NMR (400 MHz, DMSO-*d*₆) δ ppm 1.20 (t, *J* = 7.07 Hz, 3H), 2.16 (m, 1H), 2.54 (m, 1H), 2.66-2.94 (m, 4H), 3.57 (m, 1H), 4.13 (q, *J* = 7.07 Hz, 2H), 7.39 (s, 1H), 7.50 (d, *J =* 8.59 Hz, 1H), 7.76 (dd, *J*=8.59,1.52 Hz, 1H), 7.82 (s, 1H), 7.87 (s, 1H), 8.13 (d, *J* = 1.01 1Hz, 1H), 10.86 (s, 1H), 11.09 (s, 1H).

### Step B: Preparation of Ethyl 2-(7-(5-(3-Methoxy-5-(trifluoromethyl)phenyl-1,2,4-oxadiazol-3-yl)-1,2,3,4-tetrabydrocyclopenta[b]indol-3-yl)acetate.

Ethyl 2-(7-(5-(3-hydroxy-5-(trifluoromethyl)phenyl)-1,2,4-oxadiazol-3-yl)-1,2,3,4-tetrahydrocyclopenta[b]indol-3-yl)acetate (0.047 g, 0.10 mmol) was dissolved in anhydrous THF (0.500 mL). Triphenylphosphine (0.034 g, 0.130 mmol) and anhydrous methanol (5.26 µL, 0.130 mmol) were added followed by dropwise addition at 25 °C of DIAD (0.025 mL, 0.130 mmol) to give an amber solution which was stirred at 25 °C for 2 h. The reaction mixture was diluted with MTBE (25 mL), washed with water and brine, dried with MgSO₄, and the solvent was evaporated under reduced pressure to give an oil. The oil was purified by silica flash chromatography to give the title compound as a white solid (0.039 g). LCMS *m*/*z* = 486.4 [M + H]⁺; ¹H NMR (400 MHz, Acetonitrile-*d₃*) δ ppm 1.24 (t, J = 7.07 Hz, 3H), 2.15-2.25 (m, 1H), 2.64 (d, J = 7.33 Hz, 2H), 2.71-2.99 (m, 3H), 3.52-3.67 (m, 1H), 3.97 (s, 3H), 4.17 (q, J = 7.07 Hz, 2H), 7.47.7.55 (m, 2H), 7.84 (dd, *J* = 8.59, 1.52 Hz, 1H), 7.94 (s, 1H), 8.05 (s, 1H), 8.22 (s, 1H), 9.28 (bs, 1H).

### Step C: Preparation of 2-(7-(5-(3-Methoxy-5-(trifluoromethyl)phenyl)-1,2,4-oxadiazol-3-yl)-1,2,3,4-tetrahydrocyclopenta[b]indol-3-yl)acetic Acid (Compound 8).

Ethyl 2-(7-(5-(3-methoxy-5-(trifluoromethyl)phenyl)-1,2,4-oxadiazol-3-yl)-1,2,3,4-tetrahydrocyclopenta[b]indol-3-yl)acetate (39 mg, 0.080 mmol) was dissolved in 1,4-dioxane (1.0 mL). Aqueous LiOH (1.0 M, 0.32 mL, 0.32 mmol) was added and the reaction was heated' at 90°C for 1 h. The reaction mixture was acidified with 1.0 M aqueous HCl (0.40 mL, 0.40 mmol), diluted with EtOAc (25 mL) and the organic layer was washed with water twice, followed by brine, dried with MgSO₄ and the solvent was evaporated under reduced pressure to give an oil. The oil was dissolved in a minimum volume of DCM (2 mL) and co-evaporated with excess hexanes (8 mL) to give the title compound as a white solid (35 mg). LCMS *m*/*z* = 458.3 [M + H]⁺; ¹H NMR (400 MHz, Acetonitrile-*d*₃) δ ppm 2.19 (d, *J* = 3.03 Hz, 1H), 2.65 (d, *J* = 7.07 Hz, 2H), 2.73-3.00 (m, 3H), 3.60 (s, 1 H), 3.97 (s, 3H), 7.45-7.56 (m, 2H), 7.84 (d, *J* = 8.59 Hz, 1H), 7.95 (s, 1H), 8.06 (s, 1H), 8.23 (s, 1H), 9.31 (bs, 1H).

### Example 1.73: Preparation of 2-(7-(5-(3-Propoxy-5-(trifluoromethyl)phenyl)-1,2,4-oxadiazol-3-yl)-1,2,3,4-tetrahydrocyclopenta[b]indol-3-yl)acetic Acid (Compound 24).

The title compound was prepared using a similar method to the one described in **Example 1.72.** LCMS ***m*/*z*** = 486.4 [M + H]⁺.

### Example 1.74: Preparation of 2-(7-(5-(3-Isopropoxy-5-(trifluoromethyl)phenyl)-1,2,4-oxadiazol-3-yl)-1,2,3,4-tetrahydrocyclopenta[b]indol-3-yl)acetic Acid (Compound 67).

The title compound was prepared using a similar method to the one described in **Example 1.72.** LCMS ***m*/*z*** = 486.4 [M + H]⁺;¹H NMR (400 MHz, Acetonitrile-*d*₃) δ ppm 1.38 (d, *J* = 6.06 Hz, 6H), 2.17-2.26 (m, 1H), 2.65 (d, *J* = 7.07 Hz, 2H), 2.72-2.99 (m, 3H), 3.50-3.65 (m, 1H), 4.71-4.95 (m, 1H), 7.45 (s, 1H), 7.51 (d, *J* = 8.59 Hz, 1H), 7.84 (dd, *J* = 8.59,1.52 Hz, 1H), 7.91 (s, 1H), 8.02 (s, 1H), 8.23 (s, 1H), 9.31 (bs, 1H).

### Example 1.75: Preparation of 2-(7-(5-(3-(Cyclopropylmethoxy)-5-(trifluoromethyl)phenyl)-1,2,4-oxadiazol-3-yl)-1,2,3,4-tetrahydrocyclopentalblindol-3-yl)acetic Acid (Compound 28).

The title compound was prepared using a similar method to the one described in **Example 1.72.** LCMS *m*/*z* = 498.5 [M + H]⁺; ¹H NMR (400 MHz. Acetonitrile-*d*₃) δ ppm 0.40 (d, *J* = 4.80 Hz, 2H), 0.65 (q, *J* = 5.89 Hz, 2H), 2.15-2.34 (m, 2H), 2.65 (d, *J* = 7.07 Hz, 2H), 2.72-2.98 (m, 3H), 3.50-3.67 (m, 1H), 4.01 (d, *J* = 7.07 Hz, 2H), 7.47 (s, 1H), 7.51 (d, *J* = 8.34 Hz, 1H), 7.84 (dd, *J* = 8.59, 1.52 Hz, 1H), 7.92 (s, 1H), 8.04 (s, 1H), 8.22 (s**,** 1H), 9.32 (bs, 1H).

### Example 1.76: Preparation of 2-(7-(5-(3-(Benzyloxy)-5-(trifluoromethyl)phenyl)-1,2,4-oxadiazol-3-yl)-1,2,3,4-tetrahydrocyclopenta[b]indol-3-yl)acetic Acid (Compound 49).

The title compound was prepared using a similar method to the one described in **Example 1.72.** LCMS *m*/*z* = 534.4 [M + H]⁺; ¹H NMR (400 MHz, Acetonitrile-*d*₃) δ ppm 2.19 (d, *J* = 3.03 Hz, 2H), 2.65 (d, *J* = 6.82 Hz, 2H), 2.71-3.05 (m, 3H), 3.47-3.70 (m, 1H), 5.29 (s, 2H), 7.17-7.68 (m, 7H), 7.84 (dd, *J* = 8.59,1.52 Hz, 1H), 7.96-8.16 (m, 2H), 8.23 (s, 1H), 9.33 (bs, 1H).

### Example 1.77: Preparation of 2-(7-(5-(3-Cyano-4-ethoxyphenyl)-1,2,4-oxadiazol-3-yl)-1,2,3,4-tetrahydrocyclopenta[b]indol-3-yl)acetic Acid (Compound 36).

To a solution of methanol (0.027 mL, 0.465 mmol) in THF (1.0 mL) was added a 60% oil dispersion of sodium hydride (18.58 mg, 0.465 mmol). After stirring at room temperature for 10 min, ethyl 2-(7-(5-(3-cyano-4-fluorophenyl)-1,2,4-oxadiazol-3-yl)-1,2,3,4-tetrahydrocyclopenta[b]indol-3-yl)acctate (50 mg, 0.116 mmol) was added. The reaction mixture was heated at 100°C for 60 min under microwave irradiation. It was diluted with water (0.5 mL), and NaOH (0.116 mL, 0.116 mmol) was added. The mixture was heated to 70°C in a sealed vial for 2 h. The mixture was concentrated under reduced pressure and purified via preparative LCMS to give the title compound as a white solid (7.0 mg). LCMS *m*/*z* = 429.3 [M + H]⁺;¹H NMR (400 MHz, DMSO-*d*₆) δ ppm 1.43 (t, *J* = 6.95 Hz, 3H), 2.09-2.21 (m, 1H), 2.43 (dd, *J* = 16.17,8.84 Hz, 1H), 2.70-2.89 (m, 4H), 3.54 (bs, 1H), 4.35 (q, *J* = 6.99 Hz, 2H), 7.50 (dd, *J* = 8.84, 7.07 Hz, 2H), 7.74 (dd, *J* = 8.46, 1.64 Hz, 1H), 8.12 (d, *J* = 1.52 Hz, 1H), 8.44 (dd, *J* = 8.84, 2.27 Hz, 1H), 8.53 (d, *J* = 2.27 Hz, 1H), 11.08 (s, 1H), 12.27 (s, 1H).

### Example 1.78: Preparation of 2-(7-(5-(3-Cyano-4-hydroxyphenyl)-1,2,4-oxadiazol-3-yl)-1,2,3,4-tetrahydrocyclopenta[b]indol-3-yl)acetic Acid (Compound 44).

The title compound was obtained as a white solid using a similar method to the one described in **Example 1.77.** LCMS *m*/*z* = 401.4 [M + H]⁺; ¹H NMR (400 MHz, DMSO-*d*₆) δ ppm 2.08-2.21 (m, 1H), 2.43 (dd, *J* = 16.17, 8.84 Hz, 1H), 2.63-2.92 (m, 4H), 3.54 (bs, 1H), 7.26(d*, J*=8.84 Hz*,* 1H), 7.49 (d*,J =* 8.59 Hz, 1H), 7.73 (dd, *J* = 8.59,1.52 Hz, 1H), 8.11(s, 1H), 8.30 (dd, *J* = 8.84, 2.27 Hz, 1H), 8.44 (d, *J* = 2.02 Hz, 1H), 11.07 (s, 1H), 12.26 (s, 1H).

### Example 1.79: Preparation of 2-(7-(5-(3-Cyano-5-(trifluoromethoxy)phenyl)-1,2,4-oxadiazol-3-yl)-1,2,3,4-tetrahydrocyclopenta[b]indol-3-yl)acetic Acid (Compound 7).

### Step A: Preparation of Methyl 2-(2-Oxocyctopentyl)acetate.

To a solution of 2-(2-oxocyclopentyl)acetic acid (48.0 g, 338 mmol) in acetone (1000 mL) was added anhydrous K₂CO₃ (65.3 g, 473 mmol) and iodomethane (427 g, 3.009 mol). The reaction mixture was refluxed for 4 h. The solid was filtered and the filter cake was washed with acetone (2 x 50 mL). The filtrate was concentrated and DCM (1000 mL) and H₂O (400 mL) were added to the residue. The layers were separated and the aqueous layer was extracted with DCM (2 x 500 mL). The combined organic layers were dried over Na₂SO₄, decanted, concentrated, and dried to give the title compound as a colorless liquid (53.8 g). ¹H NMR (400 MHz, CDCl₃) δ ppm 1.56-1.68 (m, 1H), 1.75-1.89 (m, 1H), 2.01-2.11 (m, 1H), 2.12-2.22 (dd, *J* = 18.66, 10.73 Hz, 1H), 2.26-2.36 (m, 2H), 2.37-2.51 (m, 2H), 2.77 (d, *J* = 12.69 Hz, 1H), 3.69 (s, 3H).

### Step B: Preparation of Methyl 2-(7-Cyano-1,2,3,4-tetrahydrocyclopenta[b]indol-3-yl)acetate.

To a 1000 mL three-necked round-bottom flask filled with DMF (400 mL, deoxygenated with N₂ under reduced pressure) were sequentially added 4-amino-3-iodobenzonitrile (26.385 g, 108 mmol), pyridine 4-methylbenzenesulfonate (1.034 g, 4.11 mmol), tetraethoxysilane (30.8 mL, 137 mmol), and methyl 2-(2-oxocyclopentyl)acetate (25.4 g, 162 mmol) under N₂. The reaction mixture was heated at 135 °C overnight in the dark. To the mixture were added N-ethyl-N-isopropylpropan-2-amine (56.7 mL, 325 mmol) and palladium (II) acetate (0.729 g, 3.25 mmol) under N₂. The resulting mixture was cooled to 120°C and stirred for additional 16 h. The reaction mixture was cooled to room temperature and acidified to pH ∼5 with 1 N aqueous HCl. The solvent was evaporated under reduced pressure at 60 °C. To the liquid residue was added 500 mL of water and 1000 mL of EtOAc. The organic layer was separated and the aqueous layer was extracted with EtOAc (2 x 400 mL). The combined organic layers were dried over Na₂SO₄, decanted and concentrated under reduced pressure to afford a dark brown liquid (46.2 g). The liquid was purified *via* silica flash chromatography (0%-100% EtOAc/hexanes). The collected fractions were concentrated and further purified by precipitation in DCM/hexanes to yield the title compound as a light yellow solid (10.3 g). LCMS *m*/*z* = 255.4 [M + H]⁺; ¹HNMR(400MHz,CDCl₃) δ ppm 2.09-2.18 (m, 1H), 2.47-2.57 (dd, *J* = 17.11, 11.71 Hz, 1H), 2.75-2.94 (m, 4H), 3.51-3.63 (m, 1H), 3.77 (s, 3H), 7.34 (d, *J* = 0.97 Hz, 2H), 7.78 (s, 1H), 8.98 (s, 1H).

### Step C: Preparation of Methyl 2-(7-(N-Hydroxycarbamimidoyl)-1,2,3,4-tetrahydrocyclopenta(blindol-3-yl)acetate.

To a suspension of methyl 2-(7-cyano-1,2,3,4-tetrahydrocyclopenta[b]indol-3-yl)acetate (5.81 g, 22.85 mmol) in ethanol (156 mL) in a 200 mL round bottom flask at 70°C was added 50% aqueous solution of hydroxylamine (14.0 mL, 228.5 mmol). The resulting mixture was stirred in the closed flask at 70°C for 140 min. The mixture was cooled in an ice-bath and then poured into 250 mL of ice-water. The resulting mixture was concentrated at water bath temperature (< 22°C) to ∼ 120 mL. The aqueous suspension was extracted with DCM/IPA (3/1, 3 x 250 mL), dried over Na₂SO₄, decanted, and concentrated to give the title compound as a light orange solid (6.71 g). LCMS *m*/*z* = 288.3 [M + H]⁺; ¹H NMR (400 MHz, DMSO-*d*₆) δ ppm 2.05-2.17 (m, 1H), 2.45-2.58 (m, 1H), 2.64-2.85 (m, 4H), 3.45-3.55 (m, 1H), 3.66 (s, 3H), 5.66 (s, 2H), 7.27 (d,*J*= 8.56 Hz, 1H), 7.37 (dd,*J*= 8.56,1.48 Hz, 1H), 7.65 (s, 1H), 9.31 (s, 1H), 10.73(s, 1H).

### Step D: Preparation of Methyl 2-(7-(5-(3-Cyano-5-(trifluoromethoxy)phenyl)-1,2,4-oxadiazol-3-yl)-1,2,3,4-tetrahydrocyclopenta[b]indol-3-yl)acetate.

To a solution of methyl 2-(7-(*N*-hydroxycarbamimidoyl)-1,2,3,4-tetrahydrocyclopenta[b]indol-3-yl)acetate (6.83 g, 23.77 mmol) in dioxane (60 mL) in a 200 mL round bottom flask was added 3-cyano-5-(trifluoromethoxy)benzoyl chloride (5.93 g, 23.77 mmol) in dioxane (10 mL) and triethylamine (11.6 mL, 83 mmol). The reaction mixture was heated at 70°C for 2 h. The solvent was removed under reduced pressure and to the residue was added H₂O (200 mL) and 1 N NaOH to adjust pH to ∼3. The mixture was extracted with EtOAc (3 x 300 mL), dried over Na₂SO₄, decanted, and concentrated to give a brown residue. The brown residue was purified via silica flash chromatography (5%-100% EtOAc in hexanes). The collected fractions were concentrated and further purified by precipitation in DCM/hexanes to afford the title compound as a light yellow solid. LCMS *m*/*z* = 483.2 [M + H]⁺; ¹H NMR (400 MHz, CDCl₃) δ ppm 2.14-2.25 (m, 1H), 2.53-2.63 (dd, *J* = 17.02, 11.52 Hz, **1H),** 2.79-3.03 (m, 4H), 3.57-3.67 (m, 1H)**,** 3.80 (s, 3H), 7.44 (d, *J* = 8.53 Hz, 1H), 7.74 (m, 1H), 7.93 (dd, *J* = 8.54,1.52 Hz, 1H), 8.31 (m, 1H), 8.33 (m, 1H), 8.49 (s, 1H), 8.87 (s, 1H).

### Step E: Preparation of 2-(7-(5-(3-Cyano-5-(trifluoromethoxy)phenyl)-1,2,4-oxadiazol-3-yl)1,2,3,4-tetrahydrocyclopenta[b]indol-3-yl)acetic Acid (Compound 7).

To a solution of methyl 2-(7-(5-(3-cyano-5-(trifluoromethoxy)phenyl)-1,2,4-oxadiazol-3-yl)-1,2,3,4-tetrahydrocyclopenta[b]indol-3-yl)acetate (3.05 g, 6.32 mmol) in 2% H₂O in ACN (30 mL) in a 100 mL round bottom flask was added lithium bromide (5.49 g, 63.2 mmol) and TEA (2.63 mL, 18.97 mol). The reaction mixture was heated at 75 °C for 90 min. After cooling to room temperature, 0.5 N HCl (∼40 mL) was added to the mixture to adjust pH to ∼3, followed by EtOAc (200 mL) and H₂O (80 mL). The aqueous layer was separated and extracted with EtOAc (2 x 150 mL). The combined organic layers were dried over Na₂SO₄, decanted, and concentrated to give a light yellow solid (3.1 g). The light yellow solid was purified by precipitation in DCM/hexanes to afford the title compound as an off-white solid (2.69 g). LCMS *m*/*z* = 469.2 [M + H]⁺; ¹H NMR (400 MHz, CDCl₃) δ ppm 2.12-2.26 (m, 1H), 2.60-2.71 (dd, *J =* 17.17, 11.23 Hz, 1H), 2.79-3.01 (m, 4H), 3.58-3.69 (m, 1H), 7.43 (d,*J*= 8.46 Hz, 1H), 7.72 (m, 1H), 7.92 (dd, J = 8.50, 1.61 Hz, 1H), 8.30 (bs, 1H), 8.31 (m, 1H), 8.47 (t, J = 1.35 Hz, 1H), 8.68 (s, 1H).

### Example 1.80: Preparation of 7-(5-(3-cyano-5-(trifluoromethoxy)phenyl)-1,2,4-oxadiazol-3-yl)-1,2,3,4-tetrahydrocyclopenta[b]indole-3-carboxylic acid (Compuond 73)

### Step A: Preparation of Ethyl 7-Bromo-1,2,3,4-tetrahydrocyclopenta[b]indole-3-carboxylate

4-Bromophenylhydrazine hydrochloride (0.5 g, 2.24 mmol) and ethyl 2-oxocyclopentanecarboxylate (0.350 g, 2.24 mmol) was suspended in AcOH (4 mL) and the heterogeneous mixture was warmed to 75 °C and stirred for 16 h. The reaction mixture was poured into saturated aqueous NaHCO₃ and extracted with EtOAc. The EtOAc solution was dried over Na₂SO₄, filtered, and concentrated under reduced pressure. The concentrate was dissolved in MeOH and passed through a strong cation exchange column (Strata SCX) washing with MeOH. The filtrate was concentrated under reduced pressure and dissolved in 1:1 EtOAclhexanes and filtered through a pad of SiO₂. The filtrate was concentrated and the residue was further purified by column chromatography (SiO₂, 50% EtOAc in hexanes) to afford the title compound (218 mg). LCMS *m*/*z* = 308.0 [M+H⁺].

### Step B: Preparation of Ethyl 7-Cyano-1,2,3,4-tetrahydrocyclopenta[b]indol-3-carboxylate

Ethyl 7-bromo-1,2,3,4-tetrahydrocyclopenta[b]indole-3-carboxylate (218 mg, 0.71 mmol) was dissolved in NMP (1.0 mL) and CuCN (95 mg, 1.06 mmol) was added. The heterogeneous mixture was heated to 200°C (microwave) for 2 h. The mixture was diluted with DCM (100 mL) and filtered through celite. The filtrate was concentrated under reduced pressure and the residue was purified by column chromatography (SiO₂, 25% EtOAc in hexanes) to afford the title compound (54 mg). LCMS *m*/*z* = 255.5 [M+H⁺].

### Step C: Preparation of Ethyl 7-(N'-Hydroxycarbamimidoyl)-1,2,3,4-tetrahydrocyclopentalblindole-3-carboxylate

Ethyl 7-cyano-1,2,3,4-tetrahydrocyclopenta[b]indole-3-carboxylate (54 mg, 0.21 mmol) was dissolved in ethanol (5ml) and 50% hydroxylamine in water (0.130 mL, 2.12 mmol) was added. The reaction was warmed to 70°C and stirred for 8 h. The solution was concentrated to dryness under reduced pressure and the crude product was used without further purification. LCMS **m/z** 288.1 (M+H)⁺.

### Step D: Preparation of Ethyl 7-(5-(3-Cyano-5-(tritluoromethoxy)phenyl)-1,2,4-oxadiazol-3-yl)-1,2,3,4-tetrahydrocyclopenta[b]indole-3-carboxylate.

3-Cyano-5-(trifluoromethoxy)benzoyl chloride (0.053 g, 0.212 mmol) was dissolved in dioxane (0.89 ml) and triethylamine (0.030 ml, 0.21 mmol) and ethyl 7-(*N*'-hydroxycarbamimidoyl)-1,2,3,4-tetrahydrocyclopenta[b]indole-3-arboxylate (0.061 g, 0.21 mmol) were added. The reaction mixture was stirred for 30 minutes and then warmed to 70 °C. After stirring for 1 h, additional 3-cyano-5-(trifluoromethoxy)benzoyl chloride (0.053 g, 0.212 mmol) was added and the reaction mixture was stirred at 70°C for 2 h. The reaction was cooled to room temperature and then poured into NaHCO₃ and extracted with EtOAc. The extract was dried over Na₂SO₄, filtered, and concentrated under reduced pressure. The residue was purified by column chromatography (SiO₂, 10-25% EtOAc in hexanes) to afford the title compound (54 mg). LCMS *m*/*z* = 483.3 [M+H⁺]. ¹H NMR (400 MHz, CDCl₃) δ ppm 1.34 (t, *J* = 7.1 Hz, 3H), 2.82-2.95 (m, 3H), 2.96-3.08 (m, 1H), 4.12-4.19 (m, 1H), 4.21-4.29 (m, 2H), 7.46 (d, *J* = 8.6 Hz, 1H), 7.73 (s, 1H), 7.95 (dd, *J* = 8.6,1.8 Hz, 1H), 8.31-8.36 (m, 2H), 8.47-8.50 (m, 1H).

### Step E: Preparation of 7-(5-(3-cyano-5-(trifluoromethoxy)phenyl)-1,2,4-oxadiazol-3-yl)-1,2,3,4-tetrahydrocyclopenta[b]indole-3-carboxylic acid (Compound 73).

Ethyl 7-(5-(3-cyano-5-(trifluoromethoxy)phenyl)-1,2,4-oxadiazol-3-yl)-1,2,3,4-tetrahydrocyclopenta[b]indole-3-carboxylate (49.9 mg, 0.103 mmol) was dissolved in 2% water in acetonitrile (0.5 mL) and LiBr (90 mg, 1.03 mmol) and triethylamine (0.043 mL, 0.310 mmol) were added. The solution was warmed to 70°C and stirred for 8 h. The reaction mixture was acidified to pH4 with 1.0 M HCl and then extracted with EtOAc. The extract was dried over Na₂SO₄, filtered, and concentrated under reduced pressure. The residue was purified by HPLC to afford the title compound (22 mg). LCMS *m*/*z* = 455.2 [M+H⁺]. ¹H NMR (400 MHz, DMSO-*d*₆) δ ppm 2.64-2.96 (m, 4H), 4.07-4.14 (m, 1H), 7.50 (d, *J* = 8.6 Hz, 1H), 7.80 (dd, *J*= 8.6, 2.0 Hz, 1H), 8.19 (d*, J =* 1.0 Hz, 1H)*,* 8.44(d*, J =* 1.0 Hz, 1H), 8.47 (d*, J =* 1.0 Hz, 1H), 8.71-8.74 (m. 1H), 11.4 (s, 1H), 12.7 (bs, 1H).

### Example 1.81: Preparation of 2-(7-(5-(3-isopropoxy-5-(tritluoromethyl)phenyl)-1,2,4-oxadiazol-3-yl)-4-methyl-1,2,3,4-tetrahydrocyclopentalblindol-3-yl)acetic acid (Compound 101).

### Step A: Preparation of Methyl 2-(7-(5-(3-Isopropoxy-5(trifluoromethyl)phenyl) 1,2,4-oxadiazol-3-yl)-4-methyl-1,2,3,4-tetrahydrocyclopenta[b]indol-3-yl)acetate.

2-(7-(5-(3-Isopropoxy-5-(trifluoromethyl)phenyl)-1,2,4-oxadiazol-3-yl)-1,2,3,4-tetrahydrocyclopenta[b]indol-3-yl)acetic acid (31 mg, 0.064 mmol) was dissolved in anhydrous DMF (2.0 mL, 25.8 mmol). Sodium hydride (60% oil dispersion, 5.67 mg, 0.236 mmol) was added. The reaction was stirred at 25 °C for 20 min, then iodomethane (8.78 µL, 0.140 mmol) was added at 25°C. After stirring for 16 h additional sodium hydride (60% oil dispersion, 5.67 mg, 0.236 mmol) was added to the reaction which was stirred at 25°C for 20 min, then additional iodomethane (8.78 µL, 0.140 mmol) was added. After stirring for 5 h the reaction was acidified with aqueous 1 N HCl (2.5 mL), extracted with EtOAc (30 mL), the organic layer was washed with water, washed with brine, dried with MgSO<, and the solvent was evaporated in *vacuo* to give the title compound as a crude residue (0.033 g) which was used without purification for the next step.

### Step B: Preparation of 2-(7-(5-(3-isopropoxy-5-(trifluoromethyl)phenyl)-1,2,4-oxadiazol-3-yl)-4-methyl-1,2,3,4-tetrahydrocyclopenta[b]indol-3-yl)acetic acid (Compound 101).

Methyl 2-(7-(5-(3-isopropoxy-5-(trifluoromethyl)phenyl)-1,2,4-oxadiazol-3-yl)-4-methyl-1,2,3,4-tetrahydrocyclopenta[b]indol-3-yl)acetate (33 mg, 0.064 mmol) was dissolved in 1,4-dioxane (0.78 ml, 9.12 mmol). Aqueous 1.00 N lithium hydroxide (0.257 ml, 0.257 mmol) was added and the reaction was heated at 90°C for 1 h. The reaction mixture was acidified with aqueous 1 N HCl (0.386 ml, 0.386 mmol), diluted with MTBE (30 mL) and the organic layer was washed with water twice, washed with brine, dried with MgSO₄ and the solvent was evaporated *in vacuo* to give an oil which was purified by preparative HPLC to give the title compound as a beige solid (8 mg). LCMS *m*/*z* = 500.1 (M+H⁺); ¹H NMR (400 MHz, CD₃CN) δ ppm 1.38 (d, *J* = 6.06 Hz, 6H), 2.25-2.40 (m, 1H), 2.50 (dd, *J* = 9.98, 5.81 Hz, 1H), 2.75-2.89 (m, 3H), 2.90-3.04 (m, 1H), 3.63-3.72 (m, 1H), 3.74 (s, 3H), 4.78-4.89 (m, 1H), 7.41-7.54 (m, 2H), 7.84-7.97 (m, 2H), 8.02 (s, 1H), 8.22 (s, 1H).

### Example 1.82: Preparation of 2-(7-(5-(3,5-Bis(methylsulfonyl)phenyl)-1,2,4-oxadiazol-3-yl)-1,2,3,4-tetrahydrocyclopenta[b]indol-3-yl)acetic Acid (Compound 90).

### Step A: Preparation of Ethyl 2-(7-(5-(3,5-Bis(methylsulfonyl)phenyl)-1,2,4-oxadiazol-3-yl)-1,2,3,4-tetrahydrocyclopenta[b]indol-3-yl)acetate.

From ethyl 2-(7-(*N*'-hydroxycarbamimidoyl)-1,2,3,4-tetrahydrocyclopenta[b]indol-3-yl)acetate and 3,5-bis(methylsulfonyl)benzoyl chloride, using a similar method to the one described in **Example 1.25, Step A,** the title compound was obtained. LCMS *m*/*z* = 544.3 [M+H]⁺.

### Step B: Preparation of 2-(7-(5-(3,5-Bis(methylsulfonyl)phenyl)-1,2,4-oxadiazol-3-yl)1,2,3,4-tetrahydrocyclopenta[b]indol-3-yl)acetic Acid (Compound 90).

From ethyl 2-(7-(5-(3,5-bis(methylsulfonyl)phenyl)-1,2,4-oxadiazol-3-yl)-1,2,3,4-tetrahydrocyclopenta(b]indol-3-yl)acetate and 2.0 M aqueous sodium hydroxide, using a similar method to the one described in **Example 1.29, Step B,** the title compound was obtained as a white solid. LCMS *m*/*z* = 516.3 [M+H]⁺.

### Example 1.83: Preparation of 2-(7-(5-(3-(Cyclopropylethynyl)-5-(trifluoromethoxy)phenyl)-1,2,4-oxadiazol-3-yl)-1,2,3,4-tetrahydrocyclopenta[b]indol-3-yl)acetic Acid (Compound 86).

### Step A: Preparation of Ethyl 2-(7-(5-(3-(Cyclopropylethynyl)-5-(trifluoromethoxy)phenyl)-1,2,4-oxadiazol-3-yl)-1,2,3,4-tetrahydrocyclopenta[b]indol-3-yl)acetate.

From ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetate and ethynylcyclopropane, using a method similar to **Example 1.38, Step A,** the title compound was obtained as a yellow solid. LCMS *m*/*z* = 536.4 **[M+H]⁺.**

### Step B: Preparation of 2-(7-(5-(3-(Cyclopropylethynyl)-5-(trifluoromethoxy)phenyl)-1,2,4-oxadiazol-3-yl)-1,2,3,4-tetrahydrocyclopenta[b]indol-3-yl)acetic Acid (Compound 86).

From ethyl 2-(7-(5-(3-(cyclopropylethynyl)-5-(trifluoromethoxy)phenyl)-1,2,4-oxadiazol-3-yl)-1,2,3,4-tetrahydrocyclopenta[b]indol-3-yl)acetate, using a method similar to **Example 1.29, Step B,** the title compound was obtained as a white solid. LCMS ***m*/*z*** = 508.4 [M+H]⁺; ¹H NMR (400 MHz, CD₃CN) δ ppm: 0.82-0.85 (m, 2H), 0.93-0.98 (m, 2H), 1.49-1.56 (m, 1H), 2.13-2.22 (m, 1H), 2.63-2.65 (m, 2H), 2.76-2.93 (m, 3H), 3.54-3.61 (m, 1H), 7.50 (d, *J* = 8.3 Hz, 1 H), 7.5 3 (s, 1 H), 7.81 (dd, *J* = 8.6,1.5 Hz, 1H), 7.99 (s, 1H), 8.12 (t, *J* = 1.3 Hz, 1H), 8.19 (s, 1H), 9.38 (s, 1H).

### Example 1.84: Preparation of 2-(7-(5-(4-Isopropoxy-3-(methylsulfonyl)phenyl)-1,2,4-oxadiazol-3-yl)-1,2,3,4-tetrahydrocyclopenta[b]indol-3-yl)acetic Acid (Compound 89).

### Step A: Preparation of 4-Isopropoxy-3-(methylsulfonyl)benzoic Acid.

To a solution of 3-bromo-4-isopropoxybenzoic acid (0.50 g, 1.93 mmol), sodium methanesulfinate (0.25 g, 2.45 mmol), and N1,N2-dimethylethane-1,2-diamine (0.019 mL, 0.174 mmol) in DMSO (9.65 mL) was added copper(I) trifluoromethanesulfonate benzene complex (0.039 g, 0.077 mmol). The mixture was stirred at 120°C for 24 h and then filtered. The filtrate was purified by preparative HPLC to provide the title compound as an off-white solid (50 mg). LCMS *m*/*z* = 259.3 [M+H]⁺;¹H NMR (400 MHz, DMSO-*d*₆) δ ppm 1.37 (d, *J* = 5.8 Hz, 6H), 3.29 (s, 3H), 4.98 (sept, *J =* 6.1 Hz, **1H),** 7.43 (d, *J* = 9.1 Hz, 1H), 8.18 (dd, *J* = 8.8 Hz, 2.3 Hz, 1H), 8.35 (d, *J* = 2.3 Hz, 1H), 13.15 (s, 1H).

### Step B: Preparation of Ethyl 2-(7-(5-(4-Isopropoxy-3-(methylsulfonyl)phenyl)-1,2,4-oxadiazol-3-yl)-1,2,3,4-tetrahydrocyclopenta[b]indol-3-yl)acetate.

From ethyl 2-(7-N'-hydroxycarbamimidoyl)-1,2,3,4-tetrahydrocyclopenta[b]indol-3-yl)acetate and 4-isopropoxy-3-(methylsulfonyl)benzoic acid, using a method similar to **Example 1.25, Step A, the title compound was obtained as a white solid. LCMS m/z = 524.5 [M+H]⁺.**

### Step C: Preparation of 2-(7-(5-(4-Isopropoxy-3-(methylsulfonyl)phenyl)-1,2,4-oxadiazol-3-yl)-1,2,3,4-tetrahydrocyclopenta[b]indol-3-yl)acetic Acid (Compound 89).

From ethyl 2-(7-(5-(4-isopropoxy-3-(methylsulfonyl)phenyl)-1,2,4-oxadia2ol-3-yl)-1,2,3,4-tetrahydrocyclopenta[b]indol-3-yl)acetate, using a method similar to **Example 1.29, Step B,** the title compound was obtained as a white solid. LCMS *m*/*z* = 496.4 [M+H]⁺; ¹H NMR (400 MHz, DMSO-*d*₆) δ ppm 1.41 (d, *J* = 6.1 Hz, 6H), 2.10-2.17 (m, 1H), 2.43 (dd, *J* = 16.2, 8.8 Hz, 1H), 2.73-2.89 (m, 4H), 3.36 (s, 3H), 3.51-3.57 (m, 1H), 5.06 (quint, *J* = 6.1 Hz, 1H), 7.50 (d, *J* = 8.8 Hz, 1H), 7.61 (d, *J* =9.1 Hz, 1H), 7.75 (dd, *J* = 8.6, 1.5 Hz, 1H), 8.12 (d, *J* = 1.5 Hz, 1H), 8.45 (dd, *J* = 8.8, 2.3 Hz, 1H), 8.55 (d, *J* = 2.3 Hz, 1H), 11.11 (s, 1H), 12.10 (bs, 1H).

### Example 1.85: Preparation of 2-(7-(5-(5-Methoxypyridin-2-yl)-1,2,4-oxadiazol-3-yl)-1,2,3,4-tetrahydrocyclopenta[b]indol-3-yl)acetic Acid (Compound 83).

From ethyl 2-(7-(5-(5-bromopyridin-2-yl)-1,2,4-oxadiazol-3-yl)-1,2,3,4-tetrahydrocyclopenta(blindol-3-yl)acetate and methanol, using a method similar to **Example 1.100, Step B,** the title compound was obtained as a yellow solid. LCMS *m*/*z* = 391.3 [M+H]⁺.

### Example 1.86: Preparation of 2-(7-(5-(3-Cyano-4-(trifluoromethoxy)phenyl)-1,2,4-oxadiazol-3-yl)-1,2,3,4-tetrahydrocyclopenta[b]indol-3-yl)acetic Acid (Compound 103).

### Step A: Preparation of Ethyl 2-(7-(5-(3-Cyano-4-(trifluoromethoxy)phenyl)-1,2,4-oxadiazol-3-yl)-1,2,3,4-tetrahydrocyclopenta[b]indol-3-yl)acetate

From ethyl 2-(7-(N'-hydroxycarbamimidoyl)-1,2,3,4-tetrahydrocyclopenta[b]indol-3-yl)acetate and 3-cyano-4-(trifluoromethoxy)benzoyl chloride, using a method similar to **Example 1.25, Step A,** the title compound was obtained as a white solid. LCMS *m*/*z* = 497.3 [M+H]⁺.

### Step B: Preparation of 2-(7-(5-(3-Cyano-4-(trifluoromethoxy)phenyl)-1,2,4-oxadiazol-3-yl)-1,2,3,4-tetrahydrocyclopenta[b]indol-3-yl)acetic Acid (Compound 103).

From ethyl 2-(7-(5-(3-cyano-4-(trifluoromethoxy)phenyl)-1,2,4-oxadiazol-3-yl)-1,2,3,4-tetrahydrocyclopenta[b]indol-3-yl)acetate, using a method similar to **Example 1.25, Step B,** the title compound was obtained as a white solid. LCMS *m*/*z* = 469.3 [M+H]⁺; ¹H NMR (400 MHz, DMSO-*d*₆) δ ppm 2.11-2.18 (m, 1H), 2.43 (dd, *J* = 16.3, 8.8 Hz, 1H), 2.71-2.89 (m, 4H), 3.51-3.57 (m, 1H), 7.50 (d, *J* = 8.5 Hz, 1H), 7.75 (dd, *J* = 8.8,1.5 Hz, 1H), 7.95 (dq, *J* = 8.8, 1.5 Hz, 1H), 8.13 (d, *J* = 1.3 Hz, 1H), 8.62 (dd, *J* = 9.0, 2.2 Hz, 1H), 8.84 (d, *J* = 2.2 Hz, 1H), 11.10 (s, 1H), 12.28 (bs, 1H).

### Example 1.87: Preparation of 2-(7-(5-(4-Methoxy-3-(methylsulfonyl)phenyl)-1,2,4-oxadiazol-3-yl)-1,2,3,4-tetrahydrocyclopenta[b]indol-3-yl)acetic Acid (Compound 108).

### Step A: Preparation of 4-Methoxy-3-(methylsulfonyl)benzoic Acid

To a solution of 3-bromo-4-methoxybenzoic acid (0.50 g, 2.16 mmol), sodium methanesulfinate (0.287 g, 2.81 mmol), and N1,N2-dimethylethane-1,2-diamine (0.047 mL, 0.433 mmol) in DMSO (10 mL) was added copper(I) trifluoromethanesulfonate benzene complex (0.109 g, 0.216 mmol)**.** The mixture was stirred at 130°C for 12 h, filtered, and purified by preparative HPLC to provide the title compound as a white solid (95 mg). LCMS *m*/*z* = 231.3 [M+H]⁺.

### Step B: Preparation of Ethyl 2-(7-(5-(4-Methoxy-3-(methylsulfonyl)phenyl)-1,2,4-oxadiazol-3-yl)-1,2,3,4-tetrahydrocyclopenta[b]indol-3-yl)acetate.

To a solution of 4-methoxy-3-(methylsulfonyl)benzoic acid (38.2 mg, 0.166 mmol) in dichloromethane (1 mL) was added a 2.0 M dichloromethane solution of oxalyl chloride (0.166 mL, 0.332 mmol). The reaction was stirred at room temperature for 2 h then concentrated. The resulting acid chloride was dissolved in in dioxane (1 mL). Ethyl 2-(7-(N'-hydroxycarbamimidoyl)-1,2,3,4-tetrahydrocyclopenta[b]indol-3-yl)acetate (50 mg, 0.166 mmol)) was added followed by triethylamine (0.069 mL, 0.498 mmol). The resulting suspension was stirred at room temperature for 30 min then at 90°C for 2.5 h then concentrated. The residue was purified by silica gel column chromatography to provide the title compound as a tan solid (42 mg). LCMS *m*/*z* = 496.4 [M+H]⁺;

### Step C: Preparation of 2-(7-(5-(4-methoxy-3-(methylsulfonyl)phenyl)-1,2,4-oxadiazol-3-yl)-1,2,3,4-tetrahydrocyctopenta[b]indol-3-yl)acetic Acid (Compound 108).

From ethyl 2-(7-(5-(4-methoxy-3-(methylsulfonyl)phenyl)-1,2,4-oxadiazol-3-yl)-1,2,3,4-tetrahydrocyclopenta[b]indol-3-yl)acetate, using a method similar to **Example 1.29, Step B**, the title compound was obtained as a white solid (20 mg). LCMS *m*/*z* = 468.1 [M+H]⁺; ¹H NMR (400 MHz, DMSO-*d*₆) δ ppm 2.11-2.20 (m, 1H), 2.43 (dd, *J* = 16.2,8.8 Hz, 1H), 2.73-2.89 (m, 4H), 3.35 (s, 3H), 3.51-3.57 (m, 1H), 4.10 (s, 3H), 7.49 (d, *J* = 8.6 Hz, 1H), 7.58 (d, *J* = 8.8 Hz, 1H), 7.75 (dd, *J* = 8.6,1.6 Hz, 1H), 8.12 (d, *J* = 1.3 Hz, 1H), 8.49 (dd, *J* = 8.7, 2.2 Hz, 1H), 8.55 (d, *J* = 2.2Hz, 1H), 11.04(s, 1H), 12.22 (s, 1H).

### Example 1.88: Preparation of 2-(7-(5-(4-Hydroxy-3-methylphenyl)-1,2,4-oxadiazol-3-yl)-1,2,3,4-tetrahydrocyclopenta[b]indol-3-yl)acetic Acid (Compound 78).

### Step A: Preparation of Ethyl 2-(7-(5-(4-Hydroxy-3-methylphenyl)-1,2,4-oxadiazol-3-yl)-1,2,3,4-tetrahydrocyclopenta[b]indol-3-yl)acetate.

4-Hydroxy-3-methylbenzoic acid (0.050 g, 0.332 mmol) and CDI (0.054 g, 0.332 mmol) were stirred in dioxane for 30 minutes. To this was added a solution of ethyl 2-(7-(N-hydroxycarbamimidoyl)-1,2,3,4-tetrahydrocyclopenta[b]indol-3-yl)acetate (0.100 g, 0.332 mmol,) in dioxane. After stirring overnight the reaction was heated to 100°C for 5 hours. The mixture was concentrated and the residue was purified by silica gel column chromatography to provide the title compound as a white solid (0.0439 g). LCMS *m*/*z* = 418.3 [M+H]⁺.

### Step B: Preparation of 2-(7-(5-(4-Hydroxy-3-methylphenyl)-1,2,4-oxadiazol-3-yl)-1,2,3,4-tetrahydrocyclopenta[b]indol-3-yl)acetic Acid (Compound 78).

To a solution of ethyl 2-(7-(5-(4-hydroxy-3-methylphenyl)-1,2,4-oxadiazol-3-yl)-1,2,3,4-tetrahydrocyclopenta[b]indol-3-yl)acetate (9.7 mg, 0.023 mmol) in THF:MeOH (1:1) was added aq 1 M LiOH. After overnight stirring material was concentrated and taken up in EtOAc. Organic was washed with 1M HCl, dried over MgSO₄, filtered and concentrated to give title compound as a white solid (8.3 mg). LCMS *m*/*z* 390.2 [M+H]⁺. ¹H NMR (400 MHz, CD₃OD) δ ppm 2.16-2.25 (m, 1H), 2.28 (s, 3H), 2.55 (dd, *J* = 16.17, 7.83 Hz, 1H), 2.74 (dd, *J* = 16.29, 6.95 Hz, 1H), 2.77-2.94 (m, 3H), 3.56-3.65 (m, 1H), 6.91 (d*,J* = 8.34 Hz, 1H), 7.40 (d, *J* = 8.59 Hz, 1H), 7.76 (dd, *J* = 8.46, 1.64 Hz, 1H), 7.88 (dd, *J* = 8.46, 2.15 Hz, 1H), 7.93 (s, 1H), 8.13 (s, 1H).

### Example 1.89: Preparation of 2-(7-(5-(3-Chloro-4-hydroxyphenyl)-1,2,4-oxadiazol-3-yl-1,2,3,4-tetrahydrocyclopenta[b]indol-3-yl)acetic Acid (Compound 94).

### Step A: Preparation of Ethyl 2-(7-(5-(3-Chloro-4-hydroxyphenyl)-1,2,4-oxadiazol-3-yl)-1,2,3,4-tetrahydrocyclopenta[b]indol-3-yl)acetate.

3-Chloro-4-hydroxybenzoic acid (0.029 g, 0.166 mmol), thionyl chloride (0.121 mL, 1.659 mmol) and DCM (1.00 mL) were stirred at reflux overnight. After removal of solvent under reduced pressure the resultant acid chloride was dissolved in THF. To this was added ethyl 2-(7-(N-hydroxycarbamimidoyl)-1,2,3,4-tetrahydrocyclopenta[b]indol-3-yl)acetate (0.050 g, 0.166 mmol) and TEA (0.023 mL, 0.166 mmol). After stirring for 1 hour, the solvent was removed under reduced pressure. The residue was dissolved in DMF (5.00 mL) and the reaction was heated to 100°C for 6 h. The reaction mixture was concentrated and residue was purified by silica gel column chromatography to provide the title compound as a white solid (0.0384 g). LCMS *m*/*z* = 438.4 [M+H]⁺.

### Step B: Preparation of 2-(7-(5-(3-Chloro4-hydroxyphenyl)-1,2,4-oxadiazol-3-yl)-1,2,3,4-tetrahydrocyclopenta[b]indol-3-yl)acedc Acid (Compound 94).

From ethyl 2-(7-(5-(3-chloro-4-hydroxyphenyl)-1,2,4-oxadiazol-3-yl)-1,2,3,4-tetrahydrocyclopenta[b]indol-3-yl)acetate, using a method similar to **Example 1.88, Step B,** the title compound was obtained. LCMS *m*/*z* = 410.4 [M+H]⁺. ¹H NMR (400 MHz, CD₃OD) δ ppm 2.14-2.26 (m, 1H), 2.55 (dd, *J* = 16.29, 7.71 Hz, 1H), 2.68-2.94 (m, 4H), 3.60 (t, *J* = 6.57 Hz, 1H), 7.08 (d, *J* = 8.34 Hz, 1H), 7.39 (d, *J* = 8.59 Hz, 1H), 7.76 (d, *J =* 8.59 Hz, 1H), 7.96 (dd, *J* = 8.59, 2.02 Hz, 1H), 8.07-8.15 (m, 2H).

### Example 1.90: Preparation of 2-(7-(5-(3-Fluoro4-isopropoxyphenyl)-1,2,4-oxadiazol-3-yl)-1,2,3,4-tetrahydrocyclopenta[b]indol-3-yl)acetic Acid (Compound 81).

### Step A: Preparation of Ethyl 2-(7-(5-(3-Fluoro-4-isopropoxyphenyl)-1,2,4-oxadiazol-3-yl)-1,2,3,4-tetrahydrocyclopenta[b]indol-3-yl)acetate.

To a solution of 3-fluoro-4-isopropoxybenzoic acid (0.263 g, 1.327 mmol) and DMF (8.22 µL, 0.106 mmol) in DCM was added oxalyl chloride (0.232 mL, 2.65 mmol). After stirring 1 hour at room temperature, the solvent was removed under reduced pressure and the residue was dissolved in THF. This was added to a solution of ethyl 2-(7-(N'-hydroxycarbamimidoyl)-1,2,3,4-tetrahydrocyclopenta[b]indol-3-yl)acetate (0.400 g, 1.327 mmol), and triethylamine (0.185 mL, 1.327 mmol) in THF. The reaction was stirred overnight, then concentrated and the residue was taken up in DMF. This was stirred at 100°C for 4 hours. The solvent was removed and the residue was taken up in EtOAc. The organics were washed with 1M HCl followed by brine (thrice), dried over MgSO₄, filtered and concentrated. The residue was purified by silica gel column chromatography to yield title compound as a yellow solid (0.229 g). LCMS *m*/*z* = 464.4 [M+H]⁺. ¹H NMR (400 MHz, CD₃OD) δ ppm 1.25 (t, *J* = 7.07 Hz, 3H), 1.39 (d, *J* = 6.06 Hz, 6H), 2.13-2.25 (m, 1H), 2.56 (dd, *J* = 15.92, 7.83 Hz, 1H), 2.66-2.92 (m, 4H), 3.53-3.64 (m, 1H), 4.11-4.22 (m, 2H), 4.76 (m, 1H)**,** 7.25 (t, *J* = 8.34 Hz, 1H), 7.39 (d, *J* = 8.59 Hz, 1H), 7.75 (dd, *J* = 8.59,1.52 Hz, 1H), 7.85 (dd, *J* = 11.62, 2.02 Hz, 1H), 7.91 (d, *J* = 8.84 Hz, 1H), 8.10 (s, 1H).

### Step B: Preparation of 2-(7-(5-(3-Fluoro-4-isopropoxyphenyl)-1,2,4-oxadiazol-3-yl)-1,2,3,4-tetrahydrocyclopentalblindol-3-yl)acetic Acid (Compound 81).

From ethyl 2-(7-(5-(3-fluoro-4-isopropoxyphenyl)-1,2,4-oxadiazol-3-yl)-1,2,3,4-tetrahydrocyclopenta[b]indol-3-yl)acetate, using a method similar to **Example 1.88, Step B,** the title compound was obtained.. LCMS *m*/*z* = 436.5 [M+H]⁺**.** ¹H NMR (400 MH₂, CD₃OD) δ ppm 1.40 (d, *J*= 6.06 Hz, 6H), 2.14-2.29 (m, 1H), 2.55 (dd, J = 16.17, 7.83 Hz, 1H), 2.68-2.98 (m, 4H), 3.62 (m, 1H), 4.80 (m, Hz, 1H), 7.32 (t, *J* = 8.46 Hz, 1H), 7.42 (d, *J* = 8.59 Hz, 1H)**,** 7.79 (d, *J* = 8.49, 1.59 Hz, 1H), 7.93 (dd, *J* = 11.37, 2.02 Hz, 1H), 7.98 (d, *J* = 8.62 Hz, 1H), 8.15 (s, 1H).

### Example 1.91: Preparation of 2-(7-(5-(4-Isopropoxyphenyl)-1,2,4-oxadiazol-3-yl)-1,2,3,4-tetrahydrocyclopenta[b]indol-3-yl)acetic Acid (Compound 97).

### Step A: Preparation of Ethyl 2-(7-(5-(4-Isopropoxyphenyl)-1,2,4-oxadiazol-3-yl)-1,2,3,4-tetrahydrocyclopenta[b]indol-3-yl)acetate.

To a solution of (Z)-ethyl 2-(7-(N'-hydroxycarbamimidoyl)-1,2,3,4-tetrahydrocyclopenta[b]indol-3-yl)acetate (0.100 g, 0.332 mmol) and triethylamine (0.046 mL, 0.332 mmol) in THF (1.5 mL) was added 4-isopropoxybenzoyl chloride (0.066 g, 0.332 mmol). After stirring overnight, the solvent was removed under reduced pressure and the residue was dissolved in DMF (1.5 mL). The reaction mixture was stirred for 4 h at 100 °C, then the solvent was removed and the residue was taken up in EtOAc. The organics were washed with 1M HCl and brine (twice), dried over MgSO₄, filtered and concentrated. The residue was purified by silica gel column chromatography to provide the title compound as a yellow solid (0.0435 g). LCMS *m*/*z* = 446.3 [M+H]⁺. ¹H NMR (400 MHz, CD₃OD) δ ppm 1.24 (t, *J* = 7.07 Hz, 3H), 1.36 (d, *J* = 6.06 Hz, 6H), 2.15-2.24 (m, 1H), 2.57 (dd, *J* = 15.92, 7.83 Hz, 1H), 2.68-2.92 (m, 4H), 3.60 (dd, *J* = 6.32, 3.79 Hz, 1H), 4.17 (m, 2H), 4.73 (m, 1H), 7.07 (d, *J* = 8.84 Hz, 2H), 7.40 (d, *J* = 8.59 Hz, 1H), 7.74-7.79 (m, 1H), 8.06-8.14 (m, 3H).

### Step B: Preparation of 2-(7-(5-(4-Isopropoxyphenyl)-1,2,4-oxadiazol-3-yl)-1,2,3,4-tetrahydrocyclopenta[b]indol-3-yl)acetic Acid (Compound 97).

From ethyl 2-(7-(5-(4-isopropoxyphenyl)-1,2,4-oxadiazol-3-yl)-1,2,3,4-tetrahydrocyclopenta[b]indol-3-yl)acetate, using a method similar to **Example 1.88, Step B,** the title compound was obtained. LCMS *m*/*z* 418.5 [M+H]⁺. ¹H NMR (400 MH₂, DMSO-*d*₆) δ ppm 1.33 (d, *J* = 6.06 Hz, 6H), 2.14 (m, 1H), 2.43 (dd, *J* = 16.17, 8.84 Hz, 1H), 2.68-2.91 (m, 4H), 3.53 (bs, 1H), 4.79 (m, 1H), 7.16 (d, *J* = 8.84 Hz, 2H), 7.48 (d, *J* = 8.84 Hz, 1H), 7.73 (dd, *J* = 8.59, 1.52 Hz, 1H), 8.04-8.18 (m, 3H), 11.08 (s, 1H), 12.30 (bs, 1H)

### Example 1.92: Preparation of 2-(7-(5-(4-Isopropoxy-3-methoxyphenyt)-1,2,4-oxadiazol-3-yl)-1,2,3,4-tetrahydrocyclopenta[b]indol-3-yl)acetic Acid (Compound 76).

### Step A: Preparation of Ethyl 2-(7-(5-(4-Isopropoxy-3-methoxyphenyl)-1,2,4-oxadiazol-3-yl)-1,2,3,4-tetrahydrocyclopenta[b]indol-3-yl)acetate.

From 4-isopropoxy-3-methoxybenzoyl chloride, using a method similar to **Example 1.91 step A**, the title compound was obtained as a solid. LCMS *m*/*z* = 476.2 [M+H]⁺.

### Step B: Preparation of 2-(7-(5-(4-Isopropoxy-3-methoxyphenyl)-1,2,4-oxadiazol-3-yl)-1,2,3,4-tetrahydrocyclopenta[b]indol-3-yl)acetic Acid (Compound 76).

From ethyl 2-(7-(5-(4-isopropoxy-3-methoxyphenyl)-1,2,4-oxadiazol-3-yl)-1,2,3,4-tetrahydrocyclopenta[b]indol-3-yl)acetate, using a method similar to **Example 1.88 Step B,** the title compound was obtained as a solid. LCMS *m*/*z* = 448.6 [M+H]⁺. ¹H NMR (400 MHz, DMSO-*d*₆) δ ppm 1.32 (d, *J* = 6.06 Hz, 6H), 2.07-2.21 (m, 1H), 2.43 (dd, *J* = 16.17, 9.09 Hz, 1H), 2.68-2.92 (m, 4H), 3.55 (d, *J* = 6.57 Hz, 1H), 3.90 (s, 3H), 4.76 (m, 1H), 7.23 (d, *J* = 8.84 Hz, 1H), 7.49 (d, *J* = 8.59 Hz, 1H), 7.65 (d, *J* = 2.02 Hz, 1H), 7.76 (m, 2H), 8.11 (s, 1H), 11.08 (s, 1H), 12.32 (bs, 1H).

### Example 1.93: Preparation of 2-(7-(5-(3,4-Dimethoxyphenyl)-1,2,4-oxadiazol-3-yl)-1,2,3,4-tetrahydrocyclopenta[b]indol-3-yl)acetic Acid (Compound 85).

### Step A: Preparation of Ethyl 2-(7-(5-(3,4-Dimethoxyphenyl)-1,2,4-oxadiazol-3-yl)-1,2,3,4-tetrahydrocyclopenta[b]indol-3-yl)acetate.

From 3,4-dimethoxybenzoyl chloride, using a method similar to **Example 1.91**, **Step A**, the title compound was obtained as a solid. LCMS *m*/*z* = 448.3 [M+H]⁺. ¹H NMR (400 MHz, CDCl₃) δ ppm 1.31 (t, *J* = 7.07 Hz, 3H), 2.15 (m, 1H), 2.54 (dd, *J* = 16.93, 11.37 Hz, 1H), 2.75-2.99 (m, 4H), 3.53-3.65 (m, 1H), 3.99 (s, 6H), 4.18-4.28 (m, 2H), 7.01 (d, *J* = 8.59 Hz, 1H), 7.41 (d*, J* = 8.59 Hz, 1H), 7.73 (d, *J =* 2.02 Hz, 1H), 7.87 (dd, *J* = 8.34, 2.02 Hz, 1H), 7.93 (dd, *J =* 8.46, 1.64 Hz, 1H), 8.31 (d, *J =* 1.26 Hz, 1H), 8.81 (s, 1H).

### Step B: Preparation of 2-(7-(5-(3,4-Dimethoxyphenyl)-1,2,4-oxadiazol-3-yl)-1,2,3,4-tetrahydrocyclopenta[b]indol-3-yl)acetic Acid (Compound 85).

From ethyl 2-(7-(5-(3,4-dimethoxyphenyl)-1,2,4-oxadiazol-3-yl)-1,2,3,4-tetrahydrocyclopenta[b]indol-3-yl)acetate, using a method similar to **Example 1.88**, **Step B**, the title compound was obtained as a solid. LCMS *m*/*z* = 420.6 [M+H]⁺. ¹H NMR (400 MHz, DMSO-*d*₆) δ ppm 2.08-2.19 (m, 1H), 2.43 (dd, *J* = 16.17, 8.84 Hz, 1H), 2.68-2.90 (m, 4H), 3.54 (bs, 1H), 3.90 (d, *J* = 11.12 Hz, 6H), 7.22 (d, *J* = 8.59 Hz, 1H), 7.49 (d, *J* = 8.59 Hz, 1H), 7.66 (d, *J* = 2.02 Hz, 1H), 7.75 (dd, *J* = 8.46, 1.64 Hz, 1H), 7.82 (dd, *J* = 8.59, 2.02 Hz, 1H), 8.12 (s, 1H), 11.08 (s, 1H), 12.28 (bs, 1H).

### Example 1.94: Preparation of 2-(7-(5-(3-Fluoro4-methoxyphenyl)-1,2,4-oxadiazol-3-yl)-1,2,3,4-tetrahydrocyclopenta[b]indol-3-yl)acetic Acid (Compound 92).

### Step A: Preparation of Ethyl 2-(7-(5-(3-Fluoro-4-methoxyphenyl)-1,2,4-oxadiazol-3-yl)-1,2,3,4-tetrahydrocyclopenta[b]indol-3-yl)acetate.

From 3-fluoro-4-methoxybenzoyl chloride, using a method similar to **Example 1.91**, **Step A**, the title compound was obtained as a solid. LCMS *mlz* = 436.5 [M+H]⁺. ¹H NMR (400 MHz, CDCl₃) δ ppm 1.31 (t, *J* = 7.20 Hz, 3H), 2.15 (m, 1H), 2.54 (dd, *J* = 16.93, 11.62 Hz, 1H), 2.71-3.00 (m, 4H), 3.59 (m, 1H), 4.00 (s, 3H), 4.16-4.30 (m, 2H), 7.10 (t, *J* = 8.34 Hz, 1H), 7.41 (d, *J* = 8.08 Hz, 1H), 7.86-8.06 (m, 3H), 8.29 (s, 1H), 8.82 (s, 1H).

### Step B: Preparation of 2-(7-(5-(3-Fluoro-4-methoxyphenyl)-1,2,4-oxadiazol-3-yl)-1,2,3,4-tetrahydrocyclopenta[b]indol-3-yl)acetic Acid (Compound 92).

From ethyl 2-(7-(5-(3-fluoro-4-methoxyphenyl)-1,2,4-oxadiazol-3-yl)-1,2,3,4-tetrahydrocyclopenta[b]indol-3-yl)acetate, using a method similar to **Example 1.88**, **Step B**, the title compound was obtained as a solid. LCMS *m*/*z* = 408.3 [M+H]⁺. ¹H NMR (400 MHz, DMSO-*d₆*) δ ppm 2.09-2.19 (m, 1H), 2.43 (dd, *J* = 16.04, 8.97 Hz, 1H), 2.70-2.89 (m, 4H), 3.54 (bs, 1H), 3.98 (s, 3H), 7.45 (t, *J* = 8.59 Hz, 1H), 7.49 (d, *J* = 8.59 Hz, 1H), 7.74 (dd, *J* = 8.34, 1.52 Hz, 1H), 7.97-8.07 (m, 2H), 8.11 (d, *J* = 1.01 Hz, 1H), 11.09 (s, 1H).

### Example 1.95: Preparation of 2-(7-(5-(4-Ethoxy-3-fluorophenyl)-1,2,4-oxadiazol-3-yl)-1,2,3,4-tetrahydrocyclopenta[b]indol-3-yl)acetic Acid (Compound 102).

### Step A: Preparation of Ethyl 2-(7-(5-(4-Ethoxy-3-fluorophenyl)-1,2,4-oxadiazol-3-yl)-1,2,3,4-tetrahydrocyclopenta[b]indol-3-yl)acetate.

From 4-ethoxy-3-fluorobenzoic acid, using a method similar to **Example 1.9**, **Step A**, the title compound was obtained as a solid. LCMS *m*/*z* = 450.0 [M+H]^{+ 1}H NMR (400 MHz, CDCl₃) δ ppm 1.32 (t, *J* = 7.07 Hz, 3H), 1.52 (t, *J* = 6.99 Hz, 3H), 2.08-2.24 (m, 1H), 2.55 (dd, *J* = 16.93, 11.62 Hz, 1H), 2.73-3.01 (m, 4H), 3.59 (d, *J* = 7.58 Hz, 1H), 4.12-4.33 (m, 4H), 7.09 (t, *J* = 8.46 Hz, 1H), 7.41 (d, *J* = 8.34 Hz, 1H), 7.86-8.03 (m, 3H), 8.30 (s, 1H), 8.82 (s, 1H).

### Step B: Preparation of 2-(7-(5-(4-Ethoxy-3-fluorophenyl)-1,2,4-oxadiazol-3-yl)-1,2,3,4-tetrahydrocyclopenta[b]indol-3-yl)acetic Acid (Compound 102).

From ethyl 2-(7-(5-(4-ethoxy-3-fluorophenyl)-1,2,4-oxadiazol-3-yl)-1,2,3,4-tetrahydrocyclopenta[b]indol-3-yl)acetate, using a method similar to **Example 1.88**, **Step B**, the title compound was obtained as a solid. LCMS *m*/*z* = 422.5 [M+H]⁺. ¹H NMR (400 MHz, DMSO-*d*₆) δ ppm 1.40 (t, *J* = 6.95 Hz, 3H), 2.09-2.19 (m, 1H), 2.43 (dd, *J* = 16.17, 9.09 Hz, 1H), 2.65-2.89 (m, 4H), 3.53 (bs, 1H), 4.26 (m, 2H), 7.43 (t, *J* = 8.72 Hz, 1H), 7.49 (d, *J* = 8.34 Hz, 1H), 7.73 (dd, *J* = 8.59, 1.52 Hz, 1H), 7.97-8.04 (m, 2H), 8.11 (s, 1H), 11.08 (s, 1H), 12.26 (bs, 1H).

### Example 1.96: Preparation of 2-(7-(5-(2-Chloro-6-methoxypyridin-4-yl)-1,2,4-oxadiazol-3-yl)-1,2,3,4-tetrahydrocyclopenta[b]indol-3-yl)acetic Acid (Compound 10⁷).

### Step A: Preparation of Ethyl 2-(7-(5-(2-Chloro-6-methoxypyridin4-yl)-1,2,4-oxadiazol-3-yl)-1,2,3,4-tetrahydrocyclopenta[b]indol-3-yl)acetate.

From 2-chloro-6-methoxyisonicotinoyl, using a method similar to **Example 1.91**, **Step A**, the title compound was obtained as a solid. LCMS *m*/*z* = 453.1 [M+H]⁺. ¹H NMR (400 MHz, CDCl₃) δ ppm 1.31 (t, *J* = 7.20 Hz, 3H), 2.09-2.23 (m, 1H), 2.54 (dd, *J* = 16.86, 11.43 Hz, 1H), 2.68 (s, 3H), 2.74-2.99 (m, 4H), 3.60 (bs, 1H), 4.16-4.30 (m, 2H), 7.41 (d, *J* = 8.59 Hz, 1H), 7.83-7.96 (m, 2H), 8.10 (s, 1H), 8.29 (s, 1H), 8.85 (bs, 1H).

### Step B: Preparation of 2-(7-(5-(2-Chloro-6-methoxypyridin-4-yl)-1,2,4-oxadiazol-3-yl)-1,2,3,4-tetrahydrocyclopenta[b]indol-3-yl)acetic Acid (Compound 107).

From ethyl 2-(7-(5-(2-chloro-6-methoxypyridin-4-yl)-1,2,4-oxadiazol-3-yl)-1,2,3,4-tetrahydrocyclopenta[b]indol-3-yl)acetate, using a method similar to **Example 1.88**, **Step B**, the title compound was obtained as a solid. LCMS *m*/*z* = 425.2 [M+H]. ¹H NMR (400 MHz, DMSO-*d*₆) δ ppm 2.08-2.21 (m, 1H), 2.43 (dd, *J* = 16.04, 8.84 Hz, 1H), 2.68-2.91 (m, 4H), 3.54 (bs, 1H), 3.97 (s, 3H), 7.50 (dd, *J* = 4.55, 3.92 Hz, 2H), 7.68-7.80 (m, 2H), 8.11 (d, *J* = 1.01 Hz, 1H), 11.08 (s, 1H), 12.23 (bs, 1H).

### Example 1.97: Preparation of 2-(7-(5-(2-Bromo-6-methylpyridin-4-yl)-1,2,4-oxadiazol-3-yl)-1,2,3,4-tetrahydrocyclopenta[b]indol-3-yl)acetic Acid (Compound 119).

### Step A: Preparation of Ethyl 2-(7-(5-(2-Bromo-6-methylpyridin-4-yl)-1,2,4-oxadiazol-3-yl)-1,2,3,4-tetrahydrocyclopenta[b]indol-3-yl)acetate.

From 2-bromo-6-methylisonicotinic acid, using a method similar to **Example 1.9**, **Step A**, the title compound was obtained as a solid. LCMS *m*/*z* =481.2 [M+H]⁺; ¹H NMR (400 MHz, CDCl₃) δ ppm 1.31 (t, *J* = 7.14 Hz, 3H), 2.09-2.22 (m, 1H), 2.54 (dd, *J* = 16.86, 11.43 Hz, 1H), 2.68 (s, 3H), 2.74-2.99 (m, 4H), 3.53-3.64 (m, 1H), 4.17-4.29 (m. 2H), 7.41 (d, *J* = 8.46 Hz, 1H), 7.84-7.96 (m, 2H), 8.10 (s, 1H), 8.29 (s, 1H), 8.84 (bs, 1H).

### Step B: Preparation of 2-(7-(5-(2-Bromo-6-methylpyridin4-yl)-1,2,4-oxadiazol-3-yl)-1,2,3,4-tetrahydrocyclopenta[b]indol-3-yl)acetic Acid (Compound 119).

From ethyl 2-(7-(5-(2-bromo-6-methylpyridin-4-yl)-1,2,4-oxadiazol-3-yl)-1,2,3,4-tetrahydrocyclopenta[b]indol-3-yl)acetate, using a method similar to **Example 1.88**, **Step B**, the title compound was obtained as a solid. LCMS *m*/*z* = 453.1 [M+H]⁺.

### Example 1.98: Preparation of 2-(7-(5-(4-(Difluoromethoxy)-3-methoxyphenyl)-1,2,4-oxadiazol-3-yl)-1,2,3,4-tetrahydrocyclopenta[b]indol-3-yl)acetic Acid (Compound 120).

### Step A: Preparation of Ethyl 2-(7-(5-(4-(Difluoromethoxy)-3-methoxyphenyl)-1,2,4-oxadiazol-3-yl)-1,2,3,4-tetrahydrocyclopenta[b]indol-3-yl)acetate.

From 4-(difluoromethoxy)-3-methoxybenzoic acid, using a method similar to **Example 1.9, Step A,** the title compound was obtained as a solid. LCMS *m*/*z* = 484.4 [M+H]⁺. ¹H NMR (400 MHz, CDCl₃) δ ppm 1.31 (t, *J* = 7.14 Hz, 3H), 2.09-2.22 (m, 1H), 2.54 (dd, *J* = 16.86, 11.43 Hz, 1H), 2.72-3.00 (m, 4H), 3.52-3.65 (m, 1H), 4.03 (s, 3H), 4.23 (m, 2H), 6.42-6.90 (m, 1H), 7.28-7.35 (m, 1H), 7.41 (d, *J =* 8.46 Hz, 1H), 7.79-7.87 (m, 2H), 7.92 (dd, *J* = 8.59, 1.52 Hz, 1H), 8.30 (s, 1H), 8.81 (s, 1H).

### Step B: Preparation of 2-(7-(5-(4-(Difluoromethoxy)-3-methoxyphenyl)-1,2,4-oxadiazol-3-yl)-1,2,3,4-tetrahydrocyclopenta[b]indol-3-yl)acetic Acid (Compound 120).

From ethyl 2-(7-(5-(4-(difluoromethoxy)-3-methoxyphenyl)-1,2,4-oxadiazol-3-yl)-1,2,3,4-tetrahydrocyclopenta[b]indol-3-yl)acetate, using a method similar to **Example 1.88, Step B,** the title compound was obtained as a solid. LCMS *m*/*z* = 456.5 [M+H]⁺. ¹H NMR (400 MHz, CD₃OD) δ ppm 2.15-2.27 (m, 1H), 2.54 (dd, *J* = 16.04, 7.71 Hz, 1H), 2.66-2.95 (m, 4H), 3.56-3.66 (m, 1H), 4.01 (s, 3H), 6.64-7.11 (m, 1H), 7.35 (d, *J* = 8.34 Hz, 1H), 7.41 (d, *J* = 8.46 Hz, 1H), 7.74-7.84 (m, 2H), 7.88 (d, *J* = 1.89 Hz, 1H), 8.14 (d, *J* = 1.14 Hz, 1H).

### Example 1.99: Preparation of 2-(7-(5-(6-(Diethylamino)-4-(trifluoromethyl)pyridin-2-yl)-1,2,4-oxadiazol-3-yl)-1,2,3,4-tetrahydrocyclopenta[b]indol-3-yl)acetic Acid (Compound 80).

### Step A: Preparation of 6-Chloro-N,N-diethyl-4-(trifluoromethyl)pyridin-2-amine.

2,6-Dichloro-4-(trifluoromethyl)pyridine (1.0 g, 4.63 mmol), diethylamine (2.355 mL, 23.15 mmol), and N-ethyl-N-isopropylpropan-2-amine (1.210 mL, 6.94 mmol) were mixed in IPA (1.5 mL) in a microwave vial. The reaction was heated under microwave irradiation at 100 °C for 1 h. The reaction mixture was added water and extracted with dichloromethane (3 x 50 mL). The organic layer was dried over sodium sulfate, filtered and concentrated to give the title compound as a yellow oil (1.095 g). LCMS *m*/*z* = 253.6[M+H]⁺; ¹H NMR (400 MHz, CDCl₃) δ ppm 1.10-1.14 (t, 6H), 3.42-3.47 (q, 4H), 6.39 (s, 1H), 6.58 (s, 1H).

### Step B: Preparation of 6-(Diethylamino)-4-(trifluoromethyl)picolinonitrile.

A suspension of 6-chloro-N,N-diethyl-4-(trifluoromethyl)pyridin-2-amine (0.960 g, 3.80 mmol), dicyanozinc (0.491 g, 4.18 mmol), and tetrakis(triphenylphosphine)palladium (0.439 g, 0.380 mmol) in DMF (10 mL) was heated at 95 °C for 16 h. The reaction mixture was poured into ice, extracted with ethylacetate, washed organics with brine, dried over magnesium sulfate, filtered and concentrated to obtain an oily residue. The residue was purified by silica gel column chromatography to provide the title compound as yellow oil (0.765 g). LCMS *m*/*z* = 244.3 [M+H]⁺; ¹H NMR (400 MHz, CDCl₃); δ ppm 1.21-1.24 (t, 6H), 3.54-3.59(q, 4H), 6.79 (s, 1H), 7.03 (s, 1H).

### Step C: Preparation of 6-(Diethyamino)-4-(trifluoromethyl)picolinic Acid.

To 6-(diethylamino)-4-(trifluoromethyl)picolinonitrile (0.760 g, 3.12 mmol) was added 6N HCl in dioxane (8 mL, 48.0 mmol) and the mixture was refluxed for 2 h. To the reaction mixture was added water (15 mL) and this was basified to pH9 using 6N NaOH solution. The mixture was extracted with ethyl acetate. The ethyl acetate layer was discarded. The aqueous layer was acidified back to pH 2 and extracted with ethyl acetate. The ethyl acetate extract was washed with brine, dried over magnesium sulfate, filtered and concentrated to provide the title compound as yellow oil (500 mg). LCMS *m*/*z* = 263.3 [M+H]⁺.

### Step D: Preparation of 6-(Diethylamino)-4-(trifluoromethyl)picolinoyl Chloride.

6-(Diethylamino)-4-(trifluoromethyl)picolinic acid in dichloromethane was added one drop of DMF and 3.0 equivalents of oxalyl chloride at room temperature. The mixture was stirred for I h. The yellow solution was concentrated. The resulting residue was used as the title compound without further purification.

### Step E: Preparation of Ethyl 2-(7-(5-(6-(Diethylamino)-4-(trifluoromethyl)pyridin-2-yl)-1,2,4-oxadiazol-3-yl)-1,2,3,4-tetrahydrocyclopenta[b]indol-3-yl)acetate.

To a solution of ethyl 2-(7-(N-hydroxycarbamimidoyl)-1,2,3,4-tetrahydrocyclopenta[b]indol-3-yl)acetate (0.060 g, 0.199 mmol) in dioxane (2 mL) was added 6-(diethylamino)-4-(trifluoromethyl)picolinoyl chloride (0.101 g, 0.358 mmol) in dioxane (2 mL). The mixture was stirred at room temperature for 1 h then concentrated. The residue was dissolved in DMA (2 mL) and heated at 100 °C for 30 minutes. DMA was evaporated under reduced pressure. The residue was purified by silica gel column chromatography to provide the title compound as a yellow solid (0.032 g). LCMS *m*/*z* = 528.7 [M+H]⁺. ¹H NMR (400 MHz, CDCl₃) δ ppm 1.14-1.27 (m, 9H), 2.02-2.11 (m, 1H), 2.41-2.52 (m, 1H), 2.68-2.92 (m, 4H), 3.47-3.62 (m, 5H), 4.11-4.20 (q, 2H), 6.72 (s, 1H), 7.33 (d, *J*= 8.59 Hz, 1H), 7.64(s, 1H), 7.87 (dd, *J* = 8.59Hz, 1.77 Hz, 1H), 8.25 (s, 1H), 8.74 (s, 1H).

### Step F: Preparation of 2-(7-(5-(6-(Diethylamino)-4-(trifluoromethyl)pyridin-2-yl)-1,2,4-oxadiazol-3-yl)-1,2,3,4-tetrahydrocyclopenta[b]indol-3-yl)acetic Acid (Compound 80).

To a solution of ethyl 2-(7-(5-(6-(diethylamino)-4-(trifluoromethyl)pyridin-2-yl)-1,2,4-oxadiazol-3-yl)-1,2,3,4-tetrahydrocyclopenta[b]indol-3-yl)acetate (0.032 g, 0.061 mmol) in THF (800 µL) and methanol (800 µL) was added NaOH (152 µL, 0.303 mmol). The reaction mixture was stirred at room temperature for 3 h. The mixture was added water (10 mL), acidified by 6 N HCl to pH 3, and extracted with ethyl acetate. The organic layer was concentrated and the residue purified by preparative HPLC to provide the title compound (0.021 g). LCMS *m*/*z* = 500.0 [M+H]⁺. ¹H NMR (400 MHz, DMSO-*d*₆) δ ppm 1.15-1.22 (t, 6H), 2.10-2.19 (m, 1H), 2.39-2.48 (m, 1H), 2.70-2.91 (m, 4H), 3.48-3.74 (m, 5H), 7.15 (s, 1H), 7.50 (d, *J* = 8.59 Hz, 1H), 7.61(s, 1H), 7.76 (dd, *J* = 8.34 Hz, 1.52 Hz, 1H), 8.13 (s, 1H), 11.1 (s, 1H).

### Example 1.100: Preparation of 2-(7-(5-(6-Isopropoxypyridin-3-yl)-1,2,4-oxadiazol-3-yl)-1,2,3,4-tetrahydrocyclopenta[b]indol-3-yl)acetic Acid (Compound 93).

### Step A: Preparation of Ethyl 2-(7-(5-(6-Chloropyridin-3-yl)-1,2,4-oxadiazol-3-yl)-1,2,3,4-tetrahydrocyclopenta[b]indol-3-yl)acetate.

From ethyl 2-(7-(N-hydroxycarbamimidoyl)-1,2,3,4-tetrahydrocyclopenta[b]indol-3-yl)acetate and 6-chloronicotinoyl chloride, using a method similar to **Example 1.99**, **Step E**, the title compound was obtained as a white solid. LCMS *m*/*z* = 423.2 [M+H]⁺.

### Step B: Preparation of 2-(7-(5-(6-Isopropoxypyridin-3-yl)-1,2,4-oxadiazol-3-yl)-1,2,3,4-tetrahydrocyclopenta[b]indol-3-yl)acetic Acid (Compound 93).

To a solution of ethyl 2-(7-(5-(6-chloropyridin-3-yl)-1,2,4-oxadiazol-3-yl)-1,2,3,4-tetrahydrocyclopenta[b]indol-3-yl)acetate (0.050 g, 0.118 mmol) in DMF (0.850 mL) was added propan-2-ol (0.091 mL, 1.182 mmol) and potassium 2-methylpropan-2-olate (0.236 mL, 0.236 mmol) at 0 °C. The mixture was stirred at room temperature for 2 h, then added water (100 µL) and stirred at room temperature for 1 h. After adding aqueous 2 M NaOH (3 eq.), the mixture was stirred at room temperature for another 1 h. The mixture was purified by preparative HPLC to afford the title compound as a yellow solid (0.018 g). LCMS *m*/*z* = 419.2 [M+H]⁺. ¹H NMR (400 MHz, Methanol-*d*₄) δ ppm 1.34-1.41 (d, 6H), 2.14-2.25 (m, 1H), 2.49-2.58 (m, 1H), 2.68-2.95 (m, 4H), 3.55-3.65 (m, 1H), 5.36-5.47 (m, 1H), 6.89 (d, *J* = 8.59 Hz, 1H), 7.40 (d, *J* = 8.34 Hz, 1H), 7.77 (dd, *J* = 8.34 Hz, 1.52 Hz, 1H), 8.14 (s, 1H), 8.36 (dd, *J =* 8.59 Hz, 2.53 Hz, 1H), 8.96 (s, 1H).

### Example 1.101: Preparation of 2-(7-(5-(6-Methoxypyridin-3-yl)-1,2,4-oxadiazol-3-yl)-1,2,3,4-tetrahydrocyclopenta[b]indol-3-yl)acetic Acid (Compound 75).

To a solution of ethyl 2-(7-(5-(6-chloropyridin-3-yl)-1,2,4-oxadiazol-3-yl)-1,2,3,4-tetrahydrocyclopenta[b]indol-3-yl)acetate (0.050 g, 0.118 mmol) in DMF (0.850 mL) was added methanol (0.038 g, 1.182 mmol) and potassium 2-methylpropan-2-olate (0.177 mL, 0.177 mmol) at 0 °C. The mixture was stirred at room temperature for 3 h, then added water (100 µL) and stirred at room temperature for 1 h. After adding 2 M aqueous NaOH (3 eq.), the mixture was stirred at room temperature for 1 h. The mixture was purified by preparative HPLC to provide the title compound as a white solid (0.016 g). LCMS *m*/*z* = 391.3 [M+H]⁺. ¹H NMR (400 MHz, DMSO-*d*₆) δ ppm 2.06-2.21 (m, 1H), 2.38-2.47 (m, 1H), 2.66-2.91 (m, 4H), 3.55-3.67 (m, 1H), 3.99 (s, 3H), 7.09 (d, *J* = 8.84 Hz, 1H), 7.49 (d, *J* = 8.59 Hz, 1H), 7.74 (d, *J* = 8.59 Hz, 1H), 8.11 (s, 1H), 8.43(dd, *J* = 8.59 Hz, 2.27 Hz, 1H), 9.01 (s, 1H), 11.08 (s, 1H), 12.26 (bs, 1H).

### Example 1.102: Preparation of 2-(7-(5(5-Isopropoxypyridin-2-yl)-1,2,4-oxadiazol-3-yl)-1,2,3,4-tetrahydrocyclopenta[b]indol-3-yl)acetic Acid (Compound 84).

### Step A: Preparation of Ethyl 2-(7-(5-(5-Bromopyridin-2-yl)-1,2,4-oxadiazo-3-yl)-1,2,3,4-tetrahydrocyclopenta[b]indol-3-yl)acetate.

From ethyl 2-(7-(N-hydroxycarbamimidoyl)-1,2,3,4-tetrahydrocyclopenta[b]indol-3-yl)acetate and 5-bromonicotinoyl chloride, using a method similar to **Example 1.99**, **Step E**, the title compound was obtained as a yellow solid. LCMS *m*/*z* = 467.3 [M+H]⁺.

### Step B: Preparation of 2-(7-(5-(5-Isopropoxypyridin-2-yl)-1,2,4-oxadiazol-3-yl)-1,2,3,4-tetrahydrocyclopenta[b]indol-3-yl)acetic Acid (Compound 84).

From ethyl 2-(7-(5-(5-bromopyridin-2-yl)-1,2,4-oxadiazol-3-yl)-1,2,3,4-tetrahydrocyclopenta[b]indol-3-yl)acetate, using a method similar to **Example 1.100**, **Step B**, the title compound was obtained as a yellow solid. LCMS *m*/*z* = 419.2 [M+H]⁺. ¹H NMR (400 MHz, CD₃OD) δ ppm 1.26-1.34 (d, 6H), 2.05-2.17 (m, 1H), 2.40-2.50 (m, 1H), 2.58-2.85 (m, 4H), 3.46-3.56 (m, 1H), 4.67-4.76 (m, 1H). 7.32 (d, *J =* 8.34 Hz, 1H), 7.48 (dd, *J* = 8.84 Hz, 2.53 Hz, 1H), 7.70 (dd, *J* = 8.59 Hz, 1.26 Hz, 1H), 8.07 (s, 1H), 8.20 (d, *J* = 8.59 Hz, 1H), 8.27 (s, 1H).

### Example 1.103: Preparation of 2-(7-(5-(5-Isopropoxypyrazin-2-yl)-1,2,4-oxadiazol-3-yl) 1,2,3,4-tetrahydrocyclopenta[b]indol-3-yl)acetic Acid (Compound 82).

### Step A: Preparation of Ethyl 2-(7-(5-(5-Chloropyrazin-2-yl)-1,2,4-oxadiazol-3-yl)-1,2,3,4-tetrahydrocyclopenta[b]indol-3-yl)acetate.

From ethyl 2-(7-(N-hydroxycarbamimidoyl)-1,2,3,4-tetrahydrocyclopenta[b]indol-3-yl)acetate and 5-chloropyrazine-2-carbonyl chloride, using a method similar to **Example 1.99**, **Step E**, the title compound was obtained as a yellow solid. LCMS *m*/*z* = 424.2 [M+H]⁺.

### Step B: Preparation of 2-(7-(5-(5-Isopropoxypyrazin-2-yl)-1,2,4-oxadiazol-3-yl)-1,2,3,4-tetrahydrocyclopenta[b]indol-3-yl)acetic Acid (Compound 82).

From ethyl 2-(7-(5-(5-chloropyrazin-2-yl)-1,2,4-oxadiazol-3-yl)-1,2,3,4-tetrahydrocyclopenta[b]indol-3-yl)acetate, using a method similar to **Example 1.100**, **Step B**, the title compound was obtained as a yellow solid. LCMS *m*/*z* = 420.6 [M+H]⁺. ¹H NMR (400 MHz, DMSO-*d*₆) δ ppm 1.33-1.44 (d, 6H), 2.09-2.18 (m, 1H), 2.39-2.47 (m, 1H), 2.69-2.91 (m, 4H), 3.49-3.60 (m, 1H), 5.32-5.43 (m, 1H), 7.50 (d, *J* = 8.46 Hz, 1H), 7.76 (dd, *J* = 8.46 Hz, 1.77 Hz, 1H), 8.13 (s, 1H), 8.48 (s, 1H), 9.11 (s, 1H), 11.1 (s, 1H), 12.28 (bs, 1H).

### Example 1.104: Preparation of 2-(7-(5-(6-Isopropoxypyridazin-3-yl)-1,2,4-oxadiazol-3-yl)-1,2,3,4-tetrahydrocyclopenta[b]indol-3-yl)acetic Acid (Compound 88).

### Step A: Preparation of Ethyl 2-(7-(5-(6-Chloropyridazin-3-yl)-1,2,4-oxadiazol-3-yl-1,2,3,4-tetrahydrocyclopenta[b]indol-3-yl)acetate.

From 2-(7-(N-hydroxycarbamimidoyl)-1,2,3,4-tetrahydrocyclopenta[b]indol-3-yl)acetate and 6-chloropyridazine-3-carbonyl chloride, using a method similar to **Example 1.99**, **Step E**, the title compound was obtained as a brown solid. LCMS *m*/*z* = 424.2 [M+H]⁺.

### Step B: Preparation of 2-(7-(5-(6-Isopropoxypyridazin-3-yl)-1,2,4-oxadiaxol-3-yl)-1,2,3,4-tetrahydrocyclopenta[b]indol-3-yl)acetic Acid (Compound 88).

From ethyl 2-(7-(5-(6-chloropyridazin-3-yl)-1,2,4-oxadiazol-3-yl)-1,2,3,4-tetrahydrocyclopenta[b]indol-3-yl)acetate, using a method similar to **Example 1.100**, **Step B**, the title compound was obtained as a brownish solid. LCMS *m*/*z* = 420.6 [M+H]⁺. ¹H NMR (400 MHz, DMSO-*d*₆) δ ppm 1.35-1.46 (d, 6H), 2.10-2.20 (m, 1H), 2.37-2.48 (m, 1H), 2.69-2.91 (m, 4H), 3.47-3.60 (m, 1H), 5.54-5.64 (h, 1H), 7.42 (d, *J* = 9.47 Hz, 1H), 7.52 (d, *J* = 8.46 Hz, 1H), 7.77 (dd, *J* = 8.46 Hz, 1.64 Hz, 1H), 8.14 (s, 1H), 8.40 (d, *J* = 9.35 Hz, 1H), 11.12 (s, 1H).

### Example 1.105: Preparation of 2-(7-(5-(6-(Propylamino)-4-(trifluoromethyl)pyridin-2-yl)-1,2,4-oxadiazol-3-yl)-1,2,3,4-tetrahydrocyclopenta[b]indol-3-yl)acetic Acid (Compound 105).

### Step A: Preparation of 6-Chloro-N-propyl-4-(trifluoromethyl)pyridin-2-amine

From 2,6-dichloro-4-(trifluoromethyl)pyridine and propan-1-amine, using a method similar to **Example 1.99, Step A**, the title compound was obtained as yellow oil. LCMS *m*/*z* = 239.2 [M+H]⁺; ¹H NMR (400 MHz, CDCl₃) δ ppm 0.93 (t, 3H), 1.56-1.61 (m, 2H), 3.18 (m, 2H), 4.83-4.90 (bs, 1H), 6.35 (s, 1H), 6.67 (s, 1H).

### Step B: Preparation of 6-(Propylamino)-4-(trifluoromethyl)picolinonitrile.

From 6-Chloro-N-propyl-4-(trifluoromethyl)pyridin-2-amine and dicyanozinc, using a method similar to **Example 1.99**, **Step B**, the title compound was obtained as a yellow solid. LCMS *m*/*z* = 230.4 [M+H]⁺; ¹H NMR (400 MHz, CDCl₃); δ ppm 1.03 (t, 3H), 1.68(m, 2H), 3.35 (q, 2H), 5.08 (bs, 1H), 6.75 (s, 1H), 7.10 (s, 1H).

### Step C: Preparation of 6-(Propylamino)-4-(trifluoromethyl)picolinic Acid.

From 6-(propylamino)-4-(trifluoromethyl)picolinonitrile and propylamine, using a method similar to **Example 1.99**, **Step C**, the title compound was obtained as a yellow solid. LCMS *m*/*z* = 247.2 [M-H]⁺.

### Step D: Preparation of Ethyl 2-(7-(5-(6-(Propylamino)-4-(trifluoromethyl)pyridin-2-yl)-1,2,4-oxadiazol-3-yl)-1,2,3,4-tetrahydrocyclopenta[b]indol-3-yl)acetate.

To a solution of ethyl 2-(7-(N-hydroxycarbamimidoyl)-1,2,3,4-tetrahydrocyclopenta[b]indol-3-yl)acetate (0.60 g, 0.199 mmol), 6-(propylamino)-4-(trifluoromethyl)picolinic acid (0.054 g, 0.219 mmol), and triethylamine (0.083 mL, 0.597 mmol) in DMF (1.5 mL) was added HATU (0.083 g, 0.219 mmol) and the mixture was stirred at room temperature overnight. The reaction mixture was then heated at 80 °C for 1 h. The mixture was purified by column chromatography to provide the title compound as an off white solid. LCMS *m*/*z* = 514.4 [M+H]⁺.

### Step E: Preparation of 2-(7-(5-(6-(Propylamino)4-(trifluoromethyl)pyridin-2-yl)-1,2,4-oxadiazol-3-yl)-1,2,3,4-tetrahydrocyclopenta[b]indol-3-yl)acetic Acid (Compound 105).

From ethyl 2-(7-(5-(6-(propylamino)-4-(trifluoromethyl)pyridin-2-yl)-1,2,4-oxadiazol-3-yl)-1,2,3,4-tetrahydrocyclopenta[b]indol-3-yl)acetate, using a method similar to **Example 1.99**, **Step F**, the title compound was obtained as a yellow solid. LCMS *m*/*z* = 486.3 [M+H]⁺. ¹H NMR (400 MHz, CDCl₃) δ ppm 2.08-2.15 (m, 1H), 2.52-2.61 (m, 1H), 2.72-2.95 (m, 4H), 3.53-3.61 (m, 1H), 3.65-3.70 (m, 4H), 3.79-3.84 (m, 4H), 6.90 (s, 1H), 7.32 (d, *J* = 8.46 Hz, 1H), 7.76 (s, 1H), 7.87 (dd, *J* = 8.53, 1.58 Hz, 1H), 8.24 (s, 1H), 8.53 (s, 1H).

### Example 1.106: Preparation of 2-(7-(5-(6-Morpholino-4-(trifluoromethyl)pyridin-2-yl)-1,2,4-oxadiazol-3-yl)-1,2,3,4-tetrahydrocyclopenta[b]indol-3-yl)acetic Acid (Compound 106).

### Step A: Preparation of 4-(6-Chloro-4-(trifluoromethyl)pyridin-2-yl)morpholine

From 2,6-dichloro-4-(trifluoromethyl)pyridine and morpholine, using a method similar to **Example 1.99**, **Step A**, the title compound was obtained as yellow oil. LCMS *m*/*z* = 267.3 [M+H]⁺; ¹H NMR (400 MHz, CDCl₃) δ ppm 3.59-3.63 (m, 4H), 3.80-3.85 (m, 4H), 6.67 (s, 1H), 6.84 (s, 1H).

### Step B. Preparation of 6-Morpholino-4-(trifluoromethyl)picolinonitrile.

From 4-(6-chloro-4-(trifluoromethyl)pyridin-2-yl)morpholine and dicyanozinc, using a method similar to **Example 1.99**, **Step B**, the title compound was obtained as a yellow oil. LCMS *m*/*z* = 258.5 [M+H]⁺; ¹H NMR (400 MHz, CDCl₃) δ ppm 3.62-3.66 (m, 4H), 3.83-3.87(m, 4H), 6.98 (s, 1H), 7.16 (s, 1H).

### Step C: Preparation of 6-Morpholino-4-(trifluoromethyl)picolinic Acid.

From 6-morpholino-4-(trifluoromethyl)picolinonitrile, using a method similar to **Example 1.99**, **Step C**, the title compound was obtained as yellow solid. LCMS *m*/*z* = 277.4 [M+H]⁺.

### Step D: Preparation of 6-Morpholino-4-(trifluoromethyl)picolinoyl Chloride.

From 6-(morpholino)-4-(trifluoromethyl)picolinic acid, using a method similar to **Example 1.99**, **Step D**, the title compound was obtained.

### Step E: Preparation of Ethyl 2-(7-(5-(6-(Morpholino)-4-(trifluoromethyl)pyridin-2-yl)-1,2,4-oxadiazol-3-yl)-1,2,3,4-tetrahydrocyclopenta[b]indol-3-yl)acetate.

From ethyl 2-(7-(N-hydroxycarbamimidoyl)-1,2,3,4-tetrahydrocyclopenta[b]indol-3-yl)acetate and 6-(morpholino)-4-(trifluoromethyl)picolinoyl chloride, using a method similar to **Example 1.99**, **Step E**, the title compound was obtained as an off white solid. LCMS *m*/*z* = 540.3 [M-H]⁺.

### Step F: Preparation of 2-(7-(5-(6-Morpholino-4-(trifluoromethyl)pyridin-2-yl)-1,2,4-oxadiazol-3-yl)-1,2,3,4-tetrahydrocyclopenta[b]indol-3-yl)acetic Acid (Compound 106).

From ethyl 2-(7-(5-(6-(morpholino)-4-(trifluoromethyl)pyridin-2-yl)-1,2,4-oxadiazol-3-yl)-1,2,3,4-tetrahydrocyclopenta[b]indol-3-yl)acetate, using a method similar to **Example 1.99**, **Step F**, the title compound was obtained as white solid. LCMS *m*/*z* = 514.3 [M+H]⁺. ¹H NMR (400 MHz, CDCl₃) δ ppm 2.08-2.15 (m, 1H), 2.52-2.61 (m, 1H), 2.72-2.95 (m, 4H), 3.53-3.61 (m, 1H), 3.65-3.70 (m, 4H), 3.79-3.84 (m, 4H), 6.90 (s, 1H), 7.32 (d, *J* = 8.46 Hz, 1H), 7.76 (s, 1H), 7.87 (dd, *J* = 8.53, 1.58 Hz, 1H), 8.24 (s, 1H), 8.53 (s, 1H).

### Example 1.107: Preparation of 2-(7-(5-(5-Methoxypyrazin-2-yl)-1,2,4-oxadiazol-3-yl)-1,2,3,4-tetrahydrocyclopenta[b]indol-3-yl)acetic Acid (Compound 109).

From ethyl 2-(7-(5-(5-chloropyrazin-2-yl)-1,2,4-oxadiazol-3-yl)-1,2,3,4-tetrahydrocyclopenta[b]indol-3-yl)acetate, using a method similar to **Example 1.100**, **Step C**, the title compound was obtained as a tanned solid. LCMS *m*/*z* = 392.2 [M+H]⁺. ¹H NMR (400 MHz, DMSO-*d*₆) δ ppm 2.11-2.20 (m, 1H), 2.43 (dd, *J* = 16.11, 8.91 Hz, 1H), 2.69-2.92 (m, 4H), 3.46-3.63 (m, 1H), 4.06 (s, 3H), 7.50 (d, *J* = 8.46 Hz, 1H), 7.76 (d, 1H), 8.13 (s, 1H), 8.56 (s, 1H), 9.13 (s, 1H), 11.06 (s, 1H), 12.24 (s, 1H).

### Example 1.108: Preparation of 2-(7-(5-(3-(Pyridin-4-yl)-5-(trifluoromethoxy)phenyl)-1,2,4-oxadiazol-3-yl)-1,2,3,4-tetrahydrocyclopenta[b]indol-3-yl)acetic Acid (Compound 96).

### Step A: Preparation of Ethyl 2-(7-(5-(3-Bromo-5-(trifluoromethoxy)phenyl)-1,2,4-oxadiazol-3-yl)-1,2,3,4-tetrahydrocyclopenta[b]indol-3-yl)acetate.

To a solution of ethyl 2-(7-(N'-hydroxycarbamimidoyl)-1,2,3,4-tetrahydrocyclopenta[b]indol-3-yl)acetate (1.04 g, 3.45 mmol) in dioxane (20.79 mL) and 3-bromo-5-(trifluoromethoxy)benzoyl chloride (1.152 g, 3.80 mmol) was added TEA (1.924 mL, 13.80 mmol). The mixture was heated to reflux for 2 h, then diluted with water. The resultant precipitate was collected by filtration and washed with water to provide a brown solid. The solid was purified by column chromatography to give the title compound as a yellow solid (1.254 g).

### Step B: Preparation of 2-(7-(5-(3-Bromo-5-(trifluoromethoxy)phenyl)-1,2,4-oxadiazol-3-yl)-1,2,3,4-tetrahydrocyclopenta[b]indol-3-yl)acetic Acid.

To a solution of ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetate (0.305 g, 0.554 mmol) in dioxane (1.847 mL) and water (0.924 mL) was added NaOH (0.610 mL, 0.610 mmol) and heated to reflux for 18 h. An additional NaOH (0.610 mL, 0.610 mmol) was added, heating was continued for 18 h. The mixture was neutralized with HCl (1.219 mL, 1.219 mmol) and extracted with EtOAc (thrice). The combined extracts were washed with brine, dried over MgSO₄ and concentrated to give the title compound as a yellow solid (0.282 g). LCMS *m*/*z* = 522.3 [M+H]⁺.

### Step C: Preparation of 2-(7-(5-(3-(Pyridin-4-yl)-5-(trifluoromethoxy)phenyl)-1,2,4-oxadiazol-3-yl)-1,2,3,4-tetrahydrocyclopenta[b]indol-3-yl)acetic Acid.

To a solution of 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid (74.6 mg, 0.100 mmol) in benzene (259 µL) and ethanol (74.1 µL) was added Na₂CO₃ (100 µL, 0.200 mmol), pyridin-4-ylboronic acid (18.44 mg, 0.150 mmol), and tetrakis(triphenylphosphine)palladium(0) (3.47 mg, 3.00 µmol). The reaction mixture was heated under microwave irradiation to 100 °C for 5 h. Bu₄NBr (5 mg) was added, heating was continued for 6 h. The mixture was purified by preparative LCMS to provide the title compound as a yellow solid (14.3 mg). LCMS *m*/*z* = 521.6[M+H]⁺. ¹H NMR (400 MHz, DMSO-*d*₆) δ ppm 2.10-2.20 (m, 1H), 2.39-2.48 (m, 1H), 2.70-2.82 (m, 4H), 3.55 (bs, 1H), 7.51 (d, *J* = 8.59 Hz, 1H), 7.79 (dd, *J* = 8.46, 1.39 Hz, 1H), 8.11 (d, *J* = 5.81 Hz, 2H), 8.17 (s, 1H), 8.26 (s, 2H), 8.63 (s, 1H), 8.84 (d, *J* = 6.06 Hz, 2H), 11.10 (s, 1H).

### Example 1.109: Preparation of 2-(7-(5-(3-(Pyridin-3-yl)-5-(trifluoromethoxy)phenyl)-1,2,4-oxadiazol-3-yl)-1,2,3,4-tetrahydrocyclopenta[b]indol-3-yl)acetic Acid (Compound 79).

### Step A: Preparation of Ethyl 2-(7-(5-(3-(Pyridin-3-yl)-5-(trifluoromethoxy)phenyl)-1,2,4-oxadiazol-3-yl)-1,2,3,4-tetrahydrocyclopenta[b]indol-3-yl)acetate.

To a solution of ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetate (0.055 g, 0.100 mmol) in benzene (0.259 mL) and ethanol (0.074 mL) was added Na₂CO₃ (0.100 mL, 0.200 mmol), pyridin-3-ylboronic acid (0.015 g, 0.120 mmol), and tetrakis(triphenylphosphine)palladium (3.47 mg, 3.00 µmol). The reaction mixture was heated under microwave irradiation to 100 °C for 1 h. The organic layer was separated and the water layer was washed with EtOAc. The combined organics were concentrated *in vacuo* to provide the title compound. LCMS *m*/*z* = 549.4 [M+H]⁺.

### Step B: Preparation of 2-(7-(5-(3-(Pyridin-3-yl)-5-(trifluoromethoxy)phenyl)-1,2,4-oxadiazol-3-yl)-1,2,3,4-tetrahydrocyclopenta[b]indol-3-yl)acetic Acid.

To a solution of ethyl 2-(7-(5-(3-(pyridin-3-yl)-5-(trifluoromethoxy)phenyl)-1,2,4-oxadiazol-3-yl)-1,2,3,4-tetrahydrocyclopenta[b]indol-3-yl)acetate (55 mg, 0.100 mmol) in dioxane (334 µL) and water (167 µL) was added NaOH (21 µL, 0.211 mmol). The reaction mixture was heated to reflux for 2 h, cooled to room temperature and purified by preparative LCMS to give the title compound as a yellow solid (35.3 mg). LCMS *m*/*z* = 521.4[M+H]⁺. ¹H NMR (400 MHz, DMSO-*d*₆) δ ppm 2.07-2.21 (m, 1H), 2.44 (dd, *J* = 16.17, 8.84 Hz, 1H). 2.70-2.93 (m, 4H), 3.55 (bs, 1H), 7.51 (d, *J* = 8.59 Hz, 1H), 7.68 (dd, *J* = 8.08, 5.05 Hz, 1H), 7.79 (dd, *J* = 8.59, 1.52 Hz, 1H), 8.13-8.24 (m, 3H), 8.43 (d, *J* = 8.08 Hz, 1H), 8.55 (s, 1H), 8.75 (dd, *J* = 4.80, 1.26 Hz, 1H), 9.15 (d, *J* = 2.27 Hz, 1H), 11.09 (s, 1H).

### Example 1.110: Preparation of 2-(7-(5-(3-(2-Fluoropyridin-3-yl)-5-(trifluoromethoxy)phenyl)-1,2,4-oxadiazol-3-yl)-1,2,3,4-tetrahydrocyclopenta[b]indol-3-yl)acetic Acid (Compound 99).

### Step A: Preparation of 2-(7-(5-(3-(2-Fluoropyridin-3-yl)-5-(trifluoromethoxy)phenyl)-1,2,4-oxadiazol-3-yl)-1,2,3,4-tetrahydrocyclopeata[b]indol-3-yl)acetic Acid.

From 2-fluoropyridin-3-ylboronic acid and 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.108**, **Step C**, the title compound was obtained as a white solid. LCMS *m*/*z* = 539.3 [M+H]⁺. ¹H NMR (400 MHz, DMSO*d*₆) δ ppm 2.10-2.21 (m, *J* = 2.78 Hz, 1H), 2.43 (dd, *J* = 16.04, 8.72 Hz, 1H), 2.70-2.92 (m, 4H), 3.54 (bs, 1H), 7.51 (d, *J* = 8.59 Hz, 1H), 7.54-7.60 (m, 1H), 7.77 (dd, *J* = 8.59, 1.52 Hz, 1H), 8.05 (s, 1H), 8.15 (s, 1H), 8.21 (s, 1H), 8.30-8.40 (m. 2H), 8.45 (s, 1H), 11.09 (s, 1H), 12.27 (s, 1H).

### Example 1.111: Preparation of 2-(7-(5-(3-(2-Fluoropyridin-4-yl)-5-(trifluoromethoxy)phenyl)-1,2,4-oxadiazol-3-yl)-1,2,3,4-tetrahydrocyclopenta[b]indol-3-yl)acetic Acid (Compound 100).

### Step A: Preparation of 2-(7-(5-(3-(3-Fluoropyridin-4-yl)-5-(trifluoromethoxy)phenyl)-1,2,4-oxadiazol-3-yl)-1,2,3,4-tetrahydrocyclopenta[b]indol-3-yl)acetic Acid.

From 3-fluoropyridin-4-ylboronic acid and 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.108, Step C,** the title compound was obtained as a beige solid. LCMS *m*/*z* = 539.2 [M+H]⁺. ¹H NMR (400 MHz, DMSO-d₆) δ ppm 2.10-2.21 (m, 1H), 2.44 (dd, *J* = 16.17, 8.84 Hz, 1H), 2.70-2.92 (m, 4H), 3.54 (bs, 1H), 7.51 (d, *J* = 8.59 Hz, 1H), 7.78 (dd, *J* = 8.46, 1.64 Hz, 1H), 7.81 (s, 1H), 7.92 (d, *J* = 5.05 Hz, 1H), 8.16 (s, 1H), 8.25 (d, *J* = 9.35 Hz, 2H), 8.41 (d, *J* = 5.30 Hz, 1H), 8.62 (s, 1H). 11.09 (s, 1H), 12.27 (s, 1H).

### Example 1.112: Preparation of 2-(7-(5-(3-(6-Fluoropyridin-3-yl)-5-(trifluoromethoxy)phenyl)-1,2,4-oxadiazol-3-yl)-1,2,3,4-tetrahydrocyclopenta[b]indol-3-yl)acetic Acid (Compound 87).

### Step A: Preparation of 2-(7-(5-(3-(4-Fluoropyridin-3-yl)-5-(trifluoromethoxy)phenyl)-1,2,4-oxadiazol-3-yl)-1,2,3,4-tetrahydrocyclopenta[b]indol-3-yl)acetic Acid.

From 4-fluoropyridin-3-ylboronic acid and 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.108, Step C,** the title compound was obtained as a white solid. LCMS *m*/*z* = 539.3 [M+H]⁺. ¹H NMR (400 MHz, DMSO-*d*₆) δ ppm 2.08-2.22 (m, 1H), 2.44 (dd, *J* = 16.29,8.97 Hz, 1H), 2.67-2.95 (m, 4H), 3.54 (bs, 1H), 7.38 (dd, *J* = 8.34, 2.78 Hz, 1H), 7.51 (d, *J* = 8.59 Hz, 1H), 7.78 (dd, *J* = 8.59, 1.52 Hz, 1H), 8.17 (s, 3H), 8.43-8.65 (m, 2H), 8.78 (d, *J* = 2.53 Hz, 1H), 11.09 (s, 1H), 12.27 (s, 1H).

### Example 1.113: Preparation of 2-(7-(5-(3-(6-Methoxypyridin-3-yl)-5-(trifluoromethoxy)phenyl)-1,2,4-oxadiazol-3-yl)-1,2,3,4-tetrahydrocyclopenta[b]indol-3-yl)acetic Acid (Compound 74).

### Step A: Preparation of Ethyl 2-(7-(5-(3-(6-Methoxypyridin-3-yl)-5-(trifluoromethoxy)phenyl)-1,2,4-oxadiazol-3-yl)-1,2,3,4-tetrahydrocyclopenta[b]indol-3-yl)acetate.

From 6-methoxypyridin-3-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an orange oil. LCMS *m*/*z* = 579.5 [M+H]⁺.

### Step B: Preparation of 2-(7-(5-(3-(6-Methoxypyridin-3-yl)-5-(trifluoromethoxy)phenyl)-1,2,4-oxadiazol-3-yl)-1,2,3,4-tetrahydrocyclopenta[b]indol-3-yl)acetic Acid.

From ethyl 2-(7-(5-(3-(6-methoxypyridin-3-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a white solid. LCMS *m*/*z* = 551.7 [M+H]⁺. ¹H NMR (400 MHz, DMSO-*d*₆) δ ppm 2.10-2.20 (m, 1H), 2.44 (dd, *J* = 16.29, 8.97 Hz, 1H), 2.71-2.91 (m, 4H), 3.54 (bs, 1H), 3.94 (s, 3H), 6.99 (d, *J* = 8.59 Hz, 1H), 7.51 (d, *J* = 8.59 Hz, 1H), 7.78 (dd, *J* = 8.59, 1.52 Hz, 1H), 8.06-8.11 (m, *J* = 2.15, 1.39 Hz, 2H), 8.16 (s, 1H), 8.25 (dd, *J* = 8.72, 2.65 Hz, 1H), 8.46 (s, 1H), 8.70 (d, *J* = 2.53 Hz, 1H), 11.09 (s, 1H), 12.30 (bs, 1H).

### Example 1.114: Preparation of 2-(7-(5-(3-(1-(tert-Butoxycarbonyl)-1H-pyrrol-2-yl)-5-(trifluoromethoxy)phenyl)-1,2,4-oxadiazol-3-yl)-1,2,3,4-tetrahydrocyclopenta[b]indol-3-yl)acetic Acid (Compound 91).

### Step A: Preparation of tert-Butyl 2-(3-(3-(3-(2-Ethoxy-2-oxoethyl)-1,2,3,4-tetrahydrocyclopenta[b]indol-7-yl)-1,2,4-oxadiazol-5-yl)-5-(trifluoromethoxy)phenyl)-1H-pyrrole-1-carboxylate.

From 1-(tert-butoxycarbonyl)-1*H*-pyrrol-2-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an yellow solid. LCMS *m*/*z* = 637.4 [M+H]⁺.

### Step B: Preparation of 2-(7-(5-(3-(1-(tert-Butoxycarbonyl)-1H-pyrrol-2-yl)-5-(trifluoromethoxy)phenyl)-1,2,4-oxadiazol-3-yl)-1,2,3,4-tetrahydrocyclopenta[b]indol-3-yl)acetic Acid.

From tert-butyl 2-(3-(3-(3-(2-ethoxy-2-oxoethyl)-1,2,3,4-tetrahydrocyclopenta[b]indol-7-yl)-1,2,4-oxadiazol-5-yl)-5-(trifluoromethoxy)phenyl)-1*H*-pyrrole-1-carboxylate, using a method similar to **Example 1.109, Step B,** the title compound was obtained as a white solid. LCMS *m*/*z* = 609.6 [M+H]⁺. ¹H NMR (400 MHz, DMSO-*d*₆) δ ppm 1.36 (s, 9H), 2.09-2.20 (m, 1H), 2.43 (dd, *J* = 16.17, 8.84 Hz, 1H), 2.69-2.91 (m, 4H), 3.54 (bs, 1H), 6.38 (t, *J* = 3.28 Hz, 1H), 6.57 (dd, *J* = 3.28, 1.77 Hz, 1H), 7.47-7.49 (m, 1H), 7.50 (d, *J* = 8.59 Hz, 1H), 7.76 (dd, *J* = 8.59, 1.52 Hz, 1H), 7.79 (s, 1H), 8.07 (s, 1H), 8.12-8.19 (m, 2H), 11.09 (s, 1H), 12.26 (s, 1H).

### Example 1.115: Preparation of 2-(7-(5-(3-(Pyrimidin-5-yl)-5-(trifluoromethoxy)phenyl)-1,2,4-oxadiazol-3-yl)-1,2,3,4-tetrahydrocyclopenta[b]indol-3-yl)acetic Acid (Compound 98).

### Step A: Preparation of Ethyl 2-(7-(5-(3-(Pyrimidin-3-yl)-5-(trifluoromethoxy)phenyl)-1,2,4-oxadiazol-3-yl)-1,2,3,4-tetrahydrocyclopenta[b]indol-3-yl)acetate.

From pyrimidin-3-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an yellow solid. LCMS *m*/*z* = 550.6 [M+H]⁺.

### Step B: Preparation of 2-(7-(5-(3-(Pyrimidin-3-yl)-5-(trifluoromethoxy)phenyl)-1,2,4-oxadiazol-3-yl)-1,2,3,4-tetrahydrocyclopenta[b]indol-3-yl)acetic Acid.

From ethyl 2-(7-(5-(3-(pyrimidin-3-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a light yellow solid. LCMS *m*/*z* = 522.5 [M+H]^{+ 1}H NMR (400 MHz, DMSO-*d*₆) δ ppm 2.09-2.20 (m, 1H), 2.44 (dd, *J* = 16.29, 8.97 Hz, 1H), 2.72-2.92 (m, 4H), 3.55 (bs, 1H), 7.51 (d, *J* = 8.59 Hz, 1H), 7.79 (dd, *J* = 8.59,1.52 Hz, 1H), 8.17 (s, 1H), 8.22 (s, 1H), 8.27 (s, 1H), 8.63 (s, 1H), 9.30 (s, 1H), 9.34 (s, 2H), 11.09 (s, 1H), 12.26 (s, 1H).

### Example 1.116: Preparation of 2-(7-(5-(3-(2-Methoxypyrimidin-5-yl)-5-(trifluoromethoxy)phenyl)-1,2,4-oxadiazol-3-yl)-1,2,3,4-tetrahydrocyclopenta[b]indol-3-yl)acetic Acid (Compound 104).

### Step A: Preparation of Ethyl 2-(7-(5-(3-(2-Methoxypyrimidin-5-yl)-5-(trifluoromethoxy)phenyl)-1,2,4-oxadiazol-3-yl)-1,2,3,4-tetrahydrocyclopenta[b]indol-3-yl)acetate.

From 2-methoxypyrimidin-5-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as a yellow solid. LCMS *m*/*z* = 580.7 [M+H]⁺.

### Step B: Preparation of 2-(7-(5-(3-(2-Methoxypyrimidin-5-yl)-5-(trifluoromethoxy)phenyl)-1,2,4-oxadiazol-3-yl)-1,2,3,4-tetrahydrocyclopenta[b]indol-3-yl)acetic Acid.

From ethyl 2-(7-(5-(3-(2-methoxypyrimidin-5-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a white solid. LCMS *mlz* = 552.6 [M+H]⁺. ¹H NMR (400 MHz, DMSO-*d*₆) δ ppm 2.09-2.20 (m, 1H), 2.38-2.47 (m, 1H), 2.71-2.92 (m, 4H), 3.54 (bs, 1H), 4.01 (s, 3H), 7.51 (d, *J* = 8.59 Hz, 1H), 7.78 (dd, *J* = 8.46, 1.64 Hz, 1H), 8.14-8.19 (m, 3H), 8.56 (t, *J* = 1.45 Hz, 1H), 9.14 (s, 2H), 11.09 (s, 1H), 12.27 (s, 1H).

### Example 1.117: Preparation of 2-(7-(5-(3-(3-Fluoropyridin-4-yl)-(trifluoromethoxy)pheny)-1,2,4-oxadiazol-3-yl)-1,2,3,4-tetrahydrocyclopenta[b]indol-3-yl)acetic Acid (Compound 110).

### Step A: Preparation of Ethyl 2-(7-(5-(3-(3-Fluoropyridin-4-yl)-5-(trifluoromethoxy)phenyo-1,2,4-oxadiazol-3-yl)-1,2,3,4-tetrahydrocyclopenta[b]indol-3-yl)acetate.

From 3-fluoropyridin-4-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as a yellow solid. LCMS *m*/*z* = 567.3 [M+H]⁺.

### Step B: Preparation of 2-(7-(5-(3-(3-Fluoropyridin-4-yl)-5-(trifluoromethoxy)phenyl)-1,2,4-oxadiazol-3-yl)-1,2,3,4-tetrahydrocyclopenta[b]indol-3-yl)acetic Acid.

From ethyl 2-(7-(5-(3-(3-fluoropyridin-4-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a pink solid. LCMS *mlz* = 539.2 [M+H]⁺. ¹H NMR (400 MHz, CD₃OD) δ ppm 2.17-2.27 (m, 1H). 2.56 (dd, *J* = 16.17, 7.83 Hz, 1H), 2.74 (dd, *J* = 16.17, 7.07 Hz, 1H), 2.78-2.97 (m, 3H), 3.62 (bs, 1H), 7.42 *(d, J* = 8.46 Hz, 1H), 7.77 (dd, *J* = 6.69, 5.18 Hz, 1H), 7.81 (dd, *J* = 8.46,1.52 Hz, 1H), 7.92 (s, 1H), 8.19 (s, 1H), 8.22 (d, *J* = 1.01 Hz, 1H), 8.50 (d, *J* = 1.01 Hz, 1H), 8.56 (d, *J* = 5.05 Hz, 1H), 8.66 (d, *J* = 2.65 Hz, 1H).

### Example 1.118: Preparation of 2-(7-(5-(3-(3,5-Dimethylisoxazol-4-yl)-5-(trifluoromethoxy)phenyl)-1,2,4-oxadiazol-3-yl)-1,2,3,4-tetrahydrocyclopenta[b]indol-3-yl)acetic Acid (Compound 111).

### Step A: Preparation of Ethyl 2-(7-(5-(3-(3,5-Dimethylisoxazol-4-yl)-5-(trifluoromethoxy)phenyl)-1,2,4-oxadiazol-3-yl)-1,2,3,4-tetrahydrocyclopenta[b]indol-3-yl)acetate.

From 3,5-dimethylisoxazol-4-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as a yellow solid. LCMS *m*/*z* = 567.4 [M+H]⁺.

### Step B: Preparation 2-(7-(5-(3-(3,5-Dimethylisoxazol-4-yl)-5-(trifiuoromethoxy)phenyl)-1,2,4-oxadiazol-3-yl)-1,2,3,4-tetrahydrocyclopenta[b]indol-3-yl)acetic Acid.

From ethyl 2-(7-(5-(3-(3,5-dimethylisoxazol-4-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a brown solid. LCMS *m*/*z* = 539.4 [M+H]⁺. ¹H NMR (400 MHz, DMSO-*d*₆) δ ppm 2.07-2.21 (m, 1H), 2.31 (s, 3H), 2.43 (dd, *J* = 16.04, 8.84 Hz, 1H), 2.50 (s, 3H), 2.70-2.90 (m, 4H), 3.54 (bs, 1H), 7.50 (d, *J* = 8.84 Hz, 1H), 7.77 (dd, *J* = 8.59, 1.64 Hz, 1H), 7.82-7.84 (m, 1H), 8.11-8.15 (m, 2H), 8.16-8.19 (m, 1H), 11.06 (s, 1H), 12.23 (bs, 1H).

### Example 1.119: Preparation of 2-(7-(5-(3-(2,6-Difluoropyridin-4-yl)-5-(trifluoromethoxy)phenyl)-1,2,4-oxadiazol-3-yl)-1,2,3,4-tetrahydrocyclopenta[b]indol-3-yl)acetic Acid (Compound 112).

### Step A: Preparation of Ethyl 2-(7-(5-(3-(2,6-Difluoropyridin-4-yl)-5-(trifluoromethoxy)phenyl)-1,2,4-oxadiazol-3-yl)-1,2,3,4-tetrahydrocyclopenta[b]indol-3-yl)acetate.

From 2, 6-difluoropyridin-4-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as a yellow solid. LCMS *m*/*z* = 585.3 [M+H]⁺.

### Step B: Preparation of 2-(7-(5-(3-(2, 6-Difluoropyridin-4-yl)-5-(trifluoromethoxy)phenyl)-1,2,4-oxadiazol-3-yl)-1,2,3,4-tetrahydrocyclopenta[b]indol-3-yl)acetic Acid.

From ethyl 2-(7-(5-(3-(2,6-difluoropyridin4-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a tan solid. LCMS *m*/*z* = 557.3 [M+H]⁺. ¹H NMR (400 MHz, CD₃OD) δ ppm 2.16-2.27 (m, 1H), 2.56 (dd, *J* = 16.17, 7.71 Hz, 1H), 2.74 (dd, *J* = 16.11, 7.01 Hz, 1H), 2.78-2.94 (m, 3H), 3.55-3.65 (m, 1H), 7.38-7.42 (m, 1H), 7.41 (s, 2H), 7.78 (dd, *J* = 8.59, 1.52 Hz, 1H), 7.98 (d, *J* = 0.51 Hz, 1H), 8.15 (d, *J* = 1.14 Hz, 1H), 8.18-8.23 (m, 1H), 8.52 (t, *J* = 1.45 Hz, 1H).

### Example 1.120: Preparation of 2-(7-(5-(3-(6-Fluoro-5-methylpyridin-3-yl)-5-(trifluoromethoxy)phenyl)-1,2,4-oxadiazol-3-yl)-1,2,3,4-tetrahydrocyclopenta[b]indol-3-yl)acetic Acid (Compound 113).

### Step A: Preparation of Ethyl 2-(7-(5-(3-(6-Fluoro-5-methylpyridin-3-yl)-5-(trifluoromethoxy)phenyl)-1,2,4-oxadiazol-3-yl)-1,2,3,4-tetrahydrocyclopenta[b]indol-3-yl)acetate.

From 6-fluoro-5-methylpyridin-3-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an yellow solid. LCMS *m*/*z* = 581.4 [M+H]⁺.

### Step B: Preparation of 2-(7-(5-(3-(6-Fluoro-5-methylpyridin-3-yl)-5-(trifluoromethoxy)phenyl)-1,2,4-oxadiazol-3-yl)-1,2,3,4-tetrahydrocyclopenta[b]indol-3-yl)acetic Acid.

**From ethyl 2-(7-(5-(3-(6-fluoro-5-methylpyridin-3-yl)-5-(trifluoromethoxy)phenyl)-1,2,4-**oxadiazol-3-yl)-1,2,3,4-tetrahydrocyclopenta[b]indol-3-yl)acetate, using a method similar to **Example 1.109, Step B,** the title compound was obtained as a purple solid. LCMS *m*/*z* = 553.5 [M+H]^{+ 1}H NMR (400 MHz, DMSO-*d*₆) δ ppm 2.11-2.21 (m, 1H), 2.36 (s, 3H), 2.44 (dd, *J* = 16.11, 8.91 Hz, 1H), 2.70-2.92 (m, 4H), 3.54 (bs, 1H), 7.51 (d, *J* = 8.46 Hz, 1H), 7.78 (dd, *J* = 8.53, 1.71 Hz, 1H), 8.13-8.15 (m, *J* = 2.27, 1.01 Hz, 2H), 8.16 (d, *J* = 1.39 Hz, 1H), 8.38-8.46 (m, 1H), 8.52 (t, *J* = 1.52 Hz, 1H), 8.57 (d, *J* = 2.15 Hz, 1H), 11.07 (s, 1H), 12.24 (s, 1H).

### Example 1.121: Preparation of 2-(7-(5-(3-(1-Methyl-1H-pyrazol4-yl)-5-(trifluoromethoxy)phenyl)-1,2,4-oxadiazol-3-yl)-1,2,3,4-tetrahydrocyclopenta[b]indol-3-yl)acetic Acid (Compound 114).

### Step A: Preparation of Ethyl 2-(7-(5-(3-(1-Methyl-1H-pyrazol-4-yl)-5-(trifluoromethoxy)phenyl)-1,2,4-oxadiazol-3-yl)-1,2,3,4-tetrahydrocyclopenta[b]indol-3-yl)acetate.

From 1-methyl-4-(4,4,5,5-tetramethyl-1,3,2-dioxaborolan-2-yl)-1H-pyrazole and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an yellow solid. LCMS *m*/*z* = 552.5 [M+H]⁺.

### Step B: Preparation of 2-(7-(5-(3-(1-Methyl-1H-pyrazol-4-yl)-5-(trifluoromethoxy)phenyl)-1,2,4-oxadiazol-3-yl)-1,2,3,4-tetrahydrocyclopenta[b]indol-3-yl) acetic Acid.

From ethyl 2-(7-(5-(3-(1-methyl-1H-pyrazol-4-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a tan solid. LCMS *m*/*z* = 524.4 [M+H]⁺. ¹H NMR (400 MHz, DMSO-*d*₆) δ ppm 2.10-2.20 (m, 1H), 2.39-2.47 (m, 1H), 2.69-2.91 (m, 4H), 3.55 (bs, 1H), 3.90 (s, 3H), 7.51 (d, *J* = 8.46 Hz, 1H), 7.78 (dd, *J* = 8.59, 1.64 Hz, 1H), 7.87-7.91 (m, 1H), 7.95 (s, 1H), 8.16 (d, *J* = 0.63 Hz, 2H), 8.37 (t, *J* = 1.45 Hz, 1H), 8.51 (s, 1H), 11.07 (s, 1H), 12.23 (s, 1H).

### Example 1.122: Preparation of 2-(7-(5-(3-(2-Methylpyridin-4-yl)-5-(trifluoromethoxy)phenyl)-1,2,4-oxadiazol-3-yl)-1,2,3,4-tetrahydrocyclopenta[b]indol-3-yl)acetic Acid (Compound 115).

### Step A: Preparation of Ethyl 2-(7-(5-(3-(2-Methylpyridin-4-yl)-5-(trifluoromethoxy)phenyl)-1,2,4-oxadiazol-3-yl)-1,2,4-tetrahydrocyclopenta[b]indol-3-yl)acetate.

From 2-methylpyridin-4-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an yellow solid. LCMS *m*/*z* = 563.3 [M+H]⁺.

### Step B: Preparation of 2-(7-(-(3-(2-Methylpyridin-4-yl)-5-(trifluoromethoxy)phenyl)-1,2,4-oxadiazol-3-yl)-1,2,3,4-tetrabydrocyclopenta[b]indol-3-yl)acetic Acid.

From ethyl 2-(7-(5-(3-(2-methylpyridin4-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a yellow solid. LCMS *m*/*z* = 535.0 [M+H]⁺. ¹H NMR (400 MHz, DMSO-*d*₆) δ ppm 2.10-2.21 (m, 1H), 2.40-2.47 (m, 1H), 2.69 (s, 3H), 2.72-2.91 (m, 4H), 3.55 (bs, 1H), 7.51 (d, *J* = 8.46 Hz, 1H), 7.79 (dd, *J* = 8.46, 1.64 Hz, 1H). 8.03 (dd, *J* = 5.37, 1.33 Hz, 1H), 8.14 (s, 1H), 8.17 (d, *J* = 1.39 Hz, 1H), 8.27 (s, 2H), 8.65 (t, *J* = 1.33 Hz, 1H), 8.76 (d, *J* = 5.56 Hz, 1H), 11.08 (s, 1H), 12.30 (bs, 1H).

### Example 1.123: Preparation of 2-(7-(5-(3-(3-Methylpyridin4-yl)-5-(trifluoromethoxy)phenyl)-1,2,4-oxadiazol-3-yl)-1,2,3,4-tetrahydrocyclopenta[b]indol-3-yl)acetic Acid (Compound 116).

### Step A: Preparation of Ethyl 2-(7-(5-(3-(3-Methylpyridin-4-yl)-5-(trifluoromethoxy)phenyl)1,2,4-oxadiazol-3-yl)-1,2,3,4-tetrahydrocyclopenta[b]indol-3-yl)acetate.

From 3-methylpyridin-4-ylboronic acid and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an yellow solid. LCMS *m*/*z* = 563.4 [M+H]⁺.

### Step B: Preparation of 2-(7-(5-(3-(3-Methylpyridin-4-yl)-5-(trifluoromethoxy)phenyl)-1,2,4-oxadiazol-3-yl)-1,2,3,4-tetrahydrocyclopenta[b]indol-3-yl)acetic Acid.

From ethyl 2-(7-(5-(3-(3-methylpyridin-4-yl)-5-(trifluoromethoxy)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a yellow solid. LCMS *m*/*z* = 535.2 [M+H]⁺. ¹H NMR (400 MHz, DMSO*d*₆) δ ppm 2.10-2.20 (m, 1H), 2.37 (s, 3H), 2.43 (dd, *J* = 16.04, 8.84 Hz, 1H), 2.69-2.91 (m, 4H), 3.54 (bs, 1H), 7.50 (d, *J* = 8.46 Hz, 1H), 7.71 (d, *J* = 5.31 Hz, 1H), 7.76 (dd, *J* = 8.46, 1.64 Hz, 1H), 7.93 (s, 1H), 8.14 (d, *J* = 1.39 Hz, 1H), 8.24-8.25 (m, 1H), 8.26 (t, *J* = 1.45 Hz, 1H), 8.70 (d, *J* = 5.31 Hz, 1H), 8.76 (s, 1H), 11.08 (s, 1H), 12.28 (bs, 1H).

### Example 1.124: Preparation of 2-(7-(5-(3-(Trifluoromethoxy)-5-(1,3,5-trimethyl-1H-pyrazol-4-yl)phenyl)-1,2,4-oxadiazol-3-yl)-1,2,3,4-tetrahydrocyclopenta[b]indol-3-yl)-acetic Acid (Compound 117).

### Step A: Preparation of Ethyl 2-(7-(5-(3-(Trifluoromethoxy)-5-(1,3,5-trimethyl-1H-pyrazol-4-yl)pbenyl)-1,2,4-oxadiazol-3-yl)-1,2,3,4-tetrahydrocyclopenta[b]indol-3-yl)acetate.

From 1,3,5-trimethyl-4(4,4,5,5-tetramethyl-1,3,2-dioxaborolan-2-yl)-1H-pyrazole and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an yellow solid. LCMS *m*/*z* = 580.6 [M+H]⁺.

### Step B: Preparation of 2-(7-(5-(3-(1,3,5-Trimethyl-1H-pyrazol-4-yl)-5-(trifluorometboxy)phenyl)-1,2,4-oxadiazol-3-yl)-1,2,3,4-tetrahydrocyclopenta[b]indol-3-yl) acetic Acid.

From ethyl 2-(7-(5-(3-(trifluoromethoxy)-5-(1,3,5-trimethyl-1H-pymzol-4-yl)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a light purple solid. LCMS *m*/*z* = 552.2 [M+H]⁺. ¹H NMR (400 MHz, DMSO-*d*₆) δ ppm 2.08-2.18 (m, 1H), 2.21 (d, *J* = 2.40 Hz, 3H), 2.31 (s, 3H), 2.38-2.48 (m, 1H), 2.54 (s, 3H), 2.70-2.81 (m, 3H), 2.82-2.90 (m, 1H), 3.55 (bs, 1H), 7.50 (d, *J* = 8.59 Hz, 1H), 7.60 (s, 2H), 7.76 (dd, *J* = 8.53, 1.58 Hz, 1H), 7.97-8.04 (m, 2H), 11.06 (s, 1H).

### Example 1.125: Preparation of 2-(7-(5-(3-(1-Isobutyl-1H-pyrazol-4-yl)-5-(trifluoromethoxy)phenyl)-1,2,4-oxadiazol-3-yl)-1,2,3,4-tetrahydrocyclopenta[b]indol-3-yl)acetic Acid (Compound 118).

### Step A: Preparation of Ethyl 2-(7-(5-(3-(Trifluoromethoxy)-5-(1-isobuty-1H-pyrazol-4-yl)phenyl-1,2,4-oxadiazol-3-yl)-1,2,3,4-tetrahydrocyclopenta[b]indol-3-yl)acetate.

From 1-isobutyl-4-(4,4,5,5-tetramethyl-1,3,2-dioxaborolan-2-yl)-1*H*-pyrazole and ethyl 2-(7-(5-(3-bromo-5-(trifluoromethoxy)phenyl)-1,2,4-oxadiazol-3-yl)-1,2,3,4-tetrahydrocyclopenta[b]indol-3-yl)acetic acid, using a method similar to **Example 1.109, Step A,** the title compound was obtained as an yellow solid. LCMS *m*/*z* = 594.5 [M+H]⁺.

### Step B: Preparation of 2-(7-(5-(3-(1-Isobutyl-1H-pyrazol-4-yl)-5-(trifluoromethoxy)phenyl)-1,2,4-oxadiazol-3-yl)-1,2,3,4-tetrahydrocyclopenta[b]indol-3-yl) acetic Acid.

From ethyl 2-(7-(5-(3-(trifluoromethoxy)-5-(1-isobutyl-1*H*-pyrazol-4-yl)phenyl)-1,2,4-oxadiazol-3-yl)-1,2,3,4-tetrahydrocyclopenta[b]indol-3-yl)acetate, using a method similar to **Example 1.109, Step B,** the title compound was obtained as a yellow solid. LCMS *m*/*z* = 566.5 [M+H]⁺. ¹H NMR (400 MHz, DMSO-*d*₆) δ ppm 0.89 (d, *J* = 6.69 Hz, 6H), 2.08-2.23 (m, 2H), 2.38-2.47 (m, 1H), 2.71-2.95 (m, 4H), 3.54 (bs, 1H), 3.97 (d, *J* = 7.20 Hz, 2H), 7.5 (d, *J* = 8.59 Hz, 1H), 7.78 (dd, *J* = 8.53, 1.58 Hz, 1H), 7.87-7.91 (m, 1H), 7.97 (s, 1H), 8.16 (d, *J* = 1.39 Hz, 1H), 8.18 (s, 1H), 8.39 (t, *J* = 1.39 Hz, 1H), 8.56 (s, 1H), 11.07 (s, 1H), 12.25 (s, 1H).

### Example 1.126: Preparation of 2-(7-(5-(5-(1-methyl-1H-pyrazol-4-yl)pyridin-3-yl)-1,2,4-oxadiazol-3-yl)-1,2,3,4-tetrahydrocyclopenta[b]indol-3-yl)acetic Acid (Compound 121).

### Step A: Preparation of Ethyl 2-(7-(5-(5-Bromopyndin-3-yl)-1,2,4-oxadiazol-3-yl)-1,2,3,4-tetrahydrocyclopenta[b]indol-3-yl)acetate.

To a solution of ethyl 2-(7-(*N*'-hydroxycarbamimidoyl)-1,2,3,4-tetrahydrocyclopenta[b]indol-3-yl)acetate (0.603 g, 2.00 mmol) and 5-bromonicotinic acid (0.606 g, 3.00 mmol) in dioxane (12.05 mL) was added TEA (2.79 mL, 20.00 mmol) followed by 1-propylphosphonic acid cyclic anhydride (1.296 mL, 2.200 mmol). The reaction mixture was heated to reflux overnight. Water and EtOAc were added. The organic layer was separated. The aqueous layer was extracted with EtOAc (twice). The combined extracts were washed with brine, dried over MgSO₄ and concentrated. The residue was purified by column chromatography to provide the title compound as a white solid (0.605 g).

### Step B: Preparation of Ethyl 2-(7-(5-(5-(1-Methyl-1H-pyrazol-4-yl)pyridin-3-yl)-1,2,4-oxadiazol-3-yl)-1,2,3,4-tetrahydrocyclopenta[b]indol-3-yl)acetate.

From 1-methyl-4-(4,4,5,5-tetramethyl-1,3,2-dioxaborolan-2-yl)-1H-pyrazole and ethyl 2-(7-(5-(5-bromopyridin-3-yl)-1,2,4-oxadiazol-3-yl)-1,2,3,4-tetrahydrocyclopenta[b]indol-3-yl)acetate, using a method similar to **Example 1.109, Step A,** the title compound was obtained as an orange solid. LCMS *m*/*z* = 469.4 [M+H]⁺.

### Step C: Preparation of 2-(7-(5-(5-(1-Methyl-1H-pyrazol-4-yl)pyridin-3-yl)-1,2,4-oxadiazol-3-yl)-1,2,3,4-tetrahydrocyclopenta[b]indol-3-yl)acetic Acid.

From ethyl 2-(7-(5-(5-(1-methyl-1H-pyrazol-4-yl)pyridin-3-yl)-1,2,4-oxadiazol-3-yl)-1,2,3,4-tetrahydrocyclopenta[b]indol-3-yl)acetate, using a method similar to **Example 1.109, Step B,** the title compound was obtained as a yellow solid. LCMS *m*/*z* = 441.5 [M+H]⁺. ¹H NMR (400 MHz, DMSO-*d*₆) δ ppm 2.09-2.20 (m, 1H), 2.39-2.47 (m, 1H), 2.70-2.93 (m, 4H), 3.92 (s, 3H), 7.51 (d, *J* = 8.34 Hz, 1H), 7.78 (dd, *J* = 8.53, 1.58 Hz, 1H), 8.15-8.21 (m, 2H), 8.51 (s, 1H), 8.67 (t, *J* = 2.08 Hz, 1H), 9.15 (d, *J* = 2.15 Hz, 2H), 11.08 (s, 1H).

### Example 1.127: Preparation of 2-(7-(5-(5-Hexylpyridin-3-yl)-1,2,4-oxadiazol-3-yl)-1,2,3,4-tetrahydrocyclopenta[b]indol-3-yl)acetic Acid (Compound 122).

### Step A: Preparation of 5-Hexylnicotinic Acid.

To a solution of 5-(hex-1-ynyl)nicotinic acid (0.508 g, 2.5 mmol) was added Pd/C (0.133 g, 0.125 mmol). The reaction mixture was stirred overnight under H₂ atmosphere. The mixture was filtered through celite® and concentrated *in vacuo* to provide the title compound as a tan solid (0.493g). LCMS *m*/*z* = 208.2 [M+H]⁺. ¹H NMR (400 MHz, DMSO-*d*₆) δ ppm 0.82-0.88 (m. 3H), 1.22-1.33 (m, 6H), 1.54-1.65 (m, 2H), 2.63-2.69 (m, 2H), 8.07 (t, *J* = 2.02 Hz, 1H), 8.62 (d, *J* = 1.90 Hz, 1H), 8.88 (d, *J* = 1.64 Hz, 1H).

### Step B: Preparation of Ethyl 2-(7-(5-(5-Hexylpyridin-3-yl)-1,2,4-oxadiazol-3-yl)-1,2,3,4-tetrahydrocyclopenta[b]indol-3-yl)acetate.

From 5-hexylnicotinic acid and ethyl 2-(7-(N-hydroxycarbamimidoyl)-1,2,3,4-tetrahydrocyclopenta[b]indol-3-yl)acetate, using a method similar to **Example 1.126, Step A,** the title compound was obtained as an orange solid. LCMS *m*/*z* = 473.6 [M+H]⁺.

### Step C: Preparation of 2-(7-(5-(5-Hexylpyridin-3-yl)-1,2,4-oxadiazol-3-yl)-1,2,3,4-tetrahydrocyclopenta[b]indol-3-yl)acetic Acid.

From ethyl 2-(7-(5-(5-hexylpyridin-3-yl)-1,2,4-oxadiazol-3-yl)-1,2,3,4-tetrahydrocyclopenta[b]indol-3-yl)acetate, using a method similar to **Example 1.109, Step B,** the title compound was obtained as a yellow solid. LCMS *m*/*z* = 445.7 [M+H]⁺. ¹H NMR (400 MHz, DMSO-*d*₆) δ ppm 0.84-0.89 (m, 3H), 1.27-1.38 (m, 6H), 1.60-1.70 (m, 2H), 2.11-2.20 (m, 1H), 2.38-2.47 (m, 1H), 2.71-2.91 (m, 6H), 3.51-3.58 (m, 1H), 7.50 (d, *J* = 8.59 Hz, 1H), 7.76 (d, *J* = 8.59 Hz, 1H), 8.14 (s, 1H), 8.40 (s, 1H), 8.75 (s, 1H), 9.17 (d, *J* = 1.89 Hz, 1H), 11.07 (s, 1H).

### Example 1.128: Preparation of 2-(7-(5-(4-Cyano-3-(trifluoromethoxy)phenyl)-1,2,4-oxadiazol-3-yl)-1,2,3,4-tetrahydrocyclopenta[b]indol-3-yl)acetic Acid (Compound 95).

Ethyl 2-(7-(5-(4-cyano-3-(trifluoromethoxy)phenyl)-1,2,4-oxadiazol-3-yl)-1,2,3,4-tetrahydrocyclopenta[b]indol-3-yl)acetate (0.037 g, 0.075 mmol) was suspended in 1,4-dioxane (1.00 mL), 1.00 N aqueous lithium hydroxide (0.149 mL, 0.149 mmol) was added and the reaction was heated at 60 °C. After 60 min the reaction mixture was acidified with 1.0 N HCl (0.298 mL, 0.298 mmol) at 25 °C, diluted with EtOAc (50 mL), washed with water (3 x 10 mL), brine (10 mL), dried with MgSO₄, and the solvent was evaporated *in vacuo* to provide the title compound as a light-green solid (28 mg). LCMS *m*/*z* = 469.3 [M+H]⁺. ¹H NMR (400 MHz, CD₃CN) δ ppm 2.64 (d, *J* = 1.26 Hz, 1H), 2.66 (s, 1H), 2.73-2.95 (m, 4H), 3.52-3.64 (m, 1H), 7.51 (d, *J* = 8.59 Hz, 1H), 7.82 (dd, *J* = 8.59, 1.26 Hz, 1H), 8.08 (d, *J* = 8.08 Hz, 1H), 8.20 (s, 1H), 8.26 (s, 1H), 8.30 (d, *J* = 8.34 Hz, 1H), 9.34 (bs, 1H).

### Example 1.129: Preparation of 2-(7-(5-(3-Cyano-4-(cyclopropylmethoxy)phenyl)-1,2,4-oxadiazol-3-yl)-1,2,3,4-tetrahydrocyclopenta[b]indol-3-yl)acetic Add (Compound 77).

From ethyl 2-(7-(5-(3-cyano-4-(cyclopropylmethoxy)phenyl)-1,2,4-oxadiazol-3-yl)-1,2,3,4-tetrahydrocyclopenta[b]indol-3-yl)acetate, using a method similar to Example 1.128, the title compound was obtained as a white solid. LCMS *m*/*z* = 455.4 [M+H]⁺. ¹H NMR (400 MHz, CD₃CN) δ ppm 0.43 (d, *J* = 5.81 Hz, 2H), 0.68 (dd, *J* = 7.96, 1.64 Hz, 2H), 0.84-0.92 (m, 1H), 2.65 (d, *J* = 7.58 Hz, 2H), 2.74-2.95 (m, 3H), 3.53-3.62 (m, 1H), 4.08 (d, *J* = 7.07 Hz, 2H), 7.27 (d, *J* = 9.09 Hz, 1H), 7.49 (d, *J* = 8.59 Hz, 1H), 7.81 (dd, *J* = 8.59, 1.52 Hz, 1H), 8.17 (s, 1H), 8.37 (dd, *J* = 8.84, 2.27 Hz, 1H), 8.43 (d, *J* = 2.27 Hz, 1H), 9.30 (bs, 1H).

### Example 1.130: Preparation of 2-(7-(5-(3-Cyano-5-(trifluoromethoxy)phenyl)-1,2,4-oxadiazol-3-yl)-1,2,3,4-tetrahydrocyclopenta[b]indol-3-yl)acetic Acid (Compound 7).

### Step A: Preparation of Ethyl 2-(7-(N'-Hydroxycarbamimidoyl)-1,2,3,4-tetrahydrocyclopenta[b]indol-3-yl)acetate.

In a 2 L flask equipped with a stirrer, thermocouple and addition funnel was placed ethyl 2-(7-cyano-1,2,3,4-tetrahydrocyclopenta[b]indol-3-yl)acetate (85.8 g, 320 mmol) suspended in ethanol (850 mL). Hydroxylamine (50% in water, 209 g, 3.164 mol) was added over a period of 12 min. The reaction was slowly heated to 61 °C (internal), stirred for 75 min, cooled to room temperature and allowed to stand overnight. The solvent (~700 mL) was removed by rotary evaporator and the residue was poured into crushed ice (500 g) and extracted with ethyl acetate (3 X 200 mL). Ethyl acetate extracts were dried over MgSO₄ (60g) and concentrated. The residue was purified by silica gel column chromatography to give the title compound (84 g). LCMS *m*/*z* = 302.3.

### Step B: Preparation of Ethyl 2-(7-(5-(3-Cyano-5-(trifluoromethoxy)phenyl)-1,2,4-oxadiazol-3-yl)-1,2,3,4-tetrahydrocyclopenta[b]indol-3-yl)acetate.

In a 2 L flask equipped with condenser, thermocouple and mechanical stirrer was placed cyano-trifluormethoxy benzoic acid (62.8 g, 292 mmol) and acetonitrile (700 mL) at room temperature. CDI (42.8 g, 264 mmol) was added portion-wise over 18 min keeping the temperature below 23 °C. Upon completion, the reaction was warmed to 50 °C (internal), stirred for 40 min and cooled to 35 °C. Ethyl 2-(7-(N'-hydroxycarbamimidoyl)-1,2,3,4-tetrahydrocyclopenta[b]indol-3-yl)acetate (80 g, 264 mmol) was added and the reaction was heated at 60 °C for 1 h and 72.6 °C (internal) for 3 h. The reaction was cooled and allowed to stand at room temperature overnight. The solid was filtered and washed with ACN (3 X 60 mL). It was further purified by recrystalization from a mixture of acetonitrile (670 mL) and ethyl acetate (70 mL) to give the title compound (37.8 g). The filtrates were combined and concentrated *in vacuo*, re-suspended in water (350 mL), and extracted with ethyl acetate (460 mL). The ethyl acetate extract was washed with saturated bicarbonate, water (100 mL) and concentrated. The residue was extracted with heptanes/ethyl acetate (1:1, 700 mL) and further purified by silica gel chromatography to give additional title compounds. The total combined amount to 74.4 g of the title compound. LCMS *m*/*z* = 497.6.

### Step C: Preparation of 2-(7-(5-(3-Cyano-5-(trifluoromethoxy)phenyl)-1,2,4-oxadiazol-3-yl)-1,2,3,4-tetrahydrocyclopenta[b]indol-3-yl)acetic Acid.

In a 2 L flask equipped with a stirrer, thermocouple and condenser were placed ethyl 2-(7-(5-(3-cyano-5-(trifluoromethoxy)phenyl)-1,2,4-oxadiazol-3-yl)-1,2,3,4-tetrahydrocyclopenta[b]indol-3-yl)acetate (67.4g, 136 mmol), acetonitrile (686 mL), water (11 g) lithium bromide (122 g, 1.407 mol) and triethylamine (42.7 g, 422 mmol) at room temperature. The reaction was heated at 65 °C (internal) for 4 h. Lithium bromide (25 g, 287 mmol), ACN (300 mL) and additional amount of water (5 mL) were added. Heating was continued until nearly all the starting materials were consumed. The reaction was cooled and the solid formed was filtered and washed with acetonirile (400 mL). The solid was dissolved slowly in water (750 mL) and the solution was filtered. The deep orange filtrate was cooled in ice bath and carefully acidified with concentrated HCl to pH 3. The precipitate was extracted with ethyl acetate (3 X 200 mL), washed with water (100 mL), dried over MgSO₄ (50 g) and filtered. The solvent was removed from the filtrate and the residue was dried overnight under vacuum to give the title compound (61.5 g). LCMS *m*/*z* = 469.3.

### Example 2

### Homogeneous Time-Resolved Fluorescence (HTRF^{®}) Assay For Direct cAMP Measurement

Compounds were screened for agonists of S1P1 receptor (e.g., human S1P1 receptor) using HTRF^{®} assay for direct cAMP measurement (Gabriel et al, ASSAY and Drug Development Technologies, 1:291-303, 2003) and recombinant CHO-K1 cells stably transfected with S1P1 receptor. CHO-K1 cells were obtained from ATCC^{®} (Manassas, VA; Catalog # CCL-61). An agonist of S1P1 receptor was detected in HTRF^{®} assay for direct cAMP measurement as a compound which decreased cAMP concentration. HTRF^{®} assay also was used to determine EC₅₀ values for S1P1 receptor agonists.

**Principle of the assay:** HTRF^{®} assay kit was purchased from Cisbio-US, Inc. (Bedford; MA; Catalog # 62AM4PEC). The HTRF^{®} assay supported by the kit is a competitive immunoassay between endogenous cAMP produced by the CHO-K1 cells and tracer cAMP labeled with the dye d2. The tracer binding is visualized by a monoclonal anti-cAMP antibody labeled with Cryptate. The specific signal (i.e., fluorescence resonance energy transfer, FRET) is inversely proportional to the concentration of unlabeled cAMP in the standard or sample.

**Standard curve:** The fluorescence ratio (665 nm/620 nm) of the standards (0.17 to 712 nM cAMP) included in the assay was calculated and used to generate a cAMP standard curve according to the kit manufacturer's instructions. The fluorescence ratio of the samples (test compound or compound buffer) was calculated and used to deduce respective cAMP concentrations by reference to the cAMP standard curve.

**Setup of the assay:** HTRF^{®} assay was carried out using a two-step protocol essentially according to the kit manufacturer's instructions, in 20 µL total volume per well in 384-well plate format (ProxiPlates; PerkinElmer, Fremont, CA; catalog # 6008280). To each of the experimental wells was transferred1500 recombinant CHO-K1 cells in 5 µL phosphate buffered saline containing calcium chloride and magnesium chloride ("PBS+"; Invitrogen, Carlsbad, CA; catalog # 14040) supplemented with IBMX (250 µM) and rolipram (20 µW (phosphodiesterase inhibitors; Sigma-Aldrich, St. Louis, MO; catalog # I5879 and catalog # R6520, respectively), followed by test compound in 5 µL compound buffer (PBS+ supplemented with 10 µL NKH477 (water-soluble forskolin derivative; SignaGen Laboratories, Gaithersburg, MD; catalog # PKI-NKH477-010)) or 5 µL compound buffer. The plate was then incubated at room temperature for 1 hour. To each well was then added 5 µL cAMP-d2 conjugate in lysis buffer and 5 µL Cryptate conjugate in lysis buffer according to the kit manufacturer's instructions. The plate was then further incubated at room temperature for 1 hour, after which the assay plate was read.

**Assay readout:** HTRF^{®} readout was accomplished using a PHERAstar (BMG LABTECH Inc., Durham, NC) or EnVision™ (PerkinElmer, Fremont CA) microplate reader.

Certain compounds of the present invention and their corresponding activity values are shown in TABLE B.

**TABLE B**

| **Compound No.** | **EC₅₀ S1P1 (HTRF)** |
|---|---|
| 19" | 18.79nM |
| 32 | 42.95nM |
| 42 | 93.97nM |
| 47 | 82.99nM |
| 51 | 0.23uM |
| 52 | 75.86nM |
| 62 | 0.21uM |

| | |
|---|---|
| ^{**}1^{st} enantiomer as described in Example 1.20. | |

Certain other compounds of the invention had activity values ranging from about 20 µM to about 1 nM in this assay.

### Example 3

### Cellular/Functional Ca²⁺ Assay for Agonist Activity on S1P3 Receptor.

A compound of the invention can be shown to have no or substantially no agonist activity on S1P3 receptor in assay using a human neuroblastoma cell line which endogenously expresses S1P3 (predominantly), S1P2 and S1P5 receptors, but not S1P1 or S1P4 receptors, based on mRNA analysis (Villullas et al, J Neurosci Res, 73:215-226, 2003). Of these, S1P3 and S1P2 receptors respond to agonist, such as S1P, with an intracellular calcium increase. No or substantially no increase of intracellular calcium in response to a test compound is indicative of the test compound exhibiting no or substantially no agonist activity on S1P3 receptor. Such an assay can be performed commercially, e.g. by Caliper LifeSciences (Hopkinton, MA).

**Assay:** The human neuroblastoma cells were washed and resuspended in physiological buffer. The cells were then loaded with dye that measures intracellular calcium. S1P was used as a reference agonist. Compound 52 was included in the assay as a test compound. After addition of S1P or Compound 52, fluorescence was measured at 485 nm excitation / 525 nm emission every 2 s for at least 60 s. Calcium ionophore A23187 was then added as an internal positive control. Results are presented in Figure 1. It is apparent from inspection of Figure 1 that Compound 52 exhibited no or substantially no agonist activity on S1P3 receptor.

### Example 4

### Effect of compounds in Peripheral Lymphocyte Lowering (PLL) Assay

A compound of the invention can be shown to induce peripheral lymphocyte lowering (PLL).

### A. Mouse PLL Assay

**Animals:** Male BALB/c mice (6 to 7 weeks of age at start of study) (Charles River Laboratories, Wilmington, MA) were housed four per cage and maintained in a humidity- (40-60%) and temperature- (68-72°F) controlled facility on a 12 h:12 h light/dark cycle (lights on at 6:30 am) with free access to food (Harlan Teklad, Orange, CA, Rodent Diet 8604) and water. Mice were allowed one week of habituation to the animal facility before testing.

**PLL Assay:** Mice were administered via i.v. one of the following: saline, vehicle (100% PEG400) or 1 mg/kg Compound 52 in vehicle, in a total volume of 5ml/kg. Peripheral blood samples were collected at 5 h post dose. The mice were anesthetized with isoflurane and bled retro-orbitally (300 µL) into 1.5 ml EDTA tubes. A complete cell count (CBC) (including lymphocyte count) was obtained using CELL-DYN^{®} 3700 (Abbott Laboratories, Abbott Park, IL). Results are presented in Figure 2, in which peripheral blood lymphocyte (PBL) count is shown for the 5 hour group. Reduction of the peripheral blood lymphocyte count by the test compound in comparison with vehicle is indicative of the test compound exhibiting activity for inducing peripheral lymphocyte lowering. It is apparent from inspection of Figure 2 that Compound 52 exhibited activity for inducing peripheral lymphocyte lowering (lymphopenia) in the mouse (*P<0.05).

### B. Rat PLL Assay

**Animals:** Male Sprague-Dawley rats (7 weeks of age at start of study) (Charles River Laboratories) are housed two per cage and maintained in a humidity- (40-60%) and temperature-(68-72°F) controlled facility on a 12 h:12 h light/dark cycle (lights on at 6:30 am) with free access to food (Harlan Teklad, Orange, CA, Rodent Diet 8604) and water. Rats are allowed one week of habituation to the animal facility before testing.

**PLL Assay:** Rats are orally administered vehicle or test compound (e.g., 1 mg/kg, 3 mg/kg, 10 mg/kg or 30 mg/kg) in vehicle, in a total volume of 5ml/kg. Peripheral blood samples are collected at 1, 3, 5, 8, 16 and 24 h post dose. At each time point, the rats are anesthetized with isoflurane and bled retro-orbitally (200 µL) into 1.5 ml EDTA tubes. A complete cell count (CBC) (including lymphocyte count) is obtained using CELL-DYN^{®} 3700 (Abbott Laboratories, Abbott Park, IL). Reduction of the peripheral blood lymphocyte count by the test compound in comparison with vehicle is indicative of the test compound exhibiting activity for inducing peripheral lymphocyte lowering.

### Example 5

### Effect of compounds on experimental autoimmune encephalomyelitis (EAE)

A compound of the invention can be shown to have therapeutic efficacy in multiple sclerosis by showing it to have therapeutic efficacy in experimental autoimmune encephalomyelitis (EAE), an animal model for multiple sclerosis. In certain exemplary well-established models, EAE is induced in rodents by injection of myelin oligodendrocyte glycoprotein (MOG) peptide, by injection of myelin basic protein (MBP) or by injection of proteolipid protein (PLP) peptide.

### A. MOG-induced EAE in mice

**Animals:** Female C57BL/6 mice (8 to 10 weeks of age at start of study) (Jackson Laboratory, Bar Harbor, ME) were housed four per cage and maintained in a humidity- (40-60%) and temperature- (68-72°F) controlled facility on a 12 h:12 h light/dark cycle (lights on at 6:30 am) with free access to food (Harlan Teklad, Orange, CA, Rodent Diet 8604) and water. Mice were allowed one week of habituation to the animal facility before testing.

**Induction of EAE:** Mice were immunized subcutaneously, 50 µL per hind flank, with a total of 100 µg MOG₃₅₋₅₅ peptide emulsified 1:1 with Complete Freund's adjuvant containing 4 mg/ml heat-killed Mycobacterium tuberculosis. Mice also received 200 ng pertussis toxin intraperitoneally on the day of immunization and 48 h later.

**Clinical scoring:** Severity of disease symptoms was scored as follows (in increasing order of severity): 0 = normal; 1 = limp tail OR hind limb weakness; 2 = limp tail AND limb weakness / weakness of 2 or more limbs; 3 = severe limb weakness or single limb paralysis; 4 = paralysis of 2 or more limbs; 5 = death.

**Drug treatment:** Mice were dosed orally, with vehicle or test compound, once a day from day 3 until day 21. Dosing volume is 5ml/kg. Test compound was dosed at 30 mg/kg. Mice were weighed daily. Mice were monitored daily from day 7 onward for disease symptoms. After the last dose on day 21, disease progression was monitored daily for 2 more weeks. Reduction of the severity of disease symptoms by the test compound in comparison with vehicle was indicative of the test compound exhibiting therapeutic efficacy in EAE. Results are presented in Figure 5. It is apparent from inspection of Figure 5 that the 2^{nd} enantiomer of Compound 7 (isolated after resolution of compound 7 by HPLC, with a retention time of 24 min per the conditions reported in Example 1.22) exhibited activity in the mouse EAE assay.

### B. PLP-induced EAE in mice

**Animals:** Female SJL/J mice (8 to 10 weeks of age at start of study) (Jackson Laboratory, Bar Harbor, ME) are housed four per cage and maintained in a humidity- (40-60%) and temperature- (68-72°F) controlled facility on a 12 h:12 h light/dark cycle (lights on at 6:30 am) with free access to food (Harlan Teklad, Orange, CA, Rodent Diet 8604) and water. Mice are allowed one week of habituation to the animal facility before testing.

**Induction of EAE:** Mice are immunized subcutaneously with 100 µg PLP₁₃₉₋₁₅₁ peptide emulsified 1:1 with Complete Freund's adjuvant containing 4 mg/ml heat-killed *Mycobacterium tuberculosis.* Mice also receive 200 ng pertussis toxin intravenously on the day of immunization.

**Clinical scoring:** Severity of disease symptoms is scored as follows (in increasing order of severity): 0 = normal; 1 = limp tail OR hind limb weakness; 2 = limp tail AND limb weakness / weakness of 2 or more limbs; 3 = severe limb weakness or single limb paralysis; 4 = paralysis of 2 or more limbs; 5 = death.

**Drug treatment:** Mice are dosed orally, with vehicle or test compound, once a day from day 3 until day 21. Dosing volume is 5ml/kg. Test compound is dosed at, e.g., 1 mg/kg, 3 mg/kg, 10 mg/kg or 30 mg/kg. Mice are weighed daily. Mice are monitored daily from day 7 onward for disease symptoms. After the last dose on day 21, disease progression is monitored daily for 2 more weeks.

### C. MBP-induced EAE in rats

**Animals:** Male Lewis rats (325-375 g at start of study) (Harlan, San Diego, CA) are housed two per cage and maintained in a humidity- (30-70%) and temperature- (20-22°C) controlled facility on a 12 h:12 h light/dark cycle (lights on at 6:30 A.M.) with free access to food (Harlan-Teklad Western Res., Orange, CA, Rodent Diet 8604) and water. Rats are allowed one week of habituation to the animal facility before testing. During the study, rats are weighed daily prior to clinical scoring at 1 lam.

**Induction of EAE:** Myelin basic protein (MBP; guinea pig) is dissolved in sterile saline at a concentration of 1mg/ml, and then emulsified 1:1 with Complete Freund's adjuvant (1mg/ml). 50µL of this emulsion is administered by intraplantar (ipl) injection into both hind paws of each rat, for a total injected volume of 100 µL per rat and a total dose of 50 µg of MBP per rat.

**Clinical scoring:** Severity of disease symptoms is scored daily after body weighing and before drug dosing. Severity of disease symptoms is scored as follows (in increasing order of severity): 0 = normal; 1 = tail OR limb weakness; 2 = tail AND limb weakness; 3 = severe hind limb weakness or single limb paralysis; 4 = loss of tail tone and paralysis of 2 or more limbs; 5 = death.

**Drug treatment:** Rats are dosed orally, with vehicle or test compound, 1h prior to MBP injection on day 0 and daily thereafter, after clinical scoring, for the duration of the study. Dosing volume is 5mL/kg. Test compound is dosed at, e.g., 1 mg/kg, 3 mg/kg, 10 mg/kg or 30 mg/kg. Reduction of the severity of disease symptoms by the test compound in comparison with vehicle is indicative of the test compound exhibiting therapeutic efficacy in EAE.

### Example 6

### Effect of compounds on Type I diabetes

A compound of the invention can be shown to have therapeutic efficacy in type I diabetes using an animal model for type I diabetes, such as cyclophosphamide induced type I diabetes in mice.

**Animals:** Baseline blood glucose measurements were taken from 9-10 week old female NOD/Ltj mice (Jackson Laboratory, Bar Harbor, ME) to ensure that they were normoglycemic (blood glucose is 80-120mg/dL) prior to initiation of the experiment. Blood glucose was measured from tail bleeds using a one touch ultra kit and test strips (LifeScan, Milpitas, CA).

**Cyclophosphamide induction of Type I diabetes:** On day 0 and day 14, normoglycemic NOD mice were injected intraperitoneally with 4mg cyclophosphamide monohydrate (200mg/kg) dissolved in 0.9% saline. If mice were diabetic (blood glucose is >250mg/dL) they were not given a booster dose of cyclophosphamide on day 14.

**Drug Treatment:** Mice were dosed orally, with vehicle or test compound, once a day from day 0 until day 25. Compounds were suspended in 0.5% methyl cellulose vehicle using a sonicator to ensure uniform suspension. Mice were weighed twice weekly and were dosed according to weight. Dosing volume was 5 mL/kg. Test compound was dosed at 30 mg/kg. Blood glucose was measured twice weekly. After dosing was completed at day 25, the mice continued to be monitored and blood glucose measurements were taken once a week for 3 weeks. Promotion of normoglycemia by the test compound in comparison with vehicle was indicative of the test compound exhibiting therapeutic efficacy in type I diabetes. Results are presented in Figure 6. It is apparent from inspection of Figure 6 that the 2^{nd} enantiomer of Compound 7 (isolated after resolution of compound 7 by HPLC, with a retention time of 24 min per the conditions reported in Example 1.22) exhibited activity in the NOD mouse type I diabetes assay.

### Example 7

### Allograft survival.

A compound of the invention can be shown to have therapeutic efficacy in prolonging allograft survival by showing it to have therapeutic efficacy in prolonging, e.g., survival of skin allograft in an animal model.

**Animals:** Female BALBc/J mice (6 to 7 weeks of age at start of study) (Jackson Laboratory, Bar Harbor, ME) were housed four per cage and maintained in a humidity- (40-60%) and temperature- (68-72°F) controlled facility on a 12 h:12 h light/dark cycle (lights on at 6:30 am) with free access to food (Harlan Teklad, Orange, CA, Rodent Diet 8604) and water. Female C57BL/6 mice (8 to 10 weeks of age at start of study) (Jackson Laboratory, Bar Harbor, ME) were similarly housed and maintained. Mice were allowed one week of habituation to the animal facility before testing.

**Skin allograft:** Balbc/J and C57BL/6 mice were used as donors and recipients, respectively, in a model of skin allograft transplantation. Donor Balbc/J mice were anesthetized, and 0.5 cm - diameter full thickness areas of abdominal skin were surgically removed. Skin grafts harvested from the Balbc/J mice were sutured onto the dorsum of anesthetized recipient C57BL6 mice. Sutured allografts were covered with Vaseline gauze and Bolster dressing for 7 days. The allografted mice were divided into eight groups of 8 mice each.

**Clinical scoring:** Skin allografts were inspected and digital images recorded daily until rejection, which was defined as the first day on which more than 80% of the graft is necrotic. Histological analysis of the rejected graft was carried out on hematoxylin and eosin (H&E)-stained sections. In an optional related study, on posttransplantation day 5 isolated lymphocytes from peripheral lymph nodes and spleen were counted and characterized for activation markers (e.g., T-cell activation markers) by flow cytometry. Also on day 5, grafts were removed from transplanted recipients, cut into small fragments, digested with collagenase and sedimented over Ficoll-Paque (pharmacia Biotech, Uppsala, Sweden) to isolate graft-infiltrating lymphocytes, which were counted and characterized for activation markers (e.g., T-cell activation markers) by flow cytometry. Histopathological analysis of the graft on day 5 was carried out on hematoxylin and eosin (H&E)-stained sections.

**Drug treatment:** Mice were dosed orally, with vehicle or test compound, once a day from the day of transplantation until the end of the study, e.g. until day 14, 21 or 28. Dosing volume was 5mL/kg. Test compound was dosed at, e.g., 1 mg/kg, 3 mg/kg, 10 mg/kg or 30 mg/kg. Delay of time of rejection of the skin allograft by the test compound in comparison with vehicle was indicative of the test compound exhibiting therapeutic efficacy in prolonging skin allograft survival. The 2^{nd} enantiomer of Compound 7 (isolated after resolution of compound 7 by HPLC, with a retention time of 24 min per the conditions reported in Example 1.22) was dosed at 3, 10 and 30 mg/kg. Results are presented in Figure 3. It is apparent from inspection of Figure 3 that the 2^{nd} enantiomer of Compound 7 exhibited activity in the mouse skin allograft assay.

### Example 8

### Effect of compounds on colitis

A compound of the invention can be shown to have therapeutic efficacy in colitis using an animal model for colitis. Suitable animals models are known in the art (Boismenu et al, J Leukoc Biol, 67:267-278, 2000). A first exemplary animal model for colitis is trinitrobenzene sulfonic acid (TNBS)-induced colitis, which presents clinical and histopathological findings that resemble those in Crohn's disease (Neurath et al, J Exp Med, 182:1281-1290, 1995; Boismenu et al, J Leukoc Biol, 67:267-278, 2000). A second exemplary animal model for colitis is dextran sulfate sodium (DSS)-induced colitis, which presents clinical and histopathological findings that resemble those in ulcerative colitis (Okayasu et al, Gastroenterology, 98:694-702, 1990; Boismenu et al, J Leukoc Biol, 67:267-278, 2000). Compounds can be commercially tested for efficacy in at least DSS-induced colitis and TNBS-induced colitis, e.g. by The Jackson Laboratory (Bar Harbor, ME).

### A. Mouse model for colitis

**Animals:** Male BALB/c mice (6 weeks of age at start of study) (Jackson Laboratory, Bar Harbor, ME) are housed four per cage and maintained in a humidity- (40-60%) and temperature- (68-72°F) controlled facility on a 12 h:12 h light/dark cycle (lights on at 6:30am) with free access to food (Harlan Teklad, Orange CA, Rodent Diet 8604) and water. Mice are allowed one week of habituation to the animal facility before testing.

**TNBS induction of colitis:** Mice are weighed for baseline body weights and fasted later that day beginning at 6:15pm just prior to lights-out (day 0). Body weights are taken again the following morning (day 1) at approximately 7:30am. Mice are anesthetized with isoflurane prior to induction of colitis. Colitis is induced in the mice by intracolonic injection of about 150 mg/kg TNBS in 50% ethanol (in a volume of 150 µL) using an intubation needle (22g, 1.5in) inserted completely into the anus with the mouse held by the tail in a vertical position. The mouse is held vertically for a 30 additional sec to allow thorough absorption and minimize leakage, after which the mouse is returned to its cage. Mice are then fed, following the preceding approximately 14 hr of fasting. Each morning thereafter, the mice are weighed. In control experiments, mice receive 50% ethanol alone using the same protocol.

**Drug treatment:** Drug treatment begins on day 2. Mice are dosed orally, with vehicle or test compound, once a day from day 2 until the conclusion of the experiment on, e.g., day 7, 14 or 21. Dosing volume is 5 mL/kg. Test compound is dosed at, e.g., 1 mg/kg, 3 mg/kg, 10 mg/kg or 30 mg/kg.

**Clinical scoring:** Upon conclusion of the experiment, colons are extracted and measured. Mice are euthanized with CO₂ and colon is removed from anus to cecum. Excised colon is measured for entire length, length from anus to end ofinflamed area, and length of inflamed (affected) area. After measurements, colon is cleared of excrement by flushing with saline and then cut open to clear more thoroughly. Colon is then weighed and preserved in neutral buffered formalin (NBF; 10% formalin, pH 6.7-7.0). The colon tissue is embedded in paraffin and processed for hematoxylin and eosin (H & E)-stained sections. Severity of disease symptoms is scored histologically from the stained sections as follows: 0 = no evidence of inflammation; 1 = low level ofleukocyte infiltration with infiltration seen in <10% of high-power fields AND no structural changes; 2 = moderate leukocyte infiltration with infiltration seen in 10% to 25% of high-power fields AND crypt elongation AND bowel wall thickening that does not extend beyond the mucosal layer AND no ulcerations; 3 = high level of leukocyte infiltration seen in 25% to 50% of high-power fields AND crypt elongation AND infiltration beyond the mucosal layer AND thickening of the bowel wall AND superficial ulcerations; 4 = marked degree of transmural leukocyte infiltration seen in >50% of high-power fields AND elongated and distorted crypts AND bowel-wall thickening AND extensive ulcerations. Reduction of the severity of the disease symptoms by the test compound in comparison with vehicle is indicative of the test compound exhibiting therapeutic efficacy in colitis.

### B. Rat model for colitis

**Animals:** Male Wistar rats (175-200g at start of study) (Charles River Laboratories, Wilmington, MA) are housed two per cage and maintained in a humidity- (40-60%) and temperature- (68-72°F) controlled facility on a 12 h:12 h light/dark cycle (lights on at 6:30am) with free access to food (Harlan Teklad, Orange CA, Rodent Diet 8604) and water. Rats are allowed one week of habituation to the animal facility before testing.

**TNBS induction of colitis:** Rats are weighed for baseline body weights and fasted later that day beginning at 6:15pm just prior to lights-out (day 0). Body weights are taken again the following morning (day 1) at approximately 7:30am. Rats are anesthetized with isoflurane prior to induction of colitis. Colitis is induced in the rats by intracolonic injection of about 60 mg/kg TNBS in 50% ethanol (in a volume of 500 µL) using a fabricated intubation needle (7.5Fr umbilical catheter and 14g hub) inserted 8cm into the anus with the rat held by the tail in a vertical position. The rat is held vertically for 30 additional sec to allow thorough absorption and minimize leakage, after which the rat is returned to its cage. Rats are then fed, following the preceding approximately 14 hr of fasting. Each morning thereafter, the rats are weighed. In control experiments, rats receive 50% ethanol alone using the same protocol.

**Drug treatment:** Drug treatment begins on day 2. Rats are dosed orally, with vehicle or test compound, once a day from day 2 until the conclusion of the experiment on, e.g., day 7, 14 or 21. Dosing volume is 5 ml/kg. Test compound is dosed at, e.g., 1 mg/kg, 3 mg/kg, 10 mg/kg or 30 mg/kg.

**Clinical scoring:** Upon conclusion of the experiment, colons are extracted and measured. Rats are euthanized with CO₂ and colon is removed from anus to cecum. Excised colon is measured for entire length, length from anus to end of inflamed area, and length of inflamed (affected) area. After measurements, colon is cleared of excrement by flushing with saline and then cut open to clear more thoroughly. Colon is then weighed and preserved in neutral buffered formalin (NBF; 10% formalin, pH 6.7-7.0). The colon tissue is embedded in paraffin and processed for hematoxylin and eosin (H & E)-stained sections. Severity of disease symptoms is scored histologically from the stained sections as follows: 0 = no evidence of inflammation; 1 = low level of leukocyte infiltration with infiltration seen in <10% of high-power fields AND no structural changes; 2 = moderate leukocyte infiltration with infiltration seen in 10% to 25% of high-power fields AND crypt elongation AND bowel wall thickening that does not extend beyond the mucosal layer AND no ulcerations; 3 = high level of leukocyte infiltration seen in 25% to 50% of high-power fields AND crypt elongation AND infiltration beyond the mucosal layer AND thickening of the bowel wall AND superficial ulcerations; 4 = marked degree of transmural leukocyte infiltration seen in >50% of high-power fields AND elongated and distorted crypts AND bowel-wall thickening AND extensive ulcerations. Reduction of the severity of the disease symptoms by the test compound in comparison with vehicle is indicative of the test compound exhibiting therapeutic efficacy in colitis.

### Example 9

### Effects of compounds on cardiac telemetry in the rat.

**Animals:** Male Sprague-Dawley rats (250-300g at time of surgery) are implanted by Charles River Laboratories (Wilmington, MA) with cardiac transmitting devices (Data Sciences PhysioTel C50-PXT) into the peritoneal space, with a pressure-sensing catheter inserted into the descending aorta. Rats are allowed at least one week to recover. Rats are housed in individual cages and maintained in a humidity- (30-70%) and temperature- (20-22°C) controlled facility on a 12 h:12 h light/dark cycle (lights on at 7:00am) with free access to food (Harlan-Teklad, Orange, CA, Rodent Diet 8604) and water. Rats are allowed one week of habituation to the animal facility before testing.

**Measurement of cardiovascular parameters:** The implanted transmitting devices transmit continuous measurement of blood pressure (systolic, diastolic, mean arterial, pulse), heart rate, body temperature, and motor activity in freely moving conscious animals. These data are transmitted via radiofrequency to a computer which binned the data into 1-minute averages using DataSciences ART software. Telemetry recording occurs over a 21-hour period, starting at noon and continuing until 9:00am the following day. A maximum of eight rats are tested at a time, and the same eight rats are utilized for all treatment groups in a within-subject design.

**Drug treatment:** Rats are injected orally with vehicle or compound at 1:00pm. A full study (vehicle+3 doses) required 4 separate testing sessions, which occurred on Mondays-Tuesdays and Thursdays-Fridays. During each testing session, the eight rats are divided into four treatment groups such that each group comprises N=2 for any given session. Rats are retested in subsequent test sessions in a crossover design such that by the end of the 4 sessions, all animals receive all treatments in a pseudo-random order, and each group comprises N=8.

**Exemplary bradycardia assay:** It is expressly contemplated that the rats can be used to show that a compound of the invention has no or substantially no activity for bradycardia. By way of illustration and not limitation, the rats are administered vehicle or test compound and heart rate is then measured over a 120 minute period. No or substantially no reduction of heart rate in response to the test compound in comparison with vehicle is indicative of the test compound exhibiting no or substantially no activity for bradycardia.

### Example 10: Effect of Compounds on Arthritis.

Female Lewis rats were used in this study. Acclimated animals were anesthetized with isoflurane and given the first collagen injection (day 0). On day 6, they were anesthetized again for the second collagen injection. Collagen was prepared by making a 4 mg/mL solution in 0.01 N acetic acid. Equal volumes of collagen and incomplete Freund's adjuvant were emulsified by hand mixing until a bead of this material held its form when placed in water. Each animal received 300 µL of the mixture each time, spread over 3 subcutaneous sites on the back.

Treatment (*p.o., q.d.*, 5 mL/kg dosing volume) began on day 0 and continued through day 16 with vehicle or compounds given at 24 h intervals. Rats were weighed on days 0, 3, 6 and 9 through 17 and caliper measurements of the ankles taken on days 9 through 17. The 2^{nd} enantiomer of Compound 7 (isolated after resolution of compound 7 by HPLC, with a retention time of 24 min per the conditions reported in Example 1.22) was dosed at 3, 10 and 30 mg/kg. Results are presented in Figure 4. It is apparent from inspection of Figure 4 that the 2^{nd} enantiomer of Compound 7 exhibited activity for reducing mean ankle diameter in the rat.

Those skilled in the art will recognize that various modifications, additions, substitutions and variations to the illustrative examples set forth herein can be made without departing from the spirit of the invention and are, therefore, considered within the scope of the invention. All documents referenced above, including, but not limited to, printed publications and provisional and regular patent applications, are incorporated herein by reference in their entirety.

## Claims

1. A compound selected from compounds of Formula (Ia) and pharmaceutically acceptable salts, solvates and hydrates thereof: 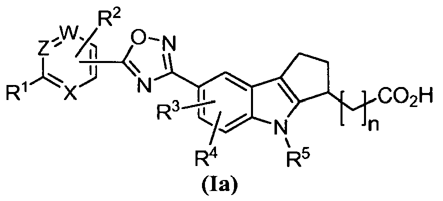 wherein:
n is 0 or 1;
W is N or CR⁶
Z is N or CR⁷;
X is N or CR⁸; provided that W, Z and X are not all N;
R¹, R², R⁶, R⁷ and R⁸ are each independently selected from H, C₁-C₆ acyl,
C₁-C₆ acyloxy, C₁-C₆ alkoxy, C₁-C₆ alkoxycarbonylamino, C₁-C₆ alkyl, C₂-C₆ alkynyl, C₁-C₆ alkylamino, C₂-C₈ dialkylamino, C₁-C₆ alkylcarboxamide, C₁-C₆ alkylsulfonamide, C₁-C₆ alkylsulfinyl, C₁-C₆ alkylsulfonyl, C₁-C₆ alkylthio, C₁-C₆ alkylureyl, amino, carbo-C₁-C₆-alkoxy, carboxamide, carboxy, cyano, C₃-C₇ cycloalkyl, C₃-C₇ cycloalkyloxy, C₃-C₇ cycloalkylthio, C₃-C₇ cycloalkylsulfinyl, C₃-C₇ cycloalkylsulfonyl, C₂-C₆ dialkylcarboxamide, C₁-C₆ haloalkoxy, C₁-C₆ haloalkyl, C₁-C₆ haloalkylsulfinyl, C₁-C₆ haloalkylsulfonyl, halogen, heteroaryl, heterocyclyl, hydroxyl, nitro and sulfonamide, wherein C₁-C₆ alkoxy, C₁-C₆ alkylthio, C₁-C₆ alkyl, C₂-C₆ alkynyl and heteroaryl are each optionally substituted with 1, 2 or 3 substituents selected independently from C₁-C₆ alkoxy, carbo-C₁-C₆-alkoxy, C₁-C₆ alkyl, cyano, C₃-C₇ cycloalkyl, halogen and phenyl;
R³ and R⁴ are each independently selected from H, C₁-C₂ alkyl, fluoro and chloro; and
R⁵ is H or C₁-C₆ alkyl.

2. A compound according to claim 1, wherein R³ and R⁴ are each independently selected from H, CH₃ and F.

3. A compound according to claim 1, wherein R³ and R⁴ are both H.

4. A compound according to any one of claims 1 to 3, wherein R⁵ is H or methyl.

5. A compound according to claim 1, wherein R³, R⁴ and R⁵ are each H.

6. A compound according to any one of claims 1 to 5, wherein n is 0.

7. A compound according to any one of claims 1 to 6, wherein n is 1.

8. A compound according to any one of claims 1 to 7, wherein R² is H.

9. A compound according to any one of claims 1 to 8, wherein R¹, R², R⁶, R⁷ and R⁸ are each independently selected from H, C₁-C₆ alkoxy, C₁-C₆ alkyl, C₂-C₆ alkynyl, C₁-C₆ alkylamino, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, carbo-C₁-C₆-alkoxy, C₁-C₆ alkyl, cyano, C₃-C₇ cycloalkyl, halogen and phenyl.

10. A compound according to any one of claims 1 to 8, wherein R¹, R², R⁶, R⁷ and R⁸ are each independently selected from H, sec-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl, 2-fluoropyridin-3-yl, 2-methoxypyrimidin-5-yl, propylamino, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl, 1-isobutyl-1*H*-pyrazol-4-yl, difluoromethoxy and hexyl.

11. A compound according to any one of claims 1 to 10, wherein R¹ is selected from H, C₁-C₆ alkoxy, C₁-C₆ alkyl, C₁-C₆ alkylamino, C₂-C₈ dialkylamino, C₁-C₆ alkylsulfonyl, carboxamide, cyano, C₂-C₆ alkynyl, C₃-C₇ cycloalkyl, C₃-C₇ cycloalkyloxy,
C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, carbo-C₁-C₆-alkoxy, C₁-C₆ alkyl, cyano, halogen, C₃-C₇ cycloalkyl and phenyl.

12. A compound according to any one of claims 1 to 10, wherein R¹ is selected from H, *sec*-butoxy, isopropoxy, methoxy, ethoxy, methyl, ethyl, propyl, butyl, diethylamino, methylsulfonyl, carboxamide, cyano, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, pyridin-3-yl, pyrrolidin-1-yl, morpholino, hydroxyl, cyclopropylmethoxy, benzyloxy, 6-methoxypyridin-3-yl and 1-(*tert*-butoxycarbonyl)-1*H*-pyrrol-2-yl.

13. A compound according to any one of claims 1 to 12, wherein R⁶ is selected from H, C₁-C₆ alkoxy, C₁-C₆ alkyl, C₁-C₆ alkylsulfonyl, carboxamide, cyano, C₂-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, carbo-C₁-C₆-alkoxy, C₁-C₆ alkyl, cyano, halogen, C₃-C₇ cycloalkyl, and phenyl.

14. A compound according to any one of claims 1 to 12, wherein R⁶ is selected from H, *sec*-butoxy, isopropoxy, methoxy, ethoxy, methyl, ethyl, propyl, butyl, diethylamino, methylsulfonyl, carboxamide, cyano, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, pyridin-3-yl, pyrrolidin-1-yl, morpholino, hydroxyl, cyclopropylmethoxy, benzyloxy, 6-methoxypyridin-3-yl and 1-(*tert*-butoxycarbonyl)-1H-pyrrol-2-yl.

15. A compound according to any one of claims 1 to 12, wherein R⁶ is H.

16. A compound according to any one of claims 1 to 16, wherein R⁷ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl.

17. A compound according to any one of claims 1 to 16, wherein R⁷ is selected from H, *sec*-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl and 2-fluoropyridin-3-yl.

18. A compound according to any one of claims 1 to 17, wherein R⁸ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, carbo-C₁-C₆-alkoxy, cyano, C₃-C₇ cycloalkyl, halogen and phenyl.

19. A compound according to any one of claims 1 to 17, wherein R⁸ is selected from H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl and 2-fluoropyridin-3-yl.

20. A compound according to any one of claims 1 to 8, wherein R¹, R², R⁶, R⁷ and R⁸ are each independently selected from H, C₁-C₆ acyl, C₁-C₆ acyloxy, C₁-C₆ alkoxy, C₁-C₆ alkoxycarbonylamino, C₁-C₆ alkyl, C₂-C₆ alkynyl, C₁-C₆ alkylamino, C₂-C₈ dialkylamino, C₁-C₆ alkylcarboxamide, C₁-C₆ alkylsulfonamide, C₁-C₆ alkylsulfinyl, C₁-C₆ alkylsulfonyl, C₁-C₆ alkylthio, C₁-C₆ alkylureyl, amino, carbo-C₁-C₆-alkoxy, carboxamide, carboxy, cyano, C₃-C₇ cycloalkyl, C₃-C₇ cycloalkyloxy, C₃-C₇ cycloalkylthio, C₃-C₇ cycloalkylsulfinyl, C₃-C₇ cycloalkylsulfonyl, C₂-C₆ dialkylcarboxamide, C₁-C₆ haloalkoxy, C₁-C₆ haloalkyl, C₁-C₆ haloalkylsulfinyl, C₁-C₆ haloalkylsulfonyl, halogen, heteroaryl, heterocyclyl, hydroxyl, nitro and sulfonamide, wherein C₁-C₆ alkoxy, C₁-C₆ alkylthio, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

21. A compound according to any one of claims 1 to 8, wherein R¹, R², R⁶, R⁷ and R⁸ are each independently selected from H, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

22. A compound according to any one of claims 1 to 8, wherein R¹, R², R⁶, R⁷ and R⁸ are each independently selected from H, *sec*-butoxy, isopropoxy, methoxy, ethoxy, propoxy, methyl, ethyl, propyl, butyl, *tert*-butyl, ethynyl, diethylamino, methylsulfonyl, carboxamide, cyano, cyclobutyl, cyclopropyl, cyclopentyloxy, trifluoromethoxy, 1,1,1,3,3,3-hexafluoropropan-2-yloxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl and 2-fluoropyridin-3-yl.

23. A compound according to any one of claims 1 to 8 and 20 to 22, wherein R' is selected from H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from C₁-C₆ alkoxy, carbo-C₁-C₆-alkoxy and C₃-C₇ cycloalkyl.

24. A compound according to any one of claims 1 to 8 and 20 to 23, wherein R⁶ is selected from H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from C₁-C₆ alkoxy, carbo-C₁-C₆-alkoxy, C₃-C₇ cycloalkyl and phenyl.

25. A compound according to any one of claims 1 to 8 and 20 to 24, wherein R⁷ is selected from H, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, cyano, carbo-C₁-C₆-alkoxy, C₃-C₇ cycloalkyl, halogen and phenyl.

26. A compound according to any one of claims 1 to 8 and 20 to 25, wherein R⁸ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

27. A compound according to any one of claims 1 to 26, wherein:
W is CR⁶;
Z is CR⁷; and
X is CR⁸.

28. A compound according to claim 1, selected from compounds of Formula **(Ig)** and pharmaceutically acceptable salts, solvates and hydrates thereof: 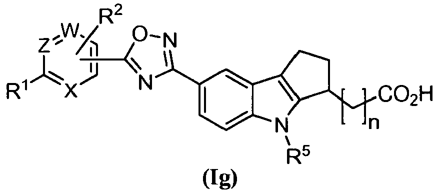 wherein:
n is 0 or 1;
W is N or CR⁶
Z is N or CR⁷;
X is N or CR⁸; provided that W, Z and X are not all N;
R¹ is selected from H, C₁-C₆ alkoxy, C₁-C₆ alkyl, C₁-C₆ alkylamino, C₂-C₈ dialkylamino, C₁-C₆ alkylsulfonyl, carboxamide, cyano, C₂-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, carbo-C₁-C₆-alkoxy, C₁-C₆ alkyl, cyano, halogen, C₃-C₇ cycloalkyl and phenyl;
R² is H;
R⁵ is H or C₁-C₆ alkyl;
R⁶ is selected from H, C₁-C₆ alkoxy, C₁-C₆ alkyl, C₁-C₆ alkylsulfonyl, carboxamide, cyano, C₂-C₆ alkynyl, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, carbo-C₁-C₆-alkoxy, C₁-C₆ alkyl, cyano, halogen, C₃-C₇ cycloalkyl, and phenyl;
R⁷ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl; and
R⁸ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, carbo-C₁-C₆-alkoxy, cyano, C₃-C₇ cycloalkyl, halogen and phenyl.

29. A compound according to claim 1, selected from compounds of Formula **(Ig)** and pharmaceutically acceptable salts, solvates and hydrates thereof: 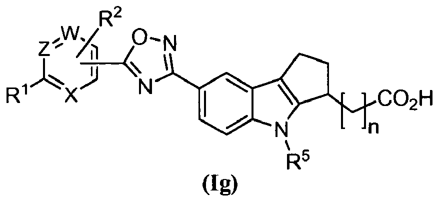 wherein:
n is 0 or 1;
W is N or CR⁶
Z is N or CR⁷;
X is N or CR⁸; provided that W, Z and X are not all N;
R¹ is selected from H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from C₁-C₆ alkoxy, carbo-C₁-C₆-alkoxy and C₃-C₇ cycloalkyl;
R² is H;
R⁵ is H or C₁-C₆ alkyl;
R⁶ is selected from H, C₁-C₆ alkoxy, C₁-C₆ alkyl, C₂-C₈ dialkylamino, C₁-C₆ alkylsulfonyl, carboxamide, cyano, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy and heteroaryl are optionally substituted with 1 substituent selected from C₁-C₆ alkoxy, carbo-C₁-C₆-alkoxy, C₃-C₇ cycloalkyl and phenyl;
R⁷ is selected from H, C₁-C₆ alkoxy, C₁-C₆ alkyl, C₂-C₆ alkynyl, C₂-C₈ dialkylamino, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, cyano, carbo-C₁-C₆-alkoxy, C₃-C₇ cycloalkyl, halogen and phenyl; and
R⁸ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1 substituent selected from C₁-C₆ alkoxy, carbo-C₁-C₆-alkoxy, cyano, C₃-C₇ cycloalkyl, halogen and phenyl.

30. A compound according to claim 1, selected from compounds of Formula **(IIa)** and pharmaceutically acceptable salts, solvates and hydrates thereof: 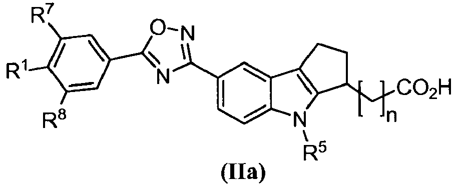 wherein:
n is 0 or 1;
R¹ is selected from H, C₁-C₆ alkoxy, cyano, C₃-C₇ cycloalkyloxy, C₁-C₆ haloalkoxy, halogen and hydroxyl, wherein C₁-C₆ alkoxy, is optionally substituted with 1, 2 or 3 substituents selected from halogen and C₃-C₇ cycloalkyl;
R⁵ is H or C₁-C₆ alkyl;
R⁷ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl; and
R⁸ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₃-C₇ cycloalkyloxy, C₁-C₆ haloalkoxy, C₁-C₆ haloalkyl, halogen, heteroaryl, heterocyclyl and hydroxyl, wherein C₁-C₆ alkoxy, C₁-C₆ alkyl, C₂-C₆ alkynyl and heteroaryl are optionally substituted with 1, 2 or 3 substituents selected from C₁-C₆ alkoxy, C₁-C₆ alkyl, cyano, carbo-C₁-C₆-alkoxy, C₃-C₇ cycloalkyl, halogen and phenyl.

31. A compound according to claim 1, selected from compounds of Formula **(IIa)** and pharmaceutically acceptable salts, solvates and hydrates thereof: 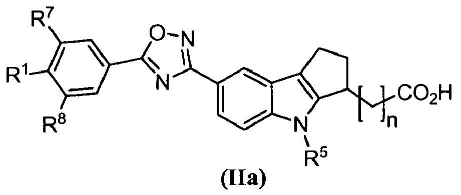 wherein:
n is 0 or 1;
R¹ is selected from H, isopropoxy, methoxy, ethoxy, cyano, cyclopentyloxy, trifluoromethoxy, 1,1,1,3,3,3-hexafluoropropan-2-yloxy, fluoro, hydroxyl, cyclopropylmethoxy, and difluoromethoxy;
R⁵ is H or methyl;
R⁷ is selected from H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-4-yl; and
R⁸ is selected from H, isopropoxy, methoxy, ethoxy, propoxy, methyl, ethyl, propyl, tert-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-4-yl.

32. A compound according to claim 1, selected from compounds of Formula **(IIa)** and pharmaceutically acceptable salts, solvates and hydrates thereof: 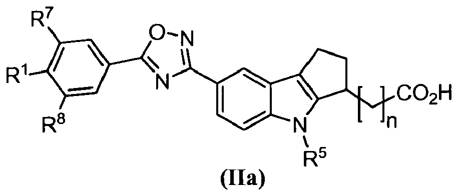 wherein:
n is 0 or 1;
R¹ is selected from H, C₁-C₆ alkoxy, cyano, C₃-C₇ cycloalkyloxy, C₁-C₆ haloalkoxy and hydroxyl, wherein C₁-C₆ alkoxy, is optionally substituted with 1 substituent selected from C₁-C₆ alkoxy;
R⁵ is H or C₁-C₆ alkyl;
R⁷ is selected from H, C₁-C₆ alkoxy, C₁-C₆ alkyl, C₁-C₆ alkylsulfonyl, cyano, C₃-C₇ cycloalkyloxy, C₁-C₆ haloalkoxy, C₁-C₆ haloalkyl, halogen, heteroaryl, heterocyclyl, wherein C₁-C₆ alkoxy and heteroaryl are optionally substituted with 1 substituent selected from C₁-C₆ alkoxy, carbo-C₁-C₆-alkoxy and phenyl; and
R⁸ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₁-C₆ haloalkoxy, C₁-C₆ haloalkyl, halogen, heteroaryl and hydroxyl, wherein C₁-C₆ alkoxy, C₁-C₆ alkyl, C₂-C₆ alkynyl and heteroaryl are optionally substituted with 1 substituent selected from cyano, C₃-C₇ cycloalkyl, halogen and phenyl.

33. A compound according to claim 1, selected from compounds of Formula **(IIa)** and pharmaceutically acceptable salts, solvates and hydrates thereof: 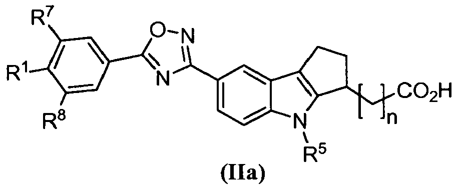 wherein:
n is 0 or 1;
R¹ is selected from H, isopropoxy, methoxy,ethoxy, cyano, cyclopentyloxy, trifluoromethoxy, 1,1,1,3,3,3-hexafluoropropan-2-yloxy, hydroxyl and cyclopropylmethoxy;
R⁵ is H or methyl;
R⁷ is selected from H, isopropoxy, methoxy,ethoxy, methyl, ethyl, propyl, methylsulfonyl, cyano, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyrimidin-5-yl, pyrrolidin-1-yl, morpholino, benzyloxy, 6-methoxypyridin-3-yl and 1-(*tert*-butoxycarbonyl)-1*H*-pyrrol-2-yl; and
R⁸ is selected from H, isopropoxy, methoxy, ethoxy, propoxy, methyl, *tert*-butyl, ethynyl, methylsulfonyl, carboxamide, cyano, cyclobutyl, cyclopropyl, trifluoromethoxy, trifluoromethyl, fluoro, chloro, bromo, pyridin-4-yl, hydroxyl, cyclopropylmethoxy, benzyloxy, 2-cyanoethyl, cyclopropylethynyl, 6-fluoropyridin-3-yl, 2-fluoropyridin-4-yl and 2-fluoropyridin-3-yl.

34. A compound according to claim 1, selected from compounds of Formula **(IIc)** and pharmaceutically acceptable salts, solvates and hydrates thereof: 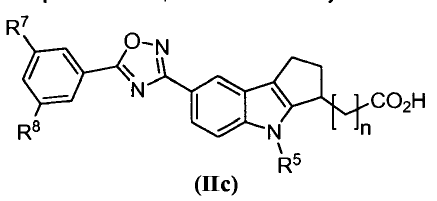 wherein:
n is 0 or 1;
R⁵ is H or methyl;
R⁷ is selected from H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-4-yl; and
R⁸ is selected from H, isopropoxy, methoxy, ethoxy, propoxy, methyl, ethyl, propyl, *tert*-butyl, ethynyl, methylsulfonyl, carboxamide, cyano, cyclobutyl, cyclopropyl, cyclopentyloxy, trifluoromethoxy, 2-fluoroethoxy, 2,2,2-trifluoroethoxy, trifluoromethyl, fluoro, chloro, bromo, 1,2,4-triazol-1-yl, 1,2,4-triazol-4-yl, pyridin-3-yl, pyridin-4-yl, pyrimidin-5-yl, pyrrolidin-1-yl, morpholino, hydroxyl, cyclopropylmethoxy, benzyloxy, 2-cyanoethyl, 6-methoxypyridin-3-yl, cyclopropylethynyl, 6-fluoropyridin-3-yl, 1-(*tert*-butoxycarbonyl)-1*H*-pyrrol-2-yl, 2-fluoropyridin-4-yl, 2-fluoropyridin-3-yl, 2-methoxypyrimidin-5-yl, 3-fluoropyridin-4-yl, 3,5-dimethylisoxazol-4-yl, 2,6-difluoropyridin-4-yl, 6-fluoro-5-methylpyridin-3-yl, 1-methyl-1*H*-pyrazol-4-yl, 2-methylpyridin-4-yl, 3-methylpyridin-4-yl, 1,3,5-trimethyl-1*H*-pyrazol-4-yl and 1-isobutyl-1*H*-pyrazol-4-yl.

35. A compound according to claim 1, selected from compounds of Formula **(IIc)** and pharmaceutically acceptable salts, solvates and hydrates thereof: 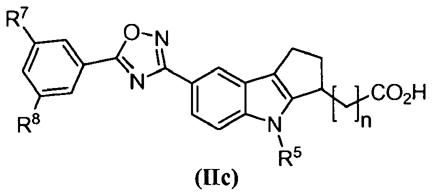 wherein:
n is 0 or 1;
R⁵ is H or methyl;
R⁷ is selected from H, C₁-C₆ alkoxy, C₁-C₆ alkyl, C₁-C₆ alkylsulfonyl, cyano, C₃-C₇ cycloalkyloxy, C₁-C₆ haloalkoxy, C₁-C₆ haloalkyl, halogen, heteroaryl, heterocyclyl, wherein C₁-C₆ alkoxy and heteroaryl are optionally substituted with 1 substituent selected from C₁-C₆ alkoxy, carbo-C₁-C₆-alkoxy and phenyl; and
R⁸ is selected from H, C₁-C₆ alkoxy, C₁-C₆ alkyl, C₂-C₆ alkynyl, C₁-C₆ alkylsulfonyl, carboxamide, cyano, C₃-C₇ cycloalkyl, C₁-C₆ haloalkoxy, C₁-C₆ haloalkyl, halogen, heteroaryl and hydroxyl, wherein C₁-C₆ alkoxy, C₁-C₆ alkyl, C₂-C₆ alkynyl and heteroaryl are optionally substituted with 1 substituent selected from cyano, C₃-C₇ cycloalkyl, halogen and phenyl.

36. A compound according to claim 1, selected from the following compounds and pharmaceutically acceptable salts, solvates and hydrates thereof:
2-(7-(5-(6-*sec*-butoxy-5-methylpyridin-3-yl)-1,2,4-oxadiazol-3-yl)-1,2,3,4-tetrahydrocyclopenta[b]indol-3-yl)acetic acid;
2-(7-(5-(pyridazin-3-yl)-1,2,4-oxadiazol-3-yl)-1,2,3,4-tetrahydrocyclopenta[b]indol-3-yl)acetic acid;
2-(7-(5-(3-cyano-5-isopropoxyphenyl)-1,2,4-oxadiazol-3-yl)-1,2,3,4-tetrahydrocyclopenta[b]indol-3-yl)acetic acid;
2-(7-(5-(3,5-difluorophenyl)-1,2,4-oxadiazol-3-yl)-1,2,3,4-tetrahydrocyclopenta[b]indol-3-yl)acetic acid;
2-(7-(5-(3-cyano-5-methoxyphenyl)-1,2,4-oxadiazol-3-yl)-1,2,3,4-tetrahydrocyclopenta[b]indol-3-yl)acetic acid;
2-(7-(5-(3-ethynyl-5-(trifluoromethoxy)phenyl)-1,2,4-oxadiazol-3-yl)-1,2,3,4-tetrahydrocyclopenta[b]indol-3-yl)acetic acid;
2-(7-(5-(3-cyano-5-(trifluoromethoxy)phenyl)-1,2,4-oxadiazol-3-yl)-1,2,3,4-tetrahydrocyclopenta[b]indol-3-yl)acetic acid;
2-(7-(5-(3-methoxy-5-(trifluoromethyl)phenyl)-1,2,4-oxadiazol-3-yl)-1,2,3,4-tetrahydrocyclopenta[b]indol-3-yl)acetic acid;
2-(7-(5-(3-cyclobutyl-5-(trifluoromethoxy)phenyl)-1,2,4-oxadiazol-3-yl)-1,2,3,4-tetrahydrocyclopenta[b]indol-3-yl)acetic acid;
2-(7-(5-(3-cyano-4-methoxyphenyl)-1,2,4-oxadiazol-3-yl)-1,2,3,4-tetrahydrocyclopenta[b]indol-3-yl)acetic acid;
2-(7-(5-(6-(trifluoromethyl)pyridin-3-yl)-1,2,4-oxadiazol-3-yl)-1,2,3,4-tetrahydrocyclopenta[b]indol-3-yl)acetic acid;
2-(7-(5-(3-fluoro-5-(trifluoromethyl)phenyl)-1,2,4-oxadiazol-3-yl)-1,2,3,4-tetrahydrocyclopenta[b]indol-3-yl)acetic acid;
2-(7-(5-(4-methoxy-3-methylphenyl)-1,2,4-oxadiazol-3-yl)-1,2,3,4-tetrahydrocyclopenta[b]indol-3-yl)acetic acid;
2-(7-(5-(3-bromo-5-(trifluoromethyl)phenyl)-1,2,4-oxadiazol-3-yl)-1,2,3,4-tetrahydrocyclopenta[b]indol-3-yl)acetic acid;
2-(7-(5-(3-cyano-4-isopropoxyphenyl)-1,2,4-oxadiazol-3-yl)-1,2,3,4-tetrahydrocyclopenta[b]indol-3-yl)acetic acid;
2-(7-(5-(4-(trifluoromethoxy)phenyl)-1,2,4-oxadiazol-3-yl)-1,2,3,4-tetrahydrocyclopenta[b]indol-3-yl)acetic acid;
2-(7-(5-(3-bromo-5-chlorophenyl)-1,2,4-oxadiazol-3-yl)-1,2,3,4-tetrahydrocyclopenta[b]indol-3-yl)acetic acid;
2-(7-(5-(3-(1H-1,2,4-triazol-1-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-cyano-4-(cyclopentyloxy)phenyl)-1,2,4-oxadiazol-3-yl)-1,2,3,4-tetrahydrocyclopenta[b]indol-3-yl)acetic acid;
2-(7-(5-(3-(pyrrolidin-1-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-cyano-5-ethoxyphenyl)-1,2,4-oxadiazol-3-yl)-1,2,3,4-tetrahydrocyclopenta[b]indol-3-yl)acetic acid;
2-(7-(5-(3-(benzyloxy)phenyl)-1,2,4-oxadiazol-3-yl)-1,2,3,4-tetrahydrocyclopenta[b]indol-3-yl)acetic acid;
2-(7-(5-(5-chloro-6-methoxypyridin-3-yl)-1,2,4-oxadiazol-3-yl)-1,2,3,4-tetrahydrocyclopenta[b]indol-3-yl)acetic acid;
2-(7-(5-(3-propoxy-5-(trifluoromethyl)phenyl)-1,2,4-oxadiazol-3-yl)-1,2,3,4-tetrahydrocyclopenta[b]indol-3-yl)acetic acid;
2-(7-(5-(3-morpholino-5-(trifluoromethoxy)phenyl)-1,2,4-oxadiazol-3-yl)-1,2,3,4-tetrahydrocyclopenta[b]indol-3-yl)acetic acid;
2-(7-(5-(6-carbamoylpyridin-3-yl)-1,2,4-oxadiazol-3-yl)-1,2,3,4-tetrahydrocyclopenta[b]indol-3-yl)acetic acid;
2-(7-(5-(3-cyano-4-(1,1,1,3,3,3-hexafluoropropan-2-yloxy)phenyl)-1,2,4-oxadiazol-3-yl)-1,2,3,4-tetrahydrocyclopenta[b]indol-3-yl)acetic acid;
2-(7-(5-(3-(cyclopropylmethoxy)-5-(trifluoromethyl)phenyl)-1,2,4-oxadiazol-3-yl)-1,2,3,4-tetrahydrocyclopenta[b]indol-3-yl)acetic acid;
2-(7-(5-(pyrazin-2-yl)-1,2,4-oxadiazol-3-yl)-1,2,3,4-tetrahydrocyclopenta[b]indol-3-yl)acetic acid;
2-(7-(5-(3-cyclopropyl-5-(trifluoromethoxy)phenyl)-1,2,4-oxadiazol-3-yl)-1,2,3,4-tetrahydrocyclopenta[b]indol-3-yl)acetic acid;
2-(7-(5-(3-ethyl-5-(trifluoromethoxy)phenyl)-1,2,4-oxadiazol-3-yl)-1,2,3,4-tetrahydrocyclopenta[b]indol-3-yl)acetic acid;
2-(7-(5-(3-cyano-5-(2-fluoroethoxy)phenyl)-1,2,4-oxadiazol-3-yl)-1,2,3,4-tetrahydrocyclopenta[b]indol-3-yl)acetic acid;
2-(7-(5-(3-isopropoxy-5-(trifluoromethoxy)phenyl)-1,2,4-oxadiazol-3-yl)-1,2,3,4-tetrahydrocyclopenta[b]indol-3-yl)acetic acid;
2-(7-(5-(2-chloro-6-methylpyridin-4-yl)-1,2,4-oxadiazol-3-yl)-1,2,3,4-tetrahydrocyclopenta[b]indol-3-yl)acetic acid;
2-(7-(5-(3,5-dichlorophenyl)-1,2,4-oxadiazol-3-yl)-1,2,3,4-tetrahydrocyclopenta[b]indol-3-yl)acetic acid;
2-(7-(5-(3-cyano-4-ethoxyphenyl)-1,2,4-oxadiazol-3-yl)-1,2,3,4-tetrahydrocyclopenta[b]indol-3-yl)acetic acid;
2-(7-(5-(3-chloro-5-cyanophenyl)-1,2,4-oxadiazol-3-yl)-1,2,3,4-tetrahydrocyclopenta[b]indol-3-yl)acetic acid;
2-(7-(5-(3,5-dimethoxyphenyl)-1,2,4-oxadiazol-3-yl)-1,2,3,4-tetrahydrocyclopenta[b]indol-3-yl)acetic acid;
2-(7-(5-(pyridin-3-yl)-1,2,4-oxadiazol-3-yl)-1,2,3,4-tetrahydrocyclopenta[b]indol-3-yl)acetic acid;
2-(7-(5-(3-propyl-5-(trifluoromethoxy)phenyl)-1,2,4-oxadiazol-3-yl)-1,2,3,4-tetrahydrocyclopenta[b]indol-3-yl)acetic acid;
2-(7-(5-(pyridin-4-yl)-1,2,4-oxadiazol-3-yl)-1,2,3,4-tetrahydrocyclopenta[b]indol-3-yl)acetic acid;
2-(7-(5-(4-methoxy-3-(trifluoromethyl)phenyl)-1,2,4-oxadiazol-3-yl)-1,2,3,4-tetrahydrocyclopenta[b]indol-3-yl)acetic acid;
2-(7-(5-(3-chloro-4-methoxyphenyl)-1,2,4-oxadiazol-3-yl)-1,2,3,4-tetrahydrocyclopenta[b]indol-3-yl)acetic acid;
2-(7-(5-(3-cyano-4-hydroxyphenyl)-1,2,4-oxadiazol-3-yl)-1,2,3,4-tetrahydrocyclopenta[b]indol-3-yl)acetic acid;
2-(7-(5-(3-chloro-5-(trifluoromethoxy)phenyl)-1,2,4-oxadiazol-3-yl)-1,2,3,4-tetrahydrocyclopenta[b]indol-3-yl)acetic acid;
2-(7-(5-(3-cyano-5-fluorophenyl)-1,2,4-oxadiazol-3-yl)-1,2,3,4-tetrahydrocyclopenta[b]indol-3-yl)acetic acid;
2-(7-(5-(6-(2,2,2-trifluoroethoxy)pyridin-3-yl)-1,2,4-oxadiazol-3-yl)-1,2,3,4-tetrahydrocyclopenta[b]indol-3-yl)acetic acid;
2-(7-(5-(6-isopropoxy-5-methylpyridin-3-yl)-1,2,4-oxadiazol-3-yl)-1,2,3,4-tetrahydrocyclopenta[b]indol-3-yl)acetic acid;
2-(7-(5-(3-(benzyloxy)-5-(trifluoromethyl)phenyl)-1,2,4-oxadiazol-3-yl)-1,2,3,4-tetrahydrocyclopenta[b]indol-3-yl)acetic acid;
2-(7-(5-(3-(trifluoromethyl)phenyl)-1,2,4-oxadiazol-3-yl)-1,2,3,4-tetrahydrocyclopenta[b]indol-3-yl)acetic acid;
2-(7-(5-(3-carbamoyl-5-(trifluoromethoxy)phenyl)-1,2,4-oxadiazol-3-yl)-1,2,3,4-tetrahydrocyclopenta[b]indol-3-yl)acetic acid;
2-(7-(5-(3,5-bis(trifluoromethyl)phenyl)-1,2,4-oxadiazol-3-yl)-1,2,3,4-tetrahydrocyclopenta[b]indol-3-yl)acetic acid;
2-(7-(5-(3-methyl-5-(trifluoromethoxy)phenyl)-1,2,4-oxadiazol-3-yl)-1,2,3,4-tetrahydrocyclopenta[b]indol-3-yl)acetic acid;
2-(7-(5-(5-butylpyridin-2-yl)-1,2,4-oxadiazol-3-yl)-1,2,3,4-tetrahydrocyclopenta[b]indol-3-yl)acetic acid;
2-(7-(5-(3-cyano-5-(2,2,2-trifluoroethoxy)phenyl)-1,2,4-oxadiazol-3-yl)-1,2,3,4-tetrahydrocyclopenta[b]indol-3-yl)acetic acid;
2-(7-(5-(pyrimidin-5-yl)-1,2,4-oxadiazol-3-yl)-1,2,3,4-tetrahydrocyclopenta[b]indol-3-yl)acetic acid;
2-(7-(5-(3-tert-butyl-5-cyanophenyl)-1,2,4-oxadiazol-3-yl)-1,2,3,4-tetrahydrocyclopenta[b]indol-3-yl)acetic acid;
2-(7-(5-(6-methoxy-5-methylpyridin-3-yl)-1,2,4-oxadiazol-3-yl)-1,2,3,4-tetrahydrocyclopenta[b]indol-3-yl)acetic acid;
2-(7-(5-(6-sec-butoxy-5-chloropyridin-3-yl)-1,2,4-oxadiazol-3-yl)-1,2,3,4-tetrahydrocyclopenta[b]indol-3-yl)acetic acid;
2-(7-(5-(3-(2-cyanoethyl)-5-(trifluoromethoxy)phenyl)-1,2,4-oxadiazol-3-yl)-1,2,3,4-tetrahydrocyclopenta[b]indol-3-yl)acetic acid;
2-(7-(5-(pyridin-2-yl)-1,2,4-oxadiazol-3-yl)-1,2,3,4-tetrahydrocyclopenta[b]indol-3-yl)acetic acid;
2-(7-(5-(6-ethoxy-5-methylpyridin-3-yl)-1,2,4-oxadiazol-3-yl)-1,2,3,4-tetrahydrocyclopenta[b]indol-3-yl)acetic acid;
2-(7-(5-(3-bromo-5-(trifluoromethoxy)phenyl)-1,2,4-oxadiazol-3-yl)-1,2,3,4-tetrahydrocyclopenta[b]indol-3-yl)acetic acid;
2-(7-(5-(3,5-dimethylphenyl)-1,2,4-oxadiazol-3-yl)-1,2,3,4-tetrahydrocyclopenta[b]indol-3-yl)acetic acid;
2-(7-(5-(3-cyano-5-(cyclopentyloxy)phenyl)-1,2,4-oxadiazol-3-yl)-1,2,3,4-tetrahydrocyclopenta[b]indol-3-yl)acetic acid;
2-(7-(5-(5-chloro-6-isopropoxypyridin-3-yl)-1,2,4-oxadiazol-3-yl)-1,2,3,4-tetrahydrocyclopenta[b]indol-3-yl)acetic acid;
2-(7-(5-(3-isopropoxy-5-(trifluoromethyl)phenyl)-1,2,4-oxadiazol-3-yl)-1,2,3,4-tetrahydrocyclopenta[b]indol-3-yl)acetic acid;
2-(7-(5-(3,5-diethoxyphenyl)-1,2,4-oxadiazol-3-yl)-1,2,3,4-tetrahydrocyclopenta[b]indol-3-yl)acetic acid;
2-(7-(5-(3-bromo-5-hydroxyphenyl)-1,2,4-oxadiazol-3-yl)-1,2,3,4-tetrahydrocyclopenta[b]indol-3-yl)acetic acid;
2-(7-(5-(5-bromopyridin-3-yl)-1,2,4-oxadiazol-3-yl)-1,2,3,4-tetrahydrocyclopenta[b]indol-3-yl)acetic acid;
2-(7-(5-(pyridazin-4-yl)-1,2,4-oxadiazol-3-yl)1,2,3,4-tetrahydrocyclopenta[b]indol-3-yl)acetic acid;
2-(7-(5-(3-(4H-1,2,4-triazol-4-yl)-5-(trifluoromethoxy)phenyl)-1,2,4-oxadiazol-3-yl)-1,2,3,4-tetrahydrocyclopenta[b]indol-3-yl)acetic acid;
7-(5-(3-cyano-5-(trifluoromethoxy)phenyl)-1,2,4-oxadiazol-3-yl)-1,2,3,4-tetrahydrocyclopenta[b]indole-3-carboxylic acid;
2-(7-(5-(3-(6-methoxypyridin-3-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(6-methoxypyridin-3-yl)-1,2,4-oxadiazol-3-yl)-1,2,3,4-tetrahydrocyclopenta[b]indol-3-yl)acetic acid;
2-(7-(5-(4-isopropoxy-3-methoxyphenyl)-1,2,4-oxadiazol-3-yl-1,2,3,4-tetrahydrocyclopenta[b]indol-3-yl)acetic acid;
2-(7-(5-(3-cyano-4-(cyclopropylmethoxy)phenyl)-1,2,4-oxadiazol-3-yl)-1,2,3,4-tetrahydrocyclopenta[b]indol-3-yl)acetic acid;
2-(7-(5-(4-hydroxy-3-methylphenyl)-1,2,4-oxadiazol-3-yl)-1,2,3,4-tetrahydrocyclopenta[b]indol-3-yl)acetic acid;
2-(7-(5-(3-(pyridin-3-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(6-(diethylamino)-4-(trifluoromethyl)pyridin-2-yl)-1,2,4-oxadiazol-3-yl)-1,2,3,4-tetrahydrocyclopenta[b]indol-3-yl)acetic acid;
2-(7-(5-(3-fluoro-4-isopropoxyphenyl)-1,2,4-oxadiazol-3-yl)-1,2,3,4-tetrahydrocyclopenta[b]indol-3-yl)acetic acid;
2-(7-(5-(5-isopropoxypyrazin-2-yl)-1,2,4-oxadiazol-3-yl)-1,2,3,4-tetrahydrocyclopenta[b]indol-3-yl)acetic acid;
2-(7-(5-(5-methoxypyridin-2-yl)-1,2,4-oxadiazol-3-yl)-1,2,3,4-tetrahydrocyclopenta[b]indol-3-yl)acetic acid;
2-(7-(5-(5-isopropoxypyridin-2-yl)-1,2,4-oxadiazol-3-yl)-1,2,3,4-tetrahydrocyclopenta[b]indol-3-yl)acetic acid;
2-(7-(5-(3,4-dimethoxyphenyl)-1,2,4-oxadiazol-3-yl)-1,2,3,4-tetrahydrocyclopenta[b]indol-3-yl)acetic acid;
2-(7-(5-(3-(cyclopropylethynyl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(6-fluoropyridin-3-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(6-isopropoxypyridazin-3-yl)-1,2,4-oxadiazol-3-yl)-1,2,3,4-tetrahydrocyclopenta[b]indol-3-yl)acetic acid;
2-(7-(5-(4-isopropoxy-3-(methylsulfonyl)phenyl)-1,2,4-oxadiazol-3-yl)-1,2,3,4-tetrahydrocyclopenta[b]indol-3-yl)acetic acid;
2-(7-(5-(3,5-bis(methylsulfonyl)phenyl)-1,2,4-oxadiazol-3-yl)-1,2,3,4-tetrahydrocyclopenta[b]indol-3-yl)acetic acid;
2-(7-(5-(3-(1-(*tert*-butoxycarbonyl)-1*H*-pyrrol-2-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-fluoro-4-methoxyphenyl)1,2,4-oxadiazol-3-yl)1,2,3,4-tetrahydrocyclopenta[b]indol-3-yl)acetic acid;
2-(7-(5-(6-isopropoxypyridin-3-yl)-1,2,4-oxadiazol-3-yl)-1,2,3,4-tetrahydrocyclopenta[b]indol-3-yl)acetic acid;
2-(7-(5-(3-chloro-4-hydroxyphenyl)-1,2,4-oxadiazol-3-yl)-1,2,3,4-tetrahydrocyclopenta[b]indol-3-yl)acetic acid;
2-(7-(5-(4-cyano-3-(trifluoromethoxy)phenyl)-1,2,4-oxadiazol-3-yl)-1,2,3,4-tetrahydrocyclopenta[b]indol-3-yl)acetic acid;
2-(7-(5-(3-(pyridin-4-yl)-5-(trifluoromethoxy)phenyl)1,2,4-oxadiazol-3-yl)-1,2,3,4-tetrahydrocyclopenta[b]indol-3-yl)acetic acid;
2-(7-(5-(4-isopropoxyphenyl)-1,2,4-oxadiazol-3-yl)-1,2,3,4-tetrahydrocyclopenta[b]indol-3-yl)acetic acid;
2-(7-(5-(3-(pyrimidin-5-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(2-fluoropyridin-3-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(2-fluoropyridin-4-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-isopropoxy-5-(trifluoromethyl)phenyl)-1,2,4-oxadiazol-3-y)-4-methyl-1,2,3,4-tetrahydrocyclopenta[b]indol-3-yl)acetic acid;
2-(7-(5-(4-ethoxy-3-fluorophenyl)-1,2,4-oxadiazol-3-yl)-1,2,3,4-tetrahydrocyclopenta[b]indol-3-yl)acetic acid;
2-(7-(5-(3-cyano-4-(trifluoromethoxy)phenyl)-1,2,4-oxadiazol-3-yl)-1,2,3,4-tetrahydrocyclopenta[b]indol-3-yl)acetic acid;
2-(7-(5-(3-(2-methoxypyrimidin-5-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(6-(propylamino)-4-(trifluoromethyl)pyridin-2-yl)-1,2,4-oxadiazol-3-yl)-1,2,3,4-tetrahydrocyclopenta[b]indol-3-yl)acetic acid;
2-(7-(5-(6-morpholino-4-(trifluoromethyl)pyridin-2-yl)-1,2,4-oxadiazol-3-yl)-1,2,3,4-tetrahydrocyclopenta[b]indol-3-yl)acetic acid;
2-(7-(5-(2-chloro-6-methoxypyridin-4-yl)-1,2,4-oxadiazol-3-yl)-1,2,3,4-tetrahydrocyclopenta[b]indol-3-yl)acetic acid;
2-(7-(5-(4-methoxy-3-(methylsulfonyl)phenyl)-1,2,4-oxadiazol-3-yl)-1,2,3,4-tetrahydrocyclopenta[b]indol-3-yl)acetic acid;
2-(7-(5-(5-methoxypyrazin-2-yl)-1,2,4-oxadiazol-3-yl)-1,2,3,4-tetrahydrocyclopenta[b]indol-3-yl)acetic acid;
2-(7-(5-(3-(3-fluoropyridin-4-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(3,5-dimethylisoxazol-4-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(2,6-difluoropyridin-4-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(6-fluoro-5-methylpyridin-3-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(1-methyl-1H-pyrazol-4-yl)-5-(trifluoromethoxy)phenyl)-1,2;4-oxadiazol-3-yl)-1,2,3,4-tetrahydrocyclopenta[b]indol-3-yl)acetic acid;
2-(7-(5-(3-(2-methylpyridin-4-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(3-methylpyridin-4-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(3-(trifluoromethoxy)-5-(3,5-trimethyl-1*H*-pyrazol-4-yl)phenyl)-1,2,4-oxadiazol-3-yl)-1,2,3,4-tetrahydrocyclopenta[b]indol-3-yl)acetic acid;
2-(7-(5-(3-(1-isobutyl-1*H*-pyrazol-4-yl)-5-(trifluoromethoxy)phenyl)-1,2,4-oxadiazol-3-yl)-1,2,3,4-tetrahydrocyclopenta[b]indol-3-yl)acetic acid;
2-(7-(5-(2-bromo-6-methylpyridin-4-yl)-1,2,4-oxadiazol-3-yl)-1,2,3,4-tetrahydrocyclopenta[b]indol-3-yl)acetic acid;
2-(7-(5-(4-(difluoromethoxy)-3-methoxyphenyl)-1,2,4-oxadiazol-3-yl)-1,2,3,4-tetrahydrocyclopenta[b]indol-3-yl)acetic acid;
2-(7-(5-(5-(1-methyl-1*H*-pyrazol-4-yl)pyridin-3-yl)-1,2,4-oxadiazol-3-yl)-1,2,3,4-tetrahydrocyclopenta[b]indol-3-yl)acetic acid; and
2-(7-(5-(5-hexylpyridin-3-yl)-1,2,4-oxadiazol-3-yl)-1,2,3,4-tetrahydrocyclopenta[b]indol-3-yl)acetic acid.

37. A compound according to any one of claims 1 to 36, wherein the stereochemistry for the ring carbon of said compound is *R*.

38. A compound according to any one of claims 1 to 36, wherein the stereochemistry for the ring carbon of said compound is *S*.

39. A compound according to claim 1, which is:
(*R*)-2-(7-(5-(3-cyano-5-(trifluoromethoxy)phenyl)-1,2,4-oxadiazol-3-yl)-1,2,3,4-tetrahydrocyclopenta[b]indol-3-yl)acetic acid;
or a pharmaceutically acceptable salt, solvate or hydrate thereof.

40. A compound according to claim 1, which is:
(*S*)-2-(7-(5-(3-cyano-5-(trifluoromethoxy)phenyl)-1,2,4-oxadiazol-3-yl)-1,2,3,4-tetrahydrocyclopenta[b]indol-3-yl)acetic acid;
or a pharmaceutically acceptable salt, solvate or hydrate thereof.

41. A compound according to claim 1, which is:
(*S*)-2-(7-(5-(3-cyano-4-isopropoxyphenyl)-1,2,4-oxadiazol-3-yl)-1,2,3,4-tetrahydrocyclopenta[b]indol-3-yl)acetic acid;
or a pharmaceutically acceptable salt, solvate or hydrate thereof.

42. A pharmaceutical composition comprising a compound according to any one of claims 1 to 41 and a pharmaceutically acceptable carrier.

43. A process for preparing a composition comprising admixing a compound according to any one of claims 1 to 41 and a pharmaceutically acceptable carrier.

44. A compound according to any one of claims 1 to 41 for use in a method of treatment of the human or animal body by therapy.

45. A compound according to any one of claims 1 to 41 for use in a method for the treatment of a S1P1 receptor associated disorder.

46. A compound according to any one of claims 1 to 41 for use in a method for the treatment of a S1 P1 receptor associated disorder wherein said S1 P1 receptor associated disorder is selected from: a disease or disorder mediated by lymphocytes, an autoimmune disease or disorder, an inflammatory disease or disorder, cancer, psoriasis, rheumatoid arthritis, Crohn's disease, transplant rejection, multiple sclerosis, systemic lupus erythematosus, ulcerative colitis, type I diabetes and acne.

47. A compound according to any one of claims 1 to 41 for use in a method for the treatment of a S1 P1 receptor associated disorder wherein said S1 P1 receptor associated disorder is a microbial infection or disease or a viral infection or disease.

48. Use of a compound according to any one of claims 1 to 41 in the manufacture of a medicament for the treatment of a S1P1 receptor associated disorder.

49. Use of a compound according to any one of claims 1 to 41 in the manufacture of a medicament for the treatment of a S1 P1 receptor associated disorder wherein said S1P1 receptor associated disorder is selected from: a disease or disorder mediated by lymphocytes, an autoimmune disease or disorder, an inflammatory disease or disorder, cancer, psoriasis, rheumatoid arthritis, Crohn's disease, transplant rejection, multiple sclerosis, systemic lupus erythematosus, ulcerative colitis, type I diabetes and acne.

50. Use of a compound according to any one of claims 1 to 41 in the manufacture of a medicament for the treatment of a S1P1 receptor associated disorder wherein said S1P1 receptor associated disorder is a microbial infection or disease or a viral infection or disease.

## Patentansprüche

1. Verbindung, ausgewählt aus Verbindungen der Formel (Ia) und pharmazeutisch annehmbaren Salzen, Solvaten und Hydraten davon: 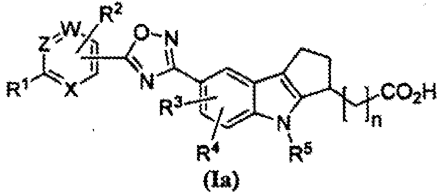 worin:
n = 0 oder 1 ist;
W N oder CR⁶ ist;
Z N oder CR⁷ ist;
X N oder CR⁸ ist; mit der Maßgabe, dass W, Z und X nicht alle N sind;
R¹, R², R⁶, R⁷ und R⁸ unabhängig voneinander aus H, C₁-C₆-Acyl, C₁-C₆-Acyloxy, C₁-C₆-Alkoxy, C₁-C₆-Alkoxycarbonylamino, C₁-C₆-Alkyl, C₂-C₆-Alkinyl, C₁-C₆-Alkylamino, C₂-C₈-Dialkylamino, C₁-C₆-Alkylcarboxamid, C₁-C₆-Alkylsulfonamid, C₁-C₆-Alkylsulfinyl, C₁-C₆-Alkylsulfonyl, C₁-C₆-Alkylthio, C₁-C₆-Alkylureyl, Amino, Carbo-C₁-C₆-alkoxy, Carboxamid, Carboxy, Cyano, C₃-C₇-Cy_{d}oalkyl, C₃-C₇-Cy_{d}oalkyloxy, C₃-C₇-Cycloalkylthio, C₃-C₇-Cycloalkylsulfinyl, C₃-C₇-Cycloalkylsulfonyl, C₂-C₆-Dialkylcarboxamid, C₁-C₆-Halogenalkoxy, C₁-C₆-Halogenalkyl, C₁-C₆-Halogenalkylsulfinyl, C₁-C₆-Halogenalkylsulfonyl, Halogen, Heteroaryl, Heterocyclyl, Hydroxyl, Nitro und Sulfonamid ausgewählt sind, wobei C₁-C₆-Alkoxy, C₁-C₆-Alkylthio, C₁-C₆-Alkyl, C₂-C₆-Alkinyl und Heteroaryl jeweils gegebenenfalls mit 1, 2 oder 3 Substituenten substituiert sind, die unabhängig voneinander aus C₁-C₆-Alkoxy, Carbo-C₁-C₆-alkoxy, C₁-C₆-Alkyl, Cyano, C₃-C₇-Cycloalkyl, Halogen und Phenyl ausgewählt sind;
R³ und R⁴ unabhängig voneinander aus H, C₁-C₂-Alkyl, Fluor und Chlor ausgewählt sind; und
R⁵ H oder C₁-C₆-Alkyl ist.

2. Verbindung nach Anspruch 1, worin R³ und R⁴ unabhängig voneinander aus H, CH₃ und F ausgewählt sind.

3. Verbindung nach Anspruch 1, worin R³ und R⁴ beide H sind.

4. Verbindung nach einem der Ansprüche 1 bis 3, worin R⁵ H oder Methyl ist.

5. Verbindung nach Anspruch 1, worin R³, R⁴ und R⁵ jeweils H sind.

6. Verbindung nach einem der Ansprüche 1 bis 5, worin n = 0 ist.

7. Verbindung nach einem der Ansprüche 1 bis 6, worin n = 1 ist.

8. Verbindung nach einem der Ansprüche 1 bis 7, worin R² H ist.

9. Verbindung nach einem der Ansprüche 1 bis 8, worin R¹, R², R⁶, R⁷ und R⁸ jeweils unabhängig voneinander aus H, C₁-C₆-Alkoxy, C₁-C₆-Alkyl, C₂-C₆-Alkinyl, C₁-C₆-Alkylamino, C₂-C₈-Dialkylamino, C₁-C₆-Alkylsulfonyl, Carboxamid, Cyano, C₃-C₇-Cyoalkyl, C₃-C₇-Cycoalkyloxy, C₁-C₆-Halogenalkoxy, C₁-C₆-Halogenalkyl, Halogen, Heteroaryl, Heterocyclyl und Hydroxyl, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, Carbo-C₁-C₆-alkoxy, C₁-C₆-Alkyl, Cyano, C₃-C₇-Cycloalkyl, Halogen und Phenyl ausgewählt sind.

10. Verbindung nach einem der Ansprüche 1 bis 8, worin R¹, R², R⁶, R⁷ und R⁸ unabhängig voneinander aus H, sec-Butoxy, Isopropoxy, Methoxy, Ethoxy, Propoxy, Methyl, Ethyl, Propyl, Butyl, tert-Butyl, Ethinyl, Diethylamino, Methylsulfonyl, Carboxamid, Cyano, Cyclobutyl, Cyclopropyl, Cyclopentyloxy, Trifluormethoxy, 1,1,1,3,3,3-Hexafluorpropan-2-yloxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyridin-4-yl, Pyrimidin-5-yl, Pyrrolidin-1-yl, Morpholino, Hydroxyl, Cyclopropylmethoxy, Benzyloxy, 2-Cyanoethyl, 6-Methoxypyridin-3-yl, Cyclopropylethinyl, 6-Fluorpyridin-3-yl, 1-(tert-Butoxycarbonyl)-1H-pyrrol-2-yl, 2-Fluorpyridin-4-yl, 2-Fluorpyridin-3-yl, 2-Methoxypyrimidin-5-yl, Propylamino, 3-Fluorpyridin-4-yl, 3,5-Dimethylisoxazol-4-yl, 2,6-Difluorpyridin-4-yl, 6-Fluor-5-methylpyridin-3-yl, 1-Methyl-1H-pyrazol-4-yl, 2-Methylpyridin-4-yl, 3-Methylpyridin-4-yl, 1,3,5-Trimethyl-1H-pyrazol-4-yl, 1-Isobutyl-1H-pyrazol-4-yl, Difluormethoxy und Hexyl ausgewählt sind.

11. Verbindung nach einem der Ansprüche 1 bis 10, worin R¹ aus H, C₁-C₆-Alkoxy, C₁-C₆-Alkyl, C₁-C₆-Alkylamino, C₂-C₈-Dialkylamino, Cₗ-C₆-Alkylsulfonyl, Carboxamid, Cyano, C₂-C₆-Alkinyl, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt ist, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, Carbo-C₁-C₆-alkoxy, C₁-C₆-Alkyl, Cyano, Halogen, C₃-C₇-Cycloalkyl und Phenyl ausgewählt sind.

12. Verbindung nach einem der Ansprüche 1 bis 10, worin R¹ aus H, sec-Butoxy, Isopropoxy, Methoxy, Ethoxy, Methyl, Ethyl, Propyl, Butyl, Diethylamino, Methylsulfonyl, Carboxamid, Cyano, Cyclopentyloxy, Trifluormethoxy, 1,1,1,3,3,3-Hexafluorpropan-2-yloxy, 2-Fluorethoxy, 2,2,2-Trifluorethoxy, Trifluormethyl, Fluor, Chlor, Brom, 1,2,4-Triazol-1-yl, Pyridin-3-yl, Pyrrolidin-1-yl, Morpholino, Hydroxyl, Cyclopropylmethoxy, Benzyloxy, 6-Methoxypyridin-3-yl und 1-(tert-Butoxycarbonyl)-1H-pyrrol-2-yl ausgewählt ist.

13. Verbindung nach einem der Ansprüche 1 bis 12, worin R⁶ aus H, C₁-C₆-Alkoxy, C₁-C₆-Alkyl, C₁-C₆-Alkylsulfonyl, Carboxamid, Cyano, C₂-C₆-Alkinyl, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt ist, wobei C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, Carbo-C₁-C₆-alkoxy, C₁-C₆-Alkyl, Cyano, Halogen, C₃-C₇-Cycloalkyl und Phenyl ausgewählt sind.

14. Verbindung nach einem der Ansprüche 1 bis 12, worin R⁶ aus H, sec-Butoxy, Isopropoxy, Methoxy, Ethoxy, Methyl, Ethyl, Propyl, Butyl, Diethylamino, Methylsulfonyl, Carboxamid, Cyano, Cyclopentyloxy, Trifluormethoxy, 1,1,1,3,3,3-Hexafluorpropan-2-yloxy, 2-Fluorethoxy, 2,2,2-Trifluorethoxy, Trifluormethyl, Fluor, Chlor, Brom, 1,2,4-Triazol-1-yl, Pyridin-3-yl, Pyrrolidin-1-yl, Morpholino, Hydroxyl, Cyclopropylmethoxy, Benzyloxy, 6-Methoxypyridin-3-yl und 1-(tert-Butoxycarbonyl)-1H-pyrrol-2-yl ausgewählt ist.

15. Verbindung nach einem der Ansprüche 1 bis 12, worin R⁶ H ist.

16. Verbindung nach einem der Ansprüche 1 bis 15, worin R⁷ aus H, C₁-C₆-Alkoxy, C₁-C₆-Alkyl, C₂-C₆-Alkinyl, C₁-C₆-Alkylsulfonyl, Carboxamid, Cyano, C₃-C₇-Cy_{d}oalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt ist, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, C₁-C₆-Alkyl, Cyano, Carbo-C₁-C₆-alkoxy, C₃-C₇-Cycloalkyl, Halogen und Phenyl ausgewählt sind.

17. Verbindung nach einem der Ansprüche 1 bis 16, worin R⁷ aus H, sec-Butoxy, Isopropoxy, Methoxy, Ethoxy, Propoxy, Methyl, Ethyl, Propyl, Butyl, tert-Butyl, Ethinyl, Diethylamino, Methylsulfonyl, Carboxamid, Cyano, Cyclobutyl, Cyclopropyl, Cyclopentyloxy, Trifluormethoxy, 1,1,1,3,3,3-Hexafluorpropan-2-yloxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyridin-4-yl, Pyrimidin-5-yl, Pyrrolidin-1-yl, Morpholino, Hydroxyl, Cyclopropylmethoxy, Benzyloxy, 2-Cyanoethyl, 6-Methoxypyridin-3-yl, Cyclopropylethinyl, 6-Fluorpyridin-3-yl, 1-(tert-Butoxycarbonyl)-1H-pyrrol-2-yl, 2-Fluorpyridin-4-yl und 2-Fluorpyridin-3-yl ausgewählt ist.

18. Verbindung nach einem der Ansprüche 1 bis 17, worin R⁸ aus H, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt ist, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, C₁-C₆-Alkyl, Carbo-C₁-C₆-alkoxy, Cyano, C₃-C₇-Cycloalkyl, Halogen und Phenyl ausgewählt sind.

19. Verbindung nach einem der Ansprüche 1 bis 17, worin R⁸ aus H, Isopropoxy, Methoxy, Ethoxy, Propoxy, Methyl, Ethyl, Propyl, tert-Butyl, Ethinyl, Methylsulfonyl, Carboxamid, Cyano, Cyclobutyl, Cyclopropyl, Cyclopentyloxy, Trifluormethoxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyridin-4-yl, Pyrimidin-5-yl, Pyrrolidin-1-yl, Morpholino, Hydroxyl, Cyclopropylmethoxy, Benzyloxy, 2-Cyanoethyl, 6-Methoxypyridin-3-yl, Cyclopropylethinyl, 6-Fluorpyridin-3-yl, 1-(tert-Butoxycarbonyl)-1H-pyrrol-2-yl, 2-Fluorpyridin-4-yl und 2-Fluorpyridin-3-yl ausgewählt ist.

20. Verbindung nach einem der Ansprüche 1 bis 8, worin R¹, R², R⁶, R⁷ und R⁸ unabhängig voneinander aus H, C₁-C₆-Acyl, C₁-C₆-Acyloxy, C₁-C₆-Alkoxy, C₁-C₆-Alkoxycarbonylamino, C₁-C₆-Alkyl, C₂-C₆-Alkinyl, C₁-C₆-Alkylamino, C₂-C₈-Dialkylamino, C₁-C₆-Alkylcarboxamid, C₁-C₆-Alkylsulfonamid, C₁-C₆-Alkylsulfinyl, C₁-C₆-Alkylsulfonyl, C₁-C₆-Alkylthio, C₁-C₆-Alkylureyl, Amino, Carbo-C₁-C₆-alkoxy, Carboxamid, Carboxy, Cyano, C₃-C₇-Cy_{d}oalkyl, C₃-C₇-Cycloalkyloxy, C₃-C₇-Cycloalkylthio, C₃-C₇-Cycloalkylsulfinyl, C₃-C₇-Cycloalkylsulfonyl, C₂-C₆-Dialkylcarboxamid, C₁-C₆-Halogenalkoxy, C₁-C₆-Halogenalkyl, C₁-C₆-Halogenalkylsulfinyl, C₁-C₆-Halogenalkylsulfonyl, Halogen, Heteroaryl, Heterocyclyl, Hydroxyl, Nitro und Sulfonamid ausgewählt sind, worin C₁-C₆-Alkoxy, C₁-C₆-Alkylthio, C₁-C₆-Alkyl, C₂-C₆-Alkinyl und Heteroaryl gegebenenfalls mit 1 Substituenten substituiert sind, der aus C₁-C₆-Alkoxy, Carbo-C₁-C₆-alkoxy, Cyano, C₃-C₇-Cycloalkyl, Halogen und Phenyl ausgewählt ist.

21. Verbindung nach einem der Ansprüche 1 bis 8, worin R¹, R², R⁶, R⁷ und R⁸ unabhängig voneinander aus H, C₁-C₆-Alkoxy, C₁-C₆-Alkyl, C₂-C₆-Alkinyl, C₂-C₈-Dialkylamino, C₁-C₆-Alkylsulfonyl, Carboxamid, Cyano, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt sind, worin C₁-C₆-Alkoxy, C₁-C₆-Alkyl, C₂-C₆-Alkinyl und Heteroaryl gegebenenfalls mit 1 Substituenten substituiert sind, der aus C₁-C₆-Alkoxy, Carbo-C₁-C₆-alkoxy, Cyano, C₃-C₇-Cyoalkyl, Halogen und Phenyl ausgewählt ist.

22. Verbindung nach einem der Ansprüche 1 bis 8, worin R¹, R², R⁶, R⁷ und R⁸ unabhängig voneinander aus H, sec-Butoxy, Isopropoxy, Methoxy, Ethoxy, Propoxy, Methyl, Ethyl, Propyl, Butyl, tert-Butyl, Ethinyl, Diethylamino, Methylsulfonyl, Carboxamid, Cyano, Cyclobutyl, Cyclopropyl, Cyclopentyloxy, Trifluormethoxy, 1,1,1,3,3,3-Hexafluorpropan-2-yloxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyridin-4-yl, Pyrimidin-5-yl, Pyrrolidin-1-yl, Morpholino, Hydroxyl, Cyclopropylmethoxy, Benzyloxy, 2-Cyanoethyl, 6-Methoxypyridin-3-yl, Cyclopropylethinyl, 6-Fluorpyridin-3-yl, 1-(tert-Butoxycarbonyl)-1H-pyrrol-2-yl, 2-Fluorpyridin-4-yl und 2-Fluorpyridin-3-yl ausgewählt sind.

23. Verbindung nach einem der Ansprüche 1 bis 8 und 20 to 22, worin R¹ aus H, C₁-C₆-Alkoxy, C₁-C₆-Alkyl, C₂-C₈-Dialkylamino, C₁-C₆-Alkylsulfonyl, Carboxamid, Cyano, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt ist, worin C₁-C₆-Alkoxy und Heteroaryl gegebenenfalls mit 1 Substituenten substituiert sind, der aus C₁-C₆-Alkoxy, Carbo-C₁-C₆-alkoxy und C₃-C₇-Cycloalkyl ausgewählt ist.

24. Verbindung nach einem der Ansprüche 1 bis 8 und 20 to 23, worin R⁶ aus H, C₁-C₆-Alkoxy, C₁-C₆-Alkyl, C₂-C₈-Dialkylamino, C₁-C₆-Alkylsulfonyl, Carboxamid, Cyano, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt ist, worin C₁-C₆-Alkoxy und Heteroaryl gegebenenfalls mit 1 Substituenten substituiert sind, der aus C₁-C₆-Alkoxy, Carbo-C₁-C₆-alkoxy, C₃-C₇-Cycloalkyl und Phenyl ist.

25. Verbindung nach einem der Ansprüche 1 bis 8 und 20 to 24, worin R⁷ aus H, C₁-C₆-Alkoxy, C1C6-Alkyl, C₂-C₆-Alkinyl, C₂-C₈-Dialkylamino, C₁-C₆-Alkylsulfonyl, Carboxamid, Cyano, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt ist, worin C₁-C₆-Alkoxy, C₁-C₆-Alkyl, C₂-C₆-Alkinyl und Heteroaryl gegebenenfalls mit 1 Substituenten substituiert sind, der aus C₁-C₆-Alkoxy, Cyano, Carbo-C₁-C₆-alkoxy, C₃-C₇-Cycloalkyl, Halogen und Phenyl ausgewählt ist.

26. Verbindung nach einem der Ansprüche 1 bis 8 und 20 to 25, worin R⁸ aus H, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₃-C₇-Cy_{d}oalkyloxy, C₁-C₆-Halogenalkoxy, C₁-C₆-Halogenalkyl, Halogen, Heteroaryl, Heterocyclyl und Hydroxyl ausgewählt ist, worin C₁-C₆-Alkoxy, C₁-C₆-Alkyl, C₂-C₆-Alkinyl und Heteroaryl gegebenenfalls mit 1 Substituenten substituiert sind, der aus C₁-C₆-Alkoxy, Carbo-C₁-C₆-alkoxy, Cyano, C₃-C₇-Cycloalkyl, Halogen und Phenyl ausgewählt ist.

27. Verbindung nach einem der Ansprüche 1 bis 26, worin:
W CR⁶ ist;
Z CR⁷ ist; und
X CR⁸ ist.

28. Verbindung nach Anspruch 1, ausgewählt aus Verbindungen der Formel (Ig) und pharmazeutisch annehmbaren Salzen, Solvaten und Hydraten davon: 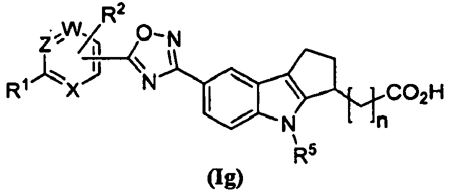 worin:
n = 0 oder 1;
W ist N oder CR⁶;
Z ist N oder CR⁷;
X ist N oder CR⁸; mit der Maßgabe, dass W, Z und X nicht alle N sind,
R¹ ist aus H, C₁-C₆-Alkoxy, C₁-C₆-Alkyl, C₁-C₆-Alkylamino, C₂-C₈-Dialkylamino, C₁-C₆-Alkylsulfonyl, Carboxamid, Cyano, C₂-C₆-Alkinyl, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, Carbo-C₁-C₆-alkoxy, C₁-C₆-Alkyl, Cyano, Halogen, C₃-C₇-Cycloalkyl und Phenyl ausgewählt sind;
R² ist H;
R⁵ ist H oder C₁-C₆-Alkyl;
R⁶ ist aus H, C₁-C₆-Alkoxy, C₁-C₆-Alkyl, C₁-C₆-Alkylsulfonyl, Carboxamid, Cyano, C₂-C₆-Alkinyl, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, Carbo-C₁-C₆-alkoxy, C₁-C₆-Alkyl, Cyano, Halogen, C₃-C₇-Cycloalkyl und Phenyl ausgewählt sind;
R⁷ ist aus H, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, C₁-C₆-Alkyl, Cyano, Carbo-C₁-C₆-alkoxy, C₃-C₇-Cycloalkyl, Halogen und Phenyl ausgewählt sind; und
R⁸ ist aus H, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, C₁-C₆-Alkyl, Carbo-C₁-C₆-alkoxy, Cyano, C₃-C₇-Cycloalkyl, Halogen und Phenyl ausgewählt sind.

29. Verbindung nach Anspruch 1, ausgewählt aus Verbindungen der Formel (Ig) und pharmazeutisch annehmbaren Salzen, Solvaten und Hydraten davon: 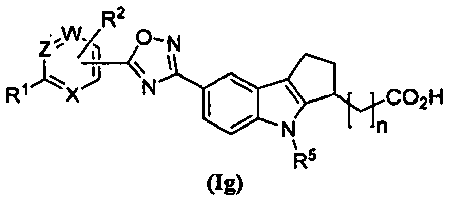 worin:
n = 0 oder 1;
W ist N oder CR⁶;
Z ist N oder CR⁷;
X ist N oder CR⁸; mit der Maßgabe, dass W, Z und X nicht alle N sind,
R¹ ist aus H, C₁-C₆-Alkoxy, C₁-C₆-Alkyl, C₂-C₈-Dialkylamino, C₁-C₆-Alkylsulfonyl, Carboxamid, Cyano, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy und Heteroaryl gegebenenfalls mit 1 Substituenten substituiert sind, der aus C₁-C₆-Alkoxy, Carbo-C₁-C₆-alkoxy und C₃-C₇-Cycloalkyl ausgewählt ist;
R² ist H;
R⁵ ist H oder C₁-C₆-Alkyl;
R⁶ ist aus H, C₁-C₆-Alkoxy, C₁-C₆-Alkyl, C₂-C₈-Dialkylamino, C₁-C₆-Alkylsulfonyl, Carboxamid, Cyano, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy und Heteroaryl gegebenenfalls mit 1 Substituenten substituiert sind, der aus C₁-C₆-Alkoxy, Carbo-C₁-C₆-alkoxy, C₃-C₇-Cycloalkyl und Phenyl ausgewählt ist;
R⁷ ist aus H, C₁-C₆-Alkoxy, C₁-C₆-Alkyl, C₂-C₆-Alkinyl, C₂-C₈-Dialkylamino, C₁-C₆-Alkylsulfonyl, Carboxamid, Cyano, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy, C₁-C₆-Alkyl, C₂-C₆-Alkinyl und Heteroaryl gegebenenfalls mit 1 Substituenten substituiert sind, der aus C₁-C₆-Alkoxy, Cyano, Carbo-C₁-C₆-alkoxy, C₃-C₇-Cycloalkyl, Halogen und Phenyl ausgewählt ist; und
R⁸ ist aus H, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy, C₁-C₆-Alkyl, C₂-C₆-Alkinyl und Heteroaryl gegebenenfalls mit 1 Substituenten substituiert sind, der aus C₁-C₆-Alkoxy, Carbo-C₁-C₆-alkoxy, Cyano, C₃-C₇-Cycloalkyl, Halogen und Phenyl ausgewählt ist.

30. Verbindung nach Anspruch 1, ausgewählt aus Verbindungen der Formel (IIa) und pharmazeutisch annehmbaren Salzen, Solvaten und Hydraten davon: 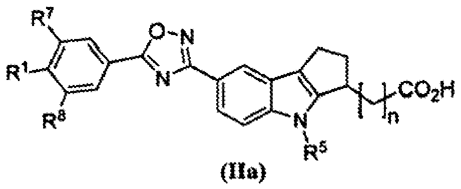 worin:
n = 0 oder 1;
R¹ ist aus H, C₁-C₆-Alkoxy, Cyano, C₃-C₇-Cycloalkyloxy, C₁-C₆-Halogenalkoxy, Halogen und Hydroxyl ausgewählt, worin C₁-C₆-Alkoxy gegebenenfalls mit 1, 2 oder 3 Substituenten substituiert ist, die aus Halogen und C₃-C₇-Cycloalkyl ausgewählt sind;
R⁵ ist H oder C₁-C₆-Alkyl;
R⁷ ist aus H, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₃-C₇-Cy_{d}oalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, C₁-C₆-Alkyl, Cyano, Carbo-C₁-C₆-alkoxy, C₃-C₇-Cycloalkyl, Halogen und Phenyl ausgewählt sind; und
R⁸ ist aus H, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₃-C₇-Cycloalkyloxy, C₁-C₆-Halogenalkoxy, C₁-C₆-Halogenalkyl, Halogen, Heteroaryl, Heterocyclyl und Hydroxyl ausgewählt, worin C₁-C₆-Alkoxy, C₁-C₆-Alkyl, C₂-C₆-Alkinyl und Heteroaryl gegebenenfalls mit 1, 2 oder 3 Substituenten substituiert sind, die aus C₁-C₆-Alkoxy, C₁-C₆-Alkyl, Cyano, Carbo-C₁-C₆-alkoxy, C₃-C₇-Cycloalkyl, Halogen und Phenyl ausgewählt sind.

31. Verbindung nach Anspruch 1, ausgewählt aus Verbindungen der Formel (IIa) und pharmazeutisch annehmbaren Salzen, Solvaten und Hydraten davon: 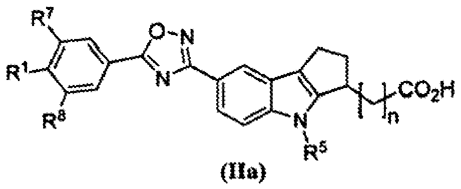 worin:
n = 0 oder 1;
R¹ ist aus H, Isopropoxy, Methoxy, Ethoxy, Cyano, Cyclopentyloxy, Trifluormethoxy, 1,1,1,3,3,3-Hexafluorpropan-2-yloxy, Fluor, Hydroxyl, Cyclopropylmethoxy und Difluormethoxy ausgewählt;
R⁵ ist H oder Methyl;
R⁷ ist aus H, Isopropoxy, Methoxy, Ethoxy, Propoxy, Methyl, Ethyl, Propyl, tert-Butyl, Ethinyl, Methylsulfonyl, Carboxamid, Cyano, Cyclobutyl, Cyclopropyl, Cyclopentyloxy, Trifluormethoxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyridin-4-yl, Pyrimidin-5-yl, Pyrrolidin-1-yl, Morpholino, Hydroxyl, Cyclopropylmethoxy, Benzyloxy, 2-Cyanoethyl, 6-Methoxypyridin-3-yl, Cyclopropylethinyl, 6-Fluorpyridin-3-yl, 1-(tert-Butoxycarbonyl)-1H-pyrrol-2-yl, 2-Fluorpyridin-4-yl, 2-Fluorpyridin-3-yl, 2-Methoxypyrimidin-5-yl, 3-Fluorpyridin-4-yl, 3,5-Dimethylisoxazol-4-yl, 2,6-Difluorpyridin-4-yl, 6-Fluor-5-methylpyridin-3-yl, 1-Methyl-1H-pyrazol-4-yl, 2-Methylpyridin-4-yl, 3-Methylpyridin-4-yl, 1,3,5-Trimethyl-1H-pyrazol-4-yl und 1-Isobutyl-1H-pyrazol-4-yl ausgewählt; und
R⁸ ist aus H, Isopropoxy, Methoxy, Ethoxy, Propoxy, Methyl, Ethyl, Propyl, tert-Butyl, Ethinyl, Methylsulfonyl, Carboxamid, Cyano, Cyclobutyl, Cyclopropyl, Cyclopentyloxy, Trifluormethoxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyridin-4-yl, Pyrimidin-5-yl, Pyrrolidin-1-yl, Morpholino, Hydroxyl, Cyclopropylmethoxy, Benzyloxy, 2-Cyanoethyl, 6-Methoxypyridin-3-yl, Cyclopropylethinyl, 6-Fluorpyridin-3-yl, 1-(tert-Butoxycarbonyl)-1H-pyrrol-2-yl, 2-Fluorpyridin-4-yl, 2-Fluorpyridin-3-yl, 2-Methoxypyrimidin-5-yl, 3-Fluorpyridin-4-yl, 3,5-Dimethylisoxazol-4-yl, 2,6-Difluorpyridin-4-yl, 6-Fluor-5-methylpyridin-3-yl, 1-Methyl-1H-pyrazol-4-yl, 2-Methylpyridin-4-yl, 3-Methylpyridin-4-yl, 1,3,5-Trimethyl-1H-pyrazol-4-yl und 1-Isobutyl-1H-pyrazol-4-yl ausgewählt.

32. Verbindung nach Anspruch 1, ausgewählt aus Verbindungen der Formel (IIa) und pharmazeutisch annehmbaren Salzen, Solvaten und Hydraten davon: 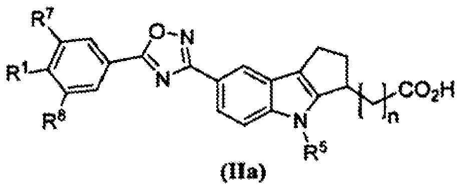 worin:
n = 0 oder 1 ;
R¹ ist aus H, C₁-C₆-Alkoxy, Cyano, C₃-C₇-Cycloalkyloxy, C₁-C₆-Halogenalkoxy und Hydroxyl ausgewählt, worin C₁-C₆-Alkoxy gegebenenfalls mit 1 Substituenten substituiert ist, der aus C₁-C₆-Alkoxy ausgewählt ist;
R⁵ ist H oder C₁-C₆-Alkyl;
R⁷ ist aus H, C₁-C₆-Alkoxy, C₁-C₆-Alkyl, C₁-C₆-Alkylsulfonyl, Cyano, C₃-C₇-Cycloalkyloxy, C₁-C₆-Halogenalkoxy, C₁-C₆-Halogenalkyl, Halogen, Heteroaryl, Heterocyclyl ausgewählt, worin C₁-C₆-Alkoxy und Heteroaryl gegebenenfalls mit 1 Substituenten substituiert sind, der aus C₁-C₆-Alkoxy, Carbo-C₁-C₆-alkoxy und Phenyl ausgewählt ist; und
R⁸ ist aus, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₁-C₆-Halogenalkoxy, C₁-C₆-Halogenalkyl, Halogen, Heteroaryl und Hydroxyl ausgewählt, worin C₁-C₆-Alkoxy, C₁-C₆-Alkyl, C₂-C₆-Alkinyl und Heteroaryl gegebenenfalls mit 1 Substituenten substituiert sind, der aus Cyano, C₃-C₇-Cycloalkyl, Halogen und Phenyl ausgewählt ist.

33. Verbindung nach Anspruch 1, ausgewählt aus Verbindungen der Formel (IIa) und pharmazeutisch annehmbaren Salzen, Solvaten und Hydraten davon: 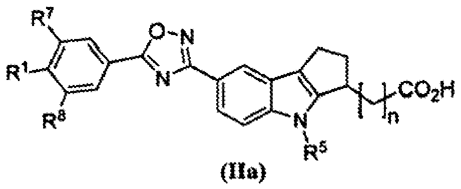 worin:
n = 0 oder 1 ;
R¹ ist aus H, Isopropoxy, Methoxy, Ethoxy, Cyano, Cyclopentyloxy, Trifluormethoxy, 1,1,1,3,3,3-Hexafluorpropan-2-yloxy, Hydroxyl und Cyclopropylmethoxy ausgewählt;
R⁵ ist H oder Methyl;
R⁷ ist aus H, Isopropoxy, Methoxy, Ethoxy, Methyl, Ethyl, Propyl, Methylsulfonyl, Cyano, Cyclopentyloxy, Trifluormethoxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyrimidin-5-yl, Pyrrolidin-1-yl, Morpholino, Benzyloxy, 6-Methoxypyridin-3-yl und 1-(tert-Butoxycarbonyl)-1H-pyrrol-2-yl ausgewählt; und
R⁸ ist aus H, Isopropoxy, Methoxy, Ethoxy, Propoxy, Methyl, tert-Butyl, Ethinyl, Methylsulfonyl, Carboxamid, Cyano, Cyclobutyl, Cyclopropyl, Trifluormethoxy, Trifluormethyl, Fluor, Chlor, Brom, Pyridin-4-yl, Hydroxyl, Cyclopropylmethoxy, Benzyloxy, 2-Cyanoethyl, Cyclopropylethinyl, 6-Fluorpyridin-3-yl, 2-Fluorpyridin-4-yl und 2-Fluorpyridin-3-yl ausgewählt.

34. Verbindung nach Anspruch 1, ausgewählt aus Verbindungen der Formel (IIc) und pharmazeutisch annehmbaren Salzen, Solvaten und Hydraten davon: 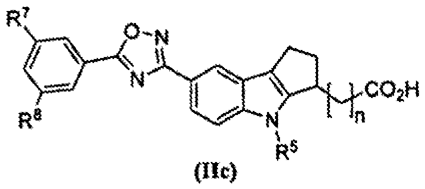 worin:
n = 0 oder 1 ;
R⁵ ist H oder Methyl;
R⁷ ist aus H, Isopropoxy, Methoxy, Ethoxy, Propoxy, Methyl, Ethyl, Propyl, tert-Butyl, Ethinyl, Methylsulfonyl, Carboxamid, Cyano, Cyclobutyl, Cyclopropyl, Cyclopentyloxy, Trifluormethoxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyridin-4-yl, Pyrimidin-5-yl, Pyrrolidin-1-yl, Morpholino, Hydroxyl, Cyclopropylmethoxy, Benzyloxy, 2-Cyanoethyl, 6-Methoxypyridin-3-yl, Cyclopropylethinyl, 6-Fluorpyridin-3-yl, 1-(tert-Butoxycarbonyl)-1H-pyrrol-2-yl, 2-Fluorpyridin-4-yl, 2-Fluorpyridin-3-yl, 2-Methoxypyrimidin-5-yl, 3-Fluorpyridin-4-yl, 3,5-Dimethylisoxazol-4-yl, 2,6-Difluorpyridin-4-yl, 6-Fluor-5-methylpyridin-3-yl, 1-Methyl-1H-pyrazol-4-yl, 2-Methylpyridin-4-yl, 3-Methylpyridin-4-yl, 1,3,5-Trimethyl-1H-pyrazol-4-yl und 1-Isobutyl-1H-pyrazol-4-yl ausgewählt; und
R⁸ ist aus H, Isopropoxy, Methoxy, Ethoxy, Propoxy, Methyl, Ethyl, Propyl, tert-Butyl, Ethinyl, Methylsulfonyl, Carboxamid, Cyano, Cyclobutyl, Cyclopropyl, Cyclopentyloxy, Trifluormethoxy, 2-Fluorethoxy, 2,2,2-Trifluorethoxy, Trifluormethyl, Fluor, Chlor, Brom, 1,2,4-Triazol-1-yl, 1,2,4-Triazol-4-yl, Pyridin-3-yl, Pyridin-4-yl, Pyrimidin-5-yl, Pyrrolidin-1-yl, Morpholino, Hydroxyl, Cyclopropylmethoxy, Benzyloxy, 2-Cyanoethyl, 6-Methoxypyridin-3-yl, Cyclopropylethinyl, 6-Fluorpyridin-3-yl, 1-(tert-Butoxycarbonyl)-1H-pyrrol-2-yl, 2-Fluorpyridin-4-yl, 2-Fluorpyridin-3-yl, 2-Methoxypyrimidin-5-yl, 3-Fluorpyridin-4-yl, 3,5-Dimethylisoxazol-4-yl, 2,6-Difluorpyridin-4-yl, 6-Fluor-5-methylpyridin-3-yl, 1-Methyl-1H-pyrazol-4-yl, 2-Methylpyridin-4-yl, 3-Methylpyridin-4-yl, 1,3,5-Trimethyl-1H-pyrazol-4-yl und 1-Isobutyl-1H-pyrazol-4-yl ausgewählt.

35. Verbindung nach Anspruch 1, ausgewählt aus Verbindungen der Formel (IIc) und pharmazeutisch annehmbaren Salzen, Solvaten und Hydraten davon: 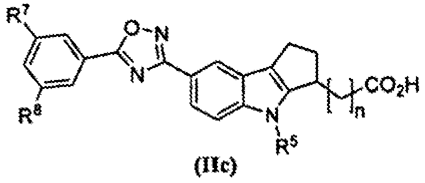 worin:
n = 0 oder 1 ;
R⁵ H oder Methyl;
R⁷ ist aus H, C₁-C₆-Alkoxy,C₁-C₆-Alkyl, C₁-C₆-Alkylsulfonyl, Cyano, C₃-C₇-Cycloalkyloxy, C₁-C₆-Halogenalkoxy, C₁-C₆-Halogenalkyl, Halogen, Heteroaryl, Heterocyclyl ausgewählt, worin C₁-C₆-Alkoxy und Heteroaryl gegebenenfalls mit 1 Substituenten substituiert sind, der aus C₁-C₆-Alkoxy, Carbo-C₁-C₆-alkoxy und Phenyl ausgewählt ist; und
R⁸ ist aus H, C₁-C₆-Alkoxy, C₁-C₆-Alkyl, C₂-C₆-Alkinyl, C₁-C₆-Alkylsulfonyl, Carboxamid, Cyano, C₃-C₇-Cycloalkyl, C₁-C₆-Halogenalkoxy, C₁-C₆-Halogenalkyl, Halogen, Heteroaryl und Hydroxyl ausgewählt, worin C₁-C₆-Alkoxy, C₁-C₆-Alkyl, C₂-C₆-Alkinyl und Heteroaryl gegebenenfalls mit 1 Substituenten substituiert sind, der aus Cyano, C₃-C₇-Cycloalkyl, Halogen und Phenyl ausgewählt ist.

36. Verbindung nach Anspruch 1, ausgewählt aus folgenden Verbindungen und pharmazeutisch annehmbaren Salzen, Solvaten und Hydraten davon:
2-(7-(5-(6-sec-Butoxy-5-methylpyridin-3-yl)-1,2,4-oxadiazol-3-yl)-1,2,3,4-tetrahydrocyclopenta[b]indol-3-yl)essigsäure;
2-(7-(5-(Pyridazin-3-yl)-1,2,4-oxadiazol-3-yl)-1,2,3,4-tetrahydrocyclopenta[b]indol-3-yl)essigsäure;
2-(7-(5-(3-Cyano-5-isopropoxyphenyl)-1,2,4-oxadiazol-3-yl)-1,2,3,4-tetrahydrocyclopenta[b]indol-3-yl)essigsäure;
2-(7-(5-(3,5-Difluorphenyl)-1,2,4-oxadiazol-3-yl)-1,2,3,4-tetrahydrocyclopenta[b]indol-3-yl)essigsäure;
2-(7-(5-(3-Cyano-5-methoxyphenyl)-1,2,4-oxadiazol-3-yl)-1,2,3,4-tetrahydrocyclopenta[b]indol-3-yl)essigsäure;
2-(7-(5-(3-Ethinyl-5-(trifluormethoxy)phenyl)-1,2,4-oxadiazol-3-yl)-1,2,3,4-tetrahydrocyclopenta[b]indol-3-yl)essigsäure;
2-(7-(5-(3-Cyano-5-(trifluormethoxy)phenyl)-1,2,4-oxadiazol-3-yl)-1,2,3,4-tetrahydrocyclopenta[b]indol-3-yl)essigsäure;
2-(7-(5-(3-Methoxy-5-(trifluormethyl)phenyl)-1,2,4-oxadiazol-3-yl)-1,2,3,4-tetrahydrocyclopenta[b]indol-3-yl)essigsäure;
2-(7-(5-(3-Cyclobutyl-5-(trifluormethoxy)phenyl)-1,2,4-oxadiazol-3-yl)-1,2,3,4-tetrahydrocyclopenta[b]indol-3-yl)essigsäure;
2-(7-(5-(3-Cyano-4-methoxyphenyl)-1,2,4-oxadiazol-3-yl)-1,2,3,4-tetrahydrocyclopenta[b]indol-3-yl)essigsäure;
2-(7-(5-(6-(Trifluormethyl)pyridin-3-yl)-1,2,4-oxadiazol-3-yl)-1,2,3,4-tetrahydrocyclopenta[b]indol-3-yl)essigsäure;
2-(7-(5-(3-Fluor-5-(trifluormethyl)phenyl)-1,2,4-oxadiazol-3-yl)-1,2,3,4-tetrahydrocyclopenta[b]indol-3-yl)essigsäure;
2-(7-(5-(4-Methoxy-3-methylphenyl)-1,2,4-oxadiazol-3-yl)-1,2,3,4-tetrahydrocyclopenta[b]indol-3-yl)essigsäure;
2-(7-(5-(3-Brom-5-(trifluormethyl)phenyl)-1,2,4-oxadiazol-3-yl)-1,2,3,4-tetrahydrocyclopenta[b]indol-3-yl)essigsäure;
2-(7-(5-(3-Cyano-4-isopropoxyphenyl)-1,2,4-oxadiazol-3-yl)-1,2,3,4-tetrahydrocyclopenta[b]indol-3-yl)essigsäure;
2-(7-(5-(4-(Trifluormethoxy)phenyl)-1,2,4-oxadiazol-3-yl)-1,2,3,4-tetrahydrocyclopenta[b]indol-3-yl)essigsäure;
2-(7-(5-(3-Brom-5-chlorphenyl)-1,2,4-oxadiazol-3-yl)-1,2,3,4-tetrahydrocyclopenta[b]indol-3-yl)essigsäure;
2-(7-(5-(3-(1H-1,2,4-Triazol-1-yl)-5-(trifluormethoxy)phenyl)-1,2,4-oxad iazol-3-yl)-1,2,3,4-tetrahydrocyclopenta[b]indol-3-yl)essigsäure;
2-(7-(5-(3-Cyano-4-(cyclopentyloxy)phenyl)-1,2,4-oxadiazol-3-yl)-1,2,3,4-tetrahydrocyclopenta[b]indol-3-yl)essigsäure;
2-(7-(5-(3-(Pyrrolidin-1-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-Cyano-5-ethoxyphenyl)-1,2,4-oxadiazol-3-yl)-1,2,3,4-tetrahydrocyclopenta[b]indol-3-yl)essigsäure;
2-(7-(5-(3-(Benzyloxy)phenyl)-1,2,4-oxadiazol-3-yl)-1,2,3,4-tetrahydrocyclopenta[b]indol-3-yl)essigsäure;
2-(7-(5-(5-Chlor-6-methoxypyridin-3-yl)-1,2,4-oxadiazol-3-yl)-1,2,3,4-tetrahydrocyclopenta[b]indol-3-yl)essigsäure;
2-(7-(5-(3-Propoxy-5-(trifluormethyl)phenyl)-1,2,4-oxadiazol-3-yl)-1,2,3,4-tetrahydrocyclopenta[b]indol-3-yl)essigsäure;
2-(7-(5-(3-Morpholino-5-(trifluormethoxy)phenyl)-1,2,4-oxadiazol-3-yl)-1,2,3,4-tetrahydrocyclopenta[b]indol-3-yl)essigsäure;
2-(7-(5-(6-Carbamoylpyridin-3-yl)-1,2,4-oxadiazol-3-yl)-1,2,3,4-tetrahydrocyclopenta[b]indol-3-yl)essigsäure;
2-(7-(5-(3-Cyano-4-(1,1,1,3,3,3-hexafluorpropan-2-yloxy)phenyl)-1,2,4-oxadiazol-3-yl)-1,2,3,4-tetrahydrocyclopenta[b]indol-3-yl)essigsäure;
2-(7-(5-(3-(Cyclopropylmethoxy)-5-(trifluormethyl)phenyl)-1,2,4-oxadiazol-3-yl)-1,2,3,4-tetrahydrocyclopenta[b]indol-3-yl)essigsäure;
2-(7-(5-(Pyrazin-2-yl)-1,2,4-oxadiazol-3-yl)-1,2,3,4-tetrahydrocyclopenta[b]indol-3-yl)essigsäure;
2-(7-(5-(3-Cyclopropyl-5-(trifluormethoxy)phenyl)-1,2,4-oxadiazol-3-yl)-1,2,3,4-tetrahydrocyclopenta[b]indol-3-yl)essigsäure;
2-(7-(5-(3-Ethyl-5-(trifluormethoxy)phenyl)-1,2,4-oxadiazol-3-yl)-1,2,3,4-tetrahydrocyclopenta[b]indol-3-yl)essigsäure;
2-(7-(5-(3-Cyano-5-(2-fluorethoxy)phenyl)-1,2,4-oxadiazol-3-yl)-1,2,3,4-tetrahydrocyclopenta[b]indol-3-yl)essigsäure;
2-(7-(5-(3-Isopropoxy-5-(trifluormethoxy)phenyl)-1,2,4-oxadiazol-3-yl)-1,2,3,4-tetrahydrocyclopenta[b]indol-3-yl)essigsäure;
2-(7-(5-(2-Chlor-6-methylpyridin-4-yl)-1,2,4-oxadiazol-3-yl)-1,2,3,4-tetrahydrocyclopenta[b]indol-3-yl)essigsäure;
2-(7-(5-(3,5-Dichlorphenyl)-1,2,4-oxadiazol-3-yl)-1,2,3,4-tetrahydrocyclopenta[b]indol-3-yl)essigsäure;
2-(7-(5-(3-Cyano-4-ethoxyphenyl)-1,2,4-oxadiazol-3-yl)-1,2,3,4-tetrahydrocyclopenta[b]indol-3-yl)essigsäure;
2-(7-(5-(3-Chlor-5-cyanophenyl)-1,2,4-oxadiazol-3-yl)-1,2,3,4-tetrahydrocyclopenta[b]indol-3-yl)essigsäure;
2-(7-(5-(3,5-Dimethoxyphenyl)-1,2,4-oxadiazol-3-yl)-1,2,3,4-tetrahydrocyclopenta[b]indol-3-yl)essigsäure;
2-(7-(5-(Pyridin-3-yl)-1,2,4-oxadiazol-3-yl)-1,2,3,4-tetrahydrocyclopenta[b]indol-3-yl)essigsäure;
2-(7-(5-(3-Propyl-5-(trifluormethoxy)phenyl)-1,2,4-oxadiazol-3-yl)-1,2,3,4-tetrahydrocyclopenta[b]indol-3-yl)essigsäure;
2-(7-(5-(Pyridin-4-yl)-1,2,4-oxadiazol-3-yl)-1,2,3,4-tetrahydrocyclopenta[b]indol-3-yl)essigsäure;
2-(7-(5-(4-Methoxy-3-(trifluormethyl)phenyl)-1,2,4-oxadiazol-3-yl)-1,2,3,4-tetrahydrocyclopenta[b]indol-3-yl)essigsäure;
2-(7-(5-(3-Chlor-4-methoxyphenyl)-1,2,4-oxadiazol-3-yl)-1,2,3,4-tetrahydrocyclopenta[b]indol-3-yl)essigsäure;
2-(7-(5-(3-Cyano-4-hydroxyphenyl)-1,2,4-oxadiazol-3-yl)-1,2,3,4-tetrahydrocyclopenta[b]indol-3-yl)essigsäure;
2-(7-(5-(3-Chlor-5-(trifluormethoxy)phenyl)-1,2,4-oxadiazol-3-yl)-1,2,3,4-tetrahydrocyclopenta[b]indol-3-yl)essigsäure;
2-(7-(5-(3-Cyano-5-fluorphenyl)-1,2,4-oxadiazol-3-yl)-1,2,3,4-tetrahydrocyclopenta[b]indol-3-yl)essigsäure;
2-(7-(5-(6-(2,2,2-Trifluorethoxy)pyridin-3-yl)-1,2,4-oxadiazol-3-yl)-1,2,3,4-tetrahydrocyclopenta[b]indol-3-yl)essigsäure;
2-(7-(5-(6-Isopropoxy-5-methyl pyridin-3-yl)-1,2,4-oxadiazol-3-yl)-1,2,3,4-tetrahydrocyclopenta[b]indol-3-yl)essigsäure;
2-(7-(5-(3-(Benzyloxy)-5-(trifluormethyl)phenyl)-1,2,4-oxadiazol-3-yl)-1,2,3,4-tetrahydrocyclopenta[b]indol-3-yl)essigsäure;
2-(7-(5-(3-(Trifluormethyl)phenyl)-1,2,4-oxadiazol-3-yl)-1,2,3,4-tetrahydrocyclopenta[b]indol-3-yl)essigsäure;
2-(7-(5-(3-Carbamoyl-5-(trifluormethoxy)phenyl)-1,2,4-oxadiazol-3-yl)-1,2,3,4-tetrahydrocyclopenta[b]indol-3-yl)essigsäure;
2-(7-(5-(3,5-Bis(trifluormethyl)phenyl)-1,2,4-oxadiazol-3-yl)-1,2,3,4-tetrahydrocyclopenta[b]indol-3-yl)essigsäure;
2-(7-(5-(3-Methyl-5-(trifluormethoxy)phenyl)-1,2,4-oxadiazol-3-yl)-1,2,3,4-tetrahydrocyclopenta[b]indol-3-yl)essigsäure;
2-(7-(5-(5-Butylpyridin-2-yl)-1,2,4-oxadiazol-3-yl)-1,2,3,4-tetrahydrocyclopenta[b]indol-3-yl)essigsäure;
2-(7-(5-(3-Cyano-5-(2,2,2-trifluorethoxy)phenyl)-1,2,4-oxadiazol-3-yl)-1,2,3,4-tetrahydrocyclopenta[b]indol-3-yl)essigsäure;
2-(7-(5-(Pyrimidin-5-yl)-1,2,4-oxadiazol-3-yl)-1,2,3,4-tetrahydrocyclopenta[b]indol-3-yl)essigsäure;
2-(7-(5-(3-tert-Butyl-5-cyanophenyl)-1,2,4-oxadiazol-3-yl)-1,2,3,4-tetrahydrocyclopenta[b]indol-3-yl)essigsäure;
2-(7-(5-(6-Methoxy-5-methylpyridin-3-yl)-1,2,4-oxadiazol-3-yl)-1,2,3,4-tetrahydrocyclopenta[b]indol-3-yl)essigsäure;
2-(7-(5-(6-sec-Butoxy-5-chlorpyridin-3-yl)-1,2,4-oxadiazol-3-yl)-1,2,3,4-tetrahydrocyclopenta[b]indol-3-yl)essigsäure;
2-(7-(5-(3-(2-Cyanoethyl)-5-(trifluormethoxy)phenyl)-1,2,4-oxadiazol-3-yl)-1,2,3,4-tetrahydrocyclopenta[b]indol-3-yl)essigsäure;
2-(7-(5-(Pyridin-2-yl)-1,2,4-oxadiazol-3-yl)-1,2,3,4-tetrahydrocyclopenta[b]indol-3-yl)essigsäure;
2-(7-(5-(6-Ethoxy-5-methylpyridin-3-yl)-1,2,4-oxadiazol-3-yl)-1,2,3,4-tetrahydrocyclopenta[b]indol-3-yl)essigsäure;
2-(7-(5-(3-Brom-5-(trifluormethoxy)phenyl)-1,2,4-oxadiazol-3-yl)-1,2,3,4-tetrahydrocyclopenta[b]indol-3-yl)essigsäure;
2-(7-(5-(3,5-Dimethylphenyl)-1,2,4-oxadiazol-3-yl)-1,2,3,4-tetrahydrocyclopenta[b]indol-3-yl)essigsäure;
2-(7-(5-(3-Cyano-5-(cyclopentyloxy)phenyl)-1,2,4-oxadiazol-3-yl)-1,2,3,4-tetrahydrocyclopenta[b]indol-3-yl)essigsäure;
2-(7-(5-(5-Chlor-6-isopropoxypyridin-3-yl)-1,2,4-oxadiazol-3-yl)-1,2,3,4-tetrahydrocyclopenta[b]indol-3-yl)essigsäure;
2-(7-(5-(3-Isopropoxy-5-(trifluormethyl)phenyl)-1,2,4-oxadiazol-3-yl)-1,2,3,4-tetrahydrocyclopenta[b]indol-3-yl)essigsäure;
2-(7-(5-(3,5-Diethoxyphenyl)-1,2,4-oxadiazol-3-yl)-1,2,3,4-tetrahydrocyclopenta[b]indol-3-yl)essigsäure;
2-(7-(5-(3-Brom-5-hydroxyphenyl)-1,2,4-oxadiazol-3-yl)-1,2,3,4-tetrahydrocyclopenta[b]indol-3-yl)essigsäure;
2-(7-(5-(5-Brompyridin-3-yl)-1,2,4-oxadiazol-3-yl)-1,2,3,4-tetrahydrocyclopenta[b]indol-3-yl)essigsäure,
2-(7-(5-(Pyridazin-4-yl)-1,2,4-oxadiazol-3-yl)-1,2,3,4-tetrahydrocyclopenta[b]indol-3-yl)essigsäure,
2-(7-(5-(3-(4H-1,2,4-Triazol-4-yl)-5-(trifluormethoxy)phenyl)-1,2,4-oxad iazol-3-yl)-1,2,3,4-tetrahydrocyclopenta[b]indol-3-yl)essigsäure;
7-(5-(3-Cyano-5-(trifluormethoxy)phenyl)-1,2,4-oxadiazol-3-yl)-1,2,3,4-tetrahydrocyclopenta[b]indol-3-carbonsäure;
2-(7-(5-(3-(6-Methoxypyridin-3-yl)-5-(trifluormethoxy)phenyl)-1,2,4-oxadiazol-3-yl)-1,2,3,4-tetrahydrocyclopenta[b]indol-3-yl)essigsäure;
2-(7-(5-(6-Methoxypyridin-3-yl)-1,2,4-oxadiazol-3-yl)-1,2,3,4-tetrahydrocyclopenta[b]indol-3-yl)essigsäure;
2-(7-(5-(4-Isopropoxy-3-methoxyphenyl)-1,2,4-oxadiazol-3-yl)-1,2,3,4-tetrahydrocyclopenta[b]indol-3-yl)essigsäure;
2-(7-(5-(3-Cyano-4-(cyclopropylmethoxy)phenyl)-1,2,4-oxadiazol-3-yl)-1,2,3,4-tetrahydrocyclopenta[b]indol-3-yl)essigsäure;
2-(7-(5-(4-Hydroxy-3-methylphenyl)-1,2,4-oxadiazol-3-yl)-1,2,3,4-tetrahydrocyclopenta[b]indol-3-yl)essigsäure;
2-(7-(5-(3-(Pyridin-3-yl)-5-(trifluormethoxy)phenyl)-1,2,4-oxadiazol-3-yl)-1,2,3,4-tetrahydrocyclopenta[b]indol-3-yl)essigsäure;
2-(7-(5-(6-(Diethylamino)-4-(trifluormethyl)pyridin-2-yl)-1,2,4-oxadiazol-3-yl)-1,2,3,4-tetrahydrocyclopenta[b]indol-3-yl)essigsäure;
2-(7-(5-(3-Fluor-4-isopropoxyphenyl)-1,2,4-oxadiazol-3-yl)-1,2,3,4-tetrahydrocyclopenta[b]indol-3-yl)essigsäure;
2-(7-(5-(5-Isopropoxypyrazin-2-yl)-1,2,4-oxadiazol-3-yl)-1,2,3,4-tetrahydrocyclopenta[b]indol-3-yl)essigsäure;
2-(7-(5-(5-Methoxypyridin-2-yl)-1,2,4-oxadiazol-3-yl)-1,2,3,4-tetrahydrocyclopenta[b]indol-3-yl)essigsäure;
2-(7-(5-(5-Isopropoxypyridin-2-yl)-1,2,4-oxadiazol-3-yl)-1,2,3,4-tetrahydrocyclopenta[b]indol-3-yl)essigsäure;
2-(7-(5-(3,4-Dimethoxyphenyl)-1,2,4-oxadiazol-3-yl)-1,2,3,4-tetrahydrocyclopenta[b]indol-3-yl)essigsäure;
2-(7-(5-(3-(Cyclopropylethinyl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(6-Fluorpyridin-3-yl)-5-(trifluormethoxy)phenyl)-1,2,4-oxadiazol-3-yl)-1,2,3,4-tetrahydrocyclopenta[b]indol-3-yl)essigsäure;
2-(7-(5-(6-Isopropoxypyridazin-3-yl)-1,2,4-oxadiazol-3-yl)-1,2,3,4-tetrahydrocyclopenta[b]indol-3-yl)essigsäure;
2-(7-(5-(4-Isopropoxy-3-(methylsulfonyl)phenyl)-1,2,4-oxadiazol-3-yl)-1,2,3,4-tetrahydrocyclopenta[b]indol-3-yl)essigsäure;
2-(7-(5-(3,5-Bis(methylsulfonyl)phenyl)-1,2,4-oxadiazol-3-yl)-1,2,3,4-tetrahydrocyclopenta[b]indol-3-yl)essigsäure;
2-(7-(5-(3-(1-(tert-Butoxycarbonyl)-1H-pyrrol-2-yl)-5-(trifluormethoxy)-phenyl)-1,2,4-oxadiazol-3-yl)-1,2,3,4-tetrahydrocyclopenta[b]indol-3-yl)essigsäure;
2-(7-(5-(3-Fluor-4-methoxyphenyl)-1,2,4-oxadiazol-3-yl)-1,2,3,4-tetrahydrocyclopenta[b]indol-3-yl)essigsäure;
2-(7-(5-(6-Isopropoxypyridin-3-yl)-1,2,4-oxadiazol-3-yl)-1,2,3,4-tetrahydrocyclopenta[b]indol-3-yl)essigsäure;
2-(7-(5-(3-Chlor-4-hydroxyphenyl)-1,2,4-oxadiazol-3-yl)-1,2,3,4-tetrahydrocyclopenta[b]indol-3-yl)essigsäure;
2-(7-(5-(4-Cyano-3-(trifluormethoxy)phenyl)-1,2,4-oxadiazol-3-yl)-1,2,3,4-tetrahydrocyclopenta[b]indol-3-yl)essigsäure;
2-(7-(5-(3-(Pyridin-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(4-Isopropoxyphenyl)-1,2,4-oxadiazol-3-yl)-1,2,3,4-tetrahydrocyclopenta[b]indol-3-yl)essigsäure;
2-(7-(5-(3-(Pyrimidin-5-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(2-Fluorpyridin-3-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(2-Fluorpyridin-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-Isopropoxy-5-(trifluormethyl)phenyl)-1,2,4-oxadiazol-3-yl)-4-methyl-1,2,3,4-tetrahydrocyclopenta[b]indol-3-yl)essigsäure.
2-(7-(5-(4-Ethoxy-3-fluorphenyl)-1,2,4-oxadiazol-3-yl)-1,2,3,4-tetrahydrocyclopenta[b]indol-3-yl)essigsäure;
2-(7-(5-(3-Cyano-4-(trifluormethoxy)phenyl)-1,2,4-oxadiazol-3-yl)-1,2,3,4-tetrahydrocyclopenta[b]indol-3-yl)essigsäure;
2-(7-(5-(3-(2-Methoxypyrimidin-5-yl)-5-(trifluormethoxy)phenyl)-1,2,4-oxadiazol-3-yl)-1,2,3,4-tetrahydrocyclopenta[b]indol-3-yl)essigsäure;
2-(7-(5-(6-(Propylamino)-4-(trifluormethyl)pyridin-2-yl)-1,2,4-oxadiazol-3-yl)-1,2,3,4-tetrahydrocyclopenta[b]indol-3-yl)essigsäure;
2-(7-(5-(6-Morpholino-4-(trifluormethyl)pyridin-2-yl)-1,2,4-oxadiazol-3-yl)-1,2,3,4-tetrahydrocyclopenta[b]indol-3-yl)essigsäure;
2-(7-(5-(2-Chlor-6-methoxypyridin-4-yl)-1,2,4-oxadiazol-3-yl)-1,2,3,4-tetrahydrocyclopenta[b]indol-3-yl)essigsäure;
2-(7-(5-(4-Methoxy-3-(methylsulfonyl)phenyl)-1,2,4-oxadiazol-3-yl)-1,2,3,4-tetrahydrocyclopenta[b]indol-3-yl)essigsäure;
2-(7-(5-(5-Methoxypyrazin-2-yl)-1,2,4-oxadiazol-3-yl)-1,2,3,4-tetrahydrocyclopenta[b]indol-3-yl)essigsäure;
2-(7-(5-(3-(3-Fluorpyridin-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(3,5-Dimethylisoxazol-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(2,6-Difluorpyridin-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(6-Fluor-5-methylpyridin-3-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(1-Methyl-1H-pyrazol-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(2-Methyl pyrid in-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(3-Methyl pyridin-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(3-(Trifluormethoxy)-5-(1,3,5-trimethyl-1H-pyrazol-4-yl)phenyl)-1,2,4-oxadiazol-3-yl)-1,2,3,4-tetrahydrocyclopenta[b]indol-3-yl)essigsäure;
2-(7-(5-(3-(1-Isobutyl-1H-pyrazol-4-yl)-5-(trifluormethoxy)phenyl)-1,2,4-oxadiazol-3-yl)-1,2,3,4-tetrahydrocyclopenta[b]indol-3-yl)essigsäure;
2-(7-(5-(2-Brom-6-methylpyridin-4-yl)-1,2,4-oxadiazol-3-yl)-1,2,3,4-tetrahydrocyclopenta[b]indol-3-yl)essigsäure;
2-(7-(5-(4-(Difluormethoxy)-3-methoxyphenyl)-1,2,4-oxadiazol-3-yl)-1,2,3,4-tetrahydrocyclopenta[b]indol-3-yl)essigsäure;
2-(7-(5-(5-(1-Methyl-1H-pyrazol-4-yl)pyridin-3-yl)-1,2,4-oxadiazol-3-yl)-1,2,3,4-tetrahydrocyclopenta[b]indol-3-yl)essigsäure; und
2-(7-(5-(5-Hexylpyridin-3-yl)-1,2,4-oxadiazol-3-yl)-1,2,3,4-tetrahydrocyclopenta[b]indol-3-yl)essigsäure.

37. Verbindung nach einem der Ansprüche 1 bis 36, worin die Stereochemie für den Ringkohlenstoff der Verbindung R ist.

38. Verbindung nach einem der Ansprüche 1 bis 36, worin die Stereochemie für den Ringkohlenstoff der Verbindung S ist.

39. Verbindung nach Anspruch 1, die
(R)-2-(7-(5-(3-Cyano-5-(trifluormethoxy)phenyl)-1,2,4-oxadiazol-3-yl)-1,2,3,4-tetrahydrocyclopenta[b]indol-3-yl)essigsäure ist,
oder ein pharmazeutisch annehmbares Salz, Solvat oder Hydrat davon.

40. Verbindung nach Anspruch 1, die
(S)-2-(7-(5-(3-Cyano-5-(trifluormethoxy)phenyl)-1,2,4-oxadiazol-3-yl)-1,2,3,4-tetrahydrocyclopenta[b]indol-3-yl)essigsäure ist,
oder ein pharmazeutisch annehmbares Salz, Solvat oder Hydrat davon.

41. Verbindung nach Anspruch 1, die
(S)-2-(7-(5-(3-Cyano-4-isopropoxyphenyl)-1,2,4-oxadiazol-3-yl)-1,2,3,4-tetrahydrocyclopenta[b]indol-3-yl)essigsäure ist,
oder ein pharmazeutisch annehmbares Salz, Solvat oder Hydrat davon.

42. Pharmazeutische Zusammensetzung, die eine Verbindung nach einem der Ansprüche 1 bis 41 und einen pharmazeutisch annehmbaren Träger umfasst.

43. Verfahren zur Herstellung einer Zusammensetzung, umfassend das Vermischen einer Verbindung nach einem der Ansprüche 1 bis 41 und eines pharmazeutisch annehmbaren Trägers.

44. Verbindung nach einem der Ansprüche 1 bis 41 zur Verwendung in einem Verfahren zur Behandlung eines menschlichen Körpers oder Tierkörpers mittels einer Therapie.

45. Verbindung nach einem der Ansprüche 1 bis 41 zur Verwendung in einem Verfahren zur Behandlung einer mit dem S1P1-Rezeptor assoziierten Störung.

46. Verbindung nach einem der Ansprüche 1 bis 41 zur Verwendung in einem Verfahren zur Behandlung einer mit dem S1P1-Rezeptor assoziierten Störung, wobei die mit dem S1P1-Rezeptor assoziierte Störung ausgewählt ist aus: einer durch Lymphozyten vermittelten Erkrankung oder Störung, einer Autoimmunerkrankung oder -störung, einer Entzündungserkrankung oder - störung, Krebs, Psoriasis, rheumatoider Arthritis, Morbus Crohn, Transplantatabstoßung, multipler Sklerose, systemischem Lupus erythematodes, ulzerativer Kolitis, Diabetes Typ 1 und Akne.

47. Verbindung nach einem der Ansprüche 1 bis 41 zur Verwendung in einem Verfahren zur Behandlung einer mit dem S1P1-Rezeptor assoziierten Störung, wobei die mit dem S1P1-Rezeptor assoziierte Störung eine mikrobielle Infektion oder Erkrankung oder eine Virusinfektion oder - erkrankung ist.

48. Verwendung einer Verbindung nach einem der Ansprüche 1 bis 41 bei der Herstellung eines Medikaments zur Behandlung einer mit dem S1 P1-Rezeptor assoziierten Störung.

49. Verwendung einer Verbindung nach einem der Ansprüche 1 bis 41 bei der Herstellung eines Medikaments zur Behandlung eines mit dem S1 P1-Rezeptor assoziierten Störung, wobei die mit dem S1P1-Rezeptor assoziierte Störung ausgewählt ist aus: einer durch Lymphozyten vermittelten Erkrankung oder Störung, einer Autoimmunerkrankung oder -störung, einer Entzündungserkrankung oder -störung, Krebs, Psoriasis, rheumatoider Arthritis, Morbus Crohn, Transplantatabstoßung, multipler Sklerose, systemischem Lupus erythematosus, ulzerativer Kolitis, Diabetes Typ 1 und Akne.

50. Verwendung einer Verbindung nach einem der Ansprüche 1 bis 41 bei der Herstellung eines Medikaments zur Behandlung einer mit dem S1P1-Rezeptor assoziierten Störung, wobei die mit dem S1P1-Rezeptor assoziierte Störung eine mikrobielle Infektion oder Erkrankung oder eine Virusinfektion oder -erkrankung ist.

## Revendications

1. Composé sélectionné parmi des composés de la formule (Ia) et des sels, solvates et hydrates pharmaceutiquement acceptables de celui-ci: 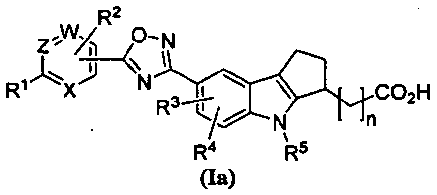 où:
n est 0 ou 1;
W est N ou CR⁶;
Z est N ou CR⁷;
X est N ou CR⁸; à condition que W, Z et X ne soient pas tous N;
R¹, R², R⁶, R⁷ et R⁸ sont chacun indépendamment sélectionnés parmi H, acyleC₁-C₆, acyloxyC₁-C₆, alkoxyC₁-C₆, alkoxycarbonylaminoC₁-C₆, alkyleC₁-C₆, alkynyleC₂-C₆, alkylaminoC₁-C₆, dialkylaminoC₂-C₈, alkylcarboxamideC₁-C₆, alkylsulfonamideC₁-C₆, alkylsulfinyleC₁-C₆, alkylsulfonyleC₁-C₆, alkylthioC₁-C₆, alkyluréyleC₁-C₆, amino, carbo-alkoxyC₁-C₆, carboxamide, carboxy, cyano, cycloalkyleC₃-C₇, cycloalkyloxyC₃-C₇, cycloalkylthioC₃-C₇, cycloalkylsulfinyleC₃-C₇, cycloalkylsulfonyleC₃-C₇, dialkylcarboxamideC₂-C₆, haloalkoxyC₁-C₆, haloalkyleC₁-C₆, haloalkylsulfinyleC₁-C₈, haloalkylsulfonyleC₁-C₆, halogène, hétéroaryle, hétérocyclyle, hydroxyle, nitro et sulfonamide, où alkoxyC₁-C₆, alkylthioC₁-C₆, alkyleC₁-C₆. alkynyleC₂-C₆ et hétéroaryle sont chacun optionnellement substitués par 1, 2 ou 3 substituants sélectionnés indépendamment parmi alcoxyC₁-C₆, carbo-alcoxyC₁-C₆, alkyleC₁-C₆, cyano, cycloalkyleC₃-C₇, halogène et phényle;
R³ et R⁴ sont chacun indépendamment sélectionnés parmi H, alkyleC₁-C₂, fluoro et chloro; et
R⁵ est H ou alkyleC₁-C₆.

2. Composé selon la revendication 1, où R³ et R⁴ sont chacun indépendamment sélectionnés parmi H, CH₃ et F.

3. Composé selon la revendication 1, où R³ et R⁴ sont tous les deux H.

4. Composé selon l'une quelconque des revendications 1 à 3, où R⁵ est H ou méthyle.

5. Composé selon la revendication 1, où R³, R⁴ et R⁵ sont chacun H.

6. Composé selon l'une quelconque des revendications 1 à 5, où n est 0.

7. Composé selon l'une quelconque des revendications 1 à 6, où n est 1.

8. Composé selon l'une quelconque des revendications 1 à 7, où R² est H.

9. Composé selon l'une quelconque des revendications 1 à 8, où R¹, R², R⁶, R⁷ et R⁸ sont chacun indépendamment sélectionné parmi H, alkoxyC₁-C₆, alkyleC₁-C₆,, alkynyleC₂-C₆, , alkylaminoC₁-C₆, dialkylamino C₂-C₈, alkylsulfonyleC₁-C₆, carboxamide, cyano, cycloalkyleC₃-C₇, cycloalkyloxyC₃-C₇, haloalkoxyC₁-C₆,, haloalkyleC₁-C₆, halogène, hétéroaryle, hétérocyclyle et hydroxyle, où alkoxy C₁-C₆, alkyleC₁-C₆, alkynyleC₂-C₆ et hétéroaryle sont optionnellement substitués par 1, 2 ou 3 substituants sélectionnés parmi alkoxyC₁-C₆,, carbo-alkoxyC₁-C₆, alkyleC₁-C₆, cyano, cycloalkyleC₃-C₇, halogène et phényle.

10. Composé selon l'une quelconque des revendications 1 à 8, où R¹, R², R⁶, R⁷ et R⁸ sont chacun indépendamment sélectionnés parmi H, sec-butoxy, isopropoxy, méthoxy, éthoxy, propoxy, méthyle, éthyle, propyle, butyle, tert-butyle, éthynyle, diéthylamino, méthylsulfonyle, carboxamide, cyano, cyclobutyle, cyclopropyle, cyclopentyloxy, trifluorométhoxy, 1,1,1,3,3,3-hexafluoropropan-2-yloxy, 2-fluoroéthoxy, 2,2,2-trifluoroéthoxy, trifluorométhyle, fluoro, chloro, bromo, 1,2,4-triazol-1-yle, 1,2,4-triazol-4-yle, pyridin-3-yle, pyridin-4-yle, pyrimidin-5-yl, pyrrolidin-1-yle, morpholino, hydroxyle, cyclopropylméthoxy, benzyloxy, 2-cyanoéthyle, 6-méthoxypyridin-3-yle, cyclopropyléthynyle, 6-fluoropyridin-3-yle, 1-(tert-butoxycarbonyl)-1H-pyrrol-2-yle, 2-fluoropyridin-4-yle, 2-fluoropyridin-3-yle, 2-methoxypyrimidin-5-yle, propylamino, 3-fluoropyridin-4-yle, 3,5-diméthylisoxazol-4-yle, 2,6-difluoropyridin-4-yle, 6-fluoro-5-méthylpyridin-3-yle, 1-méthyl-1H-pyrazol-4-yle, 2-méthylpyridin-4-yle, 3-méthylpyridin-4-yle, 1,3,5-triméthyl-1H-pyrazol-4-yle, 1-isobutyl-1H-pyrazol-4-yle, difluorométhoxy et hexyle.

11. Composé selon l'une quelconque des revendications 1 à 10, où R¹ est sélectionné parmi H, alkoxyC₁-C₆, alkyleC₁-C₆, alkylaminoC₁-C₆, dialkylaminoC₂-C₈, alkylsulfonyleC₁-C₆, carboxamide, cyano, alkynyle C₂-C₆, cycloalkyle C₃-C₇, cycloalkyloxy C₃-C₇, haloalkoxyC₁-C₆, haloalkyle C₁-C₆, halogène, hétéroaryle, hétérocyclyle et hydroxyle, où alkoxy C₁-C₆, alkyle C₁-C₆, alkynyle C₂-C₆, et hétéroaryle sont optionnellement substitués par 1, 2 ou 3 substituants sélectionnés parmi alcoxyC₁-C₆, carbo-alcoxyC₁-C₆, alkyleC₁-₆, cyano, halogène, cycloalkyleC₃-C₇ et phényle.

12. Composé selon l'une quelconque des revendications 1 à 10, où R¹ est sélectionné H, sec-butoxy, isopropoxy, méthoxy, éthoxy, méthyle, éthyle, propyle, butyle, diéthylamino, méthylsulfonyle, carboxamide, cyano, cyclopentyloxy, trifluorométhoxy, 1,1,1,3,3,3-hexafluoropropan-2-yloxy, 2-fluoroéthoxy, 2,2,2-trifluoroéthoxy, trifluorométhyle, fluoro, chloro, bromo, 1,2,4-triazol-1-yle, pyridin-3-yle, pyrrolidin-1-yle, morpholino, hydroxyle, cyclopropylméthoxy, benzyloxy, 6-methoxypyridin-3-yle et 1-(tert-butoxycarbonyl)-1H-1-pyrrol-2yle.

13. Composé selon l'une quelconque des revendications 1 à 12, où R⁶ est sélectionné parmi H, alkoxyC₁-C₆, alkyleC₁-C₆, alkylsulfonyleC₁-C₆, carboxamide, cyano, alkynyleC₂-C₆, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alcoxy C₁-C₆, alkyleC₁-C₆, alkynyleC₂-C₆ et hétéroaryle sont optionnellement substitués par 1, 2 ou 3 substituants sélectionnés parmi alkoxyC₁-C₆, carbo-alcoxyC₁-C₆, alkyleC₁-C₆, cyano, halogène, cycloalkyleC₃-C₇ et phényle.

14. Composé selon l'une quelconque des revendications 1 à 12, où R⁶ est sélectionné parmi H, sec-butoxy, isopropoxy, méthoxy, éthoxy, méthyle, éthyle, propyle, butyle, diéthylamino, méthylsulfonyle, carboxamide, cyano, cyclopentyloxy, trifluorométhoxy, 1,1,1,3,3,3-hexafluoro-propan-2-yloxy, 2-fluoroéthoxy, 2,2,2-trifluoroéthoxy, trifluorométhyle, fluoro, chloro, bromo, 1,2,4-triazol-1-yle, pyridin-3-yle, pyrrolidin-1-yle, morpholino, hydroxyle, cyclopropylméthoxy, benzyloxy, 6-méthoxypyridin-3-yle et 1-(tert-butoxycarbonyl)-1H-pyrrol-2-yle.

15. Composé selon l'une quelconque des revendications 1 à 12, où R⁶ est H.

16. Composé selon l'une quelconque des revendications 1 à 16, où R⁷ est sélectionné parmi H, alcoxyC₁-C₆, alkyleC₁-C₆, alkynyleC₂-C₆, alkylsulfonyleC₁-C₆, carboxamide, cyano, cycloalkyleC₃-C₇, cycloalkyloxy C₃-C₇, haloalkoxyC₁-C₆, haloalkyleC₁-C₆, halogène, hétéroaryle, hétérocyclyle et hydroxyle, où alkoxy C₁-C₆, alkyleC₁-C₆, alkynyleC₂-C₆ et hétéroaryle sont optionnellement substitués par 1, 2 ou 3 substituants sélectionnés parmi alcoxyC₁-C₆, alkyleC₁-C₆, cyano, carboalcoxyC₁-C₆, cycloalkyleC₃-C₇, halogène et phényle.

17. Composé selon l'une quelconque des revendications 1 à 16, où R⁷ est sélectionné parmi H, sec-butoxy, isopropoxy, méthoxy, éthoxy, propoxy, méthyle, méthylsulfonyle, carboxamide, cyano, cyclobutyle, cyclopropyle, cyclopentyloxy, trifluorométhoxy, 1,1,1,3,3,3-hexafluoropropan-2-yloxy, 2-fluoroéthoxy, 2,2,2-trifluoroéthoxy, trifluorométhyle, fluoro, chloro, bromo, 1,2,4-triazol-1-yle, 1, 2,4-triazol-4-yle, pyridin-3-yle, pyridin-4-yle, pyrimidin-5-yle, pyrrolidin-1-yle, morpholino, hydroxyle, cyclopropylméthoxy, benzyloxy, 2-cyanoéthyle, 6-methoxypyridin-3-yle, cyclopropyléthynyle, 6-fluoropyridin-3-yle, 1-(tert-butoxycarbonyl)-1H-pyrrol-2-yle, 2-fluoropyridin-4-yle et 2-fluoropyridin-3-yle.

18. Composé selon l'une quelconque des revendications 1 à 17, où R⁸ est sélectionné parmi H, alkoxyC₁-C₆, alkyle C₁-C₆, alkynyleC₂-C₆, alkylsulfonyleC₁-C₆, carboxamide, cyano, cycloalkyleC₃-C₇, cycloalkyloxy C₃-C₇, haloalkoxyC₁-C₆, haloalkyle C₁-C₆, halogène, hétéroaryle, hétérocyclyle et hydroxyle, où alcoxyC₁-C₆, alkyleC₁-C₆, alkynyleC₂-C₆, et hétéroaryle sont optionnellement substitués par 1, 2 ou 3 substituants sélectionnés parmi alkoxyC₁-C₆, alkyleC₁-C₆, carboalkoxy C₁-C₆, cyano, cycloalkyleC₃-C₇, halogène et phényle.

19. Composé selon l'une quelconque des revendications 1 à 17, où R⁸ est sélectionné parmi H, isopropoxy, méthoxy, éthoxy, propoxy, méthyle, éthyle, propyle, tert-butyle, éthynyle, méthylsulfonyle, carboxamide, cyano, cyclobutyle, cyclopropyle, cyclopentyloxy, trifluorométhoxy, 2-fluoroéthoxy, 2,2,2-trifluoroéthoxy, trifluorométhyle, fluoro, chloro, bromo, 1,2,4-triazol-1-yle, 1,2,4-triazol-4-yle, pyridin-3-yle, pyridin-4-yle, pyrimidin-5-yle, pyrrolidin-1-yle, morpholino, hydroxyle, cyclopropylméthoxy, benzyloxy, 2-cyanoéthyle, 6-méthoxypyridin-3-yle, cyclopropyléthynyle, 6-fluoropyridin-3-yle, 1-(tert-butoxycarbonyl)-1H-pyrrol-2-yle, 2-fluoropyridin-4-yle et 2-fluoropyridin-3-yle.

20. Composé selon l'une quelconque des revendications 1 à 8, où R¹, R², R⁶, R⁷ et R⁸ sont chacun indépendamment sélectionnés parmi H, acyleC₁-C₆, acyloxyC₁-C₆, alcoxyC₁-C₆, alkoxycarbonylaminoC₁-C₆, alkyleC₁-C₆, alkynyle C₂-C₈, alkylaminoC₁-C₆, dialkylaminoC₂-C₈, alkylcarboxamideC₁-C₆, alkylsulfonamideC₁-C₈, alkylsulfinyleC₁-C₆, alkylsulfonyleC₁-C₆, alkylthioC₁-C₆, alkyluréyleC₁-C₆, amino, carboalkoxyC₁-C₆, carboxamide, carboxy, cyano, cycloalkyleC₃-C₇, cycloalkyloxyC₃-C₇, cycloalkylthio C₃-C₇, cycloalkylsul-finyleC₃-C₇, cycloalkylsulfonyleC₃-C₇, dialkylcarboxamideC₂-C₆, haloalkoxyC₁-C₆, haloalkyleC₁-C₆, haloalkylsulfinyleC₁-C₆, haloalkylsulfonyleC₁-C₆, halogène, hétéroaryle, hétérocyclyle, hydroxyle, nitro et sulfonamide, où alkoxyC₁-C₆, alkylthioC₁-C₆, alkyleC₁-C₆, alkynyleC₂-C₆, et hétéroaryle sont optionnellement substitués par 1 substituant sélectionné parmi alcoxyC₁-C₆, carboalkoxyC₁-C₆, cyano, cycloalkyle C₃-C₇, halogène et phényle.

21. Composé selon l'une quelconque des revendications 1 à 8, où R¹, R², R⁶, R⁷ et R⁸ sont chacun indépendamment sélectionnés parmi H, alcoxyC₁-C₈, alkyleC₁-C₈, alkynyleC₂-C₈, dialkylaminoC₂-C₈, alkylsulfonyleC₁-C₈, carboxamide, cyano, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alcoxyC₁-C₆, alkyleC₁-C₆, alkynyleC₂-C₆ et hétéroaryle sont optionnellement substitués par un substituant sélectionné parmi alcoxyC₁-C₆, carboalcoxyC₁-C₆, cyano, cycloalkyleC₃-C₇, halogène et phényle.

22. Composé selon l'une quelconque des revendications 1 à 8, où R¹, R², R³, R⁶, R⁷ et R⁸ sont chacun indépendamment sélectionnés parmi H, sec-butoxy, isopropoxy, méthoxy, éthoxy, propoxy, méthyle, éthyle, propyle, butyle, tert-butyle, éthynyle, diéthylamino, méthylsulfonyle, carboxamide, cyano, cyclobutyle, cyclopropyle, cyclopentyloxy, trifluoro-méthoxy, 1,1,1,3,3, 3-hexafluoropropan-2-yloxy, 2-fluoroéthoxy, 2,2,2-trifluoroéthoxy, trifluorométhyle, fluoro, chloro, bromo, 1,2,4-triazol-1-yle, 1,2,4-triazol-4-yle, pyridin-3-yle, pyridin-4-yle, pyrimidin-5-yle, pyrrolidin-1-yle, morpholino, hydroxyle, cyclopropylméthoxy, benzyloxy, 2-cyanoéthyle, 6-méthoxy-pyridin-3-yle, cyciopropyléthynyle, 6-fluoropyridin-3-yle, 1-(tert-butoxycarbonyl)-1H-pyrrol-2-yle, 2-fluoropyridin-4-yle et 2-fluoropyridin-3-yle.

23. Composé selon l'une quelconque des revendications 1 à 8 et 20 à 22, où R¹ est sélectionné parmi H, alkoxyC₁-C₆, alkyleC₁-C₆, dialkylaminoC₂-C₈, alkylsulfonyleC₁-C₆, carboxamide, cyano, cycloalkyloxyC₃-C₇, haloalkoxyC₁-C₆, halogène, hétéroaryle, hétérocyclyle et hydroxyle, où alkoxyC₁-C₆ et hétéroaryle sont optionnellement substitués par un substituant sélectionné parmi alcoxyC₁-C₆, carbo-alcoxyC₁-C₆, et cycloalkyleC₃-C₇.

24. Composé selon l'une quelconque des revendications 1 à 8 et 20 à 23, où R⁶ est sélectionné parmi H, alkoxyC₁-C₆, alkyleC₁-C₆, dialkylaminoC₂-C₈, alkylsulfonyleC₁-C₆, carboxamide, cyano, cycloalkyloxyC₃-C₇, haloalkoxyC₁-C₆, halogène, hétéroaryle, hétérocyclyle et hydroxyle, où alkoxyC₁-C₆ et hétéroaryle sont optionnellement substitués par un substituant sélectionné parmi alcoxyC₁-C₆, carbo-alcoxyC₁-C₆, cycloalkyleC₃-C₇ et phényle.

25. Composé selon l'une quelconque des revendications 1 à 8 et 20 à 24, où R⁷ est sélectionné parmi H, alcoxyC₁-C₆, alkyleC₁-C₆, alkynyleC₂-C₆, dialkylaminoC₂-C₈, alkylsulfonyleC₁-C₆, carboxamide, cyano, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyleC₂-C₆ et hétéroaryle sont optionnellement substitués par 1 substituant sélectionné parmi alcoxyC₁-C₆, cyano, carbo-alcoxyC₁-C₆, cycloalkyleC₃-C₇, halogène et phényle.

26. Composé selon l'une quelconque des revendications 1 à 8 et 20 à 25, où R⁸ est sélectionné parmi H, alkoxyC₁-C₆, alkyleC₁-C₆, alkynyleC₂-C₆, alkylsulfonyleC₁-C₆, carboxamide, cyano, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alkoxyC₁-C₆ et hétéroaryle sont optionnellement substitués par 1 substituant sélectionné parmi alcoxyC₁-C₆, carbo-alcoxyC₁-C₆, cyano, cycloalkyleC₃-C₇, halogène et phényle.

27. Composé selon l'une quelconque des revendications 1 à 26, où
W est CR⁶;
Z est CR⁷; et
X est CR⁸.

28. Composé selon la revendication 1, sélectionné parmi les composés de la Formule (Ig) et de sels, solvates et hydrates pharmaceutiquement acceptables de celui-ci: 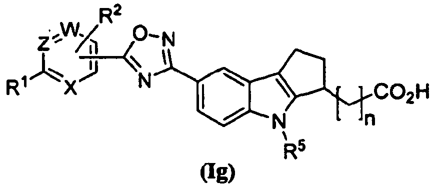 où:
n est 0 ou 1;
W est N ou CR⁶;
Z est N ou CR⁷;
X est N ou CR⁸; à condition que W, Z et X ne soient pas tous N;
R¹ est sélectionné par H, alkoxyC₁-C₆, alkyleC₁-C₆, alkylaminoC₁-C₆,, dialkylaminoC₂-C₈, alkylsulfonyleC₁-C₆,, carboxamide, cyano, alkynyleC₂-C₆, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyleC₂-C₆ et hétéroaryle sont optionnellement substitués par 1, 2 ou 3 substituants sélectionnés parmi alkoxyC₁-C₆,, carbo-alkoxyC₁-C₆, alkyleC₁-C₆, cyano, halogène, cycloalkyle C₃-C₇ et phényle;
R² is H;
R⁵ est H ou alkyleC₁-C₆;
R⁶ est sélectionné parmi H, alkoxyC₁-C₆, alkyle C₁-C₆, alkylsulfonyleC₁-C₆,, carboxamide, cyano, alkynyleC₂-C₈, cycloalkyleC₃-C₇, cycloalkyloxy C₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyle C₂-C₆ et hétéroaryle sont optionnellement substitués par 1, 2 ou 3 substituants sélectionnés parmi alkoxyC₁-C₆, carbo-alkoxyC₁-C₆, alkyleC₁-C₆, cyano, halogène, cycloalkyleC₃-C₇ et phényle;
R⁷ est sélectionné parmi H, alkoxyC₁-C₆, alkyleC₁-C₆, alkynyleC₂-C₈, alkylsulfonyleC₁-C₆, carboxamide, cyano, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyleC₂-C₆ et hétéroaryle sont optionnellement substitués par 1, 2 ou 3 substituants sélectionnés parmi alkoxyC₁-C₆, alkyle C₁-C₆, cyano, carbo-alkoxyC₁-C₆, cycloalkyleC₃-C₇, halogène et phényle; et
R⁸ est sélectionné parmi H, alkoxyC₁-C₆, alkyleC₁-C₆, alkynyle C₂-C₆, alkylsulfonyleC₁-C₆, carboxamide, cyano, cycloalkyle C₃-C₇, cycloalkyloxy C₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyleC₂-C₆, et hétéroaryle sont optionnellement substitués par 1, 2 ou 3 substituants sélectionnés parmi alkoxyC₁-C₈, alkyleC₁-C₆, carbo-alkoxyC₁-C₆, cycloalkyleC₁-C₆, cyano, cycloalkyleC₃-C₇, halogène et phényle.

29. Composé selon la revendication 1, sélectionné parmi des composés de la Formule (Ig) et de sels, solvates et hydrates pharmaceutiquement acceptables de celui-ci: 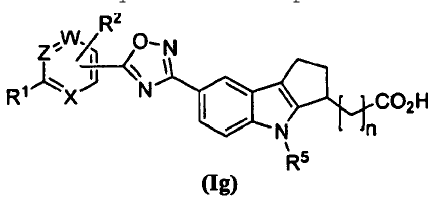 où:
n 0 ou 1;
W est N ou CR⁶;
Z est N ou CR⁷;
X est N ou CR⁸; à condition que W, Z et X ne soient pas tous N;
R¹ est sélectionné parmi H, alkoxyC₁-C₆, alkyleC₁-C₆, dialkylaminoC₂-C₈, alkylsulfonyleC₁-C₆, carboxamide, cyano, cycloalkyloxyC₃-C₇, haloalkoxyC₁-C₆, haloalkyleC₁-C₆, halogène, hétéroaryle, hétérocyclyle et hydroxyle, où alkoxyC₁-C₆ et hétéroaryle sont optionnellement substitués par 1 substituant sélectionné parmi alkoxyC₁-C₆, carbo-alkoxyC₁-C₆ et cycloalkyleC₃-C₇;
R² is H;
R⁵ est H ou alkyleC₁-C₆;
R⁶ est sélectionné parmi H, alkoxyC₁-C₆, alkyleC₁-C₆, dialkylaminoC₂-C₈, alkylsulfonyleC₁-C₆, carboxamide, cyano, cycloalkyloxyC₃-C₇, haloalkoxyC₁-C₆, haloalkyleC₁-C₆, halogène, hétéroaryle, hétérocyclyle et hydroxyle, où alkoxyC₁-C₆ et hétéroaryle sont optionnellement substitués par 1 substituant sélectionné parmi alkoxyC₁-C₆, carbo-alkoxyC₁-C₆, cycloalkyle C₃-C₇ et phényle;
R⁷ est sélectionné parmi H, alkoxyC₁-C₆, alkyleC₁-C₆, alkynyleC₂-C₈, dialkylaminoC₂-C₈, alkylsulfonyleC₁-C₆, carboxamide, cyano, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyleC₂-C₆ et hétéroaryle sont optionnellement substitués par 1 substituant sélectionné parmi alkoxyC₁-C₆, cyano, carbo-alkoxyC₁-C₆, cycloalkyleC₃-C₇, halogène et phényle; et
R⁸ est sélectionné parmi H, alkoxyC₁-C₆, alkyleC₁-C₆, alkynyleC₁-C₆, alkylsulfonyleC₁-C₆, carboxamide, cyano, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyleC₂-C₆ et hétéroaryle sont optionnellement substitués par 1 substituant sélectionné parmi alkoxyC₁-C₆, carbo-alkoxyC₁-C₆, cyano, cycloalkyleC₃-C₇, halogène et phényle.

30. Composé selon la revendication 1, sélectionné parmi des composés de la Formule (IIa) et de sels, solvates et hydrates pharmaceutiquement acceptables de celui-ci: 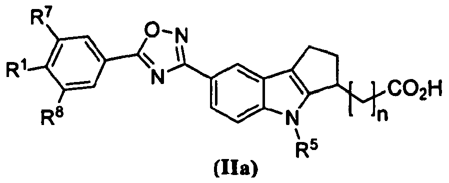 où:
n est 0 ou 1;
R¹ est sélectionné parmi H, alkoxyC₁-C₆, cyano, cycloalkyloxyC₃-C₇, haloalkoxyC₁-C₆, halogène et hydroxyle, où alkoxyC₁-C₆ est optionnellement substitué par 1, 2 ou 3 substituants sélectionnés parmi halogène et cycloalkyleC₃-C₇;
R⁵ est H ou alkyleC₁-C₆;
R⁷ est sélectionné parmi H, alkoxyC₁-C₆, alkyleC₁-C₆, alkynyleC₂-C₈, alkylsulfonyleC₁-C₆, carboxamide, cyano, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyleC₂-C₆ et hétéroaryle sont optionnellement substitués par 1, 2 ou 3 substituants sélectionnés parmi alkoxyC₁-C₆, alkyleC₁-C₆, cyano, carbo-alkoxyC₁-C₆, cycloalkyleC₃-C₇, halogène et phényle; et
R⁸ est sélectionné parmi H, alkoxyC₁-C₆, alkyleC₁-C₆, alkynyleC₂-C₆, alkylsulfonyleC₁-C₆, carboxamide, cyano, cycloalkyleC₃-C₇, cycloalkyloxyC₃-C₇, haloalkoxyC₁-C₆, haloalkyleC₁-C₆, halogène, hétéroaryle, hétérocyclyle et hydroxyle, où alkoxyC₁-C₆, alkyleC₁-C₆, alkynyleC₂-C₆, et hétéroaryle sont optionnellement substitués par 1, 2 ou 3 substituants sélectionnés parmi alkoxyC₁-C₆, alkyleC₁-C₆, cyano, carbo-alcoxyC₁-C₆, cycloalkyleC₃-C₇, halogène et phényle.

31. Composé selon la revendication 1, sélectionné parmi des composés de la Formule (IIa) et de sels, solvates et hydrates pharmaceutiquement acceptables de celui-ci: 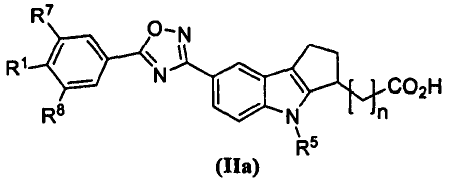 où:
n est 0 ou 1;
R¹ est sélectionné parmi H, isopropoxy, methoxy, éthoxy, cyano, cyclopentyloxy, trifluorométhoxy, 1,1,1,3,3,3-hexafluoropropan-2-yloxy, fluoro, hydroxyle, cyclopropylméthoxy, et difluorométhoxy;
R⁵ est H ou méthyle;
R⁷ est sélectionné parmi H, isopropoxy, méthoxy, éthoxy, propoxy, méthyle, éthyle, propyle, tert-butyle, éthynyle, méthylsulfonyle, carboxamide, cyano, cyclobutyle, cyclopropyle, cyclopentyloxy, trifluorométhoxy, 2-fluoroéthoxy, 2,2,2-trifluoroéthoxy, trifluorométhyle, fluoro, chloro, bromo, 1,2,4-triazol-1-yle, 1,2,4-triazol-4-yle, pyridin-3-yle, pyridin-4-yle, pyrimidin-5-yle, pyrrolidin-1-yle, morpholino, hydroxyle, cyclopropylméthoxy, benzyloxy, 2-cyanoéthyle, 6-méthoxypyridin-3-yle, cyclopropyléthynyle, 6-fluoropyridin-3-yle, 1-(tert-butoxycarbonyl)-1H-pyrrol-2-yle, 2-fluoropyridin-4-yle, 2-fluoropyridin-3-yle, 2-méthoxypyrimidin-5-yle, 3-fluoropyridin-4-yle, 3,5-diméthylisoxazol-4-yle, 2, 6-difluoropyridin-4-yle, 6-fluoro-5-méthylpyridin-3-yle, 1-méthyl-1H-pyrazol-4-yle, 2-méthylpyridin-4-yle, 3-methylpyridin-4-yle, 1,3,5-triméthyl-1H-pyrazol-4-yle et 1-isobutyl-1H-pyrazol-4-yle; et
R⁸ est sélectionné parmi H, isopropoxy, méthoxy, éthoxy, propoxy, méthyle, éthyle, propyl, tert-butyle, éthynyle méthylsulfonyle, carboxamide, cyano, cyclobutyle cyclopropyle, cyclopentyloxy, trifluorométhoxy, 2-fluoroéthoxy, 2,2,2-trifluoroéthoxy, trifluorométhyle, fluoro, chloro, bromo, 1,2,4-triazol-1-yle, 1,2,4-triazol-4-yle, pyridin-3-yle, pyridin-4-yle, pyrimidin-5-yle, pyrrolidin-1-yle, morpholino, hydroxyle, cyclopropylméthoxy, benzyloxy, 2-cyanoéthyle, 6-méthoxypyridin-3-yle, cyclopropyléthynyle, 6-fluoropyridin-3-yle, 1-(tert-butoxycarbonyl)-1H-pyrrol-2-yle, 2-fluoropyridin-4-yle, 2-fluoropyridin-3-yle, 2-méthoxypyrimidin-5-yle, 3-fluoropyridin-4-yle, 3,5-diméthylisoxazol-4-yle, 2,6-difluoropyridin-4-yle, 6-fluoro-5-méthylpyridin-3-yle, 1-méthyl-1H-pyrazol-4-yle, 2-méthylpyridin-4-yle, 3-méthylpyridin-4-yle, 1,3,5-triméthyl-1H-pyrazol-4-yle et 1-isobutyl-1H-pyrazol-4-yle.

32. Composé selon la revendication 1, sélectionné parmi des composés de la Formule (IIa) et de sels, solvates et hydrates pharmaceutiquement acceptables de celui-ci: 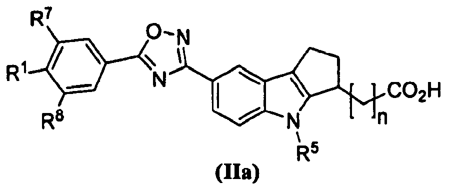 où:
n est 0 ou 1;
R¹ est sélectionné parmi H, alcoxyC₁-C₆, cyano, cycloalkyloxyC₃-C₇, haloalkoxyC₁-C₆ et hydroxyle, où alkoxyC₁-C₆ est optionnellement substitué par 1 substituant sélectionné parmi alkoxyC₁-C₆;
R⁵ est H ou alkyleC₁-C₆;
R⁷ est sélectionné parmi H, alkoxyC₁-C₆, alkyleC₁-C₆, alkylsulfonyleC₁-C₆, cyano, cycloalkyloxyC₃-C₇, haloalkoxyC₁-C₆, haloalkyleC₁-C₆, halogène, hétéroaryle, hétérocyclyle, où alkoxyC₁-C₆ et hétéroaryle sont optionnellement substitués par 1 substituant sélectionné parmi alcoxyC₁-C₆, carbo-alcoxyC₁-C₆ et phényle; et
R⁸ est sélectionné parmi H, alkoxyC₁-C₆, alkyleC₁-C₆, alkynyleC₂-C₆, alkylsulfonyleC₁-C₆, carboxamide, cyano, cycloalkyleC₃-C₇, haloalkoxyC₁-C₆, haloalkyleC₁-C₆, halogène, hétéroaryle et hydroxyle, où alkoxyC₁-C₆, alkyleC₁-C₆, alkynyleC₂-C₆ et hétéroaryle sont optionnellement substitués par 1 substituant sélectionné parmi cyano, cycloalkyleC₃-C₇, halogène et phényle.

33. Composé selon la revendication 1, sélectionné parmi des composés de la Formule (IIa) et de sels, solvates et hydrates pharmaceutiquement acceptables de celui-ci: 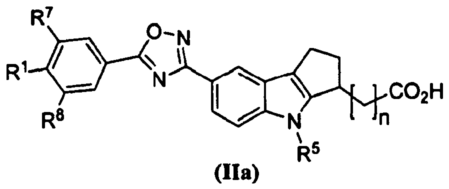 où:
n est 0 ou 1;
R¹ est sélectionné parmi H, isopropoxy, méthoxy, éthoxy, cyano, cyclopentyloxy, trifluorométhoxy, 1,1,1,3,3,3-hexafluoropropan-2-yloxy, hydroxyle et cyclopropylméthoxy;
R⁵ est H ou méthyle;
R⁷ est sélectionné parmi H, isopropoxy, méthoxy, éthoxy, méthyle, éthyle, propyle, méthylsulfonyle, cyano, cyclopentyloxy, trifluorométhoxy, 2-fluoroéthoxy, 2,2,2-trifluoroéthoxy, trifluorométhyle, fluoro, chloro, bromo, 1,2,4-triazol-1-yle, 1,2,4-triazol-4-yle, pyridin-3-yle, pyrimidin-5-yle, pyrrolidin-1-yle, morpholino, benzyloxy, 6-methoxypyridin-3-yle et 1-(tert-butoxycarbonyl)-1H-pyrrol-2-yle; et
R⁸ est sélectionné parmi H, isopropoxy, méthoxy, éthoxy, propoxy, méthyle, tert-butyle, éthynyle, méthylsulfonyle, carboxamide, cyano, cyclobutyle, cyclopropyle, trifluorométhoxy, trifluorométhyle, fluoro, chloro, bromo, pyridin-4-yle, hydroxyle, cyclopropylméthoxy, benzyloxy, 2-cyanoéthyle, cyclopropyléthynyle, 6-fluoropyridin-3-yle, 2-fluoropyridin-4-yle et 2-fluoropyridin-3-yle.

34. Composé selon la revendication 1, sélectionné parmi des composés de la Formule (IIc) et de sels, solvates et hydrates pharmaceutiquement acceptables de celui-ci: 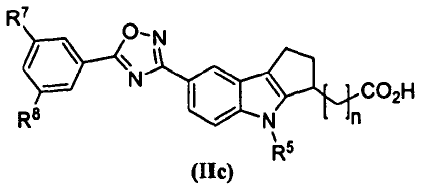 où:
n est 0 ou 1;
R⁵ est H ou méthyle;
R⁷ est sélectionné parmi H, isopropoxy, méthoxy, éthoxy, propoxy, méthyle, éthyle, propyle, tert-butyle, éthynyle, méthylsulfonyle, carboxamide, cyano, cyclobutyle, cyclopropyle, cyclopentyloxy, trifluorométhoxy, 2-fluoroéthoxy, 2,2,2-trifluoroéthoxy, trifluorométhyle, fluoro, chloro, bromo, 1,2,4-triazol-1-yle, 1,2,4-triazol-4-yle, pyridin-3-yle, pyridin-4-yle, pyrimidin-5-yle, pyrrolidin-1-yle, morpholino, hydroxyle, cyclopropylméthoxy, benzyloxy, 2-cyanoéthyle, 6-methoxypyridin-3-yle, cyclopropyléthynyle, 6-fluoropyridin-3-yle, 1-(tert-butoxycarbonyl)-1H-pyrrol-2-yle, 2-fluoropyridin-4-yle, 2-fluoropyridin-3-yle, 2-méthoxypyrimidin-5-yle, 3-fluoropyridin-4-yle, 3,5-diméthylisoxazol-4-yle, 2,6-difluoropyridin-4-yle, 6-fluoro-5-méthylpyridin-3-yle, 1-méthyl-1H-pyrazol-4-yl-méthylpyridin-4-yle, 3-méthylpyridin-4-yle, 1,3,5-triméthyl-1H-pyrazol-4-yle et 1-isobutyl-1H-pyrazol-4-yle; et
R⁸ est sélectionné parmi H, isopropoxy, méthoxy, éthoxy, propoxy, méthyle, éthyle, propyle, tert-butyle, éthynyle, méthylsulfonyle, carboxamide, cyano, cyclobutyle, cyclopropyle, cyclopentyloxy, trifluorométhoxy, 2-fluoroéthoxy, 2,2,2-trifluoroéthoxy, trifluorométhyle, fluoro, chloro, bromo, 1,2,4-triazol-1-yle, 1,2,4-triazol-4-yle, pyridin-3-yle,
pyridin-4-yle, pyrimidin-5-yle, pyrrolidin-1-yle, morpholino, hydroxyle, cyclopropylméthoxy, benzyloxy, 2-cyanoéthyle, 6-méthoxypyridin-3-yle, cyclopropyléthynyle, 6-fluoropyridin-3-yle, 1-(tert-butoxycarbonyl)-1H-pyrrol-2-yle, 2-fluoropyridin-4-yle, 2-fluoropyridin-3-yle, 2-méthoxypyrimidin-5-yle, 3-fluoropyridin-4-yle, 3,5-diméthylisoxazol-4-yle, 2,6-difluoropyridin-4-yle, 6-fluoro-5-méthylpyridin-3-yle, 1-méthyl-1H-pyrazol-4-yle, 2-méthylpyridin-4-yle, 3-méthylpyridin-4-yle, 1,3,5-triméthyl-1H-pyrazol-4-yle et 1-isobutyl-1H-pyrazol-4-yle.

35. Composé selon la revendication 1, sélectionné parmi des composés de la Formule (IIc) et de sels, solvates et hydrates pharmaceutiquement acceptables de celui-ci: 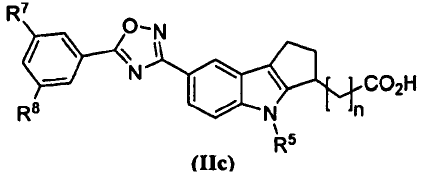 où:
n est 0 ou 1;
R⁵ est H ou méthyle;
R⁷ est sélectionné parmi H, alkoxyC₁-C₆, alkyleC₁-C₆,, alkylsulfonyleC₁-C₆, cyano, cycloalkyloxyC₃-C₇, haloalkoxyC₁-C₆, haloalkyleC₁-C₆, halogène, hétéroaryle, hétérocyclyle, où alcoxyC₁-C₆ et hétéroaryle sont optionnellement substitués par 1 substituant sélectionné parmi alcoxyC₁-C₆, carbo-alcoxyC₁-C₆ et phényle; et
R⁸ est sélectionné parmi H, alcoxyC₁-C₆, alkyleC₁-C₆, alkynyle C₂-C₆, alkylsulfonyleC₁-C₆, carboxamide, cyano, cycloalkyleC₃-C₇, haloalkoxyC₁-C₆, haloalkyleC₁-C₆, halogène, hétéroaryle et hydroxyle, où alkoxyC₁-C₆, alkyleC₁-C₆, alkynyleC₂-C₆ et hétéroaryle sont optionnellement substitués par un substituant sélectionné parmi cyano, cycloalkyleC₃-C₇, halogène et phényle.

36. Composé selon la revendication 1, sélectionné parmi les composés suivants et sels, solvates et hydrates pharmaceutiquement acceptables de celui-ci:
acide 2-(7-(5-(6-sec-butoxy-5-méthylpyridin-3-yl)-1,2,4-oxadiazol-3-yl)-1,2,3,4-tétrahydrocyclopenta[b]indol-3-yl)acétique;
acide 2-(7-(5-(pyridazin-3-yl)-1,2,4-oxadiazol-3-yl)-1,2,3,4-tétrahydrocyclopenta[b]indol-3-yl)acétique;
acide 2-(7-(5-(3-cyano-5-isopropoxyphényl)-1,2,4-oxadiazol-3-yl)-1,2,3,4-tétrahydrocyclopenta[b]indol-3-yle acétique;
acide 2-(7-(5-(3,5-difluorophényl)-1,2,4-oxadiazol-3-yl)-1,2,3,4-tétrahydrocyclopenta[b]indol-3-yl) acétique;
acide 2-(7-(5-(3-cyano-5-méthoxyphényl)-1,2,4-oxadiazol-3-yl)-1,2,3,4-tétrahydrocyclopenta[b]indol-3-yle acétique;
acide 2-(7-(5-(3-éthynyl-5-(trifluorométhoxy) phényl)-1,2,4-oxadiazol-3-yl)-1,2,3,4-tétrahydrocyclopenta [b]indol-3-yle acétique;
acide 2-(7-(5-(3-cyano-5-(trifluorométhoxy) phényl)-1,2,4-oxadiazol-3-yl)-1,2,3,4-tétrahydrocyclopenta [b]indol-3-yle acétique;
acide 2-(7-(5-(3-méthoxy-5-(trifluorométhyl)phényl)-1,2,4-oxadiazol-3-yl)-1,2,3,4-tétrahydrocyclopenta[b]indol-3-yl) acétique;
acide 2-(7-(5-(3-cyclobutyl-5-(trifluorométhoxy)phényl)-1,2,4-oxadiazol-3-yl)-1,2,3,4-tétrahydrocyclopenta[b]indol-3-yle acétique;
acide 2-(7-(5-(3-cyano-4-méthoxyphényl)-1,2,4-oxadiazol-3-yl)-1,2,3,4-tétrahydrocyclopenta[b]indol-3-yle acétique;
acide 2-(7-(5-(6-(trifluorométhyl)pyridin-3-yl)-1,2,4-oxadiazol-3-yl)-1,2,3,4-tétrahydrocyclopenta[b]indol-3-yle acétique;
acide 2-(7-(5-(3-fluoro-5-(trifluorométhyl) phényl)-1,2,4-oxadiazol-3-yl)-1,2,3,4-tétrahydrocyclopenta[b]indol-3-yle acétique;
acide 2-(7-(5-(4-méthoxy-3-méthylphényl)-1,2,4-oxadiazol-3-yl)-1,2,3,4-tétrahydrocyclopenta[b]indol-3-yle acétique;
acide 2-(7-(5-(3-bromo-5-(trifluorométhyl)phényl)-1,2,4-oxadiazol-3-yl)-1,2,3,4-tétrahydrocyclopenta[b] indol-3-yl) acétique;
acide 2-(7-(5-(3-cyano-4-isopropoxyphényl)-1,2,4-oxadiazol-3-yl)-1,2,3,4-tétrahydrocyclopenta[b]indol-3-yl) acétique;
acide 2-(7-(5-(4-(trifluorométhoxy)phényl)-1,2,4-oxadiazol-3-yl)-1,2,3,4-tétrahydrocyclopenta[b]indol-3-yl) acétique;
acide 2-(7-(5-(3-bromo-5-chlorophényl)-1,2,4-oxadiazol-3-yl)-1,2,3,4-tétrahydrocyclopenta[b]indol-3-yl) acétique;
acide 2-(7-(5-(3-(1H-1,2,4-triazol-1-yl)-5-(trifluorométhoxy)phényl)-1,2,4-oxadiazol-3-yl)-1,2,3,4-tétrahydrocyclopenta[b]indol-3-yl) acétique;
acide 2-(7-(5-(3-cyano-4-(cyclopentyloxy)phényl)-1,2,4-oxadiazol-3-yl)-1,2,3,4-tétrahydrocyclopenta [b]indol-3-yl) acétique;
acide 2-(7-(5-(3-(pyrrolidin-1-yl)-5-(trifluorométhoxy)phényl)-1,2,4-oxadiazol-3-yl)-1,2,3,4-tétrahydrocyclopenta[b]indol-3-yl) acétique;
acide 2-(7-(5-(3-cyano-5-éthoxyphényl)-1,2,4-oxadiazol-3-yl)-1,2,3,4-tétrahydrocyclopenta[b]indol-3-yl) acétique;
Acide 2-(7-(5-(3-(benzyloxy)phényl)-1,2,4-oxadiazol-3-yl)-1,2,3,4-tétrahydrocyclopenta[b]indol-3-yle acétique;
acide 2-(7-(5-(5-chloro-6-méthoxypyridin-3-yl)-1,2,4-oxadiazol-3-yl)-1,2,3,4-tétrahydrocyclopenta[b]indol-3-yle acétique;
acide 2-(7-(5-(3-propoxy-5-(trifluorométhyl)phényl)-1,2,4-oxadiazol-3-yl)-1,2,3,4-tétrahydrocyclopenta[b]indol-3-yle acétique;
acide 2-(7-(5-(3-morpholino-5-(trifluorométhoxy)phényl)-1,2,4-oxadiazol-3-yl)-1,2,3,4-tétrahydrocyclopenta[b]indol-3-yl) acétique;
acide 2-(7-(5-(6-carbamoylpyridin-3-yl)-1,2,4-oxadiazol-3-yl)-1,2,3,4-tétrahydrocyclopenta[b]indol-3-yle acétique;
acide 2-(7-(5-(3-cyano-4-(1,1,1,3,3,3-hexafluoropropan-2-yloxy)phényl)-1,2,4-oxadiazol-3-yl)-1,2,3,4-tétrahydrocyclopenta[b]indol-3-yl) acétique;
acide 2-(7-(5-(3-(cyclopropylméthoxy)-5-(trifluorométhyl) phényl)-1,2,4-oxadiazol-3-yl)-1,2,3,4-tétrahydrocyclopenta[b]indol-3-yl) acétique;
acide 2-(7-(5-(pyrazin-2-yl)-1,2,4-oxadiazol-3-yl)-1,2,3,4-tétrahydrocyclopentelindol-3-yl) acétique;
acide 2-(7-(5-(3-cyclopropyl-5-(trifluorométhoxy)phényl)-1,2,4-oxadiazol-3-yl)-1,2,3,4-tétrahydrocyclopenta[b]indol-3-yl) acétique;
acide 2-(7-(5-(3-éthyl-5-(trifluorométhoxy)phényl)-1,2,4-oxadiazol-3-yl)-1 2,3,4-tétrahydrocyclopenta[b]indol-3-yle acétique;
acide 2-(7-(5-(3-cyano-5-(2-fluoroéthoxy)phényl)-1,2,4-oxadiazol-3-yl)-1,2,3,4-tétrahydrocyclopenta[b]indol-3-yle acétique;
acide 2-(7-(5-(3-isopropoxy-5-(trifluorométhoxy)phényl)-1,2,4-oxadiazol-3-yl)-1,2,3,4-tétrahydrocyclopenta[b]indol-3-yl) acétique;
acide 2-(7-(5-(2-chloro-6-méthylpyridin-4-yl)-1,2,4-oxadiazol-3-yl)-1,2,3,4-tétrahydrocyclopenta[b]indol-3-yl) acétique;
acide 2-(7-(5-(3,5-dichlorophényl)-1,2,4-oxadiazol-3-yl)-1,2,3,4-tétrahydrocyclopenta[b]indol-3-yl) acétique;
acide 2-(7-(5-(3-cyano-4-éthoxyphényl)-1,2,4-oxadiazol-3-yl)-1,2,3,4-tétrahydrocyclopenta[b]indol-3-yl) acétique;
acide 2-(7-(5-(3-chloro-5-cyanophényl)-1,2,4-oxadiazol-3-yl)-1,2,3,4-tétrahydrocyclopenta[b]indol-3-yl) acétique;
Acide 2-(7-(5-(3,5-diméthoxyphényl)-1,2,4-oxadiazol-3-yl)-1,2,3,4-tétrahydrocyclopenta[b]indol-3-yl) acétique;
acide 2-(7-(5-(pyridin-3-yl)-1,2,4-oxadiazol-3-yl)-1,2,3,4-tétrahydrocyclopenta[b]indol-3-yl) acétique;
acide 2-(7-(5-(3-propyl-5-(trifluorométhoxy)phényl)-1,2,4-oxadiazol-3-yl)-1,2,3,4-tétrahydrocyclopenta[b]indol-3-yl) acétique;
acide 2-(7-(5-(pyridin-4-yl)-1,2,4-oxadiazol-3-yl)-1,2,3,4-tétrahydrocyclopenta[b]lndol-3-yl) acétique;
acide 2-(7-(5-(4-méthoxy-3-(trifluorométhyl)phényl)-1,2,4-oxadiazol-3-yl)-1,2,3,4-tétrahydrocyclopenta[b]indol-3-yl) acétique;
acide 2-(7-(5-(3-chloro-4-méthoxyphényl)-1,2,4-oxadiazol-3-yl)-1,2,3,4-tétrahydrocyclopenta[b]indol-3-yl) acétique;
acide 2-(7-(5-(3-cyano-4-hydroxyphényl)-1,2,4-oxadiazol-3-yl)-1,2,3,4-tétrahydrocyclopenta[b]indol-3-yl) acétique;
acide 2-(7-(5-(3-chloro-5-(trifluorométhoxy)phényl)-1,2,4-oxadiazol-3-yl)-1,2,3,4-tétrahydrocyclopenta[b]indol-3-yl) acétique;
acide 2-(7-(5-(3-cyano-5-fluorophényl)-1,2,4-oxadiazol-3-yl)-1,2,3,4-tétrahydrocyclopenta[b]indol-3-yl) acétique;
acide 2-(7-(5-(6-(2,2,2-trifluoroéthoxy)pyridin-3-yl)-1,2,4-oxadiazol-3-yl)-1,2,3,4-tétrahydrocyclopenta[b] indol-3-yl) acétique;
acide 2-(7-(5-(6-isopropoxy-5-méthylpyridin-3-yl)-1,2,4-oxadiazol-3-yl)-1,2,3,4-tétrahydrocyclopenta[b]indol-3yl) acétique;
acide 2-(7-(5-(3-(benzyloxy)-5-(trifluorométhyl)phényl)-1,2,4-oxadiazol-3-yl)-1,2,3,4-tétrahydrocyclopenta[b]indol-3-yl) acétique;
acide 2-(7-(5-(3-(trifluorométhyl)phényl)-1,2,4-oxadiazol-3-yl)-1,2,3,4-tétrahydrocyclopenta[b]indol-3-yl) acétique;
acide 2-(7-(5-(3-carbamoyl-5-(trifluorométhoxy)phényl)-1,2,4-oxadiazol-3-yl)-1,2,3,4-tétrahydrocyclopenta[b]indol-3-yl) acétique;
acide 2-(7-(5-(3,5-bis(trifluorométhyl)phényl)-1,2,4-oxadiazol-3-yl)-1,2,3,4-tétrahydrocyclopenta[b]indol-3-yl)acétique;
acide 2-(7-(5-(3-méthyl-5-(trifluorométhoxy)phényl)-1,2,4-oxadiazol-3-yl)-1,2,3,4-tétrahydrocyclopenta[b]indol-3-yl) acétique;
acide 2-(7-(5-(5-butylpyridin-2-yl)-1,2,4-oxadiazol-3-yl)-1,2,3,4-tétrahydrocyclopenta[b]indol-3-yle acétique;
acide 2-(7-(5-(3-cyano-5-(2,2,2-trifluoroéthoxy)phényl)-1,2,4-oxadiazol-3-yl)-1,2,3,4-tétrahydrocyclopenta [b]indol-3-yl) acétique;
acide 2-(7-(5-(pyrimidin-5-yl)-1,2,4-oxadiazol-3-yl)-1,2,3,4-tétrahydrocyclopenta[b]indol-3-yl) acétique;
acide 2-(7-(5-(3-tert-butyl-5-cyanophényl)-1,2,4-oxadiazol-3-yl)-1,2,3,4-tétrahydrocyclopenta[b]indol-3-yl) acétique;
acide 2-(7-(5-(6-méthoxy-5-méthylpyridin-3-yl)-1,2,4-oxadiazol-3-yl)-1,2,3,4- tétrahydrocyclopenta[b] indol-3-yl) acétique;
acide 2-(7-(5-(6-sec-butoxy-5-chloropyridin-3-yl)-1,2,4-oxadiazol-3-yl)-1,2,3,4-tétrahydrocyclopenta[b]indol-3-yl) acétique;
acide 2-(7-(5-(3-(2-cyanoéthyl)-5-(trifluorométhoxy) phényl)-1,2,4-oxadiazol-3-yl)-1,2,3,4-tétrahydrocyclopenta[b]indol-3-yl) acétique;
acide 2-(7-(5-(pyridin-2-yl)-1,2,4-oxadiazol-3-yl)-1,2,3,4- tétrahydrocyclopenta[b]indol-3-yl) acétique;
acide 2-(7-(5-(6-éthoxy-5-méthylpyridin-3-yl)-1,2,3,4-oxadiazol-3-yl)-1,2,3,4-tétrahydrocyclopenta [b]indol-3-yl) acétique;
acide 2-(7-(5-(3-bromo-5-(trifluorométhoxy)phényl)-1,2,4-oxadiazol-3-yl)-1,2,3,4-tétrahydrocyclopenta[b]indol-3-yl) acétique;
acide 2-(7-(5-(3,5-diméthylphényl)-1,2,4-oxadiazol-3-yl)-1,2,3,4-tétrahydrocyclopenta[b]indol-3-yl) acétique;
acide 2-(7-(5-(3-cyano-5-(cyclopentyloxy)phényl)-1,2,4-oxadiazol-3-yl)-1,2,3,4-tétrahydrocyclopentaplindol-3-yl) acétique;
acide 2-(7-(5-(5-chloro-6-isopropoxypyridin-3-yl)-1,2,4-oxadiazol-3-yl)-1,2,3,4-tétrahydrocyclopenta[b]indol-3-yl) acétique;
acide 2-(7-(5-(3-isopropoxy-5-(trifluorométhyl)phényl)-1,2,4-oxadiazol-3-yl)-1,2,3,4-tétrahydrocyclopenta[b]indol-3-yl) acétique;
acide 2-(7-(5-(3,5-diéthoxyphényl)-1,2,4-oxadiazol-3-yl)-1,2,3,4-tétrahydrocyclopenta[b]indol-3-yl) acétique;
acide 2-(7-(5-(3-bromo-5-hydroxyphényl)-1,2,4-oxadiazol-3-y1)-1,2,3,4-tétrahydrocyclopenta[b]indol-3-yl) acétique;
acide 2-(7-(5-(5-bromopyridin-3-yl)-1,2,4-oxadiazol-3-yl)-1,2,3,4-tétrahydrocyclopenta[b]indol-3-yl) acétique;
acide 2-(7-(5-(pyridazin-4-yl)-1,2,4-oxadiazol-3-yl)-1,2,3,4-tétrahydrocyclopenta[b]indol-3-yl) acétique;
acide 2-(7-(5-(3-(4H-1,2,4-triazol-4-yl)-5-(trifluoro-méthoxy)phényl)-1,2,4-oxadiazol-3-yl)-1,2,3,4-tétrahydrocyclopenta[b]indol-3yl) acétique;
acide 7-(5-(3-cyano-5-(trifluorométhoxy)phényl)-1,2,4-oxadiazol-3-yl)-1,2,3,4-tétrahydrocyclopenta[b]indole-3-carboxylique;
acide 2-(7-(5-(3-(6-méthoxypyridin-3-yl)-5-(trifluorométhoxy)phényl)-1,2,4-oxadiazol-3-yl)-1,2,3,4-tétrahydrocyclopentaplindol-3-yl) acétique;
acide 2-(7-(5-(6-méthoxypyridin-3-yl)-1,2,4-oxadiazol-3-yl)-1,2,3,4-tétrahydrocyclopenta[b]indol-3-yl) acétique;
acide 2-(7-(5-(4-isopropoxy-3-méthoxyphényl)-1,2,4-oxadiazol-3-yl)-1,2,3,4-tétrahydrocyclopenta[b]indol-3-yl) acétique;
acide 2-(7-(5-(3-cyano-4-(cyclopropylméthoxy)phényl)-1,2,4-oxadiazol-3-yl)-1,2,3,4-tétrahydrocyclopenta[b]indol-3yl) acétique;
acide 2-(7-(5-(4-hydroxy-3-méthylphényl)-1,2,4-oxadiazol-3-yl)-1,2,3,4-tétrahydrocyclopenta[b]indol-3-yl) acétique;
acide 2-(7-(5-(3-(pyridin-3-yl)-5-(trifluorométhoxy) phényl)-1,2,4-oxadiazol-3-yl)-1,2,3,4-tétrahydrocyclopenta[b]indol-3-yl) acétique;
acide 2-(7-(5-(6-(diéthylamino)-4-(trifluorométhyl)pyridin-2-yl)-1,2,4-oxadiazol-3-yl)-1,2,3,4-tétrahydrocyclopenta[b]indol-3-yl) acétique;
acide 2-(7-(5-(3-fluoro-4-isopropoxyphényl)-1,2,4-oxadiazol-3-yl)-1,2,3,4-tétrahydrocyclopenta[b]indol-3-yl)acétique;
acide 2-(7-(5-(5-isopropoxypyrazin-2-yl)-1,2,4-oxadiazol-3-yl)-1,2,3,4-tétrahydrocyclopenta[b]indol-3-yl)acétique;
acide 2-(7-(5-(5-méthoxypyridin-2-yl)-1,2,4-oxadiazol-3-yl)-1,2,3,4-tétrahydrocyclopenta[b]indol-3-yl)acétique;
acide 2-(7-(5-(5-isopropoxypyridin-2-yl)-1,2,4-oxadiazol-3-yl)-1,2,3,4-tétrahydrocyclopente[b]indol-3-yl)acétique;
Acide 2-(7-(5-(3,4-diméthoxyphényl)-1,2,4-oxadiazol-3-yl)-1,2,3,4-tétrahydrocyclopenta[b]indol-3-yl)acétique;
Acide 2-(7-(5-(3-(cyclopropyléthynyl)-5-(trifluorométhoxy)phényl)-1,2,4-oxadiazol-3-yl)-1,2,3,4-tétrahydrocyclopenta[b]indol-3-yl)acétique;
acide 2-(7-(5-(3-(6-fluoropyridin-3-yl)-5-(trifluorométhoxy)phényl)-1,2,4-oxadiazol-3-yl)-1,2,3,4-tétrahydrocyclopenta[b]indol-3-yl) acétique;
acide 2-(7-(5-(6-isopropoxypyridazin-3-yl)-1,2,4-oxadiazol-3-yl)-1,2,3,4-tétrahydrocyclopenta[b]indol-3-yl)acétique;
acide 2-(7-(5-(4-isopropoxy-3-(méthylsulfonyl)phényl)-1,2,4-oxadiazol-3-yl)-1,2,3,4-tétrahydrocyclopenta[b]indol-3-yl)acétique;
acide 2-(7-(5-(3,5-bis(méthylsulfonyl)phényl)-1,2,4-oxadiazol-3-yl)-1,2,3,4-tétrahydrocyclopenta[b]indol-3-yl)acétique;
acide 2-(7-(5-(3-(1-(tert-butoxycarbonyl)-1H-pyrrol-2-yl)-5-(trifluorométhoxy)phényl)-1,2,4-oxadiazol-3-yl)-1,2,3,4-tétrahydrocyclopenta[b]indol-3-yl)acétique;
acide 2-(7-(5-(3-fluoro-4-méthoxyphényl)-1,2,4-oxadiazol-3-yl)-1,2,3,4-tétrahydrocyclopenta[b]indol-3-yl)acétique;
acide 2-(7-(5-(6-isopropoxypyridin-3-yl)-1,2,4-oxadiazol-3-yl)-1,2,3,4-tétrahydrocyclopenta[b]indol-3-yl)acétique;
acide 2-(7-(5-(3-chloro-4-hydroxyphényl)-1,2,4-oxadiazol-3-yl)-1,2,3,4-tétrahydrocyclopenta[b]indol-3-yl)acétique;
acide 2-(7-(5-(4-cyano-3-(trifluorométhoxy)phényl)-1,2,4-oxadiazol-3-yl)-1,2,3,4-tétrahydrocyclopenta[b]indol-3-yl)acétique;
acide 2-(7-(5-(3-(pyridin-4-yl)-5-(trifluorométhoxy) phényl)-1,2,4-oxadiazol-3-yl)-1,2,3,4-tétrahydrocyclopenta[b]indol-3-yl)acétique;
acide 2-(7-(5-(4-isopropoxyphényl)-1,2,4-oxadiazol-3-yl)-1,2,3,4-tétrahydrocyclopenta[b]indol-3-yl)acétique;
acide 2-(7-(5-(3-(pyrimidin-5-yl)-5-(trifluorométhoxy) phényl)-1,2,4-oxadiazol-3-yl)-1,2,3,4-tétrahydrocyclopenta[b]indol-3-yl)acétique;
acide 2-(7-(5-(3-(2-fluoropyridin-3-yl)-5-(trifluorométhoxy)phényl)-1,2,4-oxadiazol-3-yl)-1,2,3,4-tétrahydrocyclopenta[b]indol-3-yl)acétique;
acide 2-(7-(5-(3-(2-fluoropyridin-4-yl)-5-(trifluoro-méthoxy)phényl)-1,2,4-oxadiazol-3-yl)-1,2,3,4-tétrahydrocyclopenta[b]indol-3-yl)acétique;
acide 2-(7-(5-(3-isopropoxy-5-(trifluorométhyl)phényl)-1,2,4-oxadiazol-3-yl)-4-méthyl-1,2,3,4-tetrahydrocyclopenta[b]indol-3-yl)acétique;
acide 2-(7-(5-(4-éthoxy-3-fluorophényl)-1,2,4-oxadiazol-3-yl)-1,2,3,4-tétrahydrocyclopenta[b]indol-3-yl)acétique;
acide 2-(7-(5-(3-cyano-4-(trifluorométhoxy)phényl)-1,2,4-oxadiazol-3-yl)-1,2,3,4-tétrahydrocyclopenta[b]indol-3-yl)acétique;
acide 2-(7-(5-(3-(2-méthoxypyrimidin-5-yl)-5-(trifluoro-méthoxy)phényl)-1,2,4-oxadiazol-3-yl)-1,2,3,4-tétrahydrocyclopenta[b]indol-3-yl)acétique;
acide 2-(7-(5-(6-(propylamino)-4-(trifluorométhyl)pyridin-2-yl)-1,2,4-oxadiazol-3-yl)-1,2,3,4-tétrahydrocyclopenta [b]indol-3-yl)acétique;
acide 2-(7-(5-(6-morpholino-4-(trifluorométhyl)pyridin-2-yl)-1,2,4-oxadiazol-3-yl)-1,2,3,4-tétrahydrocyclopenta [b]indol-3-yl)acétique;
acide 2-(7-(5-(2-chloro-6-méthoxypyridin-4-yl)-1,2,4-oxadiazol-3-yl)-1,2,3,4-tétrahydrocyclopenta[b]indol-3-yl)acétique;
acide 2-(7-(5-(4-méthoxy-3-(méthylsulfonyl)phényl)-1,2,4-oxadiazol-3-yl)-1,2,3,4-tétrahydrocyclopenta[b]indol-3-yl)acétique;
acide 2-(7-(5-(5-méthoxypyrazin-2-yl)-1,2,4-oxadiazol-3-yl)-1,2,3,4-tétrahydrocyclopenta[b]indol-3-yl)acétique;
acide 2-(7-(5-(3-(3-fluoropyridin-4-yl)-5-(trifluoro-méthoxy) phényl)-1,2,4-oxadiazol-3-yl)-1,2,3,4-tétrahydrocyclopenta[b]indol-3-yl)acétique;
acide 2-(7-(5-(3-(3,5-diméthylisoxazol-4-yl)-5-(trifluorométhoxy)phényl)-1,2,4-oxadiazol-3-yl)-1,2,3,4-tétrahydrocyclopenta[b]indol-3-yl)acétique;
acide 2-(7-(5-(3-(2,6-difluoropyridin-4-yl)-5-(trifluorométhoxy)phényl)-1,2,4-oxadiazol-3-yl)-1,2,3,4-tétrahydrocyclopenta[b]indol-3-yl)acétique;
acide 2-(7-(5-(3-(6-fluoro-5-méthylpyridin-3-yl)-5-(trifluorométhoxy)phényl)-1,2,4-oxadiazol-3-yl)-1,2,3,4-tétrahydrocyclopenta[b]indol-3-yl)acétique;
acide 2-(7-(5-(3-(1-méthyl-1H-pyrazol-4-yl)-5-(trifluoro-méthoxy)phényl)-1,2,4-oxadiazol-3-yl)-1,2,3,4-tetrahydrocyclopenta[b]indol-3-yl)acétique;
acide 2-(7-(5-(3-(2-méthylpyridin-4-yl)-5-(trifluorométhoxy)phényl)-1,2,4-oxadiazol-3-yl)-1,2,3,4-tétrahydrocyclopenta[b]indol-3-yl)acétique;
Acide 2-(7-(5-(3-(3-méthylpyridin-4-yl)-5-(trifluorométhoxy)phényl)-1,2,4-oxadiazol-3-yl)-1,2,3,4-tétrahydrocyclopenta[b]indol-3-yl)acétique;
acide 2-(7-(5-(3-(trifluorométhoxy)-5-(1,3,5-triméthyl-1H-pyrazol-4-yl)phényl)-1,2,4-oxadiazol-3-yl)-1,2,3,4-tétrahydrocyclopenta[b]indol-3-yl)acétique;
acide 2-(7-(5-(3-(1-isobutyl-1H-pyrazol-4-yl)-5-(trifluorométhoxy)phényl)-1,2,4-oxadiazol-3-yl)-1,2,3,4-tétrahydrocyclopenta[b]indol-3-yl)acétique;
acide 2-(7-(5-(2-bromo-6-méthylpyridin-4-yl)-1,2,4-oxadiazol-3-yl)-1,2,3,4-tétrahydrocyclopenta[b]indol-3-yl)acétique;
acide 2-(7-(5-(4-(difluorométhoxy)-3-méthoxyphényl)-1,2,4-oxadiazol-3-yl)-1,2,3,4-tétrahydrocyclopenta[b]indol-3-yl)acétique;
acide 2-(7-(5-(5-(1-méthyl-1H-pyrazol-4-yl)pyridin-3-yl)-1,2,4-oxadiazol-3-yl)-1,2,3,4-tétrahydrocyclopenta[b]indol-3-yl)acétique; et
acide 2-(7-(5-(5-hexylpyridin-3-yl)-1,2,4-oxadiazol-3-yl)-1,2,3,4-tétrahydrocyclopenta[b]indol-3-yl)acétique.

37. Composé selon l'une quelconque des revendications 1 à 36, où la stéréochimie pour le carbone de cycle dudit composé est R.

38. Composé selon l'une quelconque des revendications 1 à 36, où la stéréochimie pour le carbone de cycle dudit composé est S.

39. Composé selon la revendication 1, qui est:
l'acide (R)-2-(7-(5-(3-cyano-5-(trifluorométhoxy) phényl)-1,2,4-oxadiazol-3-yl)-1,2,3,4-tétrahydrocyclopenta[b]indol-3-yl)acétique;
ou un sel, solvate ou hydrate pharmaceutiquement acceptable de celui-ci.

40. Composé selon la revendication 1, qui est
l'acide (S)-2-(7-(5-(3-cyano-5-(trifluorométhoxy) phényl)-1,2,4-oxadiazol-3-yl)-1,2,3,4-tétrahydrocyclopenta[b]indol-3-yl)acétique;
ou un sel, solvate ou hydrate pharmaceutiquement acceptable de celui-ci.

41. Composé selon la revendication 1, qui est:
l'acide (S)-2-(7-(5-(3-cyano-4-isopropoxyphényl)-1,2,4-oxadiazol-3-yl)-1,2,3,4-tétrahydrocyclopenta[b]indol-3-yl)acétique;
ou un sel, solvate ou hydrate pharmaceutiquement acceptable de celui-ci.

42. Composition pharmaceutique comprenant un composé selon l'une quelconque des revendications 1 à 41 et un support pharmaceutiquement acceptable.

43. Procédé de préparation d'une composition comprenant le mélange d'un composé selon l'une quelconque des revendications 1 à 41 et d'un support pharmaceutiquement acceptable.

44. Composé selon l'une quelconque des revendications 1 à 41 pour utilisation dans une méthode de traitement du corps humain ou animal par thérapie.

45. Composé selon l'une quelconque des revendications 1 à 41 pour utilisation dans une méthode de traitement d'un trouble associé au récepteur de S1P1.

46. Composé selon l'une quelconque des revendications 1 à 41 pour utilisation dans une méthode de traitement d'un trouble associé au récepteur de S1P1, où ledit trouble associé au récepteur de S1P1 est sélectionné parmi: une maladie ou trouble provoqué par des lymphocytes, une maladie ou trouble auto-immune, une maladie ou trouble inflammatoire, le cancer, le psoriasis, la polyarthrite rhumatoïde, la maladie de Crohn, le rejet de greffe, la sclérose multiple, le lupus érythémateux dissiminé, la recto-colite hémorragique, le diabète de type I et l'acnée.

47. Composé selon l'une quelconque des revendications 1 à 41 pour utilisation dans une méthode pour le traitement d'un trouble associé au récepteur de S1P1, où ledit trouble associé au récepteur de S1P1 est une infection ou maladie microbienne ou une infection ou maladie virale.

48. Utilisation d'un composé selon l'une quelconque des revendications 1 à 41 dans la fabrication d'un médicament pour le traitement d'un trouble associé au récepteur de S1P1.

49. Utilisation d'un composé selon l'une quelconque des revendications 1 à 41 dans la fabrication d'un médicament pour le traitement d'un trouble associé au récepteur de S1P1, où ledit trouble associé au récepteur de S1P1 est sélectionné parmi: une maladie ou trouble provoqué par des lymphocytes, une maladie ou trouble auto-immune, une maladie ou trouble inflammatoire, le cancer, le psoriasis, la polyarthrite rhumatoïde, la maladie de Crohn, le rejet de greffe, la sclérose multiple, le lupus érythémateux disséminé, la recto-colite hémorrhagique, le diabète de type I et l'acnée.

50. Utilisation d'un composé selon l'une quelconque des revendications 1 à 41 dans la fabrication d'un médicament pour le traitement d'un trouble associé au récepteur de S1P1, où ledit trouble associé au récepteur de S1P1 est une infection ou maladie microbienne ou une infection ou maladie virale.
